# EUROPEAN PATENT APPLICATION

(11) **EP 2 641 978 A1**
(43) Date of publication of application: **25.09.2013**
(21) Application number: 13172903.0
(22) Date of filing: 12.05.2009
(51) Int. Cl.: C12Q 1/68, G01N 33/53, G01N 33/574

(54) **Tests to predict responsiveness of cancer patients to chemotherapy treatment options**

(30) Priority: 12.05.2008 US 52573 P; 29.05.2008 US 57182 P
(62) Divisional of application: 13159677.7
(71) Applicant: Genomic Health, Inc., Redwood City, CA 94063 (US); Aventis Inc., Greenville, DE 19807 (US)
(72) Inventor: Shak, Steve, Burlingame, CA 94010 (US); Baker, Joffre, Montara, CA 94037 (US); Yoshizawa, Carl, Redwood City, CA 94063 (US); Sparano, Joseph, Pleasantville, NY 10570 (US); Gray, Robert, Boston, MA 02115 (US)
(74) Representative: Ellis, Katherine Elizabeth Sleigh

(57) **Abstract**

The present disclosure provides methods and compositions to facilitate prediction of the likelihood of responsiveness of cancer patients to treatment including a taxane and/or a cyclophosphamide.

SEQUENCE LISTING

<110> Genomic Health, Inc.
      Aventis, Inc.

<120> TESTS TO PREDICT RESPONSIVENESS OF
  CANCER PATIENTS TO CHEMOTHERAPY TREATMENT OPTIONS

<130> KE/N30232

<140> US 12/464,797
<141> 2009-05-12

<150> US 61/052,573
<151> 2008-05-12

<150> US 61/057,182
<151> 2008-05-29

<160> 1496

<170> FastSEQ for Windows Version 4.0

<210> 1
<211> 20
<212> DNA
<213> Artificial Sequence

<220>
<223> Synthetic Oligonucleotide

<400> 1
ttacccgtgg gaactgtctc                                                  20

<210> 2
<211> 22
<212> DNA
<213> Artificial Sequence

<220>
<223> Synthetic Oligonucleotide

<400> 2
gaccagtaaa tgggtcagag ga                                               22

<210> 3
<211> 25
<212> DNA
<213> Artificial Sequence

<220>
<223> Synthetic Oligonucleotide

<400> 3
tcctctcacc aggacaacaa ccaca                                            25

<210> 4
<211> 20
<212> DNA
<213> Artificial Sequence

<220>
<223> Synthetic Oligonucleotide

<400> 4
aaacaccact ggagcattga                                                    20

<210> 5
<211> 20
<212> DNA
<213> Artificial Sequence

<220>
<223> Synthetic Oligonucleotide

<400> 5
caagcctgga acctatagcc                                                    20

<210> 6
<211> 24
<212> DNA
<213> Artificial Sequence

<220>
<223> Synthetic Oligonucleotide

<400> 6
ctcgccaatg atgctgctca agtt                                               24

<210> 7
<211> 18
<212> DNA
<213> Artificial Sequence

<220>
<223> Synthetic Oligonucleotide

<400> 7
agacagtcgc cttggtcg                                                      18

<210> 8
<211> 20
<212> DNA
<213> Artificial Sequence

<220>
<223> Synthetic Oligonucleotide

<400> 8
aacctctgca gaagctggac                                                    20

<210> 9
<211> 24
<212> DNA
<213> Artificial Sequence

<220>
<223> Synthetic Oligonucleotide

<400> 9
ccgtactctt cccactgcca ttga                                               24

<210> 10
<211> 18

<212> DNA
<213> Artificial Sequence

<220>
<223> Synthetic Oligonucleotide

<400> 10
accagtgcca caatgcag                                                    18

<210> 11
<211> 18
<212> DNA
<213> Artificial Sequence

<220>
<223> Synthetic Oligonucleotide

<400> 11
atagcgctga ccactgcc                                                    18

<210> 12
<211> 24
<212> DNA
<213> Artificial Sequence

<220>
<223> Synthetic Oligonucleotide

<400> 12
ccatgagctg tagccgaatg tcca                                             24

<210> 13
<211> 18
<212> DNA
<213> Artificial Sequence

<220>
<223> Synthetic Oligonucleotide

<400> 13
aagccacagc ctccattg                                                    18

<210> 14
<211> 19
<212> DNA
<213> Artificial Sequence

<220>
<223> Synthetic Oligonucleotide

<400> 14
ggaaggcttc acggattgt                                                   19

<210> 15
<211> 24
<212> DNA
<213> Artificial Sequence

<220>
<223> Synthetic Oligonucleotide

<400> 15
tggagacaga caccctgatc cagc                                             24

<210> 16
<211> 20
<212> DNA
<213> Artificial Sequence

<220>
<223> Synthetic Oligonucleotide

<400> 16
tgcagactgt accatgctga                                                   20

<210> 17
<211> 20
<212> DNA
<213> Artificial Sequence

<220>
<223> Synthetic Oligonucleotide

<400> 17
ggccagcacc ataatcctat                                                   20

<210> 18
<211> 22
<212> DNA
<213> Artificial Sequence

<220>
<223> Synthetic Oligonucleotide

<400> 18
ctgcacacgg ttctaggctc cg                                                22

<210> 19
<211> 20
<212> DNA
<213> Artificial Sequence

<220>
<223> Synthetic Oligonucleotide

<400> 19
tctgtggccc acctctactc                                                   20

<210> 20
<211> 20
<212> DNA
<213> Artificial Sequence

<220>
<223> Synthetic Oligonucleotide

<400> 20
gggtgtagga agtcacagcc                                                   20

<210> 21
<211> 24
<212> DNA
<213> Artificial Sequence

<220>
<223> Synthetic Oligonucleotide

<400> 21
aacctgacca agccactcct ggac                                              24

<210> 22
<211> 21
<212> DNA
<213> Artificial Sequence

<220>
<223> Synthetic Oligonucleotide

<400> 22
atgtacgtcg ccattcaagc t                                                 21

<210> 23
<211> 19
<212> DNA
<213> Artificial Sequence

<220>
<223> Synthetic Oligonucleotide

<400> 23
acgccatcac ctgaatcca                                                    19

<210> 24
<211> 20
<212> DNA
<213> Artificial Sequence

<220>
<223> Synthetic Oligonucleotide

<400> 24
ctggccgcac gacaggcatc                                                   20

<210> 25
<211> 18
<212> DNA
<213> Artificial Sequence

<220>
<223> Synthetic Oligonucleotide

<400> 25
atccgcattg aagaccca                                                     18

<210> 26
<211> 20
<212> DNA
<213> Artificial Sequence

<220>
<223> Synthetic Oligonucleotide

<400> 26
atccgctaga actgcaccac                                                   20

<210> 27
<211> 24
<212> DNA
<213> Artificial Sequence

<220>
<223> Synthetic Oligonucleotide

<400> 27
cccgcagaaa gcacatggta ttcc                                              24

<210> 28
<211> 20
<212> DNA
<213> Artificial Sequence

<220>
<223> Synthetic Oligonucleotide

<400> 28
caactgctga gagaccgaga                                                   20

<210> 29
<211> 20
<212> DNA
<213> Artificial Sequence

<220>
<223> Synthetic Oligonucleotide

<400> 29
cgctccttta ctgccttagc                                                   20

<210> 30
<211> 26
<212> DNA
<213> Artificial Sequence

<220>
<223> Synthetic Oligonucleotide

<400> 30
aggaatccct ccagaacaat ccttgg                                            26

<210> 31
<211> 27
<212> DNA
<213> Artificial Sequence

<220>
<223> Synthetic Oligonucleotide

<400> 31
ctgcatgtga ttgaataaga aacaaga                                           27

<210> 32
<211> 22
<212> DNA
<213> Artificial Sequence

<220>
<223> Synthetic Oligonucleotide

<400> 32
tgtggacctg atccctgtac ac                                                22

<210> 33

<211> 23
<212> DNA
<213> Artificial Sequence

<220>
<223> Synthetic Oligonucleotide

<400> 33
tgaccacacc aaagcctccc tgg                                               23

<210> 34
<211> 20
<212> DNA
<213> Artificial Sequence

<220>
<223> Synthetic Oligonucleotide

<400> 34
cgcttctatg gcgctgagat                                                   20

<210> 35
<211> 20
<212> DNA
<213> Artificial Sequence

<220>
<223> Synthetic Oligonucleotide

<400> 35
tcccggtaca ccacgttctt                                                   20

<210> 36
<211> 24
<212> DNA
<213> Artificial Sequence

<220>
<223> Synthetic Oligonucleotide

<400> 36
cagccctgga ctacctgcac tcgg                                              24

<210> 37
<211> 19
<212> DNA
<213> Artificial Sequence

<220>
<223> Synthetic Oligonucleotide

<400> 37
tcctgccacc cttcaaacc                                                    19

<210> 38
<211> 21
<212> DNA
<213> Artificial Sequence

<220>
<223> Synthetic Oligonucleotide

<400> 38

ggcggtaaat tcatcatcga a                                                21

<210> 39
<211> 24
<212> DNA
<213> Artificial Sequence

<220>
<223> Synthetic Oligonucleotide

<400> 39
caggtcacgt ccgaggtcga caca                                             24

<210> 40
<211> 25
<212> DNA
<213> Artificial Sequence

<220>
<223> Synthetic Oligonucleotide

<400> 40
ttgtctctgc cttggactat ctaca                                            25

<210> 41
<211> 24
<212> DNA
<213> Artificial Sequence

<220>
<223> Synthetic Oligonucleotide

<400> 41
ccagcattag attctccaac ttga                                             24

<210> 42
<211> 23
<212> DNA
<213> Artificial Sequence

<220>
<223> Synthetic Oligonucleotide

<400> 42
tcacggtaca caatctttcc gga                                              23

<210> 43
<211> 19
<212> DNA
<213> Artificial Sequence

<220>
<223> Synthetic Oligonucleotide

<400> 43
tgggagggat gaaggaaat                                                   19

<210> 44
<211> 19
<212> DNA
<213> Artificial Sequence

<220>

<223> Synthetic Oligonucleotide

<400> 44
ctcatacagg tcctgggca                                                    19

<210> 45
<211> 24
<212> DNA
<213> Artificial Sequence

<220>
<223> Synthetic Oligonucleotide

<400> 45
tgtctcacga gagcatcgtc caga                                              24

<210> 46
<211> 20
<212> DNA
<213> Artificial Sequence

<220>
<223> Synthetic Oligonucleotide

<400> 46
ggacagcagg aatgtgtttc                                                   20

<210> 47
<211> 20
<212> DNA
<213> Artificial Sequence

<220>
<223> Synthetic Oligonucleotide

<400> 47
acccactcga tttgtttctg                                                   20

<210> 48
<211> 22
<212> DNA
<213> Artificial Sequence

<220>
<223> Synthetic Oligonucleotide

<400> 48
cattggctcc ccgtgacctg ta                                                22

<210> 49
<211> 20
<212> DNA
<213> Artificial Sequence

<220>
<223> Synthetic Oligonucleotide

<400> 49
gatgaagcct ttcgcaagtt                                                   20

<210> 50
<211> 20
<212> DNA

<213> Artificial Sequence

<220>
<223> Synthetic Oligonucleotide

<400> 50
aggtctccac acagcacaag                                                    20

<210> 51
<211> 21
<212> DNA
<213> Artificial Sequence

<220>
<223> Synthetic Oligonucleotide

<400> 51
ctttcgggaa gccaggccct t                                                  21

<210> 52
<211> 21
<212> DNA
<213> Artificial Sequence

<220>
<223> Synthetic Oligonucleotide

<400> 52
ggaaacacac tggaggacaa g                                                  21

<210> 53
<211> 19
<212> DNA
<213> Artificial Sequence

<220>
<223> Synthetic Oligonucleotide

<400> 53
cgcatcttgg cagaaagtt                                                     19

<210> 54
<211> 24
<212> DNA
<213> Artificial Sequence

<220>
<223> Synthetic Oligonucleotide

<400> 54
tcatcagccg catcaaacag agtg                                               24

<210> 55
<211> 18
<212> DNA
<213> Artificial Sequence

<220>
<223> Synthetic Oligonucleotide

<400> 55
gtttatgcca tcggcacc                                                      18

<210> 56
<211> 22
<212> DNA
<213> Artificial Sequence

<220>
<223> Synthetic Oligonucleotide

<400> 56
ggaatacacg agggcatagt tc                                                22

<210> 57
<211> 24
<212> DNA
<213> Artificial Sequence

<220>
<223> Synthetic Oligonucleotide

<400> 57
actggatcct ggccaccgac tatg                                              24

<210> 58
<211> 18
<212> DNA
<213> Artificial Sequence

<220>
<223> Synthetic Oligonucleotide

<400> 58
gcctcaagag ctggttcg                                                     18

<210> 59
<211> 18
<212> DNA
<213> Artificial Sequence

<220>
<223> Synthetic Oligonucleotide

<400> 59
cctgcacctt ctccacca                                                     18

<210> 60
<211> 22
<212> DNA
<213> Artificial Sequence

<220>
<223> Synthetic Oligonucleotide

<400> 60
actggcgctg catgtcttcc ac                                                22

<210> 61
<211> 18
<212> DNA
<213> Artificial Sequence

<220>
<223> Synthetic Oligonucleotide

<400> 61
gaggtcctgg agtgcgtg                                                    18

<210> 62
<211> 18
<212> DNA
<213> Artificial Sequence

<220>
<223> Synthetic Oligonucleotide

<400> 62
aggtgccagc ttctccct                                                    18

<210> 63
<211> 24
<212> DNA
<213> Artificial Sequence

<220>
<223> Synthetic Oligonucleotide

<400> 63
ccttaagcga ggtcagctcc acca                                             24

<210> 64
<211> 18
<212> DNA
<213> Artificial Sequence

<220>
<223> Synthetic Oligonucleotide

<400> 64
ggtcctccgt cggttctc                                                    18

<210> 65
<211> 18
<212> DNA
<213> Artificial Sequence

<220>
<223> Synthetic Oligonucleotide

<400> 65
gtcgcaaact cggagacg                                                    18

<210> 66
<211> 24
<212> DNA
<213> Artificial Sequence

<220>
<223> Synthetic Oligonucleotide

<400> 66
ccacggtctg gtcttcagct accc                                             24

<210> 67
<211> 21
<212> DNA
<213> Artificial Sequence

<220>
<223> Synthetic Oligonucleotide

<400> 67
agctgcagaa gagctgcaca t                                                21

<210> 68
<211> 20
<212> DNA
<213> Artificial Sequence

<220>
<223> Synthetic Oligonucleotide

<400> 68
gcatctgcca actcctccat                                                  20

<210> 69
<211> 22
<212> DNA
<213> Artificial Sequence

<220>
<223> Synthetic Oligonucleotide

<400> 69
tgacgagcag cgaacagcca cg                                               22

<210> 70
<211> 21
<212> DNA
<213> Artificial Sequence

<220>
<223> Synthetic Oligonucleotide

<400> 70
cagcagatgt ggatcagcaa g                                                21

<210> 71
<211> 18
<212> DNA
<213> Artificial Sequence

<220>
<223> Synthetic Oligonucleotide

<400> 71
gcatttgcgg tggacgat                                                    18

<210> 72
<211> 23
<212> DNA
<213> Artificial Sequence

<220>
<223> Synthetic Oligonucleotide

<400> 72
aggagtatga cgagtccggc ccc                                              23

<210> 73
<211> 22

<212> DNA
<213> Artificial Sequence

<220>
<223> Synthetic Oligonucleotide

<400> 73
ggctcttgtg cgtactgtcc tt                                                22

<210> 74
<211> 23
<212> DNA
<213> Artificial Sequence

<220>
<223> Synthetic Oligonucleotide

<400> 74
tcagatgacg aagagcacag atg                                               23

<210> 75
<211> 29
<212> DNA
<213> Artificial Sequence

<220>
<223> Synthetic Oligonucleotide

<400> 75
aggctcagtg atgtcttccc tgtcaccag                                         29

<210> 76
<211> 19
<212> DNA
<213> Artificial Sequence

<220>
<223> Synthetic Oligonucleotide

<400> 76
gggtcaggtg cctcgagat                                                    19

<210> 77
<211> 21
<212> DNA
<213> Artificial Sequence

<220>
<223> Synthetic Oligonucleotide

<400> 77
ctgctcactc ggctcaaact c                                                 21

<210> 78
<211> 24
<212> DNA
<213> Artificial Sequence

<220>
<223> Synthetic Oligonucleotide

<400> 78
tgggcccaga gcatgttcca gatc                                              24

<210> 79
<211> 23
<212> DNA
<213> Artificial Sequence

<220>
<223> Synthetic Oligonucleotide

<400> 79
cgttgtcagc acttggaata caa                                               23

<210> 80
<211> 24
<212> DNA
<213> Artificial Sequence

<220>
<223> Synthetic Oligonucleotide

<400> 80
gttcaacctc ttcctgtgga ctgt                                              24

<210> 81
<211> 26
<212> DNA
<213> Artificial Sequence

<220>
<223> Synthetic Oligonucleotide

<400> 81
cccaattaac atgacccggc aaccat                                            26

<210> 82
<211> 20
<212> DNA
<213> Artificial Sequence

<220>
<223> Synthetic Oligonucleotide

<400> 82
ccattcccac cattctacct                                                   20

<210> 83
<211> 20
<212> DNA
<213> Artificial Sequence

<220>
<223> Synthetic Oligonucleotide

<400> 83
gggaacatag acccaccaat                                                   20

<210> 84
<211> 21
<212> DNA
<213> Artificial Sequence

<220>
<223> Synthetic Oligonucleotide

<400> 84
acaccccaga cgtcctggcc t                                                21

<210> 85
<211> 18
<212> DNA
<213> Artificial Sequence

<220>
<223> Synthetic Oligonucleotide

<400> 85
ccgccgtgga cacagact                                                    18

<210> 86
<211> 21
<212> DNA
<213> Artificial Sequence

<220>
<223> Synthetic Oligonucleotide

<400> 86
ttgccgtcag aaaacatgtc a                                                21

<210> 87
<211> 25
<212> DNA
<213> Artificial Sequence

<220>
<223> Synthetic Oligonucleotide

<400> 87
tgccactcgg aaaaagacct ctcgg                                            25

<210> 88
<211> 20
<212> DNA
<213> Artificial Sequence

<220>
<223> Synthetic Oligonucleotide

<400> 88
cctggagggt cctgtacaat                                                  20

<210> 89
<211> 19
<212> DNA
<213> Artificial Sequence

<220>
<223> Synthetic Oligonucleotide

<400> 89
ctaattgggc tccatctcg                                                   19

<210> 90
<211> 24
<212> DNA
<213> Artificial Sequence

<220>
<223> Synthetic Oligonucleotide

<400> 90
catcatggga ctcctgccct tacc                                              24

<210> 91
<211> 25
<212> DNA
<213> Artificial Sequence

<220>
<223> Synthetic Oligonucleotide

<400> 91
cagatggacc tagtacccac tgaga                                             25

<210> 92
<211> 24
<212> DNA
<213> Artificial Sequence

<220>
<223> Synthetic Oligonucleotide

<400> 92
cctatgattt aagggcattt ttcc                                              24

<210> 93
<211> 22
<212> DNA
<213> Artificial Sequence

<220>
<223> Synthetic Oligonucleotide

<400> 93
ttccacgccg aaggacagcg at                                                22

<210> 94
<211> 20
<212> DNA
<213> Artificial Sequence

<220>
<223> Synthetic Oligonucleotide

<400> 94
aattaccaag cagccgaaga                                                   20

<210> 95
<211> 20
<212> DNA
<213> Artificial Sequence

<220>
<223> Synthetic Oligonucleotide

<400> 95
caggcggaca atgtaacgta                                                   20

<210> 96

<211> 28
<212> DNA
<213> Artificial Sequence

<220>
<223> Synthetic Oligonucleotide

<400> 96
ccacccacga atggttatct tacgactg                                          28

<210> 97
<211> 20
<212> DNA
<213> Artificial Sequence

<220>
<223> Synthetic Oligonucleotide

<400> 97
cagcgacaac tctggacaag                                                   20

<210> 98
<211> 19
<212> DNA
<213> Artificial Sequence

<220>
<223> Synthetic Oligonucleotide

<400> 98
gctctcagac tgccaggaa                                                    19

<210> 99
<211> 24
<212> DNA
<213> Artificial Sequence

<220>
<223> Synthetic Oligonucleotide

<400> 99
ccccagagtc tccaactgtg acca                                              24

<210> 100
<211> 24
<212> DNA
<213> Artificial Sequence

<220>
<223> Synthetic Oligonucleotide

<400> 100
cttttgtgga actctatggg aaca                                              24

<210> 101
<211> 19
<212> DNA
<213> Artificial Sequence

<220>
<223> Synthetic Oligonucleotide

<400> 101

cagcggttga agcgttcct                                                    19

<210> 102
<211> 20
<212> DNA
<213> Artificial Sequence

<220>
<223> Synthetic Oligonucleotide

<400> 102
ttcggctctc ggctgctgca                                                   20

<210> 103
<211> 26
<212> DNA
<213> Artificial Sequence

<220>
<223> Synthetic Oligonucleotide

<400> 103
tgtactggcg aagaatattt ggtaaa                                            26

<210> 104
<211> 20
<212> DNA
<213> Artificial Sequence

<220>
<223> Synthetic Oligonucleotide

<400> 104
gccacgtgat tcttccacaa                                                   20

<210> 105
<211> 24
<212> DNA
<213> Artificial Sequence

<220>
<223> Synthetic Oligonucleotide

<400> 105
cagggcatcg atctctcacc ctgg                                              24

<210> 106
<211> 20
<212> DNA
<213> Artificial Sequence

<220>
<223> Synthetic Oligonucleotide

<400> 106
ggactgtgag gtcaacaacg                                                   20

<210> 107
<211> 20
<212> DNA
<213> Artificial Sequence

<220>

<223> Synthetic Oligonucleotide

<400> 107
ggaagccaaa caccagtagg                                                    20

<210> 108
<211> 24
<212> DNA
<213> Artificial Sequence

<220>
<223> Synthetic Oligonucleotide

<400> 108
tgtgatgcac tcatccctga ggct                                               24

<210> 109
<211> 20
<212> DNA
<213> Artificial Sequence

<220>
<223> Synthetic Oligonucleotide

<400> 109
cctgcaaaag ggaacaagag                                                    20

<210> 110
<211> 20
<212> DNA
<213> Artificial Sequence

<220>
<223> Synthetic Oligonucleotide

<400> 110
cgtggttgac tctgatctcg                                                    20

<210> 111
<211> 21
<212> DNA
<213> Artificial Sequence

<220>
<223> Synthetic Oligonucleotide

<400> 111
cttcgcctcc agatggctcc c                                                  21

<210> 112
<211> 20
<212> DNA
<213> Artificial Sequence

<220>
<223> Synthetic Oligonucleotide

<400> 112
   tcagggggct agaaatctgt                                                     20

<210> 113
<211> 20
<212> DNA

<213> Artificial Sequence

<220>
<223> Synthetic Oligonucleotide

<400> 113
ccattccagt tgatctgtgg                                                    20

<210> 114
<211> 23
<212> DNA
<213> Artificial Sequence

<220>
<223> Synthetic Oligonucleotide

<400> 114
ctatgggccc ttcaccaaca tgc                                                23

<210> 115
<211> 20
<212> DNA
<213> Artificial Sequence

<220>
<223> Synthetic Oligonucleotide

<400> 115
agttcgtgct ttgcaagatg                                                    20

<210> 116
<211> 20
<212> DNA
<213> Artificial Sequence

<220>
<223> Synthetic Oligonucleotide

<400> 116
aaggtaagct gggtctgctg                                                    20

<210> 117
<211> 29
<212> DNA
<213> Artificial Sequence

<220>
<223> Synthetic Oligonucleotide

<400> 117
cattcttcac tgcttcataa agctctgca                                          29

<210> 118
<211> 21
<212> DNA
<213> Artificial Sequence

<220>
<223> Synthetic Oligonucleotide

<400> 118
ccgaggttaa tccagcacgt a                                                  21

<210> 119
<211> 21
<212> DNA
<213> Artificial Sequence

<220>
<223> Synthetic Oligonucleotide

<400> 119
aagacatggc gctctcagtt c                                                21

<210> 120
<211> 23
<212> DNA
<213> Artificial Sequence

<220>
<223> Synthetic Oligonucleotide

<400> 120
tgctgggagc ctacacttgg ccc                                              23

<210> 121
<211> 24
<212> DNA
<213> Artificial Sequence

<220>
<223> Synthetic Oligonucleotide

<400> 121
tcaacagaag gctgaaccac taga                                             24

<210> 122
<211> 22
<212> DNA
<213> Artificial Sequence

<220>
<223> Synthetic Oligonucleotide

<400> 122
caacagagtt tgccgagaca ct                                               22

<210> 123
<211> 24
<212> DNA
<213> Artificial Sequence

<220>
<223> Synthetic Oligonucleotide

<400> 123
tacagtccca gcaccgacaa ttcc                                             24

<210> 124
<211> 22
<212> DNA
<213> Artificial Sequence

<220>
<223> Synthetic Oligonucleotide

<400> 124
ctgaagcaga tggttcatca tt                                                22

<210> 125
<211> 22
<212> DNA
<213> Artificial Sequence

<220>
<223> Synthetic Oligonucleotide

<400> 125
gctgattccc aagagtctaa cc                                                22

<210> 126
<211> 24
<212> DNA
<213> Artificial Sequence

<220>
<223> Synthetic Oligonucleotide

<400> 126
cctcgctttg tttaacagcc cagg                                              24

<210> 127
<211> 23
<212> DNA
<213> Artificial Sequence

<220>
<223> Synthetic Oligonucleotide

<400> 127
tgaggagtgg tattttggca aga                                               23

<210> 128
<211> 21
<212> DNA
<213> Artificial Sequence

<220>
<223> Synthetic Oligonucleotide

<400> 128
ctctcgggtt ctctgcattg a                                                 21

<210> 129
<211> 24
<212> DNA
<213> Artificial Sequence

<220>
<223> Synthetic Oligonucleotide

<400> 129
aaccgctctg actcccgtct ggtg                                              24

<210> 130
<211> 20
<212> DNA
<213> Artificial Sequence

<220>
<223> Synthetic Oligonucleotide

<400> 130
gtcagcgtgg tagcggtatt                                                    20

<210> 131
<211> 21
<212> DNA
<213> Artificial Sequence

<220>
<223> Synthetic Oligonucleotide

<400> 131
ggaagtcttg gctaaagagg c                                                  21

<210> 132
<211> 24
<212> DNA
<213> Artificial Sequence

<220>
<223> Synthetic Oligonucleotide

<400> 132
aacaattact gtcactgccg cgga                                               24

<210> 133
<211> 20
<212> DNA
<213> Artificial Sequence

<220>
<223> Synthetic Oligonucleotide

<400> 133
tcagctgtga gctgcggata                                                    20

<210> 134
<211> 24
<212> DNA
<213> Artificial Sequence

<220>
<223> Synthetic Oligonucleotide

<400> 134
acggtcctag gtttgaggtt aaga                                               24

<210> 135
<211> 19
<212> DNA
<213> Artificial Sequence

<220>
<223> Synthetic Oligonucleotide

<400> 135
caggtcccat tgccgggcg                                                     19

<210> 136
<211> 20

<212> DNA
<213> Artificial Sequence

<220>
<223> Synthetic Oligonucleotide

<400> 136
atcctagccc tggtttttgg                                                   20

<210> 137
<211> 20
<212> DNA
<213> Artificial Sequence

<220>
<223> Synthetic Oligonucleotide

<400> 137
ctgccttctc atctgcacaa                                                   20

<210> 138
<211> 20
<212> DNA
<213> Artificial Sequence

<220>
<223> Synthetic Oligonucleotide

<400> 138
tttgctgtca ccagcgtcgc                                                   20

<210> 139
<211> 26
<212> DNA
<213> Artificial Sequence

<220>
<223> Synthetic Oligonucleotide

<400> 139
cactaaggtt tgagacagtt ccagaa                                            26

<210> 140
<211> 23
<212> DNA
<213> Artificial Sequence

<220>
<223> Synthetic Oligonucleotide

<400> 140
gcgaattagc cctctacaac tga                                               23

<210> 141
<211> 26
<212> DNA
<213> Artificial Sequence

<220>
<223> Synthetic Oligonucleotide

<400> 141
aacccaagct caagacgcag gacgag                                            26

<210> 142
<211> 20
<212> DNA
<213> Artificial Sequence

<220>
<223> Synthetic Oligonucleotide

<400> 142
tcgttggaga tcagagtgga                                                    20

<210> 143
<211> 18
<212> DNA
<213> Artificial Sequence

<220>
<223> Synthetic Oligonucleotide

<400> 143
ttaagcacgc caaccacc                                                      18

<210> 144
<211> 24
<212> DNA
<213> Artificial Sequence

<220>
<223> Synthetic Oligonucleotide

<400> 144
tcaccatcac accacctaca gccc                                               24

<210> 145
<211> 20
<212> DNA
<213> Artificial Sequence

<220>
<223> Synthetic Oligonucleotide

<400> 145
gtggctcaac attgtgttcc                                                    20

<210> 146
<211> 20
<212> DNA
<213> Artificial Sequence

<220>
<223> Synthetic Oligonucleotide

<400> 146
caatggcctc cattttacag                                                    20

<210> 147
<211> 24
<212> DNA
<213> Artificial Sequence

<220>
<223> Synthetic Oligonucleotide

<400> 147
atttcagctg atcagtgggc ctcc                                              24

<210> 148
<211> 20
<212> DNA
<213> Artificial Sequence

<220>
<223> Synthetic Oligonucleotide

<400> 148
ttcaggttgt tgcaggagac                                                   20

<210> 149
<211> 20
<212> DNA
<213> Artificial Sequence

<220>
<223> Synthetic Oligonucleotide

<400> 149
catcttcttg ggcacacaat                                                   20

<210> 150
<211> 27
<212> DNA
<213> Artificial Sequence

<220>
<223> Synthetic Oligonucleotide

<400> 150
tgtctccatt attgatcggt tcatgca                                           27

<210> 151
<211> 21
<212> DNA
<213> Artificial Sequence

<220>
<223> Synthetic Oligonucleotide

<400> 151
gcatgttcgt ggcctctaag a                                                 21

<210> 152
<211> 22
<212> DNA
<213> Artificial Sequence

<220>
<223> Synthetic Oligonucleotide

<400> 152
cggtgtagat gcacagcttc tc                                                22

<210> 153
<211> 23
<212> DNA
<213> Artificial Sequence

<220>
<223> Synthetic Oligonucleotide

<400> 153
aaggagacca tccccctgac ggc                                              23

<210> 154
<211> 22
<212> DNA
<213> Artificial Sequence

<220>
<223> Synthetic Oligonucleotide

<400> 154
atgctgtggc tccttcctaa ct                                               22

<210> 155
<211> 27
<212> DNA
<213> Artificial Sequence

<220>
<223> Synthetic Oligonucleotide

<400> 155
acccaaattg tgatatacaa aaaggtt                                          27

<210> 156
<211> 30
<212> DNA
<213> Artificial Sequence

<220>
<223> Synthetic Oligonucleotide

<400> 156
taccaagcaa cctacatgtc aagaaagccc                                       30

<210> 157
<211> 18
<212> DNA
<213> Artificial Sequence

<220>
<223> Synthetic Oligonucleotide

<400> 157
atccaaggcc atgactgg                                                    18

<210> 158
<211> 20
<212> DNA
<213> Artificial Sequence

<220>
<223> Synthetic Oligonucleotide

<400> 158
ggaatgacct ctagggcctg                                                  20

<210> 159

<211> 24
<212> DNA
<213> Artificial Sequence

<220>
<223> Synthetic Oligonucleotide

<400> 159
acagccctgt atggccattg ttcc                                              24

<210> 160
<211> 19
<212> DNA
<213> Artificial Sequence

<220>
<223> Synthetic Oligonucleotide

<400> 160
gtgtgctagc gtactcccg                                                    19

<210> 161
<211> 18
<212> DNA
<213> Artificial Sequence

<220>
<223> Synthetic Oligonucleotide

<400> 161
gcatggtgcc ggttatct                                                     18

<210> 162
<211> 24
<212> DNA
<213> Artificial Sequence

<220>
<223> Synthetic Oligonucleotide

<400> 162
caaggaactg acgctcgagg acct                                              24

<210> 163
<211> 25
<212> DNA
<213> Artificial Sequence

<220>
<223> Synthetic Oligonucleotide

<400> 163
tgtatttcaa gacctctgtg cactt                                             25

<210> 164
<211> 23
<212> DNA
<213> Artificial Sequence

<220>
<223> Synthetic Oligonucleotide

<400> 164

ttagcctgag gaattgctgt gtt                                              23

<210> 165
<211> 25
<212> DNA
<213> Artificial Sequence

<220>
<223> Synthetic Oligonucleotide

<400> 165
tttatgaacc tgccctgctc ccaca                                            25

<210> 166
<211> 20
<212> DNA
<213> Artificial Sequence

<220>
<223> Synthetic Oligonucleotide

<400> 166
agatgaagtg gaaggcgctt                                                  20

<210> 167
<211> 21
<212> DNA
<213> Artificial Sequence

<220>
<223> Synthetic Oligonucleotide

<400> 167
tgcctctgta atcggcaact g                                                21

<210> 168
<211> 18
<212> DNA
<213> Artificial Sequence

<220>
<223> Synthetic Oligonucleotide

<400> 168
caccgcggcc atcctgca                                                    18

<210> 169
<211> 18
<212> DNA
<213> Artificial Sequence

<220>
<223> Synthetic Oligonucleotide

<400> 169
agtgggactg attgccgt                                                    18

<210> 170
<211> 19
<212> DNA
<213> Artificial Sequence

<220>

<223> Synthetic Oligonucleotide

<400> 170
gggtagcccc ctggattat                                                    19

<210> 171
<211> 24
<212> DNA
<213> Artificial Sequence

<220>
<223> Synthetic Oligonucleotide

<400> 171
tctgactcag gacaagctgt gccc                                              24

<210> 172
<211> 18
<212> DNA
<213> Artificial Sequence

<220>
<223> Synthetic Oligonucleotide

<400> 172
tggttcccag ccctgtgt                                                     18

<210> 173
<211> 19
<212> DNA
<213> Artificial Sequence

<220>
<223> Synthetic Oligonucleotide

<400> 173
ctcctccacc ctgggttgt                                                    19

<210> 174
<211> 28
<212> DNA
<213> Artificial Sequence

<220>
<223> Synthetic Oligonucleotide

<400> 174
ctccaagccc agattcagat tcgagtca                                          28

<210> 175
<211> 18
<212> DNA
<213> Artificial Sequence

<220>
<223> Synthetic Oligonucleotide

<400> 175
gagagcgacg cggttgtt                                                     18

<210> 176
<211> 26
<212> DNA

<213> Artificial Sequence

<220>
<223> Synthetic Oligonucleotide

<400> 176
gtatggtaga tcccggctta ttattc                                            26

<210> 177
<211> 18
<212> DNA
<213> Artificial Sequence

<220>
<223> Synthetic Oligonucleotide

<400> 177
tagctgccgc tgcggccg                                                     18

<210> 178
<211> 21
<212> DNA
<213> Artificial Sequence

<220>
<223> Synthetic Oligonucleotide

<400> 178
tggattggag ttctgggaat g                                                 21

<210> 179
<211> 22
<212> DNA
<213> Artificial Sequence

<220>
<223> Synthetic Oligonucleotide

<400> 179
gcttgcactc cacaggtaca ca                                                22

<210> 180
<211> 23
<212> DNA
<213> Artificial Sequence

<220>
<223> Synthetic Oligonucleotide

<400> 180
actggccgtg gcactggaca aca                                               23

<210> 181
<211> 21
<212> DNA
<213> Artificial Sequence

<220>
<223> Synthetic Oligonucleotide

<400> 181
aaacgagcag tttgccatca g                                                 21

<210> 182
<211> 20
<212> DNA
<213> Artificial Sequence

<220>
<223> Synthetic Oligonucleotide

<400> 182
gttggtgatg ttccgaagca                                                    20

<210> 183
<211> 24
<212> DNA
<213> Artificial Sequence

<220>
<223> Synthetic Oligonucleotide

<400> 183
cctcaccggc atagactgga agcg                                               24

<210> 184
<211> 19
<212> DNA
<213> Artificial Sequence

<220>
<223> Synthetic Oligonucleotide

<400> 184
gaggcacagt attgcccag                                                     19

<210> 185
<211> 21
<212> DNA
<213> Artificial Sequence

<220>
<223> Synthetic Oligonucleotide

<400> 185
gagacggttg gagagcttct t                                                  21

<210> 186
<211> 24
<212> DNA
<213> Artificial Sequence

<220>
<223> Synthetic Oligonucleotide

<400> 186
atgtttccca aggcctctgg atcc                                               24

<210> 187
<211> 21
<212> DNA
<213> Artificial Sequence

<220>
<223> Synthetic Oligonucleotide

<400> 187
tgagtgtccc ccggtatctt c                                                21

<210> 188
<211> 21
<212> DNA
<213> Artificial Sequence

<220>
<223> Synthetic Oligonucleotide

<400> 188
cagccgcttt cagattttca t                                                21

<210> 189
<211> 27
<212> DNA
<213> Artificial Sequence

<220>
<223> Synthetic Oligonucleotide

<400> 189
tgccaatccc gatgaaattg gaaattt                                          27

<210> 190
<211> 20
<212> DNA
<213> Artificial Sequence

<220>
<223> Synthetic Oligonucleotide

<400> 190
aagccctatc cgatgtaccc                                                  20

<210> 191
<211> 19
<212> DNA
<213> Artificial Sequence

<220>
<223> Synthetic Oligonucleotide

<400> 191
ctgtggcatt gagtttggg                                                   19

<210> 192
<211> 24
<212> DNA
<213> Artificial Sequence

<220>
<223> Synthetic Oligonucleotide

<400> 192
cacaacatcc ctggtgaacg tcgt                                             24

<210> 193
<211> 18
<212> DNA
<213> Artificial Sequence

<220>
<223> Synthetic Oligonucleotide

<400> 193
cggcgatcaa gaagctgt                                                    18

<210> 194
<211> 18
<212> DNA
<213> Artificial Sequence

<220>
<223> Synthetic Oligonucleotide

<400> 194
caggcgctga tctcttgc                                                    18

<210> 195
<211> 24
<212> DNA
<213> Artificial Sequence

<220>
<223> Synthetic Oligonucleotide

<400> 195
cgggcctctg atctccgatt tctt                                             24

<210> 196
<211> 21
<212> DNA
<213> Artificial Sequence

<220>
<223> Synthetic Oligonucleotide

<400> 196
tgacaatcag cacacctgca t                                                21

<210> 197
<211> 23
<212> DNA
<213> Artificial Sequence

<220>
<223> Synthetic Oligonucleotide

<400> 197
tgtgactaca gccgtgatcc tta                                              23

<210> 198
<211> 20
<212> DNA
<213> Artificial Sequence

<220>
<223> Synthetic Oligonucleotide

<400> 198
caggccctct tccgagcggt                                                  20

<210> 199
<211> 21

<212> DNA
<213> Artificial Sequence

<220>
<223> Synthetic Oligonucleotide

<400> 199
taaattcact cgtggtgtgg a                                                21

<210> 200
<211> 20
<212> DNA
<213> Artificial Sequence

<220>
<223> Synthetic Oligonucleotide

<400> 200
gcctcttgta gggccaatag                                                  20

<210> 201
<211> 21
<212> DNA
<213> Artificial Sequence

<220>
<223> Synthetic Oligonucleotide

<400> 201
cttcaattgg caagcccagg c                                                21

<210> 202
<211> 20
<212> DNA
<213> Artificial Sequence

<220>
<223> Synthetic Oligonucleotide

<400> 202
ggatgctggt gacctcttct                                                  20

<210> 203
<211> 20
<212> DNA
<213> Artificial Sequence

<220>
<223> Synthetic Oligonucleotide

<400> 203
gccaaggcac caagtaactc                                                  20

<210> 204
<211> 25
<212> DNA
<213> Artificial Sequence

<220>
<223> Synthetic Oligonucleotide

<400> 204
tccctcacgt tgcaacagga attaa                                            25

<210> 205
<211> 18
<212> DNA
<213> Artificial Sequence

<220>
<223> Synthetic Oligonucleotide

<400> 205
ctcccgtcaa cagcgttc                                                    18

<210> 206
<211> 18
<212> DNA
<213> Artificial Sequence

<220>
<223> Synthetic Oligonucleotide

<400> 206
gggtgagtct ggccttca                                                    18

<210> 207
<211> 24
<212> DNA
<213> Artificial Sequence

<220>
<223> Synthetic Oligonucleotide

<400> 207
acactggacc aggagtgcat ccag                                             24

<210> 208
<211> 20
<212> DNA
<213> Artificial Sequence

<220>
<223> Synthetic Oligonucleotide

<400> 208
ctgaaggagc tccaagacct                                                  20

<210> 209
<211> 20
<212> DNA
<213> Artificial Sequence

<220>
<223> Synthetic Oligonucleotide

<400> 209
caaaaccgct gtgtttcttc                                                  20

<210> 210
<211> 22
<212> DNA
<213> Artificial Sequence

<220>
<223> Synthetic Oligonucleotide

<400> 210
tgctgatgtg ccctctcctt gg                                                22

<210> 211
<211> 20
<212> DNA
<213> Artificial Sequence

<220>
<223> Synthetic Oligonucleotide

<400> 211
aaggaagtgg tccctctgtg                                                   20

<210> 212
<211> 20
<212> DNA
<213> Artificial Sequence

<220>
<223> Synthetic Oligonucleotide

<400> 212
gacgcagtct ttctgtctgg                                                   20

<210> 213
<211> 24
<212> DNA
<213> Artificial Sequence

<220>
<223> Synthetic Oligonucleotide

<400> 213
tgaagtctcc agctttgcct cagc                                              24

<210> 214
<211> 26
<212> DNA
<213> Artificial Sequence

<220>
<223> Synthetic Oligonucleotide

<400> 214
gataaattgg tacaagggat cagctt                                            26

<210> 215
<211> 24
<212> DNA
<213> Artificial Sequence

<220>
<223> Synthetic Oligonucleotide

<400> 215
gggtgccaag taactgacta ttca                                              24

<210> 216
<211> 26
<212> DNA
<213> Artificial Sequence

<220>
<223> Synthetic Oligonucleotide

<400> 216
ccagcccaca tgtcctgatc atatgc                                            26

<210> 217
<211> 21
<212> DNA
<213> Artificial Sequence

<220>
<223> Synthetic Oligonucleotide

<400> 217
atgtggaacc cccacctact t                                                 21

<210> 218
<211> 22
<212> DNA
<213> Artificial Sequence

<220>
<223> Synthetic Oligonucleotide

<400> 218
cagtccacag cacggttata cc                                                22

<210> 219
<211> 29
<212> DNA
<213> Artificial Sequence

<220>
<223> Synthetic Oligonucleotide

<400> 219
agtcccaaca gaaacaagaa cttcaggcg                                         29

<210> 220
<211> 24
<212> DNA
<213> Artificial Sequence

<220>
<223> Synthetic Oligonucleotide

<400> 220
ggatatttcc gtggctctta ttca                                              24

<210> 221
<211> 25
<212> DNA
<213> Artificial Sequence

<220>
<223> Synthetic Oligonucleotide

<400> 221
cttctcatca aggcagaaaa atctt                                             25

<210> 222

<211> 30
<212> DNA
<213> Artificial Sequence

<220>
<223> Synthetic Oligonucleotide

<400> 222
tctccatcaa atcctgtaaa ctccagagca                                        30

<210> 223
<211> 18
<212> DNA
<213> Artificial Sequence

<220>
<223> Synthetic Oligonucleotide

<400> 223
tcattgacca cagtggcg                                                     18

<210> 224
<211> 19
<212> DNA
<213> Artificial Sequence

<220>
<223> Synthetic Oligonucleotide

<400> 224
tcgtgtactt ctccacccg                                                    19

<210> 225
<211> 24
<212> DNA
<213> Artificial Sequence

<220>
<223> Synthetic Oligonucleotide

<400> 225
cacgtcctca tactcaccaa ggcg                                              24

<210> 226
<211> 23
<212> DNA
<213> Artificial Sequence

<220>
<223> Synthetic Oligonucleotide

<400> 226
ccccaggata cctaccacta cct                                               23

<210> 227
<211> 18
<212> DNA
<213> Artificial Sequence

<220>
<223> Synthetic Oligonucleotide

<400> 227

tgcgggactt gggaaaga                                                    18

<210> 228
<211> 20
<212> DNA
<213> Artificial Sequence

<220>
<223> Synthetic Oligonucleotide

<400> 228
cccttcagcc tgccccaccg                                                  20

<210> 229
<211> 22
<212> DNA
<213> Artificial Sequence

<220>
<223> Synthetic Oligonucleotide

<400> 229
gacatttcca gtcctgcagt ca                                               22

<210> 230
<211> 20
<212> DNA
<213> Artificial Sequence

<220>
<223> Synthetic Oligonucleotide

<400> 230
ctccgatcgc acacatttgt                                                  20

<210> 231
<211> 23
<212> DNA
<213> Artificial Sequence

<220>
<223> Synthetic Oligonucleotide

<400> 231
tgcctctctg ccccaccctt tgt                                              23

<210> 232
<211> 21
<212> DNA
<213> Artificial Sequence

<220>
<223> Synthetic Oligonucleotide

<400> 232
tccctccact cggaaggact a                                                21

<210> 233
<211> 22
<212> DNA
<213> Artificial Sequence

<220>

<223> Synthetic Oligonucleotide

<400> 233
cggttgttgc tgatctgtct ca                                                22

<210> 234
<211> 27
<212> DNA
<213> Artificial Sequence

<220>
<223> Synthetic Oligonucleotide

<400> 234
tctgacactg tccaacttga ccctctt                                           27

<210> 235
<211> 18
<212> DNA
<213> Artificial Sequence

<220>
<223> Synthetic Oligonucleotide

<400> 235
tccaccctcc tggctttg                                                     18

<210> 236
<211> 21
<212> DNA
<213> Artificial Sequence

<220>
<223> Synthetic Oligonucleotide

<400> 236
tcactcccac gttcaccttg t                                                 21

<210> 237
<211> 23
<212> DNA
<213> Artificial Sequence

<220>
<223> Synthetic Oligonucleotide

<400> 237
tcctttcgtc ttcgccatgc tgg                                               23

<210> 238
<211> 18
<212> DNA
<213> Artificial Sequence

<220>
<223> Synthetic Oligonucleotide

<400> 238
gtggccatcc agctgacc                                                     18

<210> 239
<211> 23
<212> DNA

<213> Artificial Sequence

<220>
<223> Synthetic Oligonucleotide

<400> 239
cagtggtagg tgatgttctg gga                                              23

<210> 240
<211> 21
<212> DNA
<213> Artificial Sequence

<220>
<223> Synthetic Oligonucleotide

<400> 240
tcctgcgcct gatgtccacc g                                                21

<210> 241
<211> 24
<212> DNA
<213> Artificial Sequence

<220>
<223> Synthetic Oligonucleotide

<400> 241
cagccaagaa ctggtatagg agct                                             24

<210> 242
<211> 19
<212> DNA
<213> Artificial Sequence

<220>
<223> Synthetic Oligonucleotide

<400> 242
aaactggctg ccagcattg                                                   19

<210> 243
<211> 30
<212> DNA
<213> Artificial Sequence

<220>
<223> Synthetic Oligonucleotide

<400> 243
tctcctagcc agacgtgttt cttgtccttg                                       30

<210> 244
<211> 21
<212> DNA
<213> Artificial Sequence

<220>
<223> Synthetic Oligonucleotide

<400> 244
gagagcaagc gagacattct g                                                21

<210> 245
<211> 18
<212> DNA
<213> Artificial Sequence

<220>
<223> Synthetic Oligonucleotide

<400> 245
aacagggaac tggcccac                                                    18

<210> 246
<211> 24
<212> DNA
<213> Artificial Sequence

<220>
<223> Synthetic Oligonucleotide

<400> 246
cctctttgac ggctcagcca atct                                             24

<210> 247
<211> 24
<212> DNA
<213> Artificial Sequence

<220>
<223> Synthetic Oligonucleotide

<400> 247
cgacagttgc gatgaaagtt ctaa                                             24

<210> 248
<211> 22
<212> DNA
<213> Artificial Sequence

<220>
<223> Synthetic Oligonucleotide

<400> 248
ggctgctaga gaccatggac at                                               22

<210> 249
<211> 27
<212> DNA
<213> Artificial Sequence

<220>
<223> Synthetic Oligonucleotide

<400> 249
cctcctcctg ttgctgccac taatgct                                          27

<210> 250
<211> 23
<212> DNA
<213> Artificial Sequence

<220>
<223> Synthetic Oligonucleotide

<400> 250
tctgcagagt tggaagcact cta                                              23

<210> 251
<211> 21
<212> DNA
<213> Artificial Sequence

<220>
<223> Synthetic Oligonucleotide

<400> 251
gccgaggctt ttctaccaga a                                                21

<210> 252
<211> 28
<212> DNA
<213> Artificial Sequence

<220>
<223> Synthetic Oligonucleotide

<400> 252
caggatacag ctccacagca tcgatgtc                                         28

<210> 253
<211> 20
<212> DNA
<213> Artificial Sequence

<220>
<223> Synthetic Oligonucleotide

<400> 253
acctctgtgg tccgtaggag                                                  20

<210> 254
<211> 20
<212> DNA
<213> Artificial Sequence

<220>
<223> Synthetic Oligonucleotide

<400> 254
cgaccacttg ttttccactg                                                  20

<210> 255
<211> 24
<212> DNA
<213> Artificial Sequence

<220>
<223> Synthetic Oligonucleotide

<400> 255
tcttcccagg gccctcctca tagt                                             24

<210> 256
<211> 20
<212> DNA
<213> Artificial Sequence

<220>
<223> Synthetic Oligonucleotide

<400> 256
aacttcaagg tcggagaagg                                                    20

<210> 257
<211> 20
<212> DNA
<213> Artificial Sequence

<220>
<223> Synthetic Oligonucleotide

<400> 257
tggctaaact cctgcacttg                                                    20

<210> 258
<211> 21
<212> DNA
<213> Artificial Sequence

<220>
<223> Synthetic Oligonucleotide

<400> 258
ccgtccacgg tctcctcctc a                                                  21

<210> 259
<211> 18
<212> DNA
<213> Artificial Sequence

<220>
<223> Synthetic Oligonucleotide

<400> 259
gtgctacgcc accctgtt                                                      18

<210> 260
<211> 20
<212> DNA
<213> Artificial Sequence

<220>
<223> Synthetic Oligonucleotide

<400> 260
caggggcttc tcgtagatgt                                                    20

<210> 261
<211> 22
<212> DNA
<213> Artificial Sequence

<220>
<223> Synthetic Oligonucleotide

<400> 261
ccgatgttca cgcctttggg tc                                                 22

<210> 262
<211> 20

<212> DNA
<213> Artificial Sequence

<220>
<223> Synthetic Oligonucleotide

<400> 262
gatgtgattg aggtgcatgg                                                    20

<210> 263
<211> 21
<212> DNA
<213> Artificial Sequence

<220>
<223> Synthetic Oligonucleotide

<400> 263
gaactccctg gagatgaaac c                                                  21

<210> 264
<211> 24
<212> DNA
<213> Artificial Sequence

<220>
<223> Synthetic Oligonucleotide

<400> 264
tgttcatcct ggcgctcttc atgt                                               24

<210> 265
<211> 18
<212> DNA
<213> Artificial Sequence

<220>
<223> Synthetic Oligonucleotide

<400> 265
tgcagcggct gattgaca                                                      18

<210> 266
<211> 25
<212> DNA
<213> Artificial Sequence

<220>
<223> Synthetic Oligonucleotide

<400> 266
caactgttcc tggtctacaa actca                                              25

<210> 267
<211> 28
<212> DNA
<213> Artificial Sequence

<220>
<223> Synthetic Oligonucleotide

<400> 267
tcagatggag acctcgtgcc aaattaca                                           28

<210> 268
<211> 20
<212> DNA
<213> Artificial Sequence

<220>
<223> Synthetic Oligonucleotide

<400> 268
agcttttccg gaatctgttc                                                   20

<210> 269
<211> 20
<212> DNA
<213> Artificial Sequence

<220>
<223> Synthetic Oligonucleotide

<400> 269
atttgagcat gttccagtcg                                                   20

<210> 270
<211> 24
<212> DNA
<213> Artificial Sequence

<220>
<223> Synthetic Oligonucleotide

<400> 270
catcgccagg gcttctccta tgac                                              24

<210> 271
<211> 20
<212> DNA
<213> Artificial Sequence

<220>
<223> Synthetic Oligonucleotide

<400> 271
tggcagaact acctgcaaga                                                   20

<210> 272
<211> 19
<212> DNA
<213> Artificial Sequence

<220>
<223> Synthetic Oligonucleotide

<400> 272
tgcttcaagg tgtggttgg                                                    19

<210> 273
<211> 24
<212> DNA
<213> Artificial Sequence

<220>
<223> Synthetic Oligonucleotide

<400> 273
cacctgcgtc tggatgactg tgac                                              24

<210> 274
<211> 24
<212> DNA
<213> Artificial Sequence

<220>
<223> Synthetic Oligonucleotide

<400> 274
gtacatgatc ccctgtgaga aggt                                              24

<210> 275
<211> 21
<212> DNA
<213> Artificial Sequence

<220>
<223> Synthetic Oligonucleotide

<400> 275
gggacagctt gtagcctttg c                                                 21

<210> 276
<211> 22
<212> DNA
<213> Artificial Sequence

<220>
<223> Synthetic Oligonucleotide

<400> 276
accctgcccg cgatcacact ga                                                22

<210> 277
<211> 23
<212> DNA
<213> Artificial Sequence

<220>
<223> Synthetic Oligonucleotide

<400> 277
gggaggctta tctcactgag tga                                               23

<210> 278
<211> 19
<212> DNA
<213> Artificial Sequence

<220>
<223> Synthetic Oligonucleotide

<400> 278
ccattgcagc cttcattgc                                                    19

<210> 279
<211> 29
<212> DNA
<213> Artificial Sequence

<220>
<223> Synthetic Oligonucleotide

<400> 279
ttgaggccca gagcagtcta ccagattct                                        29

<210> 280
<211> 21
<212> DNA
<213> Artificial Sequence

<220>
<223> Synthetic Oligonucleotide

<400> 280
tgtctcactg agcgagcaga a                                                21

<210> 281
<211> 19
<212> DNA
<213> Artificial Sequence

<220>
<223> Synthetic Oligonucleotide

<400> 281
accattgcag ccctgattg                                                   19

<210> 282
<211> 24
<212> DNA
<213> Artificial Sequence

<220>
<223> Synthetic Oligonucleotide

<400> 282
cttgaggacg cgaacagtcc acca                                             24

<210> 283
<211> 20
<212> DNA
<213> Artificial Sequence

<220>
<223> Synthetic Oligonucleotide

<400> 283
tgaccgcttc taccccaatg                                                  20

<210> 284
<211> 22
<212> DNA
<213> Artificial Sequence

<220>
<223> Synthetic Oligonucleotide

<400> 284
aggataaggc caaccatgat gt                                               22

<210> 285

<211> 28
<212> DNA
<213> Artificial Sequence

<220>
<223> Synthetic Oligonucleotide

<400> 285
ctgaaactgg aacacaacca cccacaag                                          28

<210> 286
<211> 19
<212> DNA
<213> Artificial Sequence

<220>
<223> Synthetic Oligonucleotide

<400> 286
ggtgcctact ccattgtgg                                                    19

<210> 287
<211> 20
<212> DNA
<213> Artificial Sequence

<220>
<223> Synthetic Oligonucleotide

<400> 287
gtggagccct tcttcctctt                                                   20

<210> 288
<211> 24
<212> DNA
<213> Artificial Sequence

<220>
<223> Synthetic Oligonucleotide

<400> 288
tactccagca ggcacacaaa cacg                                              24

<210> 289
<211> 22
<212> DNA
<213> Artificial Sequence

<220>
<223> Synthetic Oligonucleotide

<400> 289
ccagctttgt gcctgtcact at                                                22

<210> 290
<211> 20
<212> DNA
<213> Artificial Sequence

<220>
<223> Synthetic Oligonucleotide

<400> 290

gggaatgtgg tagcccaaga                                                20

<210> 291
<211> 25
<212> DNA
<213> Artificial Sequence

<220>
<223> Synthetic Oligonucleotide

<400> 291
ctcatgccac cactgccaac acctc                                           25

<210> 292
<211> 20
<212> DNA
<213> Artificial Sequence

<220>
<223> Synthetic Oligonucleotide

<400> 292
ccgtgttcaa gaggaagctc                                                20

<210> 293
<211> 20
<212> DNA
<213> Artificial Sequence

<220>
<223> Synthetic Oligonucleotide

<400> 293
agtgggatca cagggtgaag                                                20

<210> 294
<211> 24
<212> DNA
<213> Artificial Sequence

<220>
<223> Synthetic Oligonucleotide

<400> 294
ttttctcaac tcctccacaa ggca                                            24

<210> 295
<211> 30
<212> DNA
<213> Artificial Sequence

<220>
<223> Synthetic Oligonucleotide

<400> 295
agaacaagga caacatagat ccttacatat                                      30

<210> 296
<211> 19
<212> DNA
<213> Artificial Sequence

<220>

<223> Synthetic Oligonucleotide

<400> 296
gcaaacctca tgccaatgc                                                    19

<210> 297
<211> 26
<212> DNA
<213> Artificial Sequence

<220>
<223> Synthetic Oligonucleotide

<400> 297
cacacccttt ggaagtggac ccagaa                                            26

<210> 298
<211> 18
<212> DNA
<213> Artificial Sequence

<220>
<223> Synthetic Oligonucleotide

<400> 298
ccgtgtggct cgctttct                                                     18

<210> 299
<211> 24
<212> DNA
<213> Artificial Sequence

<220>
<223> Synthetic Oligonucleotide

<400> 299
ggagatttcg ctgaagagta acca                                              24

<210> 300
<211> 20
<212> DNA
<213> Artificial Sequence

<220>
<223> Synthetic Oligonucleotide

<400> 300
tgccgcttcc tctttgcggg                                                   20

<210> 301
<211> 19
<212> DNA
<213> Artificial Sequence

<220>
<223> Synthetic Oligonucleotide

<400> 301
cacaatggcg gctctgaag                                                    19

<210> 302
<211> 26
<212> DNA

<213> Artificial Sequence

<220>
<223> Synthetic Oligonucleotide

<400> 302
acacaaacac tgtctgtacc tgaaga                                            26

<210> 303
<211> 23
<212> DNA
<213> Artificial Sequence

<220>
<223> Synthetic Oligonucleotide

<400> 303
aagttacgct gcgcgacagc caa                                               23

<210> 304
<211> 20
<212> DNA
<213> Artificial Sequence

<220>
<223> Synthetic Oligonucleotide

<400> 304
caagcagtca aggagaacca                                                   20

<210> 305
<211> 20
<212> DNA
<213> Artificial Sequence

<220>
<223> Synthetic Oligonucleotide

<400> 305
agttttgctc gcctcatctt                                                   20

<210> 306
<211> 22
<212> DNA
<213> Artificial Sequence

<220>
<223> Synthetic Oligonucleotide

<400> 306
ttcttctgtc tcccgccgct tc                                                22

<210> 307
<211> 20
<212> DNA
<213> Artificial Sequence

<220>
<223> Synthetic Oligonucleotide

<400> 307
tccaattcca gcatcactgt                                                   20

<210> 308
<211> 20
<212> DNA
<213> Artificial Sequence

<220>
<223> Synthetic Oligonucleotide

<400> 308
ggcagtgaag gcgataaagt                                                   20

<210> 309
<211> 25
<212> DNA
<213> Artificial Sequence

<220>
<223> Synthetic Oligonucleotide

<400> 309
agaaaagctg tttgtctccc cagca                                             25

<210> 310
<211> 20
<212> DNA
<213> Artificial Sequence

<220>
<223> Synthetic Oligonucleotide

<400> 310
cagtgcttcc atggacaagt                                                   20

<210> 311
<211> 20
<212> DNA
<213> Artificial Sequence

<220>
<223> Synthetic Oligonucleotide

<400> 311
tggacaggga tgattgatgt                                                   20

<210> 312
<211> 24
<212> DNA
<213> Artificial Sequence

<220>
<223> Synthetic Oligonucleotide

<400> 312
atctccatca gcatgggcca gttt                                              24

<210> 313
<211> 21
<212> DNA
<213> Artificial Sequence

<220>
<223> Synthetic Oligonucleotide

<400> 313
tgcacagagg gtgtgggtta c                                                21

<210> 314
<211> 28
<212> DNA
<213> Artificial Sequence

<220>
<223> Synthetic Oligonucleotide

<400> 314
tcttcatctg atttacaagc tgtacatg                                         28

<210> 315
<211> 29
<212> DNA
<213> Artificial Sequence

<220>
<223> Synthetic Oligonucleotide

<400> 315
caatgcttcc aacaatttgt ttgcttgcc                                        29

<210> 316
<211> 24
<212> DNA
<213> Artificial Sequence

<220>
<223> Synthetic Oligonucleotide

<400> 316
ctctgagaca gtgcttcgat gact                                             24

<210> 317
<211> 19
<212> DNA
<213> Artificial Sequence

<220>
<223> Synthetic Oligonucleotide

<400> 317
ccatgaggcc caacttcct                                                   19

<210> 318
<211> 23
<212> DNA
<213> Artificial Sequence

<220>
<223> Synthetic Oligonucleotide

<400> 318
cagacttggt gccctttgac tcc                                              23

<210> 319
<211> 20
<212> DNA
<213> Artificial Sequence

<220>
<223> Synthetic Oligonucleotide

<400> 319
agacatcagc tcctggttca                                                    20

<210> 320
<211> 20
<212> DNA
<213> Artificial Sequence

<220>
<223> Synthetic Oligonucleotide

<400> 320
gacaaacacc cttcctccag                                                    20

<210> 321
<211> 26
<212> DNA
<213> Artificial Sequence

<220>
<223> Synthetic Oligonucleotide

<400> 321
cgaggccatt gacttcatag actcca                                             26

<210> 322
<211> 22
<212> DNA
<213> Artificial Sequence

<220>
<223> Synthetic Oligonucleotide

<400> 322
cagaggatga cgaggatgat ga                                                 22

<210> 323
<211> 18
<212> DNA
<213> Artificial Sequence

<220>
<223> Synthetic Oligonucleotide

<400> 323
ctgtgccgcc tccttgtc                                                      18

<210> 324
<211> 28
<212> DNA
<213> Artificial Sequence

<220>
<223> Synthetic Oligonucleotide

<400> 324
ctcctcattg tcactgccaa acaggtca                                           28

<210> 325
<211> 20

<212> DNA
<213> Artificial Sequence

<220>
<223> Synthetic Oligonucleotide

<400> 325
tgtcgatgga cttccagaac                                                    20

<210> 326
<211> 19
<212> DNA
<213> Artificial Sequence

<220>
<223> Synthetic Oligonucleotide

<400> 326
attgggacag cttggatca                                                     19

<210> 327
<211> 18
<212> DNA
<213> Artificial Sequence

<220>
<223> Synthetic Oligonucleotide

<400> 327
cacctgggca gctgccaa                                                      18

<210> 328
<211> 23
<212> DNA
<213> Artificial Sequence

<220>
<223> Synthetic Oligonucleotide

<400> 328
gatctaagat ggcgactgtc gaa                                                23

<210> 329
<211> 25
<212> DNA
<213> Artificial Sequence

<220>
<223> Synthetic Oligonucleotide

<400> 329
ttagattccg ttttctcctc ttctg                                              25

<210> 330
<211> 27
<212> DNA
<213> Artificial Sequence

<220>
<223> Synthetic Oligonucleotide

<400> 330
accaccccta ctcctaatcc cccgact                                            27

<210> 331
<211> 18
<212> DNA
<213> Artificial Sequence

<220>
<223> Synthetic Oligonucleotide

<400> 331
aagccgcggt tgaatgtg                                                    18

<210> 332
<211> 20
<212> DNA
<213> Artificial Sequence

<220>
<223> Synthetic Oligonucleotide

<400> 332
tgacgccagc ttcaatgatg                                                  20

<210> 333
<211> 25
<212> DNA
<213> Artificial Sequence

<220>
<223> Synthetic Oligonucleotide

<400> 333
tgaccctctc cctctctgga tggca                                            25

<210> 334
<211> 18
<212> DNA
<213> Artificial Sequence

<220>
<223> Synthetic Oligonucleotide

<400> 334
ctcggatcct agccctcg                                                    18

<210> 335
<211> 22
<212> DNA
<213> Artificial Sequence

<220>
<223> Synthetic Oligonucleotide

<400> 335
ggcattggaa tatccctctg ta                                               22

<210> 336
<211> 24
<212> DNA
<213> Artificial Sequence

<220>
<223> Synthetic Oligonucleotide

<400> 336
cctccatgga ctcgagtgta ccga                                              24

<210> 337
<211> 20
<212> DNA
<213> Artificial Sequence

<220>
<223> Synthetic Oligonucleotide

<400> 337
tggtccatcg ccagttatca                                                   20

<210> 338
<211> 23
<212> DNA
<213> Artificial Sequence

<220>
<223> Synthetic Oligonucleotide

<400> 338
tgttctagcg atcttgcttc aca                                               23

<210> 339
<211> 30
<212> DNA
<213> Artificial Sequence

<220>
<223> Synthetic Oligonucleotide

<400> 339
atctgtatgc ggaacctcaa aagagtccct                                        30

<210> 340
<211> 23
<212> DNA
<213> Artificial Sequence

<220>
<223> Synthetic Oligonucleotide

<400> 340
cggttatgtc atgccagata cac                                               23

<210> 341
<211> 24
<212> DNA
<213> Artificial Sequence

<220>
<223> Synthetic Oligonucleotide

<400> 341
gaactgagac ccactgaaga aagg                                              24

<210> 342
<211> 25
<212> DNA
<213> Artificial Sequence

<220>
<223> Synthetic Oligonucleotide

<400> 342
cctcaaaggt actccctcct cccgg                                          25

<210> 343
<211> 25
<212> DNA
<213> Artificial Sequence

<220>
<223> Synthetic Oligonucleotide

<400> 343
tggctcttaa tcagtttcgt tacct                                          25

<210> 344
<211> 25
<212> DNA
<213> Artificial Sequence

<220>
<223> Synthetic Oligonucleotide

<400> 344
caaggcatat cgatcctcat aaagt                                          25

<210> 345
<211> 30
<212> DNA
<213> Artificial Sequence

<220>
<223> Synthetic Oligonucleotide

<400> 345
tgtcccacga ataatgcgta aattctccag                                     30

<210> 346
<211> 20
<212> DNA
<213> Artificial Sequence

<220>
<223> Synthetic Oligonucleotide

<400> 346
gtccaggtgg atgtgaaaga                                                20

<210> 347
<211> 20
<212> DNA
<213> Artificial Sequence

<220>
<223> Synthetic Oligonucleotide

<400> 347
cggccaggat acacatctta                                                20

<210> 348

<211> 21
<212> DNA
<213> Artificial Sequence

<220>
<223> Synthetic Oligonucleotide

<400> 348
cagcaggccc tcaaggagct g                                                 21

<210> 349
<211> 20
<212> DNA
<213> Artificial Sequence

<220>
<223> Synthetic Oligonucleotide

<400> 349
acggatcaca gtggaggaag                                                   20

<210> 350
<211> 20
<212> DNA
<213> Artificial Sequence

<220>
<223> Synthetic Oligonucleotide

<400> 350
ctcatccgtc gggtcatagt                                                   20

<210> 351
<211> 20
<212> DNA
<213> Artificial Sequence

<220>
<223> Synthetic Oligonucleotide

<400> 351
cgctggctca cccctacctg                                                   20

<210> 352
<211> 18
<212> DNA
<213> Artificial Sequence

<220>
<223> Synthetic Oligonucleotide

<400> 352
acccccagac cggatcag                                                     18

<210> 353
<211> 23
<212> DNA
<213> Artificial Sequence

<220>
<223> Synthetic Oligonucleotide

<400> 353

tgtagggcag acttcctcaa aca                                              23

<210> 354
<211> 23
<212> DNA
<213> Artificial Sequence

<220>
<223> Synthetic Oligonucleotide

<400> 354
ctggccctca tgtccccttc acg                                              23

<210> 355
<211> 19
<212> DNA
<213> Artificial Sequence

<220>
<223> Synthetic Oligonucleotide

<400> 355
cgtggtgccc ctctatgac                                                   19

<210> 356
<211> 19
<212> DNA
<213> Artificial Sequence

<220>
<223> Synthetic Oligonucleotide

<400> 356
ggctagtggg cgcatgtag                                                   19

<210> 357
<211> 19
<212> DNA
<213> Artificial Sequence

<220>
<223> Synthetic Oligonucleotide

<400> 357
ctggagatgc tggacgccc                                                   19

<210> 358
<211> 21
<212> DNA
<213> Artificial Sequence

<220>
<223> Synthetic Oligonucleotide

<400> 358
ggattgctca acaaccatgc t                                                21

<210> 359
<211> 24
<212> DNA
<213> Artificial Sequence

<220>

<223> Synthetic Oligonucleotide

<400> 359
ggcattaaca cttttggacg ataa                                              24

<210> 360
<211> 30
<212> DNA
<213> Artificial Sequence

<220>
<223> Synthetic Oligonucleotide

<400> 360
tctggaccct cctacctctg gttcttacgt                                        30

<210> 361
<211> 24
<212> DNA
<213> Artificial Sequence

<220>
<223> Synthetic Oligonucleotide

<400> 361
gcactttggg attctttcca ttat                                              24

<210> 362
<211> 24
<212> DNA
<213> Artificial Sequence

<220>
<223> Synthetic Oligonucleotide

<400> 362
gcatgtaaga agaccctcac tgaa                                              24

<210> 363
<211> 29
<212> DNA
<213> Artificial Sequence

<220>
<223> Synthetic Oligonucleotide

<400> 363
acaacattct cggtgcctgt aacaaagaa                                         29

<210> 364
<211> 18
<212> DNA
<213> Artificial Sequence

<220>
<223> Synthetic Oligonucleotide

<400> 364
gcctcttcct gttcgacg                                                     18

<210> 365
<211> 18
<212> DNA

<213> Artificial Sequence

<220>
<223> Synthetic Oligonucleotide

<400> 365
gctttgcccg gtagctct                                                    18

<210> 366
<211> 24
<212> DNA
<213> Artificial Sequence

<220>
<223> Synthetic Oligonucleotide

<400> 366
tcgcccacct acgtactggc ctac                                             24

<210> 367
<211> 25
<212> DNA
<213> Artificial Sequence

<220>
<223> Synthetic Oligonucleotide

<400> 367
ggattgtaga ctgtcaccga aattc                                            25

<210> 368
<211> 28
<212> DNA
<213> Artificial Sequence

<220>
<223> Synthetic Oligonucleotide

<400> 368
ggctattcct cattttctct acaaagtg                                         28

<210> 369
<211> 30
<212> DNA
<213> Artificial Sequence

<220>
<223> Synthetic Oligonucleotide

<400> 369
cctccaggag gctaccttct tcatgttcac                                       30

<210> 370
<211> 19
<212> DNA
<213> Artificial Sequence

<220>
<223> Synthetic Oligonucleotide

<400> 370
aaggaaaggg tgcctcatc                                                   19

<210> 371
<211> 19
<212> DNA
<213> Artificial Sequence

<220>
<223> Synthetic Oligonucleotide

<400> 371
gagccacaag cagcacagt                                                    19

<210> 372
<211> 24
<212> DNA
<213> Artificial Sequence

<220>
<223> Synthetic Oligonucleotide

<400> 372
catcacccac aaggacaggt tgct                                              24

<210> 373
<211> 20
<212> DNA
<213> Artificial Sequence

<220>
<223> Synthetic Oligonucleotide

<400> 373
cacgggacat tcaccacatc                                                   20

<210> 374
<211> 19
<212> DNA
<213> Artificial Sequence

<220>
<223> Synthetic Oligonucleotide

<400> 374
gggtgccatc cacttcaca                                                    19

<210> 375
<211> 27
<212> DNA
<213> Artificial Sequence

<220>
<223> Synthetic Oligonucleotide

<400> 375
ataaaaagac aaccaacggc cgactgc                                           27

<210> 376
<211> 18
<212> DNA
<213> Artificial Sequence

<220>
<223> Synthetic Oligonucleotide

<400> 376
ccagtggagc gcttccat                                                    18

<210> 377
<211> 22
<212> DNA
<213> Artificial Sequence

<220>
<223> Synthetic Oligonucleotide

<400> 377
ctctctgggt cgtctgaaac aa                                               22

<210> 378
<211> 23
<212> DNA
<213> Artificial Sequence

<220>
<223> Synthetic Oligonucleotide

<400> 378
tcggccactt catcaggacg cag                                              23

<210> 379
<211> 22
<212> DNA
<213> Artificial Sequence

<220>
<223> Synthetic Oligonucleotide

<400> 379
ggttccagct ttctgtagct gt                                               22

<210> 380
<211> 19
<212> DNA
<213> Artificial Sequence

<220>
<223> Synthetic Oligonucleotide

<400> 380
gccgcaggtt ctagttgct                                                   19

<210> 381
<211> 24
<212> DNA
<213> Artificial Sequence

<220>
<223> Synthetic Oligonucleotide

<400> 381
attggtggcc acaccacctc ctta                                             24

<210> 382
<211> 21
<212> DNA
<213> Artificial Sequence

<220>
<223> Synthetic Oligonucleotide

<400> 382
gcgtatgatt tcccgaatga g                                                21

<210> 383
<211> 21
<212> DNA
<213> Artificial Sequence

<220>
<223> Synthetic Oligonucleotide

<400> 383
cagtgacctc gtacccattg c                                                21

<210> 384
<211> 25
<212> DNA
<213> Artificial Sequence

<220>
<223> Synthetic Oligonucleotide

<400> 384
atgttgatat gcccaaactt catga                                            25

<210> 385
<211> 21
<212> DNA
<213> Artificial Sequence

<220>
<223> Synthetic Oligonucleotide

<400> 385
cgagcccttt gatgacttcc t                                                21

<210> 386
<211> 18
<212> DNA
<213> Artificial Sequence

<220>
<223> Synthetic Oligonucleotide

<400> 386
ggagcgggct gtctcaga                                                    18

<210> 387
<211> 22
<212> DNA
<213> Artificial Sequence

<220>
<223> Synthetic Oligonucleotide

<400> 387
tcccagcatc atccaggccc ag                                               22

<210> 388
<211> 19

<212> DNA
<213> Artificial Sequence

<220>
<223> Synthetic Oligonucleotide

<400> 388
ccaccccgag caaatctgt                                                    19

<210> 389
<211> 22
<212> DNA
<213> Artificial Sequence

<220>
<223> Synthetic Oligonucleotide

<400> 389
aaatccagtc cccctacttt gg                                                22

<210> 390
<211> 23
<212> DNA
<213> Artificial Sequence

<220>
<223> Synthetic Oligonucleotide

<400> 390
cctgaatcct ggaggctcac gcc                                               23

<210> 391
<211> 26
<212> DNA
<213> Artificial Sequence

<220>
<223> Synthetic Oligonucleotide

<400> 391
ggataattca gacaacaaca ccatct                                            26

<210> 392
<211> 25
<212> DNA
<213> Artificial Sequence

<220>
<223> Synthetic Oligonucleotide

<400> 392
tgaagtaatc agccacagac tcaat                                             25

<210> 393
<211> 29
<212> DNA
<213> Artificial Sequence

<220>
<223> Synthetic Oligonucleotide

<400> 393
tcaattgtaa cattctcacc caggccttg                                         29

<210> 394
<211> 20
<212> DNA
<213> Artificial Sequence

<220>
<223> Synthetic Oligonucleotide

<400> 394
gaagcgcaga tcatgaagaa                                                    20

<210> 395
<211> 20
<212> DNA
<213> Artificial Sequence

<220>
<223> Synthetic Oligonucleotide

<400> 395
ctcctcagac accactgcat                                                    20

<210> 396
<211> 24
<212> DNA
<213> Artificial Sequence

<220>
<223> Synthetic Oligonucleotide

<400> 396
ctgaagcacg acaagctggt ccag                                               24

<210> 397
<211> 20
<212> DNA
<213> Artificial Sequence

<220>
<223> Synthetic Oligonucleotide

<400> 397
cctccaactc ctagcctcaa                                                    20

<210> 398
<211> 18
<212> DNA
<213> Artificial Sequence

<220>
<223> Synthetic Oligonucleotide

<400> 398
ggcgcatgct tgtaatcc                                                      18

<210> 399
<211> 24
<212> DNA
<213> Artificial Sequence

<220>
<223> Synthetic Oligonucleotide

<400> 399
tgatcctcct gtctcaacct ccca                                              24

<210> 400
<211> 18
<212> DNA
<213> Artificial Sequence

<220>
<223> Synthetic Oligonucleotide

<400> 400
gtgctggtga cgaatcca                                                     18

<210> 401
<211> 20
<212> DNA
<213> Artificial Sequence

<220>
<223> Synthetic Oligonucleotide

<400> 401
cccggcaaaa acaaataagt                                                   20

<210> 402
<211> 25
<212> DNA
<213> Artificial Sequence

<220>
<223> Synthetic Oligonucleotide

<400> 402
ttcatctcaa tggaaggatc ctgcc                                             25

<210> 403
<211> 20
<212> DNA
<213> Artificial Sequence

<220>
<223> Synthetic Oligonucleotide

<400> 403
accctcgaca agaccacact                                                   20

<210> 404
<211> 20
<212> DNA
<213> Artificial Sequence

<220>
<223> Synthetic Oligonucleotide

<400> 404
tgggagttca tgggtacaga                                                   20

<210> 405
<211> 24
<212> DNA
<213> Artificial Sequence

<220>
<223> Synthetic Oligonucleotide

<400> 405
aacttcagcc ccagctccca agtc                                              24

<210> 406
<211> 25
<212> DNA
<213> Artificial Sequence

<220>
<223> Synthetic Oligonucleotide

<400> 406
aagggcaatc acagtgttaa aagaa                                             25

<210> 407
<211> 26
<212> DNA
<213> Artificial Sequence

<220>
<223> Synthetic Oligonucleotide

<400> 407
ggagaacttc aatgaagaat tttcca                                            26

<210> 408
<211> 29
<212> DNA
<213> Artificial Sequence

<220>
<223> Synthetic Oligonucleotide

<400> 408
cataggagca gcctgcaaca tttcagcat                                         29

<210> 409
<211> 20
<212> DNA
<213> Artificial Sequence

<220>
<223> Synthetic Oligonucleotide

<400> 409
attccaccca tggcaaattc                                                   20

<210> 410
<211> 22
<212> DNA
<213> Artificial Sequence

<220>
<223> Synthetic Oligonucleotide

<400> 410
gatgggattt ccattgatga ca                                                22

<210> 411

<211> 22
<212> DNA
<213> Artificial Sequence

<220>
<223> Synthetic Oligonucleotide

<400> 411
ccgttctcag ccttgacggt gc                                                22

<210> 412
<211> 23
<212> DNA
<213> Artificial Sequence

<220>
<223> Synthetic Oligonucleotide

<400> 412
caaaggagct cactgtggtg tct                                               23

<210> 413
<211> 26
<212> DNA
<213> Artificial Sequence

<220>
<223> Synthetic Oligonucleotide

<400> 413
gagtcagaat ggcttattca cagatg                                            26

<210> 414
<211> 24
<212> DNA
<213> Artificial Sequence

<220>
<223> Synthetic Oligonucleotide

<400> 414
tgttccaacc actgaatctg gacc                                              24

<210> 415
<211> 20
<212> DNA
<213> Artificial Sequence

<220>
<223> Synthetic Oligonucleotide

<400> 415
ttgggaaata tttgggcatt                                                   20

<210> 416
<211> 20
<212> DNA
<213> Artificial Sequence

<220>
<223> Synthetic Oligonucleotide

<400> 416

agaagctagg gtggttgtcc                                                    20

<210> 417
<211> 26
<212> DNA
<213> Artificial Sequence

<220>
<223> Synthetic Oligonucleotide

<400> 417
ttgggacatt gtagacttgg ccagac                                            26

<210> 418
<211> 19
<212> DNA
<213> Artificial Sequence

<220>
<223> Synthetic Oligonucleotide

<400> 418
gcatgggaac catcaacca                                                    19

<210> 419
<211> 21
<212> DNA
<213> Artificial Sequence

<220>
<223> Synthetic Oligonucleotide

<400> 419
tgaggagttt gccttgattc g                                                 21

<210> 420
<211> 30
<212> DNA
<213> Artificial Sequence

<220>
<223> Synthetic Oligonucleotide

<400> 420
ccatggacca acttcactat gtgacagagc                                        30

<210> 421
<211> 20
<212> DNA
<213> Artificial Sequence

<220>
<223> Synthetic Oligonucleotide

<400> 421
ctgttgcagg aaggcattga                                                   20

<210> 422
<211> 28
<212> DNA
<213> Artificial Sequence

<220>

<223> Synthetic Oligonucleotide

<400> 422
gtcagtgggt ctctaataaa gagatgag                                          28

<210> 423
<211> 21
<212> DNA
<213> Artificial Sequence

<220>
<223> Synthetic Oligonucleotide

<400> 423
catctcctgg cccagcatgt t                                                 21

<210> 424
<211> 21
<212> DNA
<213> Artificial Sequence

<220>
<223> Synthetic Oligonucleotide

<400> 424
cgctccagac ctatgatgac t                                                 21

<210> 425
<211> 20
<212> DNA
<213> Artificial Sequence

<220>
<223> Synthetic Oligonucleotide

<400> 425
acagtggaag gaccaggact                                                   20

<210> 426
<211> 24
<212> DNA
<213> Artificial Sequence

<220>
<223> Synthetic Oligonucleotide

<400> 426
tgttagccaa agactgccac tgca                                              24

<210> 427
<211> 18
<212> DNA
<213> Artificial Sequence

<220>
<223> Synthetic Oligonucleotide

<400> 427
ctccgacgtg gctttcca                                                     18

<210> 428
<211> 23
<212> DNA

<213> Artificial Sequence

<220>
<223> Synthetic Oligonucleotide

<400> 428
cgtaattggc agaattgatg aca                                              23

<210> 429
<211> 25
<212> DNA
<213> Artificial Sequence

<220>
<223> Synthetic Oligonucleotide

<400> 429
tggcccacag catctatgga atccc                                            25

<210> 430
<211> 23
<212> DNA
<213> Artificial Sequence

<220>
<223> Synthetic Oligonucleotide

<400> 430
ggagctctgc agtaaccaca gaa                                              23

<210> 431
<211> 20
<212> DNA
<213> Artificial Sequence

<220>
<223> Synthetic Oligonucleotide

<400> 431
caatgccatc cagctcttga                                                  20

<210> 432
<211> 22
<212> DNA
<213> Artificial Sequence

<220>
<223> Synthetic Oligonucleotide

<400> 432
aggccccatg ctgacgtccc tc                                               22

<210> 433
<211> 20
<212> DNA
<213> Artificial Sequence

<220>
<223> Synthetic Oligonucleotide

<400> 433
ggtgaaggtt gtctcttccg                                                  20

<210> 434
<211> 19
<212> DNA
<213> Artificial Sequence

<220>
<223> Synthetic Oligonucleotide

<400> 434
cagcccttcc acttgtctg                                                    19

<210> 435
<211> 24
<212> DNA
<213> Artificial Sequence

<220>
<223> Synthetic Oligonucleotide

<400> 435
aagagccctg tcttcagaag gccc                                              24

<210> 436
<211> 20
<212> DNA
<213> Artificial Sequence

<220>
<223> Synthetic Oligonucleotide

<400> 436
tacccttcca tgtgctggat                                                   20

<210> 437
<211> 18
<212> DNA
<213> Artificial Sequence

<220>
<223> Synthetic Oligonucleotide

<400> 437
gctgaagagg cccaggtt                                                     18

<210> 438
<211> 24
<212> DNA
<213> Artificial Sequence

<220>
<223> Synthetic Oligonucleotide

<400> 438
cgggactccc tggtcagcta catc                                              24

<210> 439
<211> 18
<212> DNA
<213> Artificial Sequence

<220>
<223> Synthetic Oligonucleotide

<400> 439
gcttatgacc gaccccaa                                                    18

<210> 440
<211> 20
<212> DNA
<213> Artificial Sequence

<220>
<223> Synthetic Oligonucleotide

<400> 440
aaagttccag gcaacatcgt                                                  20

<210> 441
<211> 24
<212> DNA
<213> Artificial Sequence

<220>
<223> Synthetic Oligonucleotide

<400> 441
ctcatcacct ggtctccggt gtgt                                             24

<210> 442
<211> 20
<212> DNA
<213> Artificial Sequence

<220>
<223> Synthetic Oligonucleotide

<400> 442
ccatctgcat ccatcttgtt                                                  20

<210> 443
<211> 20
<212> DNA
<213> Artificial Sequence

<220>
<223> Synthetic Oligonucleotide

<400> 443
ggccaccagg gtattatctg                                                  20

<210> 444
<211> 23
<212> DNA
<213> Artificial Sequence

<220>
<223> Synthetic Oligonucleotide

<400> 444
ctccccaccc ttgagaagtg cct                                              23

<210> 445
<211> 18
<212> DNA
<213> Artificial Sequence

<220>
<223> Synthetic Oligonucleotide

<400> 445
gacaaggacg gcagcaag                                                    18

<210> 446
<211> 18
<212> DNA
<213> Artificial Sequence

<220>
<223> Synthetic Oligonucleotide

<400> 446
ttgtggcctg tctggacc                                                    18

<210> 447
<211> 24
<212> DNA
<213> Artificial Sequence

<220>
<223> Synthetic Oligonucleotide

<400> 447
ccaggagttg ccaccactgt tgtc                                             24

<210> 448
<211> 20
<212> DNA
<213> Artificial Sequence

<220>
<223> Synthetic Oligonucleotide

<400> 448
gtgatcccaa gcccacaata                                                  20

<210> 449
<211> 18
<212> DNA
<213> Artificial Sequence

<220>
<223> Synthetic Oligonucleotide

<400> 449
tgtggcgatc aggatgtg                                                    18

<210> 450
<211> 24
<212> DNA
<213> Artificial Sequence

<220>
<223> Synthetic Oligonucleotide

<400> 450
tcagtgggaa aaagtacacc gccc                                             24

<210> 451
<211> 20

<212> DNA
<213> Artificial Sequence

<220>
<223> Synthetic Oligonucleotide

<400> 451
ggcccagctt gaatttttca                                                   20

<210> 452
<211> 27
<212> DNA
<213> Artificial Sequence

<220>
<223> Synthetic Oligonucleotide

<400> 452
aagctatgag gaaaagaagt acacgat                                           27

<210> 453
<211> 30
<212> DNA
<213> Artificial Sequence

<220>
<223> Synthetic Oligonucleotide

<400> 453
tcagccactg gcttctgtca taatcaggag                                        30

<210> 454
<211> 20
<212> DNA
<213> Artificial Sequence

<220>
<223> Synthetic Oligonucleotide

<400> 454
gagaccctgc tgtcccagaa                                                   20

<210> 455
<211> 23
<212> DNA
<213> Artificial Sequence

<220>
<223> Synthetic Oligonucleotide

<400> 455
ggttgtagtc agcgaaggag atc                                               23

<210> 456
<211> 26
<212> DNA
<213> Artificial Sequence

<220>
<223> Synthetic Oligonucleotide

<400> 456
tcccacaatg aaggtcttgc ctccct                                            26

<210> 457
<211> 20
<212> DNA
<213> Artificial Sequence

<220>
<223> Synthetic Oligonucleotide

<400> 457
cccactcagt agccaagtca                                                    20

<210> 458
<211> 20
<212> DNA
<213> Artificial Sequence

<220>
<223> Synthetic Oligonucleotide

<400> 458
cacgcaggtg gtatcagtct                                                    20

<210> 459
<211> 27
<212> DNA
<213> Artificial Sequence

<220>
<223> Synthetic Oligonucleotide

<400> 459
tcaagtaaac gggctgtttt ccaaaca                                            27

<210> 460
<211> 18
<212> DNA
<213> Artificial Sequence

<220>
<223> Synthetic Oligonucleotide

<400> 460
tcctgtgctc tggaagcc                                                      18

<210> 461
<211> 21
<212> DNA
<213> Artificial Sequence

<220>
<223> Synthetic Oligonucleotide

<400> 461
ctccacggtc tcagttgatc t                                                  21

<210> 462
<211> 24
<212> DNA
<213> Artificial Sequence

<220>
<223> Synthetic Oligonucleotide

<400> 462
caagaacctc ccagaagggc tcaa                                              24

<210> 463
<211> 20
<212> DNA
<213> Artificial Sequence

<220>
<223> Synthetic Oligonucleotide

<400> 463
cggtgtgaga agtgcagcaa                                                   20

<210> 464
<211> 19
<212> DNA
<213> Artificial Sequence

<220>
<223> Synthetic Oligonucleotide

<400> 464
cctctcgcaa gtgctccat                                                    19

<210> 465
<211> 24
<212> DNA
<213> Artificial Sequence

<220>
<223> Synthetic Oligonucleotide

<400> 465
ccagaccata gcacactcgg gcac                                              24

<210> 466
<211> 24
<212> DNA
<213> Artificial Sequence

<220>
<223> Synthetic Oligonucleotide

<400> 466
tgaacataaa gtctgcaaca tgga                                              24

<210> 467
<211> 28
<212> DNA
<213> Artificial Sequence

<220>
<223> Synthetic Oligonucleotide

<400> 467
tgaggttggt tactgttggt atcatata                                          28

<210> 468
<211> 24
<212> DNA
<213> Artificial Sequence

<220>
<223> Synthetic Oligonucleotide

<400> 468
ttgcactgca caggccacat tcac                                              24

<210> 469
<211> 24
<212> DNA
<213> Artificial Sequence

<220>
<223> Synthetic Oligonucleotide

<400> 469
tccaggatgt taggaactgt gaag                                              24

<210> 470
<211> 22
<212> DNA
<213> Artificial Sequence

<220>
<223> Synthetic Oligonucleotide

<400> 470
gcgtgtctgc gtagtagctg tt                                                22

<210> 471
<211> 24
<212> DNA
<213> Artificial Sequence

<220>
<223> Synthetic Oligonucleotide

<400> 471
agtcgctggt ttcatgccct tcca                                              24

<210> 472
<211> 19
<212> DNA
<213> Artificial Sequence

<220>
<223> Synthetic Oligonucleotide

<400> 472
ggacgaatac gaccccact                                                    19

<210> 473
<211> 18
<212> DNA
<213> Artificial Sequence

<220>
<223> Synthetic Oligonucleotide

<400> 473
gcacgtctcc ccatcaat                                                     18

<210> 474

<211> 24
<212> DNA
<213> Artificial Sequence

<220>
<223> Synthetic Oligonucleotide

<400> 474
accacctgct tccggtagga atcc                                              24

<210> 475
<211> 19
<212> DNA
<213> Artificial Sequence

<220>
<223> Synthetic Oligonucleotide

<400> 475
ctgctgcgac agtccacta                                                    19

<210> 476
<211> 18
<212> DNA
<213> Artificial Sequence

<220>
<223> Synthetic Oligonucleotide

<400> 476
caggttcgct ctgggaag                                                     18

<210> 477
<211> 24
<212> DNA
<213> Artificial Sequence

<220>
<223> Synthetic Oligonucleotide

<400> 477
agagtgactc ccgttgtccc aagg                                              24

<210> 478
<211> 19
<212> DNA
<213> Artificial Sequence

<220>
<223> Synthetic Oligonucleotide

<400> 478
ggtccgcttc gtctttcga                                                    19

<210> 479
<211> 19
<212> DNA
<213> Artificial Sequence

<220>
<223> Synthetic Oligonucleotide

<400> 479

gcacaggttc gctctggaa                                                    19

<210> 480
<211> 20
<212> DNA
<213> Artificial Sequence

<220>
<223> Synthetic Oligonucleotide

<400> 480
tgactcccgc ggtcccaagg                                                   20

<210> 481
<211> 19
<212> DNA
<213> Artificial Sequence

<220>
<223> Synthetic Oligonucleotide

<400> 481
gcaggtgtga ttgctggac                                                    19

<210> 482
<211> 18
<212> DNA
<213> Artificial Sequence

<220>
<223> Synthetic Oligonucleotide

<400> 482
accataggca atggcagc                                                     18

<210> 483
<211> 24
<212> DNA
<213> Artificial Sequence

<220>
<223> Synthetic Oligonucleotide

<400> 483
aagaatcatc aatgagccca cggc                                              24

<210> 484
<211> 24
<212> DNA
<213> Artificial Sequence

<220>
<223> Synthetic Oligonucleotide

<400> 484
ggctagtaga actggatccc aaca                                              24

<210> 485
<211> 20
<212> DNA
<213> Artificial Sequence

<220>

<223> Synthetic Oligonucleotide

<400> 485
ggtctgccca aatgcttttc                                                    20

<210> 486
<211> 30
<212> DNA
<213> Artificial Sequence

<220>
<223> Synthetic Oligonucleotide

<400> 486
taattagacc taggcctcag ctgcactgcc                                         30

<210> 487
<211> 19
<212> DNA
<213> Artificial Sequence

<220>
<223> Synthetic Oligonucleotide

<400> 487
ggccactaaa gatgctggc                                                     19

<210> 488
<211> 18
<212> DNA
<213> Artificial Sequence

<220>
<223> Synthetic Oligonucleotide

<400> 488
agcagctgtg ggctcatt                                                      18

<210> 489
<211> 24
<212> DNA
<213> Artificial Sequence

<220>
<223> Synthetic Oligonucleotide

<400> 489
atcacccgaa gcacattcag tcca                                               24

<210> 490
<211> 19
<212> DNA
<213> Artificial Sequence

<220>
<223> Synthetic Oligonucleotide

<400> 490
ccgactggag gagcataaa                                                     19

<210> 491
<211> 19
<212> DNA

<213> Artificial Sequence

<220>
<223> Synthetic Oligonucleotide

<400> 491
atgctggctg actctgctc                                                    19

<210> 492
<211> 24
<212> DNA
<213> Artificial Sequence

<220>
<223> Synthetic Oligonucleotide

<400> 492
cgcacttttc tgagcagacg tcca                                              24

<210> 493
<211> 20
<212> DNA
<213> Artificial Sequence

<220>
<223> Synthetic Oligonucleotide

<400> 493
caaaaggcag aggctgataa                                                   20

<210> 494
<211> 20
<212> DNA
<213> Artificial Sequence

<220>
<223> Synthetic Oligonucleotide

<400> 494
agcgcagttt cataaagcaa                                                   20

<210> 495
<211> 27
<212> DNA
<213> Artificial Sequence

<220>
<223> Synthetic Oligonucleotide

<400> 495
tgaccagatc cttcacagac ttgtcgt                                           27

<210> 496
<211> 19
<212> DNA
<213> Artificial Sequence

<220>
<223> Synthetic Oligonucleotide

<400> 496
agaaccgcaa ggtgagcaa                                                    19

<210> 497
<211> 21
<212> DNA
<213> Artificial Sequence

<220>
<223> Synthetic Oligonucleotide

<400> 497
tccaactgaa ggtccctgat g                                                21

<210> 498
<211> 26
<212> DNA
<213> Artificial Sequence

<220>
<223> Synthetic Oligonucleotide

<400> 498
tggagattct ccagcacgtc atcgac                                           26

<210> 499
<211> 20
<212> DNA
<213> Artificial Sequence

<220>
<223> Synthetic Oligonucleotide

<400> 499
cacgcagaaa accacacttc                                                  20

<210> 500
<211> 20
<212> DNA
<213> Artificial Sequence

<220>
<223> Synthetic Oligonucleotide

<400> 500
catgttcctc cttgtgcatc                                                  20

<210> 501
<211> 24
<212> DNA
<213> Artificial Sequence

<220>
<223> Synthetic Oligonucleotide

<400> 501
caacacttcc ttccccaaag ccag                                             24

<210> 502
<211> 21
<212> DNA
<213> Artificial Sequence

<220>
<223> Synthetic Oligonucleotide

<400> 502
gcatggtagc cgaagatttc a                                                21

<210> 503
<211> 30
<212> DNA
<213> Artificial Sequence

<220>
<223> Synthetic Oligonucleotide

<400> 503
tttccggtaa tagtctgtct catagatatc                                       30

<210> 504
<211> 28
<212> DNA
<213> Artificial Sequence

<220>
<223> Synthetic Oligonucleotide

<400> 504
cgcgtcatac caaaatctcc gattttga                                         28

<210> 505
<211> 19
<212> DNA
<213> Artificial Sequence

<220>
<223> Synthetic Oligonucleotide

<400> 505
gtggacagca ccatgaaca                                                   19

<210> 506
<211> 20
<212> DNA
<213> Artificial Sequence

<220>
<223> Synthetic Oligonucleotide

<400> 506
ccttcatacc cgacttgagg                                                  20

<210> 507
<211> 20
<212> DNA
<213> Artificial Sequence

<220>
<223> Synthetic Oligonucleotide

<400> 507
cttccggcca gcactgcctc                                                  20

<210> 508
<211> 17
<212> DNA
<213> Artificial Sequence

<220>
<223> Synthetic Oligonucleotide

<400> 508
acgcaccggg tgtctga                                                      17

<210> 509
<211> 24
<212> DNA
<213> Artificial Sequence

<220>
<223> Synthetic Oligonucleotide

<400> 509
tgccctttct tgatgatgat tatc                                              24

<210> 510
<211> 24
<212> DNA
<213> Artificial Sequence

<220>
<223> Synthetic Oligonucleotide

<400> 510
   cccaagttcc accccctcca ttca                                             24

<210> 511
<211> 22
<212> DNA
<213> Artificial Sequence

<220>
<223> Synthetic Oligonucleotide

<400> 511
tggacaagta cgggatgaag ct                                                22

<210> 512
<211> 21
<212> DNA
<213> Artificial Sequence

<220>
<223> Synthetic Oligonucleotide

<400> 512
cgaaggtgtg gcactgaaag t                                                 21

<210> 513
<211> 24
<212> DNA
<213> Artificial Sequence

<220>
<223> Synthetic Oligonucleotide

<400> 513
cccgtcaacg tactccatgc ctgg                                              24

<210> 514
<211> 19

<212> DNA
<213> Artificial Sequence

<220>
<223> Synthetic Oligonucleotide

<400> 514
gcggtgattc ggaaattcg                                                    19

<210> 515
<211> 19
<212> DNA
<213> Artificial Sequence

<220>
<223> Synthetic Oligonucleotide

<400> 515
ctctcctggg cacctgctt                                                    19

<210> 516
<211> 23
<212> DNA
<213> Artificial Sequence

<220>
<223> Synthetic Oligonucleotide

<400> 516
ctctggtcct catccaggtg cgc                                               23

<210> 517
<211> 26
<212> DNA
<213> Artificial Sequence

<220>
<223> Synthetic Oligonucleotide

<400> 517
aaggaaccat ctcactgtgt gtaaac                                            26

<210> 518
<211> 20
<212> DNA
<213> Artificial Sequence

<220>
<223> Synthetic Oligonucleotide

<400> 518
atcaggaagg ctgccaagag                                                   20

<210> 519
<211> 22
<212> DNA
<213> Artificial Sequence

<220>
<223> Synthetic Oligonucleotide

<400> 519
tgacttccaa gctggccgtg gc                                                22

<210> 520
<211> 20
<212> DNA
<213> Artificial Sequence

<220>
<223> Synthetic Oligonucleotide

<400> 520
tctgcgtggt tcctttctca                                                    20

<210> 521
<211> 25
<212> DNA
<213> Artificial Sequence

<220>
<223> Synthetic Oligonucleotide

<400> 521
ttgaaggatg tttattaggc aacgt                                              25

<210> 522
<211> 26
<212> DNA
<213> Artificial Sequence

<220>
<223> Synthetic Oligonucleotide

<400> 522
cgcttctgac tgctgattct cccgtt                                             26

<210> 523
<211> 20
<212> DNA
<213> Artificial Sequence

<220>
<223> Synthetic Oligonucleotide

<400> 523
cctgaacctt ccaaagatgg                                                    20

<210> 524
<211> 20
<212> DNA
<213> Artificial Sequence

<220>
<223> Synthetic Oligonucleotide

<400> 524
accaggcaag tctcctcatt                                                    20

<210> 525
<211> 27
<212> DNA
<213> Artificial Sequence

<220>
<223> Synthetic Oligonucleotide

<400> 525
ccagattgga agcatccatc tttttca                                          27

<210> 526
<211> 20
<212> DNA
<213> Artificial Sequence

<220>
<223> Synthetic Oligonucleotide

<400> 526
ccgctccgtc actgatgtct                                                  20

<210> 527
<211> 21
<212> DNA
<213> Artificial Sequence

<220>
<223> Synthetic Oligonucleotide

<400> 527
gcaaggtcag ggcaaagagt a                                                21

<210> 528
<211> 24
<212> DNA
<213> Artificial Sequence

<220>
<223> Synthetic Oligonucleotide

<400> 528
cctgctgaac ctagccttgg ccga                                             24

<210> 529
<211> 20
<212> DNA
<213> Artificial Sequence

<220>
<223> Synthetic Oligonucleotide

<400> 529
ctcaggattt tctcgcatcc                                                  20

<210> 530
<211> 19
<212> DNA
<213> Artificial Sequence

<220>
<223> Synthetic Oligonucleotide

<400> 530
aggagcaggt ggagactgg                                                   19

<210> 531
<211> 24
<212> DNA
<213> Artificial Sequence

<220>
<223> Synthetic Oligonucleotide

<400> 531
atgtgctccc agtgctaggt gcct                                              24

<210> 532
<211> 20
<212> DNA
<213> Artificial Sequence

<220>
<223> Synthetic Oligonucleotide

<400> 532
agccatcact ctcagtgcag                                                   20

<210> 533
<211> 20
<212> DNA
<213> Artificial Sequence

<220>
<223> Synthetic Oligonucleotide

<400> 533
actgcagagt cagggtctcc                                                   20

<210> 534
<211> 22
<212> DNA
<213> Artificial Sequence

<220>
<223> Synthetic Oligonucleotide

<400> 534
caggtcctat cgtggcccct ga                                                22

<210> 535
<211> 21
<212> DNA
<213> Artificial Sequence

<220>
<223> Synthetic Oligonucleotide

<400> 535
gccaggatac tggagtccat c                                                 21

<210> 536
<211> 18
<212> DNA
<213> Artificial Sequence

<220>
<223> Synthetic Oligonucleotide

<400> 536
cttgaccctt ggggtcct                                                     18

<210> 537

<211> 23
<212> DNA
<213> Artificial Sequence

<220>
<223> Synthetic Oligonucleotide

<400> 537
tgagctccct gatggctaca ccc                                               23

<210> 538
<211> 18
<212> DNA
<213> Artificial Sequence

<220>
<223> Synthetic Oligonucleotide

<400> 538
ggatggagtt cgcctcct                                                     18

<210> 539
<211> 20
<212> DNA
<213> Artificial Sequence

<220>
<223> Synthetic Oligonucleotide

<400> 539
cgctccatgg acttgatctt                                                   20

<210> 540
<211> 24
<212> DNA
<213> Artificial Sequence

<220>
<223> Synthetic Oligonucleotide

<400> 540
cgtgatcaca gacctgaccc agct                                              24

<210> 541
<211> 20
<212> DNA
<213> Artificial Sequence

<220>
<223> Synthetic Oligonucleotide

<400> 541
ccacagctca ccttctgtca                                                   20

<210> 542
<211> 20
<212> DNA
<213> Artificial Sequence

<220>
<223> Synthetic Oligonucleotide

<400> 542

cctcagtgcc agtctcttcc                                                    20

<210> 543
<211> 20
<212> DNA
<213> Artificial Sequence

<220>
<223> Synthetic Oligonucleotide

<400> 543
tccatcccag ctccagccag                                                    20

<210> 544
<211> 20
<212> DNA
<213> Artificial Sequence

<220>
<223> Synthetic Oligonucleotide

<400> 544
tcagaattgg atttggctca                                                    20

<210> 545
<211> 20
<212> DNA
<213> Artificial Sequence

<220>
<223> Synthetic Oligonucleotide

<400> 545
cctgagctta gctggtgttg                                                    20

<210> 546
<211> 30
<212> DNA
<213> Artificial Sequence

<220>
<223> Synthetic Oligonucleotide

<400> 546
tgctaatgta aggcatcaca gtcttttcca                                         30

<210> 547
<211> 26
<212> DNA
<213> Artificial Sequence

<220>
<223> Synthetic Oligonucleotide

<400> 547
tgaggaagaa ggagaggaat actaat                                             26

<210> 548
<211> 21
<212> DNA
<213> Artificial Sequence

<220>

<223> Synthetic Oligonucleotide

<400> 548
ctgaaattct gggagcatga c                                                21

<210> 549
<211> 24
<212> DNA
<213> Artificial Sequence

<220>
<223> Synthetic Oligonucleotide

<400> 549
tatccattcc ttttggccct gcag                                             24

<210> 550
<211> 20
<212> DNA
<213> Artificial Sequence

<220>
<223> Synthetic Oligonucleotide

<400> 550
gaggacgaag gcctctacac                                                  20

<210> 551
<211> 20
<212> DNA
<213> Artificial Sequence

<220>
<223> Synthetic Oligonucleotide

<400> 551
aaaaatgcct ccacttttgc                                                  20

<210> 552
<211> 24
<212> DNA
<213> Artificial Sequence

<220>
<223> Synthetic Oligonucleotide

<400> 552
caggcatgca gtgttcttgg ctgt                                             24

<210> 553
<211> 19
<212> DNA
<213> Artificial Sequence

<220>
<223> Synthetic Oligonucleotide

<400> 553
cggactttgg gtgcgactt                                                   19

<210> 554
<211> 24
<212> DNA

<213> Artificial Sequence

<220>
<223> Synthetic Oligonucleotide

<400> 554
ttacaactct tccactggga cgat                                              24

<210> 555
<211> 23
<212> DNA
<213> Artificial Sequence

<220>
<223> Synthetic Oligonucleotide

<400> 555
ccacttgtcg aaccaccgct cgt                                               23

<210> 556
<211> 20
<212> DNA
<213> Artificial Sequence

<220>
<223> Synthetic Oligonucleotide

<400> 556
tggaggttgt aagccaatgt                                                   20

<210> 557
<211> 20
<212> DNA
<213> Artificial Sequence

<220>
<223> Synthetic Oligonucleotide

<400> 557
tgccttacgt ccatctgatt                                                   20

<210> 558
<211> 28
<212> DNA
<213> Artificial Sequence

<220>
<223> Synthetic Oligonucleotide

<400> 558
cagtgatgtc tgaacttgaa gcctcaca                                          28

<210> 559
<211> 20
<212> DNA
<213> Artificial Sequence

<220>
<223> Synthetic Oligonucleotide

<400> 559
ctaaggcact tgctggaagg                                                   20

<210> 560
<211> 18
<212> DNA
<213> Artificial Sequence

<220>
<223> Synthetic Oligonucleotide

<400> 560
tcttcccagc tcctgtgg                                                    18

<210> 561
<211> 24
<212> DNA
<213> Artificial Sequence

<220>
<223> Synthetic Oligonucleotide

<400> 561
tccataggca agcacactgg catt                                             24

<210> 562
<211> 25
<212> DNA
<213> Artificial Sequence

<220>
<223> Synthetic Oligonucleotide

<400> 562
aattcctgct ccaaaagaaa gtctt                                            25

<210> 563
<211> 20
<212> DNA
<213> Artificial Sequence

<220>
<223> Synthetic Oligonucleotide

<400> 563
cgtgatgcga agctctgaga                                                  20

<210> 564
<211> 22
<212> DNA
<213> Artificial Sequence

<220>
<223> Synthetic Oligonucleotide

<400> 564
aagccgctcc actcgcatgt cc                                               22

<210> 565
<211> 20
<212> DNA
<213> Artificial Sequence

<220>
<223> Synthetic Oligonucleotide

<400> 565
ccacagggtt gaagaaccag                                                   20

<210> 566
<211> 20
<212> DNA
<213> Artificial Sequence

<220>
<223> Synthetic Oligonucleotide

<400> 566
cacctgatgt gccagacttc                                                   20

<210> 567
<211> 24
<212> DNA
<213> Artificial Sequence

<220>
<223> Synthetic Oligonucleotide

<400> 567
agccagttcc tgctgttcct gtcc                                              24

<210> 568
<211> 18
<212> DNA
<213> Artificial Sequence

<220>
<223> Synthetic Oligonucleotide

<400> 568
gcccagaggc tccatcgt                                                     18

<210> 569
<211> 23
<212> DNA
<213> Artificial Sequence

<220>
<223> Synthetic Oligonucleotide

<400> 569
cagaggtttg aacagtgcag aca                                               23

<210> 570
<211> 23
<212> DNA
<213> Artificial Sequence

<220>
<223> Synthetic Oligonucleotide

<400> 570
cctcttcctc cccagtcggc tga                                               23

<210> 571
<211> 20
<212> DNA
<213> Artificial Sequence

<220>
<223> Synthetic Oligonucleotide

<400> 571
caaacagagg gtggcagaag                                                    20

<210> 572
<211> 18
<212> DNA
<213> Artificial Sequence

<220>
<223> Synthetic Oligonucleotide

<400> 572
gaggctctca cggctcct                                                      18

<210> 573
<211> 24
<212> DNA
<213> Artificial Sequence

<220>
<223> Synthetic Oligonucleotide

<400> 573
cgcttctcca tgttctcagg gtca                                               24

<210> 574
<211> 18
<212> DNA
<213> Artificial Sequence

<220>
<223> Synthetic Oligonucleotide

<400> 574
agccgaggct ttgttgaa                                                      18

<210> 575
<211> 19
<212> DNA
<213> Artificial Sequence

<220>
<223> Synthetic Oligonucleotide

<400> 575
tgggctatga gcacagctt                                                     19

<210> 576
<211> 24
<212> DNA
<213> Artificial Sequence

<220>
<223> Synthetic Oligonucleotide

<400> 576
ttcatatcca gtaccggcga tcgg                                               24

<210> 577
<211> 20

<212> DNA
<213> Artificial Sequence

<220>
<223> Synthetic Oligonucleotide

<400> 577
atgtgccagt gagcttgagt                                                   20

<210> 578
<211> 20
<212> DNA
<213> Artificial Sequence

<220>
<223> Synthetic Oligonucleotide

<400> 578
tgagcccctg gttaacagta                                                   20

<210> 579
<211> 24
<212> DNA
<213> Artificial Sequence

<220>
<223> Synthetic Oligonucleotide

<400> 579
ccttggagaa acacaagcac ctgc                                              24

<210> 580
<211> 20
<212> DNA
<213> Artificial Sequence

<220>
<223> Synthetic Oligonucleotide

<400> 580
ggcctgctga gatcaaagac                                                   20

<210> 581
<211> 20
<212> DNA
<213> Artificial Sequence

<220>
<223> Synthetic Oligonucleotide

<400> 581
gtccactgtg gctgtgagaa                                                   20

<210> 582
<211> 26
<212> DNA
<213> Artificial Sequence

<220>
<223> Synthetic Oligonucleotide

<400> 582
tgttcctcag gtcctcaatg gtcttg                                            26

<210> 583
<211> 21
<212> DNA
<213> Artificial Sequence

<220>
<223> Synthetic Oligonucleotide

<400> 583
cgaggattgg ttcttcagca a                                                21

<210> 584
<211> 24
<212> DNA
<213> Artificial Sequence

<220>
<223> Synthetic Oligonucleotide

<400> 584
cacctcgcgg ttcagttcct ctgt                                             24

<210> 585
<211> 22
<212> DNA
<213> Artificial Sequence

<220>
<223> Synthetic Oligonucleotide

<400> 585
actctgcacc agctcactgt tg                                               22

<210> 586
<211> 21
<212> DNA
<213> Artificial Sequence

<220>
<223> Synthetic Oligonucleotide

<400> 586
tgagcggcag aatcaggagt a                                                21

<210> 587
<211> 19
<212> DNA
<213> Artificial Sequence

<220>
<223> Synthetic Oligonucleotide

<400> 587
tgcggtaggt ggcaatctc                                                   19

<210> 588
<211> 24
<212> DNA
<213> Artificial Sequence

<220>
<223> Synthetic Oligonucleotide

<400> 588
ctcatggaca tcaagtcgcg gctg                                              24

<210> 589
<211> 20
<212> DNA
<213> Artificial Sequence

<220>
<223> Synthetic Oligonucleotide

<400> 589
tcagtggaga aggagttgga                                                   20

<210> 590
<211> 20
<212> DNA
<213> Artificial Sequence

<220>
<223> Synthetic Oligonucleotide

<400> 590
tgccatatcc agaggaaaca                                                   20

<210> 591
<211> 28
<212> DNA
<213> Artificial Sequence

<220>
<223> Synthetic Oligonucleotide

<400> 591
ccagtcaaca tctctgttgt cacaagca                                          28

<210> 592
<211> 18
<212> DNA
<213> Artificial Sequence

<220>
<223> Synthetic Oligonucleotide

<400> 592
cttgctggcc aatgccta                                                     18

<210> 593
<211> 18
<212> DNA
<213> Artificial Sequence

<220>
<223> Synthetic Oligonucleotide

<400> 593
tgattgtccg cagtcagg                                                     18

<210> 594
<211> 24
<212> DNA
<213> Artificial Sequence

<220>
<223> Synthetic Oligonucleotide

<400> 594
atctacgttg tccagctgcc agcc                                              24

<210> 595
<211> 21
<212> DNA
<213> Artificial Sequence

<220>
<223> Synthetic Oligonucleotide

<400> 595
actcaagcgg aaattgaagc a                                                 21

<210> 596
<211> 21
<212> DNA
<213> Artificial Sequence

<220>
<223> Synthetic Oligonucleotide

<400> 596
actccctgaa gccgagacac t                                                 21

<210> 597
<211> 28
<212> DNA
<213> Artificial Sequence

<220>
<223> Synthetic Oligonucleotide

<400> 597
aggtcttatc agcacagtct ccgcctcc                                          28

<210> 598
<211> 18
<212> DNA
<213> Artificial Sequence

<220>
<223> Synthetic Oligonucleotide

<400> 598
agcgatgaag atggtcgc                                                     18

<210> 599
<211> 18
<212> DNA
<213> Artificial Sequence

<220>
<223> Synthetic Oligonucleotide

<400> 599
gacatggcag cacaagca                                                     18

<210> 600

<211> 24
<212> DNA
<213> Artificial Sequence

<220>
<223> Synthetic Oligonucleotide

<400> 600
ctggacgcgg ttctactcca acag                                              24

<210> 601
<211> 20
<212> DNA
<213> Artificial Sequence

<220>
<223> Synthetic Oligonucleotide

<400> 601
gcttcaggtg ttgtgactgc                                                   20

<210> 602
<211> 20
<212> DNA
<213> Artificial Sequence

<220>
<223> Synthetic Oligonucleotide

<400> 602
aagagctgcc catccttctc                                                   20

<210> 603
<211> 24
<212> DNA
<213> Artificial Sequence

<220>
<223> Synthetic Oligonucleotide

<400> 603
tgcctccctg tcgcaccagt acta                                              24

<210> 604
<211> 18
<212> DNA
<213> Artificial Sequence

<220>
<223> Synthetic Oligonucleotide

<400> 604
ctttgggcca ggaggaat                                                     18

<210> 605
<211> 18
<212> DNA
<213> Artificial Sequence

<220>
<223> Synthetic Oligonucleotide

<400> 605

ctcccacaat ccactgcc                                                    18

<210> 606
<211> 24
<212> DNA
<213> Artificial Sequence

<220>
<223> Synthetic Oligonucleotide

<400> 606
actcgaatcc acccaggaac tccc                                              24

<210> 607
<211> 20
<212> DNA
<213> Artificial Sequence

<220>
<223> Synthetic Oligonucleotide

<400> 607
agaagctgtc cctgcaagag                                                   20

<210> 608
<211> 20
<212> DNA
<213> Artificial Sequence

<220>
<223> Synthetic Oligonucleotide

<400> 608
agccgtacca gctcagactt                                                   20

<210> 609
<211> 25
<212> DNA
<213> Artificial Sequence

<220>
<223> Synthetic Oligonucleotide

<400> 609
catgttcttc acaatcgctg catcc                                             25

<210> 610
<211> 18
<212> DNA
<213> Artificial Sequence

<220>
<223> Synthetic Oligonucleotide

<400> 610
ccgggagcag ggaatcac                                                     18

<210> 611
<211> 20
<212> DNA
<213> Artificial Sequence

<220>

<223> Synthetic Oligonucleotide

<400> 611
atgctgttga tgccgaatga                                                    20

<210> 612
<211> 19
<212> DNA
<213> Artificial Sequence

<220>
<223> Synthetic Oligonucleotide

<400> 612
cggccacgat ttcggcgct                                                     19

<210> 613
<211> 20
<212> DNA
<213> Artificial Sequence

<220>
<223> Synthetic Oligonucleotide

<400> 613
ctgtcatgtg gcagaccttc                                                    20

<210> 614
<211> 20
<212> DNA
<213> Artificial Sequence

<220>
<223> Synthetic Oligonucleotide

<400> 614
taaatgtcac tggtgcctgg                                                    20

<210> 615
<211> 24
<212> DNA
<213> Artificial Sequence

<220>
<223> Synthetic Oligonucleotide

<400> 615
cgaaccacgg cttgggaaga ctac                                               24

<210> 616
<211> 19
<212> DNA
<213> Artificial Sequence

<220>
<223> Synthetic Oligonucleotide

<400> 616
gcccagtgga gaaattcgt                                                     19

<210> 617
<211> 19
<212> DNA

<213> Artificial Sequence

<220>
<223> Synthetic Oligonucleotide

<400> 617
gcgagtctga gctgatgga                                                    19

<210> 618
<211> 24
<212> DNA
<213> Artificial Sequence

<220>
<223> Synthetic Oligonucleotide

<400> 618
tttgagatca cccagcctcc actg                                              24

<210> 619
<211> 20
<212> DNA
<213> Artificial Sequence

<220>
<223> Synthetic Oligonucleotide

<400> 619
cctcagaaat tgccaggact                                                   20

<210> 620
<211> 24
<212> DNA
<213> Artificial Sequence

<220>
<223> Synthetic Oligonucleotide

<400> 620
ttcccgtgat cttcagcaaa gcct                                              24

<210> 621
<211> 20
<212> DNA
<213> Artificial Sequence

<220>
<223> Synthetic Oligonucleotide

<400> 621
ccaaagacca gctgcaagta                                                   20

<210> 622
<211> 20
<212> DNA
<213> Artificial Sequence

<220>
<223> Synthetic Oligonucleotide

<400> 622
aggactccag caaccaagaa                                                   20

<210> 623
<211> 20
<212> DNA
<213> Artificial Sequence

<220>
<223> Synthetic Oligonucleotide

<400> 623
gagtgctgct tggaactcag                                                    20

<210> 624
<211> 24
<212> DNA
<213> Artificial Sequence

<220>
<223> Synthetic Oligonucleotide

<400> 624
caagcacctt ccctgacctg gagt                                               24

<210> 625
<211> 18
<212> DNA
<213> Artificial Sequence

<220>
<223> Synthetic Oligonucleotide

<400> 625
cggaccacca ggtcagag                                                      18

<210> 626
<211> 20
<212> DNA
<213> Artificial Sequence

<220>
<223> Synthetic Oligonucleotide

<400> 626
caggggtagt gggtgttgag                                                    20

<210> 627
<211> 24
<212> DNA
<213> Artificial Sequence

<220>
<223> Synthetic Oligonucleotide

<400> 627
ccactcttcc ctgctctgcg aatt                                               24

<210> 628
<211> 20
<212> DNA
<213> Artificial Sequence

<220>
<223> Synthetic Oligonucleotide

<400> 628
gccctccaat gtccttatca                                                    20

<210> 629
<211> 21
<212> DNA
<213> Artificial Sequence

<220>
<223> Synthetic Oligonucleotide

<400> 629
gtagccactg atgccaaagt c                                                  21

<210> 630
<211> 24
<212> DNA
<213> Artificial Sequence

<220>
<223> Synthetic Oligonucleotide

<400> 630
cacatcttca catggccctc cttg                                               24

<210> 631
<211> 18
<212> DNA
<213> Artificial Sequence

<220>
<223> Synthetic Oligonucleotide

<400> 631
gccggtcagg cacacaag                                                      18

<210> 632
<211> 23
<212> DNA
<213> Artificial Sequence

<220>
<223> Synthetic Oligonucleotide

<400> 632
gcagcataca cacaacaaaa tgg                                                23

<210> 633
<211> 24
<212> DNA
<213> Artificial Sequence

<220>
<223> Synthetic Oligonucleotide

<400> 633
accaaccagt ccacgctcca aggg                                               24

<210> 634
<211> 18
<212> DNA
<213> Artificial Sequence

<220>
<223> Synthetic Oligonucleotide

<400> 634
ccctcaaccg gcaaatcc                                                    18

<210> 635
<211> 19
<212> DNA
<213> Artificial Sequence

<220>
<223> Synthetic Oligonucleotide

<400> 635
cgtccatgcc ctgaattca                                                   19

<210> 636
<211> 23
<212> DNA
<213> Artificial Sequence

<220>
<223> Synthetic Oligonucleotide

<400> 636
tggcgagtgc agtgagcagc tcc                                              23

<210> 637
<211> 23
<212> DNA
<213> Artificial Sequence

<220>
<223> Synthetic Oligonucleotide

<400> 637
tgagtggaaa agcctgacct atg                                              23

<210> 638
<211> 23
<212> DNA
<213> Artificial Sequence

<220>
<223> Synthetic Oligonucleotide

<400> 638
ggactccatc tcttcttggt caa                                              23

<210> 639
<211> 27
<212> DNA
<213> Artificial Sequence

<220>
<223> Synthetic Oligonucleotide

<400> 639
tgaagtcatc agctttgtgc caccacc                                          27

<210> 640
<211> 22

<212> DNA
<213> Artificial Sequence

<220>
<223> Synthetic Oligonucleotide

<400> 640
caacacccgt acatcaatgt ct                                                22

<210> 641
<211> 22
<212> DNA
<213> Artificial Sequence

<220>
<223> Synthetic Oligonucleotide

<400> 641
tcatctaact gcttgtcagg ga                                                22

<210> 642
<211> 24
<212> DNA
<213> Artificial Sequence

<220>
<223> Synthetic Oligonucleotide

<400> 642
ctgaagcaga agctccacca ccaa                                              24

<210> 643
<211> 20
<212> DNA
<213> Artificial Sequence

<220>
<223> Synthetic Oligonucleotide

<400> 643
gaccttggaa cctttggaac                                                   20

<210> 644
<211> 20
<212> DNA
<213> Artificial Sequence

<220>
<223> Synthetic Oligonucleotide

<400> 644
cctaggccta tgagggttca                                                   20

<210> 645
<211> 26
<212> DNA
<213> Artificial Sequence

<220>
<223> Synthetic Oligonucleotide

<400> 645
cagccctgta agacctgttg acagca                                            26

<210> 646
<211> 18
<212> DNA
<213> Artificial Sequence

<220>
<223> Synthetic Oligonucleotide

<400> 646
cacaagctga ccttccgc                                                    18

<210> 647
<211> 20
<212> DNA
<213> Artificial Sequence

<220>
<223> Synthetic Oligonucleotide

<400> 647
acttgtacac gatctccgcc                                                  20

<210> 648
<211> 24
<212> DNA
<213> Artificial Sequence

<220>
<223> Synthetic Oligonucleotide

<400> 648
agaacgccaa agccaagaca gacc                                             24

<210> 649
<211> 23
<212> DNA
<213> Artificial Sequence

<220>
<223> Synthetic Oligonucleotide

<400> 649
cagatggcca ctttgagaac att                                              23

<210> 650
<211> 22
<212> DNA
<213> Artificial Sequence

<220>
<223> Synthetic Oligonucleotide

<400> 650
ggcagcatta accacaagga tt                                               22

<210> 651
<211> 28
<212> DNA
<213> Artificial Sequence

<220>
<223> Synthetic Oligonucleotide

<400> 651
agctgacaac agtgtgaacg accagacc                                          28

<210> 652
<211> 20
<212> DNA
<213> Artificial Sequence

<220>
<223> Synthetic Oligonucleotide

<400> 652
cttcggaaac tggacatcaa                                                   20

<210> 653
<211> 20
<212> DNA
<213> Artificial Sequence

<220>
<223> Synthetic Oligonucleotide

<400> 653
gtcgctgaaa acatggatca                                                   20

<210> 654
<211> 26
<212> DNA
<213> Artificial Sequence

<220>
<223> Synthetic Oligonucleotide

<400> 654
tcactcgaga caacgatttc acatcg                                            26

<210> 655
<211> 21
<212> DNA
<213> Artificial Sequence

<220>
<223> Synthetic Oligonucleotide

<400> 655
gacttttgcc cgctaccttt c                                                 21

<210> 656
<211> 26
<212> DNA
<213> Artificial Sequence

<220>
<223> Synthetic Oligonucleotide

<400> 656
gccactaact gcttcagtat gaagag                                            26

<210> 657
<211> 24
<212> DNA
<213> Artificial Sequence

<220>
<223> Synthetic Oligonucleotide

<400> 657
acagctcatt gttgtcacgc cgga                                              24

<210> 658
<211> 24
<212> DNA
<213> Artificial Sequence

<220>
<223> Synthetic Oligonucleotide

<400> 658
tgatggtcct atgtgtcaca ttca                                              24

<210> 659
<211> 20
<212> DNA
<213> Artificial Sequence

<220>
<223> Synthetic Oligonucleotide

<400> 659
tgggacagga aacacaccaa                                                   20

<210> 660
<211> 30
<212> DNA
<213> Artificial Sequence

<220>
<223> Synthetic Oligonucleotide

<400> 660
caggtttcat accaacacag gcttcagcac                                        30

<210> 661
<211> 19
<212> DNA
<213> Artificial Sequence

<220>
<223> Synthetic Oligonucleotide

<400> 661
cgctcagcca gatgcaatc                                                    19

<210> 662
<211> 25
<212> DNA
<213> Artificial Sequence

<220>
<223> Synthetic Oligonucleotide

<400> 662
gcactgagat cttcctattg gtgaa                                             25

<210> 663

<211> 21
<212> DNA
<213> Artificial Sequence

<220>
<223> Synthetic Oligonucleotide

<400> 663
tgccccagtc acctgctgtt a                                                21

<210> 664
<211> 20
<212> DNA
<213> Artificial Sequence

<220>
<223> Synthetic Oligonucleotide

<400> 664
gtgaaatgaa acgcaccaca                                                  20

<210> 665
<211> 20
<212> DNA
<213> Artificial Sequence

<220>
<223> Synthetic Oligonucleotide

<400> 665
gaccctgctc acaaccagac                                                  20

<210> 666
<211> 20
<212> DNA
<213> Artificial Sequence

<220>
<223> Synthetic Oligonucleotide

<400> 666
cagccctttg gggaagctgg                                                  20

<210> 667
<211> 19
<212> DNA
<213> Artificial Sequence

<220>
<223> Synthetic Oligonucleotide

<400> 667
ccaacgcttg ccaaatcct                                                   19

<210> 668
<211> 24
<212> DNA
<213> Artificial Sequence

<220>
<223> Synthetic Oligonucleotide

<400> 668

acggtagtga cagcatcaaa actc                                              24

<210> 669
<211> 24
<212> DNA
<213> Artificial Sequence

<220>
<223> Synthetic Oligonucleotide

<400> 669
aaccagctct ctgtgacccc aatt                                              24

<210> 670
<211> 20
<212> DNA
<213> Artificial Sequence

<220>
<223> Synthetic Oligonucleotide

<400> 670
ccatgatgga gaggcagaca                                                   20

<210> 671
<211> 21
<212> DNA
<213> Artificial Sequence

<220>
<223> Synthetic Oligonucleotide

<400> 671
ggagtccgtc cttaccgtca a                                                 21

<210> 672
<211> 24
<212> DNA
<213> Artificial Sequence

<220>
<223> Synthetic Oligonucleotide

<400> 672
ctgggagcat ggcgatggat accc                                              24

<210> 673
<211> 20
<212> DNA
<213> Artificial Sequence

<220>
<223> Synthetic Oligonucleotide

<400> 673
gagaaccaat ctcaccgaca                                                   20

<210> 674
<211> 20
<212> DNA
<213> Artificial Sequence

<220>

<223> Synthetic Oligonucleotide

<400> 674
cacccgagtg taaccatagc                                                    20

<210> 675
<211> 24
<212> DNA
<213> Artificial Sequence

<220>
<223> Synthetic Oligonucleotide

<400> 675
acaggtattc ctctgccagc tgcc                                               24

<210> 676
<211> 18
<212> DNA
<213> Artificial Sequence

<220>
<223> Synthetic Oligonucleotide

<400> 676
gccatgctgg ctcagtct                                                      18

<210> 677
<211> 20
<212> DNA
<213> Artificial Sequence

<220>
<223> Synthetic Oligonucleotide

<400> 677
cacccagacc cacctaactg                                                    20

<210> 678
<211> 24
<212> DNA
<213> Artificial Sequence

<220>
<223> Synthetic Oligonucleotide

<400> 678
cactagctga tgtggcccac agct                                               24

<210> 679
<211> 18
<212> DNA
<213> Artificial Sequence

<220>
<223> Synthetic Oligonucleotide

<400> 679
tcatggtgcc cgtcaatg                                                      18

<210> 680
<211> 23
<212> DNA

<213> Artificial Sequence

<220>
<223> Synthetic Oligonucleotide

<400> 680
cgattgtctt tgctcttcat gtg                                               23

<210> 681
<211> 29
<212> DNA
<213> Artificial Sequence

<220>
<223> Synthetic Oligonucleotide

<400> 681
acctgatacg tcttggtctt catcgccat                                         29

<210> 682
<211> 20
<212> DNA
<213> Artificial Sequence

<220>
<223> Synthetic Oligonucleotide

<400> 682
aggggatgac ttggacacat                                                   20

<210> 683
<211> 20
<212> DNA
<213> Artificial Sequence

<220>
<223> Synthetic Oligonucleotide

<400> 683
aaaactgcat ggctttgtca                                                   20

<210> 684
<211> 25
<212> DNA
<213> Artificial Sequence

<220>
<223> Synthetic Oligonucleotide

<400> 684
ctgccattcg acatgactgc aattt                                             25

<210> 685
<211> 22
<212> DNA
<213> Artificial Sequence

<220>
<223> Synthetic Oligonucleotide

<400> 685
tcatcctggc gatctacttc ct                                                22

<210> 686
<211> 20
<212> DNA
<213> Artificial Sequence

<220>
<223> Synthetic Oligonucleotide

<400> 686
ccgttgagtg gaatcagcaa                                                    20

<210> 687
<211> 26
<212> DNA
<213> Artificial Sequence

<220>
<223> Synthetic Oligonucleotide

<400> 687
tctgtcctgg ctggagtcgc tttcat                                             26

<210> 688
<211> 21
<212> DNA
<213> Artificial Sequence

<220>
<223> Synthetic Oligonucleotide

<400> 688
tgattaccat catggctcag a                                                  21

<210> 689
<211> 20
<212> DNA
<213> Artificial Sequence

<220>
<223> Synthetic Oligonucleotide

<400> 689
cttgtgaaaa tgccatccac                                                    20

<210> 690
<211> 24
<212> DNA
<213> Artificial Sequence

<220>
<223> Synthetic Oligonucleotide

<400> 690
tcccaattgt cgcttcttct gcag                                               24

<210> 691
<211> 20
<212> DNA
<213> Artificial Sequence

<220>
<223> Synthetic Oligonucleotide

<400> 691
ggccaggatc tgtggtggta                                                   20

<210> 692
<211> 20
<212> DNA
<213> Artificial Sequence

<220>
<223> Synthetic Oligonucleotide

<400> 692
ctccacgtcg tggacattga                                                   20

<210> 693
<211> 23
<212> DNA
<213> Artificial Sequence

<220>
<223> Synthetic Oligonucleotide

<400> 693
ctctggcctt ccgagaaggt acc                                               23

<210> 694
<211> 23
<212> DNA
<213> Artificial Sequence

<220>
<223> Synthetic Oligonucleotide

<400> 694
gaaggaatgg gaatcagtca tga                                               23

<210> 695
<211> 26
<212> DNA
<213> Artificial Sequence

<220>
<223> Synthetic Oligonucleotide

<400> 695
gtctattaga gtcagatccg ggacat                                            26

<210> 696
<211> 23
<212> DNA
<213> Artificial Sequence

<220>
<223> Synthetic Oligonucleotide

<400> 696
tcaccctgga gatcagctcc cga                                               23

<210> 697
<211> 18
<212> DNA
<213> Artificial Sequence

<220>
<223> Synthetic Oligonucleotide

<400> 697
gccgagatcg ccaagatg                                                    18

<210> 698
<211> 27
<212> DNA
<213> Artificial Sequence

<220>
<223> Synthetic Oligonucleotide

<400> 698
cttttgatgg tagagttcca gtgattc                                          27

<210> 699
<211> 24
<212> DNA
<213> Artificial Sequence

<220>
<223> Synthetic Oligonucleotide

<400> 699
cagcattgtc tgtcctccct ggca                                             24

<210> 700
<211> 27
<212> DNA
<213> Artificial Sequence

<220>
<223> Synthetic Oligonucleotide

<400> 700
cggtacttct cagggctaat atatacg                                          27

<210> 701
<211> 21
<212> DNA
<213> Artificial Sequence

<220>
<223> Synthetic Oligonucleotide

<400> 701
ccgagtagat gggcaggtgt t                                                21

<210> 702
<211> 26
<212> DNA
<213> Artificial Sequence

<220>
<223> Synthetic Oligonucleotide

<400> 702
ctcttctgcg tggtggtcaa ccccta                                           26

<210> 703
<211> 18

<212> DNA
<213> Artificial Sequence

<220>
<223> Synthetic Oligonucleotide

<400> 703
gtgaggcagc gcgactct                                                    18

<210> 704
<211> 23
<212> DNA
<213> Artificial Sequence

<220>
<223> Synthetic Oligonucleotide

<400> 704
tgccaatggt gtacaacact tca                                              23

<210> 705
<211> 21
<212> DNA
<213> Artificial Sequence

<220>
<223> Synthetic Oligonucleotide

<400> 705
tgccttcccg ggctgaggac t                                                21

<210> 706
<211> 20
<212> DNA
<213> Artificial Sequence

<220>
<223> Synthetic Oligonucleotide

<400> 706
cagaccaagg agatggacct                                                  20

<210> 707
<211> 18
<212> DNA
<213> Artificial Sequence

<220>
<223> Synthetic Oligonucleotide

<400> 707
agctgccagt gctatccg                                                    18

<210> 708
<211> 24
<212> DNA
<213> Artificial Sequence

<220>
<223> Synthetic Oligonucleotide

<400> 708
aagctgtaaa catgagccgc acca                                             24

<210> 709
<211> 19
<212> DNA
<213> Artificial Sequence

<220>
<223> Synthetic Oligonucleotide

<400> 709
ctgccgggat ggcttctat                                                    19

<210> 710
<211> 22
<212> DNA
<213> Artificial Sequence

<220>
<223> Synthetic Oligonucleotide

<400> 710
ccaggttctg gaaactgtgg at                                                22

<210> 711
<211> 21
<212> DNA
<213> Artificial Sequence

<220>
<223> Synthetic Oligonucleotide

<400> 711
ctgagctctg cccggaccgc t                                                 21

<210> 712
<211> 18
<212> DNA
<213> Artificial Sequence

<220>
<223> Synthetic Oligonucleotide

<400> 712
cactgacacc cgcaacac                                                     18

<210> 713
<211> 19
<212> DNA
<213> Artificial Sequence

<220>
<223> Synthetic Oligonucleotide

<400> 713
ggctgcaatc tctctgctg                                                    19

<210> 714
<211> 24
<212> DNA
<213> Artificial Sequence

<220>
<223> Synthetic Oligonucleotide

<400> 714
aaccacagag tccgaaccat gggt                                              24

<210> 715
<211> 19
<212> DNA
<213> Artificial Sequence

<220>
<223> Synthetic Oligonucleotide

<400> 715
ctgcttcttt cccgagctt                                                    19

<210> 716
<211> 19
<212> DNA
<213> Artificial Sequence

<220>
<223> Synthetic Oligonucleotide

<400> 716
agcaggaatg tagctgcca                                                    19

<210> 717
<211> 24
<212> DNA
<213> Artificial Sequence

<220>
<223> Synthetic Oligonucleotide

<400> 717
acttcgttat ccgcgatgcg tttc                                              24

<210> 718
<211> 20
<212> DNA
<213> Artificial Sequence

<220>
<223> Synthetic Oligonucleotide

<400> 718
ggctgtggct gaggctgtag                                                   20

<210> 719
<211> 21
<212> DNA
<213> Artificial Sequence

<220>
<223> Synthetic Oligonucleotide

<400> 719
ggagcattcg aggtcaaatc a                                                 21

<210> 720
<211> 28
<212> DNA
<213> Artificial Sequence

<220>
<223> Synthetic Oligonucleotide

<400> 720
ttcccagagt gtctcacctc cagcagag                                          28

<210> 721
<211> 20
<212> DNA
<213> Artificial Sequence

<220>
<223> Synthetic Oligonucleotide

<400> 721
cggacctgaa tgtaatgcaa                                                   20

<210> 722
<211> 20
<212> DNA
<213> Artificial Sequence

<220>
<223> Synthetic Oligonucleotide

<400> 722
ctttgccagg tgactaagca                                                   20

<210> 723
<211> 28
<212> DNA
<213> Artificial Sequence

<220>
<223> Synthetic Oligonucleotide

<400> 723
aatgaacaga acaagcaaaa tgaccagc                                          28

<210> 724
<211> 20
<212> DNA
<213> Artificial Sequence

<220>
<223> Synthetic Oligonucleotide

<400> 724
caaaggaaga gcaacggaag                                                   20

<210> 725
<211> 19
<212> DNA
<213> Artificial Sequence

<220>
<223> Synthetic Oligonucleotide

<400> 725
attcccaaaa cctttgctt                                                    19

<210> 726

<211> 24
<212> DNA
<213> Artificial Sequence

<220>
<223> Synthetic Oligonucleotide

<400> 726
ttctcctttc gttcttgctc gcgt                                              24

<210> 727
<211> 21
<212> DNA
<213> Artificial Sequence

<220>
<223> Synthetic Oligonucleotide

<400> 727
tggagactct cagggtcgaa a                                                 21

<210> 728
<211> 22
<212> DNA
<213> Artificial Sequence

<220>
<223> Synthetic Oligonucleotide

<400> 728
ggcgtttgga gtggtagaaa tc                                                22

<210> 729
<211> 20
<212> DNA
<213> Artificial Sequence

<220>
<223> Synthetic Oligonucleotide

<400> 729
cggcggcaga ccagcatgac                                                   20

<210> 730
<211> 21
<212> DNA
<213> Artificial Sequence

<220>
<223> Synthetic Oligonucleotide

<400> 730
cggtggacca cgaagagtta a                                                 21

<210> 731
<211> 19
<212> DNA
<213> Artificial Sequence

<220>
<223> Synthetic Oligonucleotide

<400> 731

ggctcgcctc ttccatgtc                                                    19

<210> 732
<211> 23
<212> DNA
<213> Artificial Sequence

<220>
<223> Synthetic Oligonucleotide

<400> 732
ccgggacttg gagaagcact gca                                              23

<210> 733
<211> 20
<212> DNA
<213> Artificial Sequence

<220>
<223> Synthetic Oligonucleotide

<400> 733
tcactaccag ctgacatccg                                                  20

<210> 734
<211> 20
<212> DNA
<213> Artificial Sequence

<220>
<223> Synthetic Oligonucleotide

<400> 734
agatggggct attgagggtc                                                  20

<210> 735
<211> 24
<212> DNA
<213> Artificial Sequence

<220>
<223> Synthetic Oligonucleotide

<400> 735
cttctccagg tagccctcag gcag                                             24

<210> 736
<211> 20
<212> DNA
<213> Artificial Sequence

<220>
<223> Synthetic Oligonucleotide

<400> 736
ccagctctcc ttccagctac                                                  20

<210> 737
<211> 20
<212> DNA
<213> Artificial Sequence

<220>

<223> Synthetic Oligonucleotide

<400> 737
gggtggctct cacttagctc                                                    20

<210> 738
<211> 24
<212> DNA
<213> Artificial Sequence

<220>
<223> Synthetic Oligonucleotide

<400> 738
atcaatgtcc ctgtccgagt gctg                                               24

<210> 739
<211> 19
<212> DNA
<213> Artificial Sequence

<220>
<223> Synthetic Oligonucleotide

<400> 739
gagaagcacg ccatgaaac                                                     19

<210> 740
<211> 20
<212> DNA
<213> Artificial Sequence

<220>
<223> Synthetic Oligonucleotide

<400> 740
ggctgtgagc tgctgaatct                                                    20

<210> 741
<211> 24
<212> DNA
<213> Artificial Sequence

<220>
<223> Synthetic Oligonucleotide

<400> 741
tgactcatca tttccatgac ggcc                                               24

<210> 742
<211> 24
<212> DNA
<213> Artificial Sequence

<220>
<223> Synthetic Oligonucleotide

<400> 742
agagccagtt gctgtagaac tcaa                                               24

<210> 743
<211> 21
<212> DNA

<213> Artificial Sequence

<220>
<223> Synthetic Oligonucleotide

<400> 743
ctgggcctac acagtccttc a                                                21

<210> 744
<211> 27
<212> DNA
<213> Artificial Sequence

<220>
<223> Synthetic Oligonucleotide

<400> 744
tctctgctgg gcaaggatgt tctgttc                                          27

<210> 745
<211> 20
<212> DNA
<213> Artificial Sequence

<220>
<223> Synthetic Oligonucleotide

<400> 745
gacattgtgg taggggaagg                                                  20

<210> 746
<211> 20
<212> DNA
<213> Artificial Sequence

<220>
<223> Synthetic Oligonucleotide

<400> 746
cggcagtcaa agataattgg                                                  20

<210> 747
<211> 26
<212> DNA
<213> Artificial Sequence

<220>
<223> Synthetic Oligonucleotide

<400> 747
tcattttctc atcccgtggg tacaga                                           26

<210> 748
<211> 23
<212> DNA
<213> Artificial Sequence

<220>
<223> Synthetic Oligonucleotide

<400> 748
cacactagca ttttctccgc ata                                              23

<210> 749
<211> 21
<212> DNA
<213> Artificial Sequence

<220>
<223> Synthetic Oligonucleotide

<400> 749
ataccaatca ccgcacaaac c                                                21

<210> 750
<211> 30
<212> DNA
<213> Artificial Sequence

<220>
<223> Synthetic Oligonucleotide

<400> 750
tgcgctgtga ctggacttaa caaatagcct                                       30

<210> 751
<211> 20
<212> DNA
<213> Artificial Sequence

<220>
<223> Synthetic Oligonucleotide

<400> 751
ctgctcaagg acaccgtcta                                                  20

<210> 752
<211> 18
<212> DNA
<213> Artificial Sequence

<220>
<223> Synthetic Oligonucleotide

<400> 752
ggatccactc tggagggc                                                    18

<210> 753
<211> 24
<212> DNA
<213> Artificial Sequence

<220>
<223> Synthetic Oligonucleotide

<400> 753
tacactcggg tcccatcgaa gtcc                                             24

<210> 754
<211> 19
<212> DNA
<213> Artificial Sequence

<220>
<223> Synthetic Oligonucleotide

<400> 754
tggggacatt ccctttgag                                                    19

<210> 755
<211> 18
<212> DNA
<213> Artificial Sequence

<220>
<223> Synthetic Oligonucleotide

<400> 755
gacatgggct gggaagtg                                                     18

<210> 756
<211> 24
<212> DNA
<213> Artificial Sequence

<220>
<223> Synthetic Oligonucleotide

<400> 756
cagcttccag aatctcctgg tccc                                              24

<210> 757
<211> 20
<212> DNA
<213> Artificial Sequence

<220>
<223> Synthetic Oligonucleotide

<400> 757
cccatggatg ctcctctgaa                                                   20

<210> 758
<211> 20
<212> DNA
<213> Artificial Sequence

<220>
<223> Synthetic Oligonucleotide

<400> 758
ccggtggcta ccagacattg                                                   20

<210> 759
<211> 22
<212> DNA
<213> Artificial Sequence

<220>
<223> Synthetic Oligonucleotide

<400> 759
cattgactgc cgaggcccca tg                                                22

<210> 760
<211> 18
<212> DNA
<213> Artificial Sequence

<220>
<223> Synthetic Oligonucleotide

<400> 760
ccaagttctg ggtggtgg                                                    18

<210> 761
<211> 19
<212> DNA
<213> Artificial Sequence

<220>
<223> Synthetic Oligonucleotide

<400> 761
gtgaacgcca gtccatgtt                                                   19

<210> 762
<211> 24
<212> DNA
<213> Artificial Sequence

<220>
<223> Synthetic Oligonucleotide

<400> 762
ccagacccac ttctacctgg gcag                                             24

<210> 763
<211> 26
<212> DNA
<213> Artificial Sequence

<220>
<223> Synthetic Oligonucleotide

<400> 763
aatgaataca gtattcccaa gcacat                                           26

<210> 764
<211> 23
<212> DNA
<213> Artificial Sequence

<220>
<223> Synthetic Oligonucleotide

<400> 764
tgtctgaagc atcttctgga tga                                              23

<210> 765
<211> 18
<212> DNA
<213> Artificial Sequence

<220>
<223> Synthetic Oligonucleotide

<400> 765
aaccccgtgg ccgcctcc                                                    18

<210> 766
<211> 21

<212> DNA
<213> Artificial Sequence

<220>
<223> Synthetic Oligonucleotide

<400> 766
cttacggttt tcgtggagaa g                                                21

<210> 767
<211> 20
<212> DNA
<213> Artificial Sequence

<220>
<223> Synthetic Oligonucleotide

<400> 767
agcagccgtt cttgttgtaa                                                  20

<210> 768
<211> 29
<212> DNA
<213> Artificial Sequence

<220>
<223> Synthetic Oligonucleotide

<400> 768
cctcagctat acaacaaatt gaccccaag                                        29

<210> 769
<211> 20
<212> DNA
<213> Artificial Sequence

<220>
<223> Synthetic Oligonucleotide

<400> 769
gatgtggact gccattcaaa                                                  20

<210> 770
<211> 20
<212> DNA
<213> Artificial Sequence

<220>
<223> Synthetic Oligonucleotide

<400> 770
tgcgagatta gttggctgag                                                  20

<210> 771
<211> 28
<212> DNA
<213> Artificial Sequence

<220>
<223> Synthetic Oligonucleotide

<400> 771
tcgaaattta catccggtat cttcctgg                                         28

<210> 772
<211> 19
<212> DNA
<213> Artificial Sequence

<220>
<223> Synthetic Oligonucleotide

<400> 772
ccacataccg tccagccta                                                    19

<210> 773
<211> 18
<212> DNA
<213> Artificial Sequence

<220>
<223> Synthetic Oligonucleotide

<400> 773
gaggtcatgt gcgggagt                                                     18

<210> 774
<211> 24
<212> DNA
<213> Artificial Sequence

<220>
<223> Synthetic Oligonucleotide

<400> 774
ccgctcctct tcttcgttct ccag                                              24

<210> 775
<211> 20
<212> DNA
<213> Artificial Sequence

<220>
<223> Synthetic Oligonucleotide

<400> 775
gcaatcatgg aacttgacga                                                   20

<210> 776
<211> 18
<212> DNA
<213> Artificial Sequence

<220>
<223> Synthetic Oligonucleotide

<400> 776
atgtggctcg cctctacg                                                     18

<210> 777
<211> 24
<212> DNA
<213> Artificial Sequence

<220>
<223> Synthetic Oligonucleotide

<400> 777
tttcttgcag tttgacccag cacc                                              24

<210> 778
<211> 20
<212> DNA
<213> Artificial Sequence

<220>
<223> Synthetic Oligonucleotide

<400> 778
gcatcaggct gtcattatgg                                                   20

<210> 779
<211> 20
<212> DNA
<213> Artificial Sequence

<220>
<223> Synthetic Oligonucleotide

<400> 779
agtagttgtg ctgcccttcc                                                   20

<210> 780
<211> 28
<212> DNA
<213> Artificial Sequence

<220>
<223> Synthetic Oligonucleotide

<400> 780
tgtccttacc tgtgggagct gtaaggtc                                          28

<210> 781
<211> 19
<212> DNA
<213> Artificial Sequence

<220>
<223> Synthetic Oligonucleotide

<400> 781
aggactggga cccatgaac                                                    19

<210> 782
<211> 20
<212> DNA
<213> Artificial Sequence

<220>
<223> Synthetic Oligonucleotide

<400> 782
cccataatcc tgagcaatgg                                                   20

<210> 783
<211> 24
<212> DNA
<213> Artificial Sequence

<220>
<223> Synthetic Oligonucleotide

<400> 783
tcctttggta tcagacccga agcg                                              24

<210> 784
<211> 20
<212> DNA
<213> Artificial Sequence

<220>
<223> Synthetic Oligonucleotide

<400> 784
ggtgtccttc gccagatcac                                                   20

<210> 785
<211> 18
<212> DNA
<213> Artificial Sequence

<220>
<223> Synthetic Oligonucleotide

<400> 785
cagccgcaga gcctcatc                                                     18

<210> 786
<211> 26
<212> DNA
<213> Artificial Sequence

<220>
<223> Synthetic Oligonucleotide

<400> 786
ttaatgattt gcctgtggga cgctcc                                            26

<210> 787
<211> 21
<212> DNA
<213> Artificial Sequence

<220>
<223> Synthetic Oligonucleotide

<400> 787
caagcaatgc gtcatcaatg t                                                 21

<210> 788
<211> 20
<212> DNA
<213> Artificial Sequence

<220>
<223> Synthetic Oligonucleotide

<400> 788
gtaaatccgc cccctcttct                                                   20

<210> 789

<211> 25
<212> DNA
<213> Artificial Sequence

<220>
<223> Synthetic Oligonucleotide

<400> 789
cagcctctgc ggaatggatc acact                                          25

<210> 790
<211> 20
<212> DNA
<213> Artificial Sequence

<220>
<223> Synthetic Oligonucleotide

<400> 790
ctgacacttg ccgcagagaa                                                20

<210> 791
<211> 20
<212> DNA
<213> Artificial Sequence

<220>
<223> Synthetic Oligonucleotide

<400> 791
aggtggtcct tggtctggaa                                                20

<210> 792
<211> 26
<212> DNA
<213> Artificial Sequence

<220>
<223> Synthetic Oligonucleotide

<400> 792
ccctttctca cccacctcat ctgcac                                         26

<210> 793
<211> 18
<212> DNA
<213> Artificial Sequence

<220>
<223> Synthetic Oligonucleotide

<400> 793
gggttctaga cgcccctc                                                  18

<210> 794
<211> 21
<212> DNA
<213> Artificial Sequence

<220>
<223> Synthetic Oligonucleotide

<400> 794

ggacggctgt agaggctgta t                                                21

<210> 795
<211> 24
<212> DNA
<213> Artificial Sequence

<220>
<223> Synthetic Oligonucleotide

<400> 795
caagcgttcc tggccttctg aact                                             24

<210> 796
<211> 21
<212> DNA
<213> Artificial Sequence

<220>
<223> Synthetic Oligonucleotide

<400> 796
ctccacctat gagcgtctgt c                                                21

<210> 797
<211> 20
<212> DNA
<213> Artificial Sequence

<220>
<223> Synthetic Oligonucleotide

<400> 797
cacactttcc ctccttttgg                                                  20

<210> 798
<211> 24
<212> DNA
<213> Artificial Sequence

<220>
<223> Synthetic Oligonucleotide

<400> 798
tcctgttaac atcccaagcc caca                                             24

<210> 799
<211> 19
<212> DNA
<213> Artificial Sequence

<220>
<223> Synthetic Oligonucleotide

<400> 799
gccctcccag tgtgcaaat                                                   19

<210> 800
<211> 25
<212> DNA
<213> Artificial Sequence

<220>

<223> Synthetic Oligonucleotide

<400> 800
cgtcgatggt attaggatag aagca                                          25

<210> 801
<211> 23
<212> DNA
<213> Artificial Sequence

<220>
<223> Synthetic Oligonucleotide

<400> 801
tgctgtttcg acgacaccgt tcg                                            23

<210> 802
<211> 25
<212> DNA
<213> Artificial Sequence

<220>
<223> Synthetic Oligonucleotide

<400> 802
tggctaagtg aagatgacaa tcatg                                          25

<210> 803
<211> 25
<212> DNA
<213> Artificial Sequence

<220>
<223> Synthetic Oligonucleotide

<400> 803
tgcacatatc attacaccag ttcgt                                          25

<210> 804
<211> 29
<212> DNA
<213> Artificial Sequence

<220>
<223> Synthetic Oligonucleotide

<400> 804
cctttccagc tttacagtga attgctgca                                      29

<210> 805
<211> 23
<212> DNA
<213> Artificial Sequence

<220>
<223> Synthetic Oligonucleotide

<400> 805
cgcttgccta actcatactt tcc                                            23

<210> 806
<211> 20
<212> DNA

<213> Artificial Sequence

<220>
<223> Synthetic Oligonucleotide

<400> 806
ccattcagac tgcgccactt                                                    20

<210> 807
<211> 19
<212> DNA
<213> Artificial Sequence

<220>
<223> Synthetic Oligonucleotide

<400> 807
tccacgcagc gtggcactg                                                     19

<210> 808
<211> 28
<212> DNA
<213> Artificial Sequence

<220>
<223> Synthetic Oligonucleotide

<400> 808
ggctactctg atctatgttg ataaggaa                                           28

<210> 809
<211> 20
<212> DNA
<213> Artificial Sequence

<220>
<223> Synthetic Oligonucleotide

<400> 809
gcttcagccc atccttagca                                                    20

<210> 810
<211> 22
<212> DNA
<213> Artificial Sequence

<220>
<223> Synthetic Oligonucleotide

<400> 810
cacacgggtg cctggttctc ca                                                 22

<210> 811
<211> 18
<212> DNA
<213> Artificial Sequence

<220>
<223> Synthetic Oligonucleotide

<400> 811
gggacactgc gggacaag                                                      18

<210> 812
<211> 18
<212> DNA
<213> Artificial Sequence

<220>
<223> Synthetic Oligonucleotide

<400> 812
gcccatggcg tctctgaa                                                    18

<210> 813
<211> 25
<212> DNA
<213> Artificial Sequence

<220>
<223> Synthetic Oligonucleotide

<400> 813
cggttccgga gtctcaccac tgcat                                            25

<210> 814
<211> 19
<212> DNA
<213> Artificial Sequence

<220>
<223> Synthetic Oligonucleotide

<400> 814
ctgaaggacc ctacgctcg                                                   19

<210> 815
<211> 19
<212> DNA
<213> Artificial Sequence

<220>
<223> Synthetic Oligonucleotide

<400> 815
atgcaaagcc agtgtgctc                                                   19

<210> 816
<211> 24
<212> DNA
<213> Artificial Sequence

<220>
<223> Synthetic Oligonucleotide

<400> 816
cttctcaaag tgaggtgcca ggcc                                             24

<210> 817
<211> 21
<212> DNA
<213> Artificial Sequence

<220>
<223> Synthetic Oligonucleotide

<400> 817
gcgacagctc ctctagttcc a                                                21

<210> 818
<211> 19
<212> DNA
<213> Artificial Sequence

<220>
<223> Synthetic Oligonucleotide

<400> 818
ttcccgaagt ctccgcccg                                                   19

<210> 819
<211> 22
<212> DNA
<213> Artificial Sequence

<220>
<223> Synthetic Oligonucleotide

<400> 819
ggaacaccag cttgaatttc ct                                               22

<210> 820
<211> 20
<212> DNA
<213> Artificial Sequence

<220>
<223> Synthetic Oligonucleotide

<400> 820
gaggagtggt gacagtctgc                                                  20

<210> 821
<211> 20
<212> DNA
<213> Artificial Sequence

<220>
<223> Synthetic Oligonucleotide

<400> 821
gctgcagaaa tcaaagtcca                                                  20

<210> 822
<211> 27
<212> DNA
<213> Artificial Sequence

<220>
<223> Synthetic Oligonucleotide

<400> 822
tcaatacaca ggaacctgag gagaccc                                          27

<210> 823
<211> 23
<212> DNA
<213> Artificial Sequence

<220>
<223> Synthetic Oligonucleotide

<400> 823
agctagcctc agtgacacac atg                                              23

<210> 824
<211> 18
<212> DNA
<213> Artificial Sequence

<220>
<223> Synthetic Oligonucleotide

<400> 824
ccggatctga cggctgtt                                                    18

<210> 825
<211> 24
<212> DNA
<213> Artificial Sequence

<220>
<223> Synthetic Oligonucleotide

<400> 825
acacaacgtc ggcagtgcaa cctg                                             24

<210> 826
<211> 20
<212> DNA
<213> Artificial Sequence

<220>
<223> Synthetic Oligonucleotide

<400> 826
cgtcgtatgc gagagtctgt                                                  20

<210> 827
<211> 20
<212> DNA
<213> Artificial Sequence

<220>
<223> Synthetic Oligonucleotide

<400> 827
tgaaggcgtg aggtgtagaa                                                  20

<210> 828
<211> 26
<212> DNA
<213> Artificial Sequence

<220>
<223> Synthetic Oligonucleotide

<400> 828
tccaggatgc ctgttagttc tcagca                                           26

<210> 829
<211> 26

<212> DNA
<213> Artificial Sequence

<220>
<223> Synthetic Oligonucleotide

<400> 829
ggtgtcagat ataaatgtgc aaatgc                                            26

<210> 830
<211> 24
<212> DNA
<213> Artificial Sequence

<220>
<223> Synthetic Oligonucleotide

<400> 830
ttcgatattg ccagcagcta taaa                                              24

<210> 831
<211> 28
<212> DNA
<213> Artificial Sequence

<220>
<223> Synthetic Oligonucleotide

<400> 831
tgctgtcctg tcggtctcag tacgttca                                          28

<210> 832
<211> 20
<212> DNA
<213> Artificial Sequence

<220>
<223> Synthetic Oligonucleotide

<400> 832
tgtggatgct ggattgattt                                                   20

<210> 833
<211> 20
<212> DNA
<213> Artificial Sequence

<220>
<223> Synthetic Oligonucleotide

<400> 833
aagcagcact tcctggtctt                                                   20

<210> 834
<211> 25
<212> DNA
<213> Artificial Sequence

<220>
<223> Synthetic Oligonucleotide

<400> 834
ccactggtgc agctgctaaa tagca                                             25

<210> 835
<211> 20
<212> DNA
<213> Artificial Sequence

<220>
<223> Synthetic Oligonucleotide

<400> 835
agtgggagac acctgacctt                                                    20

<210> 836
<211> 20
<212> DNA
<213> Artificial Sequence

<220>
<223> Synthetic Oligonucleotide

<400> 836
tgatctgggc attgtactcc                                                    20

<210> 837
<211> 29
<212> DNA
<213> Artificial Sequence

<220>
<223> Synthetic Oligonucleotide

<400> 837
ttgatcttct gctcaatctc agcttgaga                                          29

<210> 838
<211> 20
<212> DNA
<213> Artificial Sequence

<220>
<223> Synthetic Oligonucleotide

<400> 838
cgaagccctt acaagtttcc                                                    20

<210> 839
<211> 20
<212> DNA
<213> Artificial Sequence

<220>
<223> Synthetic Oligonucleotide

<400> 839
ggactcttca ggggtgaaat                                                    20

<210> 840
<211> 24
<212> DNA
<213> Artificial Sequence

<220>
<223> Synthetic Oligonucleotide

<400> 840
cccttacgga ttcctggagg gaac                                              24

<210> 841
<211> 20
<212> DNA
<213> Artificial Sequence

<220>
<223> Synthetic Oligonucleotide

<400> 841
cccagtgtac gaggaacaag                                                   20

<210> 842
<211> 20
<212> DNA
<213> Artificial Sequence

<220>
<223> Synthetic Oligonucleotide

<400> 842
ttagcgagga aggagttgct                                                   20

<210> 843
<211> 24
<212> DNA
<213> Artificial Sequence

<220>
<223> Synthetic Oligonucleotide

<400> 843
acagaccgag atctccaacc ggac                                              24

<210> 844
<211> 18
<212> DNA
<213> Artificial Sequence

<220>
<223> Synthetic Oligonucleotide

<400> 844
gggctgggtg agaatgtg                                                     18

<210> 845
<211> 19
<212> DNA
<213> Artificial Sequence

<220>
<223> Synthetic Oligonucleotide

<400> 845
cacaacatcc tccggaatg                                                    19

<210> 846
<211> 24
<212> DNA
<213> Artificial Sequence

<220>
<223> Synthetic Oligonucleotide

<400> 846
ataccagggc cggcttcttc ctct                                              24

<210> 847
<211> 18
<212> DNA
<213> Artificial Sequence

<220>
<223> Synthetic Oligonucleotide

<400> 847
cctacaagtc ctggggca                                                     18

<210> 848
<211> 20
<212> DNA
<213> Artificial Sequence

<220>
<223> Synthetic Oligonucleotide

<400> 848
tgaggtcagg ctcacacagt                                                   20

<210> 849
<211> 24
<212> DNA
<213> Artificial Sequence

<220>
<223> Synthetic Oligonucleotide

<400> 849
tggagcccca agcagtgtta atcc                                              24

<210> 850
<211> 20
<212> DNA
<213> Artificial Sequence

<220>
<223> Synthetic Oligonucleotide

<400> 850
gcgaggagct ctacttgctc                                                   20

<210> 851
<211> 18
<212> DNA
<213> Artificial Sequence

<220>
<223> Synthetic Oligonucleotide

<400> 851
gccctgctga acaccact                                                     18

<210> 852

<211> 24
<212> DNA
<213> Artificial Sequence

<220>
<223> Synthetic Oligonucleotide

<400> 852
tgtcctcttt ctgcaccttg tcgc                                              24

<210> 853
<211> 21
<212> DNA
<213> Artificial Sequence

<220>
<223> Synthetic Oligonucleotide

<400> 853
aagcatgaac aggacttgac c                                                 21

<210> 854
<211> 18
<212> DNA
<213> Artificial Sequence

<220>
<223> Synthetic Oligonucleotide

<400> 854
cctccccaag tcagttgc                                                     18

<210> 855
<211> 24
<212> DNA
<213> Artificial Sequence

<220>
<223> Synthetic Oligonucleotide

<400> 855
ctttccaacc cctggggaag acat                                              24

<210> 856
<211> 18
<212> DNA
<213> Artificial Sequence

<220>
<223> Synthetic Oligonucleotide

<400> 856
cccgttcggt ctgaggaa                                                     18

<210> 857
<211> 22
<212> DNA
<213> Artificial Sequence

<220>
<223> Synthetic Oligonucleotide

<400> 857

gagcactcaa ggtagccaaa gg                                                22

<210> 858
<211> 19
<212> DNA
<213> Artificial Sequence

<220>
<223> Synthetic Oligonucleotide

<400> 858
tccggttcgc catgtcccg                                                    19

<210> 859
<211> 20
<212> DNA
<213> Artificial Sequence

<220>
<223> Synthetic Oligonucleotide

<400> 859
ccagacgagc gattagaagc                                                   20

<210> 860
<211> 20
<212> DNA
<213> Artificial Sequence

<220>
<223> Synthetic Oligonucleotide

<400> 860
tcctcctctt cctcctcctc                                                   20

<210> 861
<211> 23
<212> DNA
<213> Artificial Sequence

<220>
<223> Synthetic Oligonucleotide

<400> 861
tgtgaggtga atgatttggg gga                                               23

<210> 862
<211> 22
<212> DNA
<213> Artificial Sequence

<220>
<223> Synthetic Oligonucleotide

<400> 862
tgtgcacata ggaatgagct tc                                                22

<210> 863
<211> 20
<212> DNA
<213> Artificial Sequence

<220>

<223> Synthetic Oligonucleotide

<400> 863
gtttagcacg caattgctca                                                    20

<210> 864
<211> 24
<212> DNA
<213> Artificial Sequence

<220>
<223> Synthetic Oligonucleotide

<400> 864
tcactctctt tgctggccaa ctgc                                               24

<210> 865
<211> 19
<212> DNA
<213> Artificial Sequence

<220>
<223> Synthetic Oligonucleotide

<400> 865
gatctggcac tgtggttcc                                                     19

<210> 866
<211> 20
<212> DNA
<213> Artificial Sequence

<220>
<223> Synthetic Oligonucleotide

<400> 866
tgacagcgga agtggtattg                                                    20

<210> 867
<211> 22
<212> DNA
<213> Artificial Sequence

<220>
<223> Synthetic Oligonucleotide

<400> 867
caccgccagc cactgtcttc at                                                 22

<210> 868
<211> 24
<212> DNA
<213> Artificial Sequence

<220>
<223> Synthetic Oligonucleotide

<400> 868
ccattctatc atcaacgggt acaa                                               24

<210> 869
<211> 23
<212> DNA

<213> Artificial Sequence

<220>
<223> Synthetic Oligonucleotide

<400> 869
tcagcaagtg ggaaggtgta atc                                               23

<210> 870
<211> 25
<212> DNA
<213> Artificial Sequence

<220>
<223> Synthetic Oligonucleotide

<400> 870
tctccacaga caaggccagg actcg                                             25

<210> 871
<211> 19
<212> DNA
<213> Artificial Sequence

<220>
<223> Synthetic Oligonucleotide

<400> 871
ctgtcttcct gttggccct                                                    19

<210> 872
<211> 20
<212> DNA
<213> Artificial Sequence

<220>
<223> Synthetic Oligonucleotide

<400> 872
gtgaggtagt gagtgggcgt                                                   20

<210> 873
<211> 24
<212> DNA
<213> Artificial Sequence

<220>
<223> Synthetic Oligonucleotide

<400> 873
acaatcatag ccagcacctg ggct                                              24

<210> 874
<211> 25
<212> DNA
<213> Artificial Sequence

<220>
<223> Synthetic Oligonucleotide

<400> 874
gctcattatg aaaaacatcc caaac                                             25

<210> 875
<211> 26
<212> DNA
<213> Artificial Sequence

<220>
<223> Synthetic Oligonucleotide

<400> 875
aagaaacaga agttgtctgg ctttct                                            26

<210> 876
<211> 24
<212> DNA
<213> Artificial Sequence

<220>
<223> Synthetic Oligonucleotide

<400> 876
cacaccaacc aataatttcg catt                                              24

<210> 877
<211> 20
<212> DNA
<213> Artificial Sequence

<220>
<223> Synthetic Oligonucleotide

<400> 877
gctctgttgt gtcctgttgc                                                   20

<210> 878
<211> 21
<212> DNA
<213> Artificial Sequence

<220>
<223> Synthetic Oligonucleotide

<400> 878
gtacggagaa gccacttcac a                                                 21

<210> 879
<211> 23
<212> DNA
<213> Artificial Sequence

<220>
<223> Synthetic Oligonucleotide

<400> 879
tcagtcacag gaaggccaga gcc                                               23

<210> 880
<211> 18
<212> DNA
<213> Artificial Sequence

<220>
<223> Synthetic Oligonucleotide

<400> 880
cgaggagatg cctgtgga                                                    18

<210> 881
<211> 18
<212> DNA
<213> Artificial Sequence

<220>
<223> Synthetic Oligonucleotide

<400> 881
caacgccctg gtcactct                                                    18

<210> 882
<211> 24
<212> DNA
<213> Artificial Sequence

<220>
<223> Synthetic Oligonucleotide

<400> 882
ctgttccaca gcaagctctg cctc                                             24

<210> 883
<211> 20
<212> DNA
<213> Artificial Sequence

<220>
<223> Synthetic Oligonucleotide

<400> 883
acaccaaaat gccatctcaa                                                  20

<210> 884
<211> 20
<212> DNA
<213> Artificial Sequence

<220>
<223> Synthetic Oligonucleotide

<400> 884
tttatcccca gcgaatttgt                                                  20

<210> 885
<211> 24
<212> DNA
<213> Artificial Sequence

<220>
<223> Synthetic Oligonucleotide

<400> 885
cacgccatgg aaaccatgat gttt                                             24

<210> 886
<211> 18
<212> DNA
<213> Artificial Sequence

<220>
<223> Synthetic Oligonucleotide

<400> 886
cttctgagcc cgtctccc                                                    18

<210> 887
<211> 18
<212> DNA
<213> Artificial Sequence

<220>
<223> Synthetic Oligonucleotide

<400> 887
gagagctccc cttccgag                                                    18

<210> 888
<211> 24
<212> DNA
<213> Artificial Sequence

<220>
<223> Synthetic Oligonucleotide

<400> 888
cgcttctgga gcagcttcct caac                                             24

<210> 889
<211> 20
<212> DNA
<213> Artificial Sequence

<220>
<223> Synthetic Oligonucleotide

<400> 889
cgcgagcccc tcattataca                                                  20

<210> 890
<211> 19
<212> DNA
<213> Artificial Sequence

<220>
<223> Synthetic Oligonucleotide

<400> 890
cactcgccgt tgacatcct                                                   19

<210> 891
<211> 22
<212> DNA
<213> Artificial Sequence

<220>
<223> Synthetic Oligonucleotide

<400> 891
ctccccacag cgcatcgagg aa                                               22

<210> 892
<211> 20

<212> DNA
<213> Artificial Sequence

<220>
<223> Synthetic Oligonucleotide

<400> 892
gagagagcca gtctgatcca                                                    20

<210> 893
<211> 19
<212> DNA
<213> Artificial Sequence

<220>
<223> Synthetic Oligonucleotide

<400> 893
aggctgccat gtcctcata                                                     19

<210> 894
<211> 22
<212> DNA
<213> Artificial Sequence

<220>
<223> Synthetic Oligonucleotide

<400> 894
ctgctctgcc agcttggcct tc                                                 22

<210> 895
<211> 23
<212> DNA
<213> Artificial Sequence

<220>
<223> Synthetic Oligonucleotide

<400> 895
cagtggagac cagttgggta gtg                                                23

<210> 896
<211> 20
<212> DNA
<213> Artificial Sequence

<220>
<223> Synthetic Oligonucleotide

<400> 896
ccttgaagag cgtcccatca                                                    20

<210> 897
<211> 28
<212> DNA
<213> Artificial Sequence

<220>
<223> Synthetic Oligonucleotide

<400> 897
cacacatgca gagcttgtag cgtaccca                                           28

<210> 898
<211> 18
<212> DNA
<213> Artificial Sequence

<220>
<223> Synthetic Oligonucleotide

<400> 898
tccgcaagac acctcctg                                                    18

<210> 899
<211> 18
<212> DNA
<213> Artificial Sequence

<220>
<223> Synthetic Oligonucleotide

<400> 899
tgaagtcatc cttggccc                                                    18

<210> 900
<211> 23
<212> DNA
<213> Artificial Sequence

<220>
<223> Synthetic Oligonucleotide

<400> 900
tgtcctgtcc ttctgcagga ggc                                              23

<210> 901
<211> 20
<212> DNA
<213> Artificial Sequence

<220>
<223> Synthetic Oligonucleotide

<400> 901
tgcattcgtt ggtttctctt                                                  20

<210> 902
<211> 20
<212> DNA
<213> Artificial Sequence

<220>
<223> Synthetic Oligonucleotide

<400> 902
tttctgaatg gcaaactgct                                                  20

<210> 903
<211> 30
<212> DNA
<213> Artificial Sequence

<220>
<223> Synthetic Oligonucleotide

<400> 903
tgcacctcct tcagaagact tatttttgtg                                        30

<210> 904
<211> 20
<212> DNA
<213> Artificial Sequence

<220>
<223> Synthetic Oligonucleotide

<400> 904
ccaacacctt cttggcttct                                                   20

<210> 905
<211> 20
<212> DNA
<213> Artificial Sequence

<220>
<223> Synthetic Oligonucleotide

<400> 905
ctgttatccc aacccaaacc                                                   20

<210> 906
<211> 24
<212> DNA
<213> Artificial Sequence

<220>
<223> Synthetic Oligonucleotide

<400> 906
cctgtgttct tgctgagcac cctc                                              24

<210> 907
<211> 20
<212> DNA
<213> Artificial Sequence

<220>
<223> Synthetic Oligonucleotide

<400> 907
agctggggtg tctgtttcat                                                   20

<210> 908
<211> 20
<212> DNA
<213> Artificial Sequence

<220>
<223> Synthetic Oligonucleotide

<400> 908
acagcaaggc gagcataaat                                                   20

<210> 909
<211> 23
<212> DNA
<213> Artificial Sequence

<220>
<223> Synthetic Oligonucleotide

<400> 909
cctgacttca ggtcaaggga tgg                                              23

<210> 910
<211> 18
<212> DNA
<213> Artificial Sequence

<220>
<223> Synthetic Oligonucleotide

<400> 910
gtggggagcc catcatct                                                    18

<210> 911
<211> 20
<212> DNA
<213> Artificial Sequence

<220>
<223> Synthetic Oligonucleotide

<400> 911
ccagtccaca ctgagtgcat                                                  20

<210> 912
<211> 24
<212> DNA
<213> Artificial Sequence

<220>
<223> Synthetic Oligonucleotide

<400> 912
ccaagccatc acaggctctg cata                                             24

<210> 913
<211> 20
<212> DNA
<213> Artificial Sequence

<220>
<223> Synthetic Oligonucleotide

<400> 913
tctgccagtg ctgaattctt                                                  20

<210> 914
<211> 20
<212> DNA
<213> Artificial Sequence

<220>
<223> Synthetic Oligonucleotide

<400> 914
ttcgaacctt agcagcttcc                                                  20

<210> 915

<211> 24
<212> DNA
<213> Artificial Sequence

<220>
<223> Synthetic Oligonucleotide

<400> 915
tgctgacatt gccctggctc ctat                                              24

<210> 916
<211> 20
<212> DNA
<213> Artificial Sequence

<220>
<223> Synthetic Oligonucleotide

<400> 916
cccaactcag gtccttggta                                                   20

<210> 917
<211> 18
<212> DNA
<213> Artificial Sequence

<220>
<223> Synthetic Oligonucleotide

<400> 917
caagagggtc accccaag                                                     18

<210> 918
<211> 24
<212> DNA
<213> Artificial Sequence

<220>
<223> Synthetic Oligonucleotide

<400> 918
atcctgcaag ccaatcccag tcat                                              24

<210> 919
<211> 22
<212> DNA
<213> Artificial Sequence

<220>
<223> Synthetic Oligonucleotide

<400> 919
agatgcatac ttggaagcac ag                                                22

<210> 920
<211> 22
<212> DNA
<213> Artificial Sequence

<220>
<223> Synthetic Oligonucleotide

<400> 920

aacctaggac caggtaacca ca                                              22

<210> 921
<211> 24
<212> DNA
<213> Artificial Sequence

<220>
<223> Synthetic Oligonucleotide

<400> 921
catatcacac tgggaggcaa tgca                                            24

<210> 922
<211> 18
<212> DNA
<213> Artificial Sequence

<220>
<223> Synthetic Oligonucleotide

<400> 922
gcgcagaggc cagttaaa                                                   18

<210> 923
<211> 18
<212> DNA
<213> Artificial Sequence

<220>
<223> Synthetic Oligonucleotide

<400> 923
agctgagctg tgggttgc                                                   18

<210> 924
<211> 24
<212> DNA
<213> Artificial Sequence

<220>
<223> Synthetic Oligonucleotide

<400> 924
agatcacctc ctcgaaccca ctcc                                            24

<210> 925
<211> 19
<212> DNA
<213> Artificial Sequence

<220>
<223> Synthetic Oligonucleotide

<400> 925
ggctggccaa acataagca                                                  19

<210> 926
<211> 26
<212> DNA
<213> Artificial Sequence

<220>

<223> Synthetic Oligonucleotide

<400> 926
tccttgtcac agtatttaca gctgaa                                          26

<210> 927
<211> 29
<212> DNA
<213> Artificial Sequence

<220>
<223> Synthetic Oligonucleotide

<400> 927
ctgcactgcg atgcccagtc tagaaaatc                                       29

<210> 928
<211> 21
<212> DNA
<213> Artificial Sequence

<220>
<223> Synthetic Oligonucleotide

<400> 928
agtgacaaat gttggaggag c                                               21

<210> 929
<211> 19
<212> DNA
<213> Artificial Sequence

<220>
<223> Synthetic Oligonucleotide

<400> 929
ccctccactg tcttctggg                                                  19

<210> 930
<211> 24
<212> DNA
<213> Artificial Sequence

<220>
<223> Synthetic Oligonucleotide

<400> 930
tactgctgtc acacccgtca ccac                                            24

<210> 931
<211> 19
<212> DNA
<213> Artificial Sequence

<220>
<223> Synthetic Oligonucleotide

<400> 931
acccaccatg gatgtcttc                                                  19

<210> 932
<211> 19
<212> DNA

<213> Artificial Sequence

<220>
<223> Synthetic Oligonucleotide

<400> 932
cctgcttggt cttttccac                                                    19

<210> 933
<211> 24
<212> DNA
<213> Artificial Sequence

<220>
<223> Synthetic Oligonucleotide

<400> 933
aagaagggct tctccatcgc caag                                              24

<210> 934
<211> 20
<212> DNA
<213> Artificial Sequence

<220>
<223> Synthetic Oligonucleotide

<400> 934
tgaagagagg catgttggag                                                   20

<210> 935
<211> 20
<212> DNA
<213> Artificial Sequence

<220>
<223> Synthetic Oligonucleotide

<400> 935
aatagacaca tcggccacac                                                   20

<210> 936
<211> 24
<212> DNA
<213> Artificial Sequence

<220>
<223> Synthetic Oligonucleotide

<400> 936
tttgtcagca gtcacattgc ccaa                                              24

<210> 937
<211> 24
<212> DNA
<213> Artificial Sequence

<220>
<223> Synthetic Oligonucleotide

<400> 937
atacagcacc aatggaaaga tcac                                              24

<210> 938
<211> 24
<212> DNA
<213> Artificial Sequence

<220>
<223> Synthetic Oligonucleotide

<400> 938
tgtctgtaag agccctcagt ttga                                              24

<210> 939
<211> 24
<212> DNA
<213> Artificial Sequence

<220>
<223> Synthetic Oligonucleotide

<400> 939
ttcgacgact acatcgcctg ctgc                                              24

<210> 940
<211> 20
<212> DNA
<213> Artificial Sequence

<220>
<223> Synthetic Oligonucleotide

<400> 940
gggctcagct ttcagaagtg                                                   20

<210> 941
<211> 20
<212> DNA
<213> Artificial Sequence

<220>
<223> Synthetic Oligonucleotide

<400> 941
acatgttcag ctggtccaca                                                   20

<210> 942
<211> 25
<212> DNA
<213> Artificial Sequence

<220>
<223> Synthetic Oligonucleotide

<400> 942
tggcagtttt cttctgtcac caaaa                                             25

<210> 943
<211> 20
<212> DNA
<213> Artificial Sequence

<220>
<223> Synthetic Oligonucleotide

<400> 943
tcacatgcca ctttggtgtt                                                    20

<210> 944
<211> 20
<212> DNA
<213> Artificial Sequence

<220>
<223> Synthetic Oligonucleotide

<400> 944
cttgcaggaa gcggctatac                                                    20

<210> 945
<211> 22
<212> DNA
<213> Artificial Sequence

<220>
<223> Synthetic Oligonucleotide

<400> 945
tcctgggaga gattgaccag ca                                                 22

<210> 946
<211> 19
<212> DNA
<213> Artificial Sequence

<220>
<223> Synthetic Oligonucleotide

<400> 946
caagagggac tcggactgc                                                     19

<210> 947
<211> 21
<212> DNA
<213> Artificial Sequence

<220>
<223> Synthetic Oligonucleotide

<400> 947
caggtcagtg gagagattgg t                                                  21

<210> 948
<211> 24
<212> DNA
<213> Artificial Sequence

<220>
<223> Synthetic Oligonucleotide

<400> 948
cctctgcacc tctgagagca tgga                                               24

<210> 949
<211> 18
<212> DNA
<213> Artificial Sequence

<220>
<223> Synthetic Oligonucleotide

<400> 949
ggactcggag acgctgtg                                                    18

<210> 950
<211> 20
<212> DNA
<213> Artificial Sequence

<220>
<223> Synthetic Oligonucleotide

<400> 950
gggatccttc gcaacttctt                                                  20

<210> 951
<211> 24
<212> DNA
<213> Artificial Sequence

<220>
<223> Synthetic Oligonucleotide

<400> 951
ttcttgagga tcttgacggc cctc                                             24

<210> 952
<211> 20
<212> DNA
<213> Artificial Sequence

<220>
<223> Synthetic Oligonucleotide

<400> 952
catcttccag gaggaccact                                                  20

<210> 953
<211> 20
<212> DNA
<213> Artificial Sequence

<220>
<223> Synthetic Oligonucleotide

<400> 953
tccgaccttc aatcatttca                                                  20

<210> 954
<211> 24
<212> DNA
<213> Artificial Sequence

<220>
<223> Synthetic Oligonucleotide

<400> 954
ctctgtggca ccctggacta cctg                                             24

<210> 955
<211> 20

<212> DNA
<213> Artificial Sequence

<220>
<223> Synthetic Oligonucleotide

<400> 955
aatacccaac gcacaaatga                                                   20

<210> 956
<211> 20
<212> DNA
<213> Artificial Sequence

<220>
<223> Synthetic Oligonucleotide

<400> 956
ggagacaatg caaaccacac                                                   20

<210> 957
<211> 23
<212> DNA
<213> Artificial Sequence

<220>
<223> Synthetic Oligonucleotide

<400> 957
cacgttctct gccccgtttc ttg                                               23

<210> 958
<211> 20
<212> DNA
<213> Artificial Sequence

<220>
<223> Synthetic Oligonucleotide

<400> 958
cctggaggct gcaacatacc                                                   20

<210> 959
<211> 23
<212> DNA
<213> Artificial Sequence

<220>
<223> Synthetic Oligonucleotide

<400> 959
tacaatggct ttggaggata gca                                               23

<210> 960
<211> 25
<212> DNA
<213> Artificial Sequence

<220>
<223> Synthetic Oligonucleotide

<400> 960
   atcctcctga agcccttttc gcagc                                              25

<210> 961
<211> 20
<212> DNA
<213> Artificial Sequence

<220>
<223> Synthetic Oligonucleotide

<400> 961
tgttttgatt cccgggctta                                                    20

<210> 962
<211> 24
<212> DNA
<213> Artificial Sequence

<220>
<223> Synthetic Oligonucleotide

<400> 962
caaagctgtc agctctagca aaag                                               24

<210> 963
<211> 28
<212> DNA
<213> Artificial Sequence

<220>
<223> Synthetic Oligonucleotide

<400> 963
tgccttcttc ctccctcact tctcacct                                           28

<210> 964
<211> 20
<212> DNA
<213> Artificial Sequence

<220>
<223> Synthetic Oligonucleotide

<400> 964
   cacccttgga ctggaaaact                                                   20

<210> 965
<211> 20
<212> DNA
<213> Artificial Sequence

<220>
<223> Synthetic Oligonucleotide

<400> 965
ccttgatgag ctgttggttc                                                    20

<210> 966
<211> 24
<212> DNA
<213> Artificial Sequence

<220>
<223> Synthetic Oligonucleotide

<400> 966
cacacccggt ctggacacag aaag                                              24

<210> 967
<211> 19
<212> DNA
<213> Artificial Sequence

<220>
<223> Synthetic Oligonucleotide

<400> 967
gatcctcgcg tgagacaga                                                    19

<210> 968
<211> 21
<212> DNA
<213> Artificial Sequence

<220>
<223> Synthetic Oligonucleotide

<400> 968
   cactttttct ttgaccaacc g                                                  21

<210> 969
<211> 23
<212> DNA
<213> Artificial Sequence

<220>
<223> Synthetic Oligonucleotide

<400> 969
ttcaccacgc cggtcttgat ctt                                               23

<210> 970
<211> 20
<212> DNA
<213> Artificial Sequence

<220>
<223> Synthetic Oligonucleotide

<400> 970
ctgttttcct ggaggactgc                                                   20

<210> 971
<211> 20
<212> DNA
<213> Artificial Sequence

<220>
<223> Synthetic Oligonucleotide

<400> 971
actgtgtatg cggagctgtt                                                   20

<210> 972
<211> 21
<212> DNA
<213> Artificial Sequence

<220>
<223> Synthetic Oligonucleotide

<400> 972
ccactgccag cacgcagtca c                                                21

<210> 973
<211> 21
<212> DNA
<213> Artificial Sequence

<220>
<223> Synthetic Oligonucleotide

<400> 973
cagccaggtg tacgagacat t                                                21

<210> 974
<211> 18
<212> DNA
<213> Artificial Sequence

<220>
<223> Synthetic Oligonucleotide

<400> 974
acctcgtcca gcacatcc                                                    18

<210> 975
<211> 24
<212> DNA
<213> Artificial Sequence

<220>
<223> Synthetic Oligonucleotide

<400> 975
ctcacctaca gtgacgtcgt gggc                                             24

<210> 976
<211> 19
<212> DNA
<213> Artificial Sequence

<220>
<223> Synthetic Oligonucleotide

<400> 976
tcccgagaga ggaaagcat                                                   19

<210> 977
<211> 18
<212> DNA
<213> Artificial Sequence

<220>
<223> Synthetic Oligonucleotide

<400> 977
gtcgggtgcc tgcattta                                                    18

<210> 978

<211> 24
<212> DNA
<213> Artificial Sequence

<220>
<223> Synthetic Oligonucleotide

<400> 978
atacacagtc ccttcgtggc tccc                                              24

<210> 979
<211> 22
<212> DNA
<213> Artificial Sequence

<220>
<223> Synthetic Oligonucleotide

<400> 979
cctggttgaa gcctgttaat gc                                                22

<210> 980
<211> 26
<212> DNA
<213> Artificial Sequence

<220>
<223> Synthetic Oligonucleotide

<400> 980
tgcagacttc tcatatttgc taaagg                                            26

<210> 981
<211> 23
<212> DNA
<213> Artificial Sequence

<220>
<223> Synthetic Oligonucleotide

<400> 981
ccgctgggtt ttccacacgt tga                                               23

<210> 982
<211> 22
<212> DNA
<213> Artificial Sequence

<220>
<223> Synthetic Oligonucleotide

<400> 982
ccagtgtgtg taacagggtc ac                                                22

<210> 983
<211> 18
<212> DNA
<213> Artificial Sequence

<220>
<223> Synthetic Oligonucleotide

<400> 983

tatagccttg ggccaccc                                                    18

<210> 984
<211> 24
<212> DNA
<213> Artificial Sequence

<220>
<223> Synthetic Oligonucleotide

<400> 984
agaattcgac agccagatgc tcca                                            24

<210> 985
<211> 20
<212> DNA
<213> Artificial Sequence

<220>
<223> Synthetic Oligonucleotide

<400> 985
gccaactgct ttcatttgtg                                                 20

<210> 986
<211> 20
<212> DNA
<213> Artificial Sequence

<220>
<223> Synthetic Oligonucleotide

<400> 986
actcaggccc atttccttta                                                 20

<210> 987
<211> 28
<212> DNA
<213> Artificial Sequence

<220>
<223> Synthetic Oligonucleotide

<400> 987
agggatctga accaatacag agcagaca                                        28

<210> 988
<211> 22
<212> DNA
<213> Artificial Sequence

<220>
<223> Synthetic Oligonucleotide

<400> 988
catccgcaaa gtgactgaag ag                                              22

<210> 989
<211> 27
<212> DNA
<213> Artificial Sequence

<220>

<223> Synthetic Oligonucleotide

<400> 989
gtactgaact ccgttgtgat agcatag                                          27

<210> 990
<211> 22
<212> DNA
<213> Artificial Sequence

<220>
<223> Synthetic Oligonucleotide

<400> 990
ccaatgagct gaggcggcct cc                                               22

<210> 991
<211> 20
<212> DNA
<213> Artificial Sequence

<220>
<223> Synthetic Oligonucleotide

<400> 991
gccgggaaga ccgtaattgt                                                  20

<210> 992
<211> 18
<212> DNA
<213> Artificial Sequence

<220>
<223> Synthetic Oligonucleotide

<400> 992
cagcggcacc aggttcag                                                    18

<210> 993
<211> 26
<212> DNA
<213> Artificial Sequence

<220>
<223> Synthetic Oligonucleotide

<400> 993
caaatggctt cctctggaag gtccca                                           26

<210> 994
<211> 20
<212> DNA
<213> Artificial Sequence

<220>
<223> Synthetic Oligonucleotide

<400> 994
aatccaaggg ggagagtgat                                                  20

<210> 995
<211> 20
<212> DNA

<213> Artificial Sequence

<220>
<223> Synthetic Oligonucleotide

<400> 995
gtacagattt tgcccgagga                                                    20

<210> 996
<211> 26
<212> DNA
<213> Artificial Sequence

<220>
<223> Synthetic Oligonucleotide

<400> 996
catatggact ttgactcagc tgtggc                                             26

<210> 997
<211> 18
<212> DNA
<213> Artificial Sequence

<220>
<223> Synthetic Oligonucleotide

<400> 997
gtgatgcctt ccctgtgg                                                      18

<210> 998
<211> 19
<212> DNA
<213> Artificial Sequence

<220>
<223> Synthetic Oligonucleotide

<400> 998
tcaggtagtc gggtgggtt                                                     19

<210> 999
<211> 24
<212> DNA
<213> Artificial Sequence

<220>
<223> Synthetic Oligonucleotide

<400> 999
tgcttctcca gcatcttcac ctcg                                               24

<210> 1000
<211> 24
<212> DNA
<213> Artificial Sequence

<220>
<223> Synthetic Oligonucleotide

<400> 1000
ctatatgcag ccagagatgt gaca                                               24

<210> 1001
<211> 24
<212> DNA
<213> Artificial Sequence

<220>
<223> Synthetic Oligonucleotide

<400> 1001
ccacgagttt cttactgaga atgg                                              24

<210> 1002
<211> 23
<212> DNA
<213> Artificial Sequence

<220>
<223> Synthetic Oligonucleotide

<400> 1002
acagcctgcc actcatcaca gcc                                               23

<210> 1003
<211> 20
<212> DNA
<213> Artificial Sequence

<220>
<223> Synthetic Oligonucleotide

<400> 1003
tgctgttgct gagtctgttg                                                   20

<210> 1004
<211> 20
<212> DNA
<213> Artificial Sequence

<220>
<223> Synthetic Oligonucleotide

<400> 1004
cttgcctggc ttcacagata                                                   20

<210> 1005
<211> 24
<212> DNA
<213> Artificial Sequence

<220>
<223> Synthetic Oligonucleotide

<400> 1005
ccagtcccca gaagaccatg tctg                                              24

<210> 1006
<211> 22
<212> DNA
<213> Artificial Sequence

<220>
<223> Synthetic Oligonucleotide

<400> 1006
ggtgtcgata ttgtcatgaa cc                                                22

<210> 1007
<211> 27
<212> DNA
<213> Artificial Sequence

<220>
<223> Synthetic Oligonucleotide

<400> 1007
gtaatctttg atgtacttct tgtaggc                                           27

<210> 1008
<211> 27
<212> DNA
<213> Artificial Sequence

<220>
<223> Synthetic Oligonucleotide

<400> 1008
tcacctgcag gaaacaagtt tcacaaa                                           27

<210> 1009
<211> 21
<212> DNA
<213> Artificial Sequence

<220>
<223> Synthetic Oligonucleotide

<400> 1009
gacgctggtc tggtgaagat g                                                 21

<210> 1010
<211> 20
<212> DNA
<213> Artificial Sequence

<220>
<223> Synthetic Oligonucleotide

<400> 1010
tgggtggttg ttggacaatg                                                   20

<210> 1011
<211> 23
<212> DNA
<213> Artificial Sequence

<220>
<223> Synthetic Oligonucleotide

<400> 1011
ccaggaaacc acgagcctcc agc                                               23

<210> 1012
<211> 22
<212> DNA
<213> Artificial Sequence

<220>
<223> Synthetic Oligonucleotide

<400> 1012
cttcacagtg ctcctgcagt ct                                                22

<210> 1013
<211> 21
<212> DNA
<213> Artificial Sequence

<220>
<223> Synthetic Oligonucleotide

<400> 1013
catctgcttc agctcgttgg t                                                 21

<210> 1014
<211> 26
<212> DNA
<213> Artificial Sequence

<220>
<223> Synthetic Oligonucleotide

<400> 1014
aagtacacgt aagttacagc cacaca                                            26

<210> 1015
<211> 18
<212> DNA
<213> Artificial Sequence

<220>
<223> Synthetic Oligonucleotide

<400> 1015
gcctcggtgt gcctttca                                                     18

<210> 1016
<211> 19
<212> DNA
<213> Artificial Sequence

<220>
<223> Synthetic Oligonucleotide

<400> 1016
cgtgatgtgc gcaatcatg                                                    19

<210> 1017
<211> 22
<212> DNA
<213> Artificial Sequence

<220>
<223> Synthetic Oligonucleotide

<400> 1017
catcgccagc tacgccctgc tc                                                22

<210> 1018
<211> 19

<212> DNA
<213> Artificial Sequence

<220>
<223> Synthetic Oligonucleotide

<400> 1018
ctggtcagcc ttcagggac 19

<210> 1019
<211> 18
<212> DNA
<213> Artificial Sequence

<220>
<223> Synthetic Oligonucleotide

<400> 1019
cttcagttct gggctggc 18

<210> 1020
<211> 23
<212> DNA
<213> Artificial Sequence

<220>
<223> Synthetic Oligonucleotide

<400> 1020
ctgagcaccg cctggtctct ttc 23

<210> 1021
<211> 23
<212> DNA
<213> Artificial Sequence

<220>
<223> Synthetic Oligonucleotide

<400> 1021
tgcttgtcag ttgtcaacac ctt 23

<210> 1022
<211> 23
<212> DNA
<213> Artificial Sequence

<220>
<223> Synthetic Oligonucleotide

<400> 1022
tggagtggca agtatttgat gct 23

<210> 1023
<211> 23
<212> DNA
<213> Artificial Sequence

<220>
<223> Synthetic Oligonucleotide

<400> 1023
tggccaacct gcctgtttcc agc 23

<210> 1024
<211> 19
<212> DNA
<213> Artificial Sequence

<220>
<223> Synthetic Oligonucleotide

<400> 1024
tgtcaccccg actcaacgt                                                    19

<210> 1025
<211> 22
<212> DNA
<213> Artificial Sequence

<220>
<223> Synthetic Oligonucleotide

<400> 1025
acgtggactg agatgcattc ac                                                22

<210> 1026
<211> 21
<212> DNA
<213> Artificial Sequence

<220>
<223> Synthetic Oligonucleotide

<400> 1026
agacgcaccg cccggactca c                                                 21

<210> 1027
<211> 20
<212> DNA
<213> Artificial Sequence

<220>
<223> Synthetic Oligonucleotide

<400> 1027
agctcaacat gcgtgagtgt                                                   20

<210> 1028
<211> 19
<212> DNA
<213> Artificial Sequence

<220>
<223> Synthetic Oligonucleotide

<400> 1028
attgccgatc tggactcct                                                    19

<210> 1029
<211> 23
<212> DNA
<213> Artificial Sequence

<220>
<223> Synthetic Oligonucleotide

<400> 1029
atctctatcc acgtggggca ggc                                              23

<210> 1030
<211> 23
<212> DNA
<213> Artificial Sequence

<220>
<223> Synthetic Oligonucleotide

<400> 1030
ctcttacatc gaccgcctaa gag                                              23

<210> 1031
<211> 18
<212> DNA
<213> Artificial Sequence

<220>
<223> Synthetic Oligonucleotide

<400> 1031
gctgatggcg gagacgaa                                                    18

<210> 1032
<211> 27
<212> DNA
<213> Artificial Sequence

<220>
<223> Synthetic Oligonucleotide

<400> 1032
cgcgctgtaa gaagcaacaa cctctcc                                          27

<210> 1033
<211> 20
<212> DNA
<213> Artificial Sequence

<220>
<223> Synthetic Oligonucleotide

<400> 1033
gaggagggtg agttctccaa                                                  20

<210> 1034
<211> 20
<212> DNA
<213> Artificial Sequence

<220>
<223> Synthetic Oligonucleotide

<400> 1034
atgcccacct ccttgtaatc                                                  20

<210> 1035
<211> 24
<212> DNA
<213> Artificial Sequence

<220>
<223> Synthetic Oligonucleotide

<400> 1035
ccatgaggat atgactgccc tgga                                              24

<210> 1036
<211> 19
<212> DNA
<213> Artificial Sequence

<220>
<223> Synthetic Oligonucleotide

<400> 1036
gtcccttcgc ctccttcac                                                    19

<210> 1037
<211> 20
<212> DNA
<213> Artificial Sequence

<220>
<223> Synthetic Oligonucleotide

<400> 1037
cgtggatgga gatgcactca                                                   20

<210> 1038
<211> 28
<212> DNA
<213> Artificial Sequence

<220>
<223> Synthetic Oligonucleotide

<400> 1038
ccgcagaccc cttcaagttc tagtcatg                                          28

<210> 1039
<211> 20
<212> DNA
<213> Artificial Sequence

<220>
<223> Synthetic Oligonucleotide

<400> 1039
cgccctacct ataccaacct                                                   20

<210> 1040
<211> 20
<212> DNA
<213> Artificial Sequence

<220>
<223> Synthetic Oligonucleotide

<400> 1040
cggagagaag cagtgattga                                                   20

<210> 1041

<211> 25
<212> DNA
<213> Artificial Sequence

<220>
<223> Synthetic Oligonucleotide

<400> 1041
caaccgcctc atcagtcaga ttgtg                                             25

<210> 1042
<211> 20
<212> DNA
<213> Artificial Sequence

<220>
<223> Synthetic Oligonucleotide

<400> 1042
cgaggacgag gcttaaaaac                                                   20

<210> 1043
<211> 20
<212> DNA
<213> Artificial Sequence

<220>
<223> Synthetic Oligonucleotide

<400> 1043
accatgcttg aggacaacag                                                   20

<210> 1044
<211> 29
<212> DNA
<213> Artificial Sequence

<220>
<223> Synthetic Oligonucleotide

<400> 1044
tctcagatca atcgtgcatc cttagtgaa                                         29

<210> 1045
<211> 21
<212> DNA
<213> Artificial Sequence

<220>
<223> Synthetic Oligonucleotide

<400> 1045
cgccctcctg cagtatttat g                                                 21

<210> 1046
<211> 22
<212> DNA
<213> Artificial Sequence

<220>
<223> Synthetic Oligonucleotide

<400> 1046

acagagacag gagcagctca ca                                              22

<210> 1047
<211> 23
<212> DNA
<213> Artificial Sequence

<220>
<223> Synthetic Oligonucleotide

<400> 1047
cctcgtcctc cccacctagg cca                                             23

<210> 1048
<211> 21
<212> DNA
<213> Artificial Sequence

<220>
<223> Synthetic Oligonucleotide

<400> 1048
acactgactg gcatcctgct t                                               21

<210> 1049
<211> 25
<212> DNA
<213> Artificial Sequence

<220>
<223> Synthetic Oligonucleotide

<400> 1049
gctctgtagc tccccatgta ctagt                                           25

<210> 1050
<211> 24
<212> DNA
<213> Artificial Sequence

<220>
<223> Synthetic Oligonucleotide

<400> 1050
agcctccaga agagccaggt gcct                                            24

<210> 1051
<211> 20
<212> DNA
<213> Artificial Sequence

<220>
<223> Synthetic Oligonucleotide

<400> 1051
gtggcctaga gccttcagtc                                                 20

<210> 1052
<211> 21
<212> DNA
<213> Artificial Sequence

<220>

<223> Synthetic Oligonucleotide

<400> 1052
caggctggga gtgaataaag a                                                21

<210> 1053
<211> 24
<212> DNA
<213> Artificial Sequence

<220>
<223> Synthetic Oligonucleotide

<400> 1053
ttcacactgc ttccctgctt tccc                                             24

<210> 1054
<211> 18
<212> DNA
<213> Artificial Sequence

<220>
<223> Synthetic Oligonucleotide

<400> 1054
acaggcccca tgcatcct                                                    18

<210> 1055
<211> 25
<212> DNA
<213> Artificial Sequence

<220>
<223> Synthetic Oligonucleotide

<400> 1055
tgtttctctc ccagataagc taagg                                            25

<210> 1056
<211> 21
<212> DNA
<213> Artificial Sequence

<220>
<223> Synthetic Oligonucleotide

<400> 1056
tgcctcactc ccctcagccc c                                                21

<210> 1057
<211> 19
<212> DNA
<213> Artificial Sequence

<220>
<223> Synthetic Oligonucleotide

<400> 1057
ccctatggcc ctgaatggt                                                   19

<210> 1058
<211> 26
<212> DNA

<213> Artificial Sequence

<220>
<223> Synthetic Oligonucleotide

<400> 1058
actaattaca tgacttggct gcattt                                            26

<210> 1059
<211> 23
<212> DNA
<213> Artificial Sequence

<220>
<223> Synthetic Oligonucleotide

<400> 1059
tgagggccg acaccaacac aat                                                23

<210> 1060
<211> 20
<212> DNA
<213> Artificial Sequence

<220>
<223> Synthetic Oligonucleotide

<400> 1060
agtggaatcc ttccctttcc                                                   20

<210> 1061
<211> 20
<212> DNA
<213> Artificial Sequence

<220>
<223> Synthetic Oligonucleotide

<400> 1061
cccttgatcc ctttctctga                                                   20

<210> 1062
<211> 24
<212> DNA
<213> Artificial Sequence

<220>
<223> Synthetic Oligonucleotide

<400> 1062
cctcactcag ctcctttccc ctga                                              24

<210> 1063
<211> 24
<212> DNA
<213> Artificial Sequence

<220>
<223> Synthetic Oligonucleotide

<400> 1063
ggaaggaaag aagcatggtc tact                                              24

<210> 1064
<211> 24
<212> DNA
<213> Artificial Sequence

<220>
<223> Synthetic Oligonucleotide

<400> 1064
aaaaagtgac aggcaacagt gaag                                              24

<210> 1065
<211> 24
<212> DNA
<213> Artificial Sequence

<220>
<223> Synthetic Oligonucleotide

<400> 1065
caccagagac ccagcgcaca ccta                                              24

<210> 1066
<211> 19
<212> DNA
<213> Artificial Sequence

<220>
<223> Synthetic Oligonucleotide

<400> 1066
gatgccgagg gaaatcatc                                                    19

<210> 1067
<211> 20
<212> DNA
<213> Artificial Sequence

<220>
<223> Synthetic Oligonucleotide

<400> 1067
ccagaactcg aacccaatct                                                   20

<210> 1068
<211> 24
<212> DNA
<213> Artificial Sequence

<220>
<223> Synthetic Oligonucleotide

<400> 1068
attgccgcac tggcccaact gtag                                              24

<210> 1069
<211> 18
<212> DNA
<213> Artificial Sequence

<220>
<223> Synthetic Oligonucleotide

<400> 1069
gcgctgcgga agatcatc                                                    18

<210> 1070
<211> 22
<212> DNA
<213> Artificial Sequence

<220>
<223> Synthetic Oligonucleotide

<400> 1070
gcttgagggt ctgaatcttg ct                                               22

<210> 1071
<211> 21
<212> DNA
<213> Artificial Sequence

<220>
<223> Synthetic Oligonucleotide

<400> 1071
ccacgctgcc ctcggacaag c                                                21

<210> 1072
<211> 18
<212> DNA
<213> Artificial Sequence

<220>
<223> Synthetic Oligonucleotide

<400> 1072
cagtgtggag ggatgga                                                     18

<210> 1073
<211> 19
<212> DNA
<213> Artificial Sequence

<220>
<223> Synthetic Oligonucleotide

<400> 1073
caagttctgg accacagcc                                                   19

<210> 1074
<211> 24
<212> DNA
<213> Artificial Sequence

<220>
<223> Synthetic Oligonucleotide

<400> 1074
cttcacccac tggatgtcag gctc                                             24

<210> 1075
<211> 23
<212> DNA
<213> Artificial Sequence

<220>
<223> Synthetic Oligonucleotide

<400> 1075
agttgtcgtg tgttctggat tca                                               23

<210> 1076
<211> 22
<212> DNA
<213> Artificial Sequence

<220>
<223> Synthetic Oligonucleotide

<400> 1076
cgtcagcgtg atcttaaagg aa                                                22

<210> 1077
<211> 22
<212> DNA
<213> Artificial Sequence

<220>
<223> Synthetic Oligonucleotide

<400> 1077
tccggcacca ccatgtcgaa gg                                                22

<210> 1078
<211> 19
<212> DNA
<213> Artificial Sequence

<220>
<223> Synthetic Oligonucleotide

<400> 1078
gtggatgtgc cctgaagga                                                    19

<210> 1079
<211> 20
<212> DNA
<213> Artificial Sequence

<220>
<223> Synthetic Oligonucleotide

<400> 1079
ctgcggatcc agggtaagaa                                                   20

<210> 1080
<211> 28
<212> DNA
<213> Artificial Sequence

<220>
<223> Synthetic Oligonucleotide

<400> 1080
aagccaggcg tctacacgag agtctcac                                          28

<210> 1081
<211> 24

<212> DNA
<213> Artificial Sequence

<220>
<223> Synthetic Oligonucleotide

<400> 1081
ggttgtgctc tacgagctta tgac                                              24

<210> 1082
<211> 22
<212> DNA
<213> Artificial Sequence

<220>
<223> Synthetic Oligonucleotide

<400> 1082
cggcccacca taaagataat ct                                                22

<210> 1083
<211> 24
<212> DNA
<213> Artificial Sequence

<220>
<223> Synthetic Oligonucleotide

<400> 1083
tgccttacag ccacattggc tgcc                                              24

<210> 1084
<211> 21
<212> DNA
<213> Artificial Sequence

<220>
<223> Synthetic Oligonucleotide

<400> 1084
tggcttcagg agctgaatac c                                                 21

<210> 1085
<211> 24
<212> DNA
<213> Artificial Sequence

<220>
<223> Synthetic Oligonucleotide

<400> 1085
tgctgtcgtg atgagaaaat agtg                                              24

<210> 1086
<211> 26
<212> DNA
<213> Artificial Sequence

<220>
<223> Synthetic Oligonucleotide

<400> 1086
caggcacaca caggtgggac acaaat                                            26

<210> 1087
<211> 20
<212> DNA
<213> Artificial Sequence

<220>
<223> Synthetic Oligonucleotide

<400> 1087
ctgctgtctt gggtgcattg                                                   20

<210> 1088
<211> 18
<212> DNA
<213> Artificial Sequence

<220>
<223> Synthetic Oligonucleotide

<400> 1088
gcagcctggg accacttg                                                     18

<210> 1089
<211> 25
<212> DNA
<213> Artificial Sequence

<220>
<223> Synthetic Oligonucleotide

<400> 1089
ttgccttgct gctctacctc cacca                                             25

<210> 1090
<211> 19
<212> DNA
<213> Artificial Sequence

<220>
<223> Synthetic Oligonucleotide

<400> 1090
tgacgatggc ctggagtgt                                                    19

<210> 1091
<211> 22
<212> DNA
<213> Artificial Sequence

<220>
<223> Synthetic Oligonucleotide

<400> 1091
ggtaccggat catgaggatc tg                                                22

<210> 1092
<211> 21
<212> DNA
<213> Artificial Sequence

<220>
<223> Synthetic Oligonucleotide

<400> 1092
ctgggcagca ccaagtccgg a                                                21

<210> 1093
<211> 25
<212> DNA
<213> Artificial Sequence

<220>
<223> Synthetic Oligonucleotide

<400> 1093
cctcagcaag acgttatttg aaatt                                            25

<210> 1094
<211> 24
<212> DNA
<213> Artificial Sequence

<220>
<223> Synthetic Oligonucleotide

<400> 1094
aagtgtgatt ggcaaaactg attg                                             24

<210> 1095
<211> 26
<212> DNA
<213> Artificial Sequence

<220>
<223> Synthetic Oligonucleotide

<400> 1095
cctctctctc aaggccccaa accagt                                           26

<210> 1096
<211> 19
<212> DNA
<213> Artificial Sequence

<220>
<223> Synthetic Oligonucleotide

<400> 1096
cggttggtga cttgtctgc                                                   19

<210> 1097
<211> 20
<212> DNA
<213> Artificial Sequence

<220>
<223> Synthetic Oligonucleotide

<400> 1097
aagacttgtc cctgcctcac                                                  20

<210> 1098
<211> 24
<212> DNA
<213> Artificial Sequence

<220>
<223> Synthetic Oligonucleotide

<400> 1098
atgcctcagt cttcccaaag cagg                                              24

<210> 1099
<211> 21
<212> DNA
<213> Artificial Sequence

<220>
<223> Synthetic Oligonucleotide

<400> 1099
tgcccttaaa ggaaccaatg a                                                 21

<210> 1100
<211> 22
<212> DNA
<213> Artificial Sequence

<220>
<223> Synthetic Oligonucleotide

<400> 1100
gcttcaacgg caaagttctc tt                                                22

<210> 1101
<211> 22
<212> DNA
<213> Artificial Sequence

<220>
<223> Synthetic Oligonucleotide

<400> 1101
atttcacgca tctggcgttc ca                                                22

<210> 1102
<211> 18
<212> DNA
<213> Artificial Sequence

<220>
<223> Synthetic Oligonucleotide

<400> 1102
ctctccagtg tgggcacc                                                     18

<210> 1103
<211> 19
<212> DNA
<213> Artificial Sequence

<220>
<223> Synthetic Oligonucleotide

<400> 1103
gcggtgtagc tcccagagt                                                    19

<210> 1104

<211> 24
<212> DNA
<213> Artificial Sequence

<220>
<223> Synthetic Oligonucleotide

<400> 1104
ccagaacaga tgcgagcagt ccat                                              24

<210> 1105
<211> 25
<212> DNA
<213> Artificial Sequence

<220>
<223> Synthetic Oligonucleotide

<400> 1105
gtatcaggac cacatgcagt acatc                                             25

<210> 1106
<211> 22
<212> DNA
<213> Artificial Sequence

<220>
<223> Synthetic Oligonucleotide

<400> 1106
tgtcggaatt gatactggca tt                                                22

<210> 1107
<211> 24
<212> DNA
<213> Artificial Sequence

<220>
<223> Synthetic Oligonucleotide

<400> 1107
ttgatgcctg tcttcgcgcc ttct                                              24

<210> 1108
<211> 23
<212> DNA
<213> Artificial Sequence

<220>
<223> Synthetic Oligonucleotide

<400> 1108
gcagttggaa gacacaggaa agt                                               23

<210> 1109
<211> 21
<212> DNA
<213> Artificial Sequence

<220>
<223> Synthetic Oligonucleotide

<400> 1109

tgcgtggcac tattttcaag a                                                21

<210> 1110
<211> 27
<212> DNA
<213> Artificial Sequence

<220>
<223> Synthetic Oligonucleotide

<400> 1110
tccccaaatt gcagatttat caacggc                                          27

<210> 1111
<211> 20
<212> DNA
<213> Artificial Sequence

<220>
<223> Synthetic Oligonucleotide

<400> 1111
caggtcaggc agaggaagtc                                                  20

<210> 1112
<211> 20
<212> DNA
<213> Artificial Sequence

<220>
<223> Synthetic Oligonucleotide

<400> 1112
cctaacaagc cccaaatcaa                                                  20

<210> 1113
<211> 27
<212> DNA
<213> Artificial Sequence

<220>
<223> Synthetic Oligonucleotide

<400> 1113
tgcattgcat gagctaaacc tatctga                                          27

<210> 1114
<211> 20
<212> DNA
<213> Artificial Sequence

<220>
<223> Synthetic Oligonucleotide

<400> 1114
tggtcagatg ctgctgaagt                                                  20

<210> 1115
<211> 20
<212> DNA
<213> Artificial Sequence

<220>

<223> Synthetic Oligonucleotide

<400> 1115
atcacagcat gaagggatgg                                                    20

<210> 1116
<211> 24
<212> DNA
<213> Artificial Sequence

<220>
<223> Synthetic Oligonucleotide

<400> 1116
tatccttagg ccgctggcat cttg                                               24

<210> 1117
<211> 19
<212> DNA
<213> Artificial Sequence

<220>
<223> Synthetic Oligonucleotide

<400> 1117
gagggagcag acagtgggt                                                     19

<210> 1118
<211> 21
<212> DNA
<213> Artificial Sequence

<220>
<223> Synthetic Oligonucleotide

<400> 1118
tcagagccct tgctaagtca c                                                  21

<210> 1119
<211> 24
<212> DNA
<213> Artificial Sequence

<220>
<223> Synthetic Oligonucleotide

<400> 1119
ccacgatctc cgtaaccatt tgca                                               24

<210> 1120
<211> 20
<212> DNA
<213> Artificial Sequence

<220>
<223> Synthetic Oligonucleotide

<400> 1120
tagaggccat caaaattggc                                                    20

<210> 1121
<211> 18
<212> DNA

<213> Artificial Sequence

<220>
<223> Synthetic Oligonucleotide

<400> 1121
tccgtttcct ctgggctt                                                    18

<210> 1122
<211> 24
<212> DNA
<213> Artificial Sequence

<220>
<223> Synthetic Oligonucleotide

<400> 1122
accaaggccc tgactcagat ggag                                             24

<210> 1123
<211> 82
<212> DNA
<213> Artificial Sequence

<220>
<223> Synthetic Oligonucleotide

<400> 1123
ttacccgtgg gaactgtctc caaatacata cttcctctca ccaggacaac aaccacagga 60
tcctctgacc catttactgg tc                                               82

<210> 1124
<211> 77
<212> DNA
<213> Artificial Sequence

<220>
<223> Synthetic Oligonucleotide

<400> 1124
aaacaccact ggagcattga ctaccaggct cgccaatgat gctgctcaag ttaaaggggc 60
tataggttcc aggcttg                                                     77

<210> 1125
<211> 67
<212> DNA
<213> Artificial Sequence

<220>
<223> Synthetic Oligonucleotide

<400> 1125
agacagtcgc cttggtcggt ctcaatggca gtgggaagag tacggtagtc cagcttctgc 60
agaggtt                                                                67

<210> 1126
<211> 68
<212> DNA
<213> Artificial Sequence

<220>
<223> Synthetic Oligonucleotide

<400> 1126
accagtgcca caatgcagtg gctggacatt cggctacagc tcatgggggc ggcagtggtc 60
agcgctat 68

<210> 1127
<211> 66
<212> DNA
<213> Artificial Sequence

<220>
<223> Synthetic Oligonucleotide

<400> 1127
aagccacagc ctccattgac atggagacag acaccctgat ccagcgcaca atccgtgaag 60
ccttcc 66

<210> 1128
<211> 76
<212> DNA
<213> Artificial Sequence

<220>
<223> Synthetic Oligonucleotide

<400> 1128
tgcagactgt accatgctga ccattgccca tcgcctgcac acggttctag gctccgatag 60
gattatggtg ctggcc 76

<210> 1129
<211> 67
<212> DNA
<213> Artificial Sequence

<220>
<223> Synthetic Oligonucleotide

<400> 1129
tctgtggccc acctctactc caacctgacc aagccactcc tggacgtggc tgtgacttcc 60
tacaccc 67

<210> 1130
<211> 83
<212> DNA
<213> Artificial Sequence

<220>
<223> Synthetic Oligonucleotide

<400> 1130
atgtacgtcg ccattcaagc tgtgctctcc ctctatgcct ctggccgcac gacaggcatc 60
gtcctggatt caggtgatgg cgt 83

<210> 1131
<211> 66
<212> DNA
<213> Artificial Sequence

<220>
<223> Synthetic Oligonucleotide

<400> 1131
atccgcattg aagacccacc ccgcagaaag cacatggtat tcctgggtgg tgcagttcta 60
gcggat 66

<210> 1132
<211> 74
<212> DNA
<213> Artificial Sequence

<220>
<223> Synthetic Oligonucleotide

<400> 1132
caactgctga gagaccgaga agtaggaatc cctccagaac aatccttgga aactgctaag 60
gcagtaaagg agcg 74

<210> 1133
<211> 78
<212> DNA
<213> Artificial Sequence

<220>
<223> Synthetic Oligonucleotide

<400> 1133
ctgcatgtga ttgaataaga aacaagaaag tgaccacacc aaagcctccc tggctggtgt 60
acagggatca ggtccaca 78

<210> 1134
<211> 71
<212> DNA
<213> Artificial Sequence

<220>
<223> Synthetic Oligonucleotide

<400> 1134
cgcttctatg gcgctgagat tgtgtcagcc ctggactacc tgcactcgga gaagaacgtg 60
gtgtaccggg a 71

<210> 1135
<211> 71
<212> DNA
<213> Artificial Sequence

<220>
<223> Synthetic Oligonucleotide

<400> 1135
tcctgccacc cttcaaacct caggtcacgt ccgaggtcga cacaaggtac ttcgatgatg 60
aatttaccgc c 71

<210> 1136
<211> 75
<212> DNA
<213> Artificial Sequence

<220>
<223> Synthetic Oligonucleotide

<400> 1136
ttgtctctgc cttggactat ctacattccg gaaagattgt gtaccgtgat ctcaagttgg 60
agaatctaat gctgg 75

<210> 1137
<211> 67

<212> DNA
<213> Artificial Sequence

<220>
<223> Synthetic Oligonucleotide

<400> 1137
tgggagggat gaaggaaatt atctggacga tgctctcgtg agacaggatg cccaggacct 60
gtatgag 67

<210> 1138
<211> 69
<212> DNA
<213> Artificial Sequence

<220>
<223> Synthetic Oligonucleotide

<400> 1138
ggacagcagg aatgtgtttc tccatacagg tcacggggag ccaatggttc agaaacaaat 60
cgagtgggt 69

<210> 1139
<211> 68
<212> DNA
<213> Artificial Sequence

<220>
<223> Synthetic Oligonucleotide

<400> 1139
gatgaagcct ttcgcaagtt cctgaagggc ctggcttccc gaaagcccct tgtgctgtgt 60
ggagacct 68

<210> 1140
<211> 74
<212> DNA
<213> Artificial Sequence

<220>
<223> Synthetic Oligonucleotide

<400> 1140
ggaaacacac tggaggacaa ggctcgggaa ctcatcagcc gcatcaaaca gagtgaactt 60
tctgccaaga tgcg 74

<210> 1141
<211> 67
<212> DNA
<213> Artificial Sequence

<220>
<223> Synthetic Oligonucleotide

<400> 1141
gtttatgcca tcggcaccgt actggatcct ggccaccgac tatgagaact atgccctcgt 60
gtattcc 67

<210> 1142
<211> 75
<212> DNA
<213> Artificial Sequence

<220>
<223> Synthetic Oligonucleotide

<400> 1142
gcctcaagag ctggttcgag cccctggtgg aagacatgca gcgccagtgg gccgggctgg 60
tggagaaggt gcagg 75

<210> 1143
<211> 66
<212> DNA
<213> Artificial Sequence

<220>
<223> Synthetic Oligonucleotide

<400> 1143
gaggtcctgg agtgcgtgag cctggtggag ctgacctcgc ttaagggcag ggagaagctg 60
gcacct 66

<210> 1144
<211> 73
<212> DNA
<213> Artificial Sequence

<220>
<223> Synthetic Oligonucleotide

<400> 1144
ggtcctccgt cggttctctc attagtccac ggtctggtct tcagctaccc gccttcgtct 60
ccgagtttgc gac 73

<210> 1145
<211> 67
<212> DNA
<213> Artificial Sequence

<220>
<223> Synthetic Oligonucleotide

<400> 1145
agctgcagaa gagctgcaca tttgacgagc agcgaacagc cacgatcatg gaggagttgg 60
cagatgc 67

<210> 1146
<211> 66
<212> DNA
<213> Artificial Sequence

<220>
<223> Synthetic Oligonucleotide

<400> 1146
cagcagatgt ggatcagcaa gcaggagtat gacgagtccg gcccctccat cgtccaccgc 60
aaatgc 66

<210> 1147
<211> 73
<212> DNA
<213> Artificial Sequence

<220>
<223> Synthetic Oligonucleotide

<400> 1147
gggtcaggtg cctcgagatc gggcttgggc ccagagcatg ttccagatcc cagagtttga 60
gccgagtgag cag 73

<210> 1148
<211> 81
<212> DNA
<213> Artificial Sequence

<220>
<223> Synthetic Oligonucleotide

<400> 1148
cgttgtcagc acttggaata caagatggtt gccgggtcat gttaattggg aaaaagaaca 60
gtccacagga agaggttgaa c 81

<210> 1149
<211> 66
<212> DNA
<213> Artificial Sequence

<220>
<223> Synthetic Oligonucleotide

<400> 1149
ccattcccac cattctacct gaggccagga cgtctggggt gtggggattg gtgggtctat 60
gttccc 66

<210> 1150
<211> 70
<212> DNA
<213> Artificial Sequence

<220>
<223> Synthetic Oligonucleotide

<400> 1150
ccgccgtgga cacagactcc ccccgagagg tctttttccg agtggcagct gacatgtttt 60
ctgacggcaa 70

<210> 1151
<211> 83
<212> DNA
<213> Artificial Sequence

<220>
<223> Synthetic Oligonucleotide

<400> 1151
cctggagggt cctgtacaat ctcatcatgg gactcctgcc cttacccagg ggccacagag 60
cccccgagat ggagcccaat tag 83

<210> 1152
<211> 80
<212> DNA
<213> Artificial Sequence

<220>
<223> Synthetic Oligonucleotide

<400> 1152
ggctcttgtg cgtactgtcc ttcgggctgg tgacagggaa gacatcactg agcctgccat 60
ctgtgctctt cgtcatctga 80

<210> 1153
<211> 73
<212> DNA
<213> Artificial Sequence

<220>
<223> Synthetic Oligonucleotide

<400> 1153
cagatggacc tagtacccac tgagatttcc acgccgaagg acagcgatgg gaaaaatgcc 60
cttaaatcat agg 73

<210> 1154
<211> 69
<212> DNA
<213> Artificial Sequence

<220>
<223> Synthetic Oligonucleotide

<400> 1154
aattaccaag cagccgaaga ccacccacga atggttatct tacgactgtt acgttacatt 60
gtccgcctg 69

<210> 1155
<211> 70
<212> DNA
<213> Artificial Sequence

<220>
<223> Synthetic Oligonucleotide

<400> 1155
cagcgacaac tctggacaag tcagtccccc agagtctcca actgtgacca cttcctggca 60
gtctgagagc 70

<210> 1156
<211> 70
<212> DNA
<213> Artificial Sequence

<220>
<223> Synthetic Oligonucleotide

<400> 1156
cttttgtgga actctatggg aacaatgcag cagccgagag ccgaaagggc caggaacgct 60
tcaaccgctg 70

<210> 1157
<211> 74
<212> DNA
<213> Artificial Sequence

<220>
<223> Synthetic Oligonucleotide

<400> 1157
tgtactggcg aagaatattt ggtaaagcag ggcatcgatc tctcaccctg gggcttgtgg 60
aagaatcacg tggc 74

<210> 1158
<211> 71

<212> DNA
<213> Artificial Sequence

<220>
<223> Synthetic Oligonucleotide

<400> 1158
ggactgtgag gtcaacaacg gttccagcct cagggatgag tgcatcacaa acctactggt 60
gtttggcttc c 71

<210> 1159
<211> 76
<212> DNA
<213> Artificial Sequence

<220>
<223> Synthetic Oligonucleotide

<400> 1159
cctgcaaaag ggaacaagag cccttcgcct ccagatggct cccctgccgc cacccccgag 60
atcagagtca accacg 76

<210> 1160
<211> 65
<212> DNA
<213> Artificial Sequence

<220>
<223> Synthetic Oligonucleotide

<400> 1160
tcaggggcct agaaatctgt tgctatgggc ccttcaccaa catgcccaca gatcaactgg 60
aatgg 65

<210> 1161
<211> 70
<212> DNA
<213> Artificial Sequence

<220>
<223> Synthetic Oligonucleotide

<400> 1161
agttcgtgct ttgcaagatg gtgcagagct ttatgaagca gtgaagaatg cagcagaccc 60
agcttacctt 70

<210> 1162
<211> 68
<212> DNA
<213> Artificial Sequence

<220>
<223> Synthetic Oligonucleotide

<400> 1162
ccgaggttaa tccagcacgt atggggccaa gtgtaggctc ccagcaggaa ctgagagcgc 60
catgtctt 68

<210> 1163
<211> 82
<212> DNA
<213> Artificial Sequence

<220>
<223> Synthetic Oligonucleotide

<400> 1163
tcaacagaag gctgaaccac tagaaagact acagtcccag caccgacaat tccaagctcg 60
agtgtctcgg caaactctgt tg 82

<210> 1164
<211> 73
<212> DNA
<213> Artificial Sequence

<220>
<223> Synthetic Oligonucleotide

<400> 1164
ctgaagcaga tggttcatca tttcctgggc tgttaaacaa agcgaggtta aggttagact 60
cttgggaatc agc 73

<210> 1165
<211> 69
<212> DNA
<213> Artificial Sequence

<220>
<223> Synthetic Oligonucleotide

<400> 1165
gtcagcgtgg tagcggtatt ctccgcggca gtgacagtaa ttgtttttgc ctctttagcc 60
aagacttcc 69

<210> 1166
<211> 65
<212> DNA
<213> Artificial Sequence

<220>
<223> Synthetic Oligonucleotide

<400> 1166
tcagctgtga gctgcggata ccgcccggca atgggacctg ctcttaacct caaacctagg 60
accgt 65

<210> 1167
<211> 72
<212> DNA
<213> Artificial Sequence

<220>
<223> Synthetic Oligonucleotide

<400> 1167
atcctagccc tggtttttgg cctccttttt gctgtcacca gcgtcgcgtt ccttgtgcag 60
atgagaaggc ag 72

<210> 1168
<211> 78
<212> DNA
<213> Artificial Sequence

<220>
<223> Synthetic Oligonucleotide

<400> 1168
cactaaggtt tgagacagtt ccagaaagaa cccaagctca agacgcagga cgagctcagt 60
tgtagagggc taattcgc 78

<210> 1169
<211> 66
<212> DNA
<213> Artificial Sequence

<220>
<223> Synthetic Oligonucleotide

<400> 1169
tcgttggaga tcagagtgga agttcaccat cacaccacct acagcccagg tggttggcgt 60
gcttaa 66

<210> 1170
<211> 74
<212> DNA
<213> Artificial Sequence

<220>
<223> Synthetic Oligonucleotide

<400> 1170
gtggctcaac attgtgttcc catttcagct gatcagtggg cctccaagga ggggctgtaa 60
aatggaggcc attg 74

<210> 1171
<211> 84
<212> DNA
<213> Artificial Sequence

<220>
<223> Synthetic Oligonucleotide

<400> 1171
ttcaggttgt tgcaggagac catgtacatg actgtctcca ttattgatcg gttcatgcag 60
aataattgtg tgcccaagaa gatg 84

<210> 1172
<211> 69
<212> DNA
<213> Artificial Sequence

<220>
<223> Synthetic Oligonucleotide

<400> 1172
gcatgttcgt ggcctctaag atgaaggaga ccatccccct gacggccgag aagctgtgca 60
tctacaccg 69

<210> 1173
<211> 82
<212> DNA
<213> Artificial Sequence

<220>
<223> Synthetic Oligonucleotide

<400> 1173
atgctgtggc tccttcctaa ctggggcttt cttgacatgt aggttgcttg gtaataacct 60
ttttgtatat cacaatttgg gt 82

<210> 1174
<211> 69
<212> DNA
<213> Artificial Sequence

<220>
<223> Synthetic Oligonucleotide

<400> 1174
atccaaggcc atgactggtg tggaacaatg gccatacagg gctgttgccc aggccctaga 60
ggtcattcc 69

<210> 1175
<211> 66
<212> DNA
<213> Artificial Sequence

<220>
<223> Synthetic Oligonucleotide

<400> 1175
gtgtgctagc gtactcccgc ctcaaggaac tgacgctcga ggacctaaag ataaccggca 60
ccatgc 66

<210> 1176
<211> 75
<212> DNA
<213> Artificial Sequence

<220>
<223> Synthetic Oligonucleotide

<400> 1176
tgtatttcaa gacctctgtg cacttattta tgaacctgcc ctgctcccac agaacacagc 60
aattcctcag gctaa 75

<210> 1177
<211> 65
<212> DNA
<213> Artificial Sequence

<220>
<223> Synthetic Oligonucleotide

<400> 1177
agatgaagtg gaaggcgctt ttcaccgcgg ccatcctgca ggcacagttg ccgattacag 60
aggca 65

<210> 1178
<211> 71
<212> DNA
<213> Artificial Sequence

<220>
<223> Synthetic Oligonucleotide

<400> 1178
agtgggactg attgccgtgg gtgtcggggc acagcttgtc ctgagtcaga ccataatcca 60
gggggctacc c 71

<210> 1179
<211> 74

<212> DNA
<213> Artificial Sequence

<220>
<223> Synthetic Oligonucleotide

<400> 1179
tggttcccag ccctgtgtcc acctccaagc ccagattcag attcgagtca tgtacacaac 60
ccagggtgga ggag 74

<210> 1180
<211> 68
<212> DNA
<213> Artificial Sequence

<220>
<223> Synthetic Oligonucleotide

<400> 1180
gagagcgacg cggttgttgt agctgccgct gcggccgccg cggaataata agccgggatc 60
taccatac 68

<210> 1181
<211> 68
<212> DNA
<213> Artificial Sequence

<220>
<223> Synthetic Oligonucleotide

<400> 1181
tggattggag ttctgggaat gtactggccg tggcactgga caacagtgtg tacctgtgga 60
gtgcaagc 68

<210> 1182
<211> 85
<212> DNA
<213> Artificial Sequence

<220>
<223> Synthetic Oligonucleotide

<400> 1182
aaacgagcag tttgccatca gacgcttcca gtctatgccg gtgaggctgc tgggccacag 60
ccccgtgctt cggaacatca ccaac 85

<210> 1183
<211> 67
<212> DNA
<213> Artificial Sequence

<220>
<223> Synthetic Oligonucleotide

<400> 1183
gaggcacagt attgcccagc tggatccaga ggccttggga aacattaaga agctctccaa 60
ccgtctc 67

<210> 1184
<211> 81
<212> DNA
<213> Artificial Sequence

<220>
<223> Synthetic Oligonucleotide

<400> 1184
tgagtgtccc ccggtatctt ccccgccctg ccaatcccga tgaaattgga aattttattg 60
atgaaaatct gaaagcggct g 81

<210> 1185
<211> 67
<212> DNA
<213> Artificial Sequence

<220>
<223> Synthetic Oligonucleotide

<400> 1185
aagccctatc cgatgtaccc ggccacaaca tccctggtga acgtcgtgcc caaactcaat 60
gccacag 67

<210> 1186
<211> 69
<212> DNA
<213> Artificial Sequence

<220>
<223> Synthetic Oligonucleotide

<400> 1186
cggcgatcaa gaagctgtcc gggcctctga tctccgattt cttcgccaag cgcaagagat 60
cagcgcctg 69

<210> 1187
<211> 77
<212> DNA
<213> Artificial Sequence

<220>
<223> Synthetic Oligonucleotide

<400> 1187
tgacaatcag cacacctgca ttcaccgctc ggaagagggc ctgagctgca tgaataagga 60
tcacggctgt agtcaca 77

<210> 1188
<211> 63
<212> DNA
<213> Artificial Sequence

<220>
<223> Synthetic Oligonucleotide

<400> 1188
taaattcact cgtggtgtgg acttcaattg gcaagcccag gccctattgg ccctacaaga 60
ggc 63

<210> 1189
<211> 83
<212> DNA
<213> Artificial Sequence

<220>
<223> Synthetic Oligonucleotide

<400> 1189
ggatgctggt gacctcttct tccctcacgt tgcaacagga attaaaggct aaaagaaaac 60
gaagagttac ttggtgcctt ggc 83

<210> 1190
<211> 68
<212> DNA
<213> Artificial Sequence

<220>
<223> Synthetic Oligonucleotide

<400> 1190
ctcccgtcaa cagcgttctt tccaaacact ggaccaggag tgcatccaga tgaaggccag 60
actcaccc 68

<210> 1191
<211> 76
<212> DNA
<213> Artificial Sequence

<220>
<223> Synthetic Oligonucleotide

<400> 1191
ctgaaggagc tccaagacct cgctctccaa ggcgccaagg agagggcaca tcagcagaag 60
aaacacagcg gttttg 76

<210> 1192
<211> 76
<212> DNA
<213> Artificial Sequence

<220>
<223> Synthetic Oligonucleotide

<400> 1192
aaggaagtgg tccctctgtg gcaagtgatg aagtctccag ctttgcctca gctctcccag 60
acagaaagac tgcgtc 76

<210> 1193
<211> 82
<212> DNA
<213> Artificial Sequence

<220>
<223> Synthetic Oligonucleotide

<400> 1193
gataaattgg tacaagggat cagcttttcc cagcccacat gtcctgatca tatgcttttg 60
aatagtcagt tacttggcac cc 82

<210> 1194
<211> 78
<212> DNA
<213> Artificial Sequence

<220>
<223> Synthetic Oligonucleotide

<400> 1194
atgtggaacc cccacctact tggcgcctga agttcttgtt tctgttggga ctgctgggta 60
taaccgtgct gtggactg 78

<210> 1195
<211> 86
<212> DNA
<213> Artificial Sequence

<220>
<223> Synthetic Oligonucleotide

<400> 1195
ggatatttcc gtggctctta ttcaaactct ccatcaaatc ctgtaaactc cagagcaaat 60
caagattttt ctgccttgat gagaag 86

<210> 1196
<211> 66
<212> DNA
<213> Artificial Sequence

<220>
<223> Synthetic Oligonucleotide

<400> 1196
tcattgacca cagtggcgcc cgccttggtg agtatgagga cgtgtcccgg gtggagaagt 60
acacga 66

<210> 1197
<211> 76
<212> DNA
<213> Artificial Sequence

<220>
<223> Synthetic Oligonucleotide

<400> 1197
ccccaggata cctaccacta cctgcccttc agcctgcccc accggaggcc tcacttcttc 60
tttcccaagt cccgca 76

<210> 1198
<211> 86
<212> DNA
<213> Artificial Sequence

<220>
<223> Synthetic Oligonucleotide

<400> 1198
gacatttcca gtcctgcagt caatgcctct ctgccccacc ctttgttcag tgtggctggt 60
gccacgacaa atgtgtgcga tcggag 86

<210> 1199
<211> 84
<212> DNA
<213> Artificial Sequence

<220>
<223> Synthetic Oligonucleotide

<400> 1199
tccctccact cggaaggact atcctgctgc caagagggtc aagttggaca gtgtcagagt 60
cctgagacag atcagcaaca accg 84

<210> 1200
<211> 64

<212> DNA
<213> Artificial Sequence

<220>
<223> Synthetic Oligonucleotide

<400> 1200
tccaccctcc tggctttggc cagcatggcg aagacgaaag gaaacaaggt gaacgtggga 60
gtga 64

<210> 1201
<211> 68
<212> DNA
<213> Artificial Sequence

<220>
<223> Synthetic Oligonucleotide

<400> 1201
gtggccatcc agctgacctt cctgcgcctg atgtccaccg aggcctccca gaacatcacc 60
taccactg 68

<210> 1202
<211> 80
<212> DNA
<213> Artificial Sequence

<220>
<223> Synthetic Oligonucleotide

<400> 1202
cagccaagaa ctggtatagg agctccaagg acaagaaaca cgtctggcta ggagaaacta 60
tcaatgctgg cagccagttt 80

<210> 1203
<211> 66
<212> DNA
<213> Artificial Sequence

<220>
<223> Synthetic Oligonucleotide

<400> 1203
gagagcaagc gagacattct gttcctcttt gacggctcag ccaatcttgt gggccagttc 60
cctgtt 66

<210> 1204
<211> 81
<212> DNA
<213> Artificial Sequence

<220>
<223> Synthetic Oligonucleotide

<400> 1204
cgacagttgc gatgaaagtt ctaatctctt ccctcctcct gttgctgcca ctaatgctga 60
tgtccatggt ctctagcagc c 81

<210> 1205
<211> 79
<212> DNA
<213> Artificial Sequence

<220>
<223> Synthetic Oligonucleotide

<400> 1205
tctgcagagt tggaagcact ctatggtgac atcgatgctg tggagctgta tcctgccctt 60
ctggtagaaa agcctcggc 79

<210> 1206
<211> 78
<212> DNA
<213> Artificial Sequence

<220>
<223> Synthetic Oligonucleotide

<400> 1206
acctctgtgg tccgtaggag ccactatgag gagggccctg ggaagaattt gccattttca 60
gtggaaaaca agtggtcg 78

<210> 1207
<211> 67
<212> DNA
<213> Artificial Sequence

<220>
<223> Synthetic Oligonucleotide

<400> 1207
aacttcaagg tcggagaagg ctttgaggag gagaccgtgg acggacgcaa gtgcaggagt 60
ttagcca 67

<210> 1208
<211> 76
<212> DNA
<213> Artificial Sequence

<220>
<223> Synthetic Oligonucleotide

<400> 1208
gtgctacgcc accctgttcg gacccaaagg cgtgaacatc gggggcgcgg gctcctacat 60
ctacgagaag cccctg 76

<210> 1209
<211> 69
<212> DNA
<213> Artificial Sequence

<220>
<223> Synthetic Oligonucleotide

<400> 1209
gatgtgattg aggtgcatgg aaaacatgaa gagcgccagg atgaacatgg tttcatctcc 60
agggagttc 69

<210> 1210
<211> 74
<212> DNA
<213> Artificial Sequence

<220>
<223> Synthetic Oligonucleotide

<400> 1210
tgcagcggct gattgacagt cagatggaga cctcgtgcca aattacattt gagtttgtag 60
accaggaaca gttg 74

<210> 1211
<211> 72
<212> DNA
<213> Artificial Sequence

<220>
<223> Synthetic Oligonucleotide

<400> 1211
agcttttccg gaatctgttc catcgccagg gcttctccta tgactacgtg ttcgactgga 60
acatgctcaa at 72

<210> 1212
<211> 78
<212> DNA
<213> Artificial Sequence

<220>
<223> Synthetic Oligonucleotide

<400> 1212
tggcagaact acctgcaaga aaaaccagca cctgcgtctg gatgactgtg acttccaaac 60
caaccacacc ttgaagca 78

<210> 1213
<211> 80
<212> DNA
<213> Artificial Sequence

<220>
<223> Synthetic Oligonucleotide

<400> 1213
gtacatgatc ccctgtgaga aggtgtccac cctgcccgcg atcacactga agctgggagg 60
caaaggctac aagctgtccc 80

<210> 1214
<211> 74
<212> DNA
<213> Artificial Sequence

<220>
<223> Synthetic Oligonucleotide

<400> 1214
gggaggctta tctcactgag tgagcagaat ctggtagact gctctgggcc tcaaggcaat 60
gaaggctgca atgg 74

<210> 1215
<211> 67
<212> DNA
<213> Artificial Sequence

<220>
<223> Synthetic Oligonucleotide

<400> 1215
tgtctcactg agcgagcaga atctggtgga ctgttcgcgt cctcaaggca atcagggctg 60
caatggt 67

<210> 1216
<211> 72
<212> DNA
<213> Artificial Sequence

<220>
<223> Synthetic Oligonucleotide

<400> 1216
tgaccgcttc taccccaatg acttgtgggt ggttgtgttc cagtttcagc acatcatggt 60
tggccttatc ct 72

<210> 1217
<211> 77
<212> DNA
<213> Artificial Sequence

<220>
<223> Synthetic Oligonucleotide

<400> 1217
ggtgcctact ccattgtggc gggcgtgttt gtgtgcctgc tggagtaccc ccgggggaag 60
aggaagaagg gctccac 77

<210> 1218
<211> 71
<212> DNA
<213> Artificial Sequence

<220>
<223> Synthetic Oligonucleotide

<400> 1218
ccagctttgt gcctgtcact attcctcatg ccaccactgc caacacctct gtcttgggct 60
accacattcc c 71

<210> 1219
<211> 73
<212> DNA
<213> Artificial Sequence

<220>
<223> Synthetic Oligonucleotide

<400> 1219
ccgtgttcaa gaggaagctc actgccttgt ggaggagttg agaaaaacca aggcttcacc 60
ctgtgatccc act 73

<210> 1220
<211> 79
<212> DNA
<213> Artificial Sequence

<220>
<223> Synthetic Oligonucleotide

<400> 1220
agaacaagga caacatagat ccttacatat acacaccctt tggaagtgga cccagaaact 60
gcattggcat gaggtttgc 79

<210> 1221
<211> 70

<212> DNA
<213> Artificial Sequence

<220>
<223> Synthetic Oligonucleotide

<400> 1221
ccgtgtggct cgctttctgc agtgccgctt cctctttgcg gggccctggt tactcttcag 60
cgaaatctcc 70

<210> 1222
<211> 73
<212> DNA
<213> Artificial Sequence

<220>
<223> Synthetic Oligonucleotide

<400> 1222
cacaatggcg gctctgaaga gttggctgtc gcgcagcgta acttcattct tcaggtacag 60
acagtgtttg tgt 73

<210> 1223
<211> 62
<212> DNA
<213> Artificial Sequence

<220>
<223> Synthetic Oligonucleotide

<400> 1223
caagcagtca aggagaacca gaagcggcgg gagacagaag aaaagatgag gcgagcaaaa 60
ct 62

<210> 1224
<211> 68
<212> DNA
<213> Artificial Sequence

<220>
<223> Synthetic Oligonucleotide

<400> 1224
tccaattcca gcatcactgt ggagaaaagc tgtttgtctc cccagcatac tttatcgcct 60
tcactgcc 68

<210> 1225
<211> 75
<212> DNA
<213> Artificial Sequence

<220>
<223> Synthetic Oligonucleotide

<400> 1225
cagtgcttcc atggacaagt ccttgtcaaa actggcccat gctgatggag atcaaacatc 60
aatcatccct gtcca 75

<210> 1226
<211> 83
<212> DNA
<213> Artificial Sequence

<220>
<223> Synthetic Oligonucleotide

<400> 1226
tgcacagagg gtgtgggtta caccaatgct tccaacaatt tgtttgcttg cctcccatgt 60
acagcttgta aatcagatga aga 83

<210> 1227
<211> 84
<212> DNA
<213> Artificial Sequence

<220>
<223> Synthetic Oligonucleotide

<400> 1227
ctctgagaca gtgcttcgat gactttgcag acttggtgcc ctttgactcc tgggagccgc 60
tcatgaggaa gttgggcctc atgg 84

<210> 1228
<211> 76
<212> DNA
<213> Artificial Sequence

<220>
<223> Synthetic Oligonucleotide

<400> 1228
agacatcagc tcctggttca acgaggccat tgacttcata gactccatca agaatgctgg 60
aggaagggtg tttgtc 76

<210> 1229
<211> 74
<212> DNA
<213> Artificial Sequence

<220>
<223> Synthetic Oligonucleotide

<400> 1229
cagaggatga cgaggatgat gacattgacc tgtttggcag tgacaatgag gaggaggaca 60
aggaggcggc acag 74

<210> 1230
<211> 62
<212> DNA
<213> Artificial Sequence

<220>
<223> Synthetic Oligonucleotide

<400> 1230
tgtcgatgga cttccagaac cacctgggca gctgccaaaa gtgtgatcca agctgtccca 60
at 62

<210> 1231
<211> 82
<212> DNA
<213> Artificial Sequence

<220>
<223> Synthetic Oligonucleotide

<400> 1231
gatctaagat ggcgactgtc gaaccggaaa ccacccctac tcctaatccc ccgactacag 60
aagaggagaa aacggaatct aa 82

<210> 1232
<211> 67
<212> DNA
<213> Artificial Sequence

<220>
<223> Synthetic Oligonucleotide

<400> 1232
aagccgcggt tgaatgtgcc atgaccctct ccctctctgg atggcaccat cattgaagct 60
ggcgtca 67

<210> 1233
<211> 67
<212> DNA
<213> Artificial Sequence

<220>
<223> Synthetic Oligonucleotide

<400> 1233
ctcggatcct agccctcgtg cctccatgga ctcgagtgta ccgagtacag agggatattc 60
caatgcc 67

<210> 1234
<211> 76
<212> DNA
<213> Artificial Sequence

<220>
<223> Synthetic Oligonucleotide

<400> 1234
tggtccatcg ccagttatca catctgtatg cggaacctca aaagagtccc tggtgtgaag 60
caagatcgct agaaca 76

<210> 1235
<211> 81
<212> DNA
<213> Artificial Sequence

<220>
<223> Synthetic Oligonucleotide

<400> 1235
cggttatgtc atgccagata cacacctcaa aggtactccc tcctcccggg aaggcaccct 60
ttcttcagtg ggtctcagtt c 81

<210> 1236
<211> 86
<212> DNA
<213> Artificial Sequence

<220>
<223> Synthetic Oligonucleotide

<400> 1236
tggctcttaa tcagtttcgt tacctgcctc tggagaattt acgcattatt cgtgggacaa 60
aactttatga ggatcgatat gccttg 86

<210> 1237
<211> 67
<212> DNA
<213> Artificial Sequence

<220>
<223> Synthetic Oligonucleotide

<400> 1237
gtccaggtgg atgtgaaaga tccccagcag gccctcaagg agctggctaa gatgtgtatc 60
ctggccg 67

<210> 1238
<211> 67
<212> DNA
<213> Artificial Sequence

<220>
<223> Synthetic Oligonucleotide

<400> 1238
acggatcaca gtggaggaag cgctggctca cccctacctg gagcagtact atgacccgac 60
ggatgag 67

<210> 1239
<211> 70
<212> DNA
<213> Artificial Sequence

<220>
<223> Synthetic Oligonucleotide

<400> 1239
acccccagac cggatcaggc aagctggccc tcatgtcccc ttcacggtgt ttgaggaagt 60
ctgccctaca 70

<210> 1240
<211> 68
<212> DNA
<213> Artificial Sequence

<220>
<223> Synthetic Oligonucleotide

<400> 1240
cgtggtgccc ctctatgacc tgctgctgga gatgctggac gcccaccgcc tacatgcgcc 60
cactagcc 68

<210> 1241
<211> 91
<212> DNA
<213> Artificial Sequence

<220>
<223> Synthetic Oligonucleotide

<400> 1241
ggattgctca acaaccatgc tgggcatctg gaccctccta cctctggttc ttacgtctgt 60
tgctagatta tcgtccaaaa gtgttaatgc c 91

<210> 1242
<211> 80

<212> DNA
<213> Artificial Sequence

<220>
<223> Synthetic Oligonucleotide

<400> 1242
gcactttggg attctttcca ttatgattct ttgttacagg caccgagaat gttgtattca 60
gtgagggtct tcttacatgc 80

<210> 1243
<211> 66
<212> DNA
<213> Artificial Sequence

<220>
<223> Synthetic Oligonucleotide

<400> 1243
gcctcttcct gttcgacggc tcgcccacct acgtactggc ctacacccag agctaccggg 60
caaagc 66

<210> 1244
<211> 90
<212> DNA
<213> Artificial Sequence

<220>
<223> Synthetic Oligonucleotide

<400> 1244
ggctattcct cattttctct acaaagtggc ctcagtgaac atgaagaagg tagcctcctg 60
gaggagaatt tcggtgacag tctacaatcc 90

<210> 1245
<211> 67
<212> DNA
<213> Artificial Sequence

<220>
<223> Synthetic Oligonucleotide

<400> 1245
aaggaaaggg tgcctcatcc cagcaacctg tccttgtggg tgatgatcac tgtgctgctt 60
gtggctc 67

<210> 1246
<211> 74
<212> DNA
<213> Artificial Sequence

<220>
<223> Synthetic Oligonucleotide

<400> 1246
cacgggacat tcaccacatc gactactata aaaagacaac caacggccga ctgcctgtga 60
agtggatggc accc 74

<210> 1247
<211> 67
<212> DNA
<213> Artificial Sequence

<220>
<223> Synthetic Oligonucleotide

<400> 1247
ccagtggagc gcttccatga cctgcgtcct gatgaagtgg ccgatttgtt tcagacgacc 60
cagagag 67

<210> 1248
<211> 72
<212> DNA
<213> Artificial Sequence

<220>
<223> Synthetic Oligonucleotide

<400> 1248
ggttccagct ttctgtagct gtaagcattg gtggccacac cacctcctta caaagcaact 60
agaacctgcg gc 72

<210> 1249
<211> 73
<212> DNA
<213> Artificial Sequence

<220>
<223> Synthetic Oligonucleotide

<400> 1249
gcgtatgatt tcccgaatga gtcaaaatgt tgatatgccc aaacttcatg atgcaatggg 60
tacgaggtca ctg 73

<210> 1250
<211> 67
<212> DNA
<213> Artificial Sequence

<220>
<223> Synthetic Oligonucleotide

<400> 1250
cgagcccttt gatgacttcc tgttcccagc atcatccagg cccagtggct ctgagacagc 60
ccgctcc 67

<210> 1251
<211> 82
<212> DNA
<213> Artificial Sequence

<220>
<223> Synthetic Oligonucleotide

<400> 1251
ccaccccgag caaatctgtc ctccccagaa cccctgaatc ctggaggctc acgcccccag 60
ccaaagtagg gggactggat tt 82

<210> 1252
<211> 80
<212> DNA
<213> Artificial Sequence

<220>
<223> Synthetic Oligonucleotide

<400> 1252
ggataattca gacaacaaca ccatctttgt gcaaggcctg ggtgagaatg ttacaattga 60
gtctgtggct gattacttca 80

<210> 1253
<211> 69
<212> DNA
<213> Artificial Sequence

<220>
<223> Synthetic Oligonucleotide

<400> 1253
gaagcgcaga tcatgaagaa gctgaagcac gacaagctgg tccagctcta tgcagtggtg 60
tctgaggag 69

<210> 1254
<211> 67
<212> DNA
<213> Artificial Sequence

<220>
<223> Synthetic Oligonucleotide

<400> 1254
cctccaactc ctagcctcaa gtgatcctcc tgtctcaacc tcccaagtag gattacaagc 60
atgcgcc 67

<210> 1255
<211> 73
<212> DNA
<213> Artificial Sequence

<220>
<223> Synthetic Oligonucleotide

<400> 1255
gtgctggtga cgaatccaca ttcatctcaa tggaaggatc ctgccttaag tcaacttatt 60
tgtttttgcc ggg 73

<210> 1256
<211> 70
<212> DNA
<213> Artificial Sequence

<220>
<223> Synthetic Oligonucleotide

<400> 1256
accctcgaca agaccacact ttgggacttg ggagctgggg ctgaagttgc tctgtaccca 60
tgaactccca 70

<210> 1257
<211> 84
<212> DNA
<213> Artificial Sequence

<220>
<223> Synthetic Oligonucleotide

<400> 1257
aagggcaatc acagtgttaa aagaagacat gctgaaatgt tgcaggctgc tcctatgttg 60
gaaaattctt cattgaagtt ctcc 84

<210> 1258
<211> 74
<212> DNA
<213> Artificial Sequence

<220>
<223> Synthetic Oligonucleotide

<400> 1258
attccaccca tggcaaattc catggcaccg tcaaggctga gaacgggaag cttgtcatca 60
atggaaatcc catc 74

<210> 1259
<211> 75
<212> DNA
<213> Artificial Sequence

<220>
<223> Synthetic Oligonucleotide

<400> 1259
caaaggagct cactgtggtg tctgtgttcc aaccactgaa tctggacccc atctgtgaat 60
aagccattct gactc 75

<210> 1260
<211> 73
<212> DNA
<213> Artificial Sequence

<220>
<223> Synthetic Oligonucleotide

<400> 1260
ttgggaaata tttgggcatt ggtctggcca agtctacaat gtcccaatat caaggacaac 60
caccctagct tct 73

<210> 1261
<211> 83
<212> DNA
<213> Artificial Sequence

<220>
<223> Synthetic Oligonucleotide

<400> 1261
gcatgggaac catcaaccag caggccatgg accaacttca ctatgtgaca gagctgacag 60
atcgaatcaa ggcaaactcc tca 83

<210> 1262
<211> 71
<212> DNA
<213> Artificial Sequence

<220>
<223> Synthetic Oligonucleotide

<400> 1262
ctgttgcagg aaggcattga tcatctcctg gcccagcatg ttgctcatct ctttattaga 60
gacccactga c 71

<210> 1263
<211> 72

<212> DNA
<213> Artificial Sequence

<220>
<223> Synthetic Oligonucleotide

<400> 1263
cgctccagac ctatgatgac ttgttagcca aagactgcca ctgcatatga gcagtcctgg 60
tccttccact gt 72

<210> 1264
<211> 70
<212> DNA
<213> Artificial Sequence

<220>
<223> Synthetic Oligonucleotide

<400> 1264
ctccgacgtg gctttccagt ggcccacagc atctatggaa tcccatctgt catcaattct 60
gccaattacg 70

<210> 1265
<211> 67
<212> DNA
<213> Artificial Sequence

<220>
<223> Synthetic Oligonucleotide

<400> 1265
ggagctctgc agtaaccaca gaacaggccc catgctgacg tccctcctca agagctggat 60
ggcattg 67

<210> 1266
<211> 67
<212> DNA
<213> Artificial Sequence

<220>
<223> Synthetic Oligonucleotide

<400> 1266
ggtgaaggtt gtctcttccg agggccttct gaagacaggg ctcttgaaca gacaagtgga 60
agggctg 67

<210> 1267
<211> 66
<212> DNA
<213> Artificial Sequence

<220>
<223> Synthetic Oligonucleotide

<400> 1267
tacccttcca tgtgctggat ccgggactcc ctggtcagct acatcaccaa cctgggcctc 60
ttcagc 66

<210> 1268
<211> 67
<212> DNA
<213> Artificial Sequence

<220>
<223> Synthetic Oligonucleotide

<400> 1268
gcttatgacc gaccccaagc tcatcacctg gtctccggtg tgtcgcaacg atgttgcctg 60
gaacttt 67

<210> 1269
<211> 67
<212> DNA
<213> Artificial Sequence

<220>
<223> Synthetic Oligonucleotide

<400> 1269
ccatctgcat ccatcttgtt tgggctcccc acccttgaga agtgcctcag ataataccct 60
ggtggcc 67

<210> 1270
<211> 66
<212> DNA
<213> Artificial Sequence

<220>
<223> Synthetic Oligonucleotide

<400> 1270
gacaaggacg gcagcaaggt gacaacagtg gtggcaactc ctgggcaggg tccagacagg 60
ccacaa 66

<210> 1271
<211> 68
<212> DNA
<213> Artificial Sequence

<220>
<223> Synthetic Oligonucleotide

<400> 1271
gtgatcccaa gcccacaata gaggtcagtg ggaaaaagta caccgcccca cacatcctga 60
tcgccaca 68

<210> 1272
<211> 86
<212> DNA
<213> Artificial Sequence

<220>
<223> Synthetic Oligonucleotide

<400> 1272
aagctatgag gaaaagaagt acacgatggg ggacgctcct gattatgaca gaagccagtg 60
gctgaatgaa aaattcaagc tgggcc 86

<210> 1273
<211> 76
<212> DNA
<213> Artificial Sequence

<220>
<223> Synthetic Oligonucleotide

<400> 1273
gagaccctgc tgtcccagaa ccagggaggc aagaccttca ttgtgggaga ccagatctcc 60
ttcgctgact acaacc 76

<210> 1274
<211> 73
<212> DNA
<213> Artificial Sequence

<220>
<223> Synthetic Oligonucleotide

<400> 1274
cccactcagt agccaagtca caatgtttgg aaaacagccc gtttacttga gcaagactga 60
taccacctgc gtg 73

<210> 1275
<211> 66
<212> DNA
<213> Artificial Sequence

<220>
<223> Synthetic Oligonucleotide

<400> 1275
tcctgtgctc tggaagccct tgagcccttc tgggaggttc ttgtgagatc aactgagacc 60
gtggag 66

<210> 1276
<211> 70
<212> DNA
<213> Artificial Sequence

<220>
<223> Synthetic Oligonucleotide

<400> 1276
cggtgtgaga agtgcagcaa gccctgtgcc cgagtgtgct atggtctggg catggagcac 60
ttgcgagagg 70

<210> 1277
<211> 82
<212> DNA
<213> Artificial Sequence

<220>
<223> Synthetic Oligonucleotide

<400> 1277
tgaacataaa gtctgcaaca tggaaggtat tgcactgcac aggccacatt cacgtatatg 60
ataccaacag taaccaacct ca 82

<210> 1278
<211> 73
<212> DNA
<213> Artificial Sequence

<220>
<223> Synthetic Oligonucleotide

<400> 1278
tccaggatgt taggaactgt gaagatggaa gggcatgaaa ccagcgactg gaacagctac 60
tacgcagaca cgc 73

<210> 1279
<211> 67
<212> DNA
<213> Artificial Sequence

<220>
<223> Synthetic Oligonucleotide

<400> 1279
ggacgaatac gaccccacta tagaggattc ctaccggaag caggtggtca ttgatgggga 60
gacgtgc 67

<210> 1280
<211> 70
<212> DNA
<213> Artificial Sequence

<220>
<223> Synthetic Oligonucleotide

<400> 1280
ctgctgcgac agtccactac ctttttcgag agtgactccc gttgtcccaa ggcttcccag 60
agcgaacctg 70

<210> 1281
<211> 63
<212> DNA
<213> Artificial Sequence

<220>
<223> Synthetic Oligonucleotide

<400> 1281
ggtccgcttc gtctttcgag agtgactccc gcggtcccaa ggctttccag agcgaacctg 60
tgc 63

<210> 1282
<211> 72
<212> DNA
<213> Artificial Sequence

<220>
<223> Synthetic Oligonucleotide

<400> 1282
gcaggtgtga ttgctggact taatgtgcta agaatcatca atgagcccac ggctgctgcc 60
attgcctatg gt 72

<210> 1283
<211> 84
<212> DNA
<213> Artificial Sequence

<220>
<223> Synthetic Oligonucleotide

<400> 1283
ggctagtaga actggatccc aacaccaaac tcttaattag acctaggcct cagctgcact 60
gcccgaaaag catttgggca gacc 84

<210> 1284
<211> 70

<212> DNA
<213> Artificial Sequence

<220>
<223> Synthetic Oligonucleotide

<400> 1284
ggccactaaa gatgctggcc agatatctgg actgaatgtg cttcgggtga ttaatgagcc 60
cacagctgct 70

<210> 1285
<211> 84
<212> DNA
<213> Artificial Sequence

<220>
<223> Synthetic Oligonucleotide

<400> 1285
ccgactggag gagcataaaa gcgcagccga gcccagcgcc ccgcactttt ctgagcagac 60
gtccagagca gagtcagcca gcat 84

<210> 1286
<211> 71
<212> DNA
<213> Artificial Sequence

<220>
<223> Synthetic Oligonucleotide

<400> 1286
caaaaggcag aggctgataa gaacgacaag tctgtgaagg atctggtcat cttgctttat 60
gaaactgcgc t 71

<210> 1287
<211> 70
<212> DNA
<213> Artificial Sequence

<220>
<223> Synthetic Oligonucleotide

<400> 1287
agaaccgcaa ggtgagcaag gtggagattc tccagcacgt catcgactac atcagggacc 60
ttcagttgga 70

<210> 1288
<211> 79
<212> DNA
<213> Artificial Sequence

<220>
<223> Synthetic Oligonucleotide

<400> 1288
cacgcagaaa accacacttc tcaaaccttc actcaacact tccttcccca aagccagaag 60
atgcacaagg aggaacatg 79

<210> 1289
<211> 83
<212> DNA
<213> Artificial Sequence

<220>
<223> Synthetic Oligonucleotide

<400> 1289
gcatggtagc cgaagatttc acagtcaaaa tcggagattt tggtatgacg cgagatatct 60
atgagacaga ctattaccgg aaa 83

<210> 1290
<211> 73
<212> DNA
<213> Artificial Sequence

<220>
<223> Synthetic Oligonucleotide

<400> 1290
gtggacagca ccatgaacat gttgggcggg ggaggcagtg ctggccggaa gcccctcaag 60
tcgggtatga agg 73

<210> 1291
<211> 68
<212> DNA
<213> Artificial Sequence

<220>
<223> Synthetic Oligonucleotide

<400> 1291
acgcaccggg tgtctgatcc caagttccac cccctccatt caaagataat catcatcaag 60
aaagggca 68

<210> 1292
<211> 69
<212> DNA
<213> Artificial Sequence

<220>
<223> Synthetic Oligonucleotide

<400> 1292
tggacaagta cgggatgaag ctgccaggca tggagtacgt tgacggggac tttcagtgcc 60
acaccttcg 69

<210> 1293
<211> 74
<212> DNA
<213> Artificial Sequence

<220>
<223> Synthetic Oligonucleotide

<400> 1293
tctgcgtggt tcctttctca gccgcttctg actgctgatt ctcccgttca cgttgcctaa 60
taaacatcct tcaa 74

<210> 1294
<211> 72
<212> DNA
<213> Artificial Sequence

<220>
<223> Synthetic Oligonucleotide

<400> 1294
cctgaacctt ccaaagatgg ctgaaaaaga tggatgcttc caatctggat tcaatgagga 60
gacttgcctg gt 72

<210> 1295
<211> 71
<212> DNA
<213> Artificial Sequence

<220>
<223> Synthetic Oligonucleotide

<400> 1295
gcggtgattc ggaaattcgc gaattcctct ggtcctcatc caggtgcgcg ggaagcaggt 60
gcccaggaga g 71

<210> 1296
<211> 70
<212> DNA
<213> Artificial Sequence

<220>
<223> Synthetic Oligonucleotide

<400> 1296
aaggaaccat ctcactgtgt gtaaacatga cttccaagct ggccgtggct ctcttggcag 60
ccttcctgat 70

<210> 1297
<211> 68
<212> DNA
<213> Artificial Sequence

<220>
<223> Synthetic Oligonucleotide

<400> 1297
ccgctccgtc actgatgtct acctgctgaa cctagccttg gccgacctac tctttgccct 60
gaccttgc 68

<210> 1298
<211> 66
<212> DNA
<213> Artificial Sequence

<220>
<223> Synthetic Oligonucleotide

<400> 1298
ctcaggattt tctcgcatcc aaatgtgctc ccagtgctag gtgcctgcca gtctccacct 60
gctcct 66

<210> 1299
<211> 63
<212> DNA
<213> Artificial Sequence

<220>
<223> Synthetic Oligonucleotide

<400> 1299
agccatcact ctcagtgcag ccaggtccta tcgtggcccc tgaggagacc ctgactctgc 60
agt 63

<210> 1300
<211> 67
<212> DNA
<213> Artificial Sequence

<220>
<223> Synthetic Oligonucleotide

<400> 1300
gccaggatac tggagtccat cacagtgagc tccctgatgg ctacacccca ggaccccaag 60
ggtcaag 67

<210> 1301
<211> 67
<212> DNA
<213> Artificial Sequence

<220>
<223> Synthetic Oligonucleotide

<400> 1301
ggatggagtt cgcctcctac gtgatcacag acctgaccca gctgcggaag atcaagtcca 60
tggagcg 67

<210> 1302
<211> 74
<212> DNA
<213> Artificial Sequence

<220>
<223> Synthetic Oligonucleotide

<400> 1302
ccacagctca ccttctgtca ggtgtccatc ccagctccag ccagctccca gagaggaaga 60
gactggcact gagg 74

<210> 1303
<211> 74
<212> DNA
<213> Artificial Sequence

<220>
<223> Synthetic Oligonucleotide

<400> 1303
tcagaattgg atttggctca tttgtggaaa agactgtgat gccttacatt agcacaacac 60
cagctaagct cagg 74

<210> 1304
<211> 74
<212> DNA
<213> Artificial Sequence

<220>
<223> Synthetic Oligonucleotide

<400> 1304
tgaggaagaa ggagaggaat actaattatc cattcctttt ggccctgcag catgtcatgc 60
tcccagaatt tcag 74

<210> 1305
<211> 68

<212> DNA
<213> Artificial Sequence

<220>
<223> Synthetic Oligonucleotide

<400> 1305
gaggacgaag gcctctacac ctgccaggca tgcagtgttc ttggctgtgc aaaagtggag 60
gcattttt 68

<210> 1306
<211> 80
<212> DNA
<213> Artificial Sequence

<220>
<223> Synthetic Oligonucleotide

<400> 1306
cggactttgg gtgcgacttg acgagcggtg gttcgacaag tggccttgcg ggccggatcg 60
tcccagtgga agagttgtaa 80

<210> 1307
<211> 71
<212> DNA
<213> Artificial Sequence

<220>
<223> Synthetic Oligonucleotide

<400> 1307
tggaggttgt aagccaatgt tgtgaggctt caagttcaga catcactgag aaatcagatg 60
gacgtaaggc a 71

<210> 1308
<211> 67
<212> DNA
<213> Artificial Sequence

<220>
<223> Synthetic Oligonucleotide

<400> 1308
ctaaggcact tgctggaagg gcagaatgcc agtgtgcttg cctatggacc cacaggagct 60
gggaaga 67

<210> 1309
<211> 73
<212> DNA
<213> Artificial Sequence

<220>
<223> Synthetic Oligonucleotide

<400> 1309
aattcctgct ccaaaagaaa gtcttcgaag ccgctccact cgcatgtcca ctgtctcaga 60
gcttcgcatc acg 73

<210> 1310
<211> 66
<212> DNA
<213> Artificial Sequence

<220>
<223> Synthetic Oligonucleotide

<400> 1310
ccacagggtt gaagaaccag aagccagttc ctgctgttcc tgtccagaag tctggcacat 60
caggtg 66

<210> 1311
<211> 78
<212> DNA
<213> Artificial Sequence

<220>
<223> Synthetic Oligonucleotide

<400> 1311
gcccagaggc tccatcgtcc atcctcttcc tccccagtcg gctgaactct ccccttgtct 60
gcactgttca aacctctg 78

<210> 1312
<211> 70
<212> DNA
<213> Artificial Sequence

<220>
<223> Synthetic Oligonucleotide

<400> 1312
caaacagagg gtggcagaag tgctcaatga ccctgagaac atggagaagc gcaggagccg 60
tgagagcctc 70

<210> 1313
<211> 72
<212> DNA
<213> Artificial Sequence

<220>
<223> Synthetic Oligonucleotide

<400> 1313
agccgaggct ttgttgaaga agcttcatat ccagtaccgg cgatcgggca cagaagctgt 60
gctcatagcc ca 72

<210> 1314
<211> 71
<212> DNA
<213> Artificial Sequence

<220>
<223> Synthetic Oligonucleotide

<400> 1314
atgtgccagt gagcttgagt ccttggagaa acacaagcac ctgctagaaa gtactgttaa 60
ccaggggctc a 71

<210> 1315
<211> 83
<212> DNA
<213> Artificial Sequence

<220>
<223> Synthetic Oligonucleotide

<400> 1315
ggcctgctga gatcaaagac tacagtccct acttcaagac cattgaggac ctgaggaaca 60
agattctcac agccacagtg gac 83

<210> 1316
<211> 73
<212> DNA
<213> Artificial Sequence

<220>
<223> Synthetic Oligonucleotide

<400> 1316
cgaggattgg ttcttcagca agacagagga actgaaccgc gaggtggcca ccaacagtga 60
gctggtgcag agt 73

<210> 1317
<211> 77
<212> DNA
<213> Artificial Sequence

<220>
<223> Synthetic Oligonucleotide

<400> 1317
tgagcggcag aatcaggagt accagcggct catggacatc aagtcgcggc tggagcagga 60
gattgccacc taccgca 77

<210> 1318
<211> 69
<212> DNA
<213> Artificial Sequence

<220>
<223> Synthetic Oligonucleotide

<400> 1318
tcagtggaga aggagttgga ccagtcaaca tctctgttgt cacaagcagt gtttcctctg 60
gatatggca 69

<210> 1319
<211> 66
<212> DNA
<213> Artificial Sequence

<220>
<223> Synthetic Oligonucleotide

<400> 1319
cttgctggcc aatgcctaca tctacgttgt ccagctgcca gccaagatcc tgactgcgga 60
caatca 66

<210> 1320
<211> 80
<212> DNA
<213> Artificial Sequence

<220>
<223> Synthetic Oligonucleotide

<400> 1320
actcaagcgg aaattgaagc agataggtct tatcagcaca gtctccgcct cctggattca 60
gtgtctcggc ttcagggagt 80

<210> 1321
<211> 67
<212> DNA
<213> Artificial Sequence

<220>
<223> Synthetic Oligonucleotide

<400> 1321
agcgatgaag atggtcgcgc cctggacgcg gttctactcc aacagctgct gcttgtgctg 60
ccatgtc 67

<210> 1322
<211> 67
<212> DNA
<213> Artificial Sequence

<220>
<223> Synthetic Oligonucleotide

<400> 1322
gcttcaggtg ttgtgactgc agtgcctccc tgtcgcacca gtactatgag aaggatgggc 60
agctctt 67

<210> 1323
<211> 66
<212> DNA
<213> Artificial Sequence

<220>
<223> Synthetic Oligonucleotide

<400> 1323
ctttgggcca ggaggaatct gttactcgaa tccacccagg aactccctgg cagtggattg 60
tgggag 66

<210> 1324
<211> 67
<212> DNA
<213> Artificial Sequence

<220>
<223> Synthetic Oligonucleotide

<400> 1324
agaagctgtc cctgcaagag caggatgcag cgattgtgaa gaacatgaag tctgagctgg 60
tacggct 67

<210> 1325
<211> 75
<212> DNA
<213> Artificial Sequence

<220>
<223> Synthetic Oligonucleotide

<400> 1325
ccgggagcag ggaatcaccc tgcgcgggag cgccgaaatc gtggccgagt tcttctcatt 60
cggcatcaac agcat 75

<210> 1326
<211> 76

<212> DNA
<213> Artificial Sequence

<220>
<223> Synthetic Oligonucleotide

<400> 1326
ctgtcatgtg gcagaccttc catccgaacc acggcttggg aagactacat ttggttccag 60
gcaccagtga cattta 76

<210> 1327
<211> 66
<212> DNA
<213> Artificial Sequence

<220>
<223> Synthetic Oligonucleotide

<400> 1327
gcccagtgga gaaattcgtc tttgagatca cccagcctcc actgctgtcc atcagctcag 60
actcgc 66

<210> 1328
<211> 72
<212> DNA
<213> Artificial Sequence

<220>
<223> Synthetic Oligonucleotide

<400> 1328
cctcagaaat tgccaggact tctttcccgt gatcttcagc aaagcctccg agtacttgca 60
gctggtcttt gg 72

<210> 1329
<211> 74
<212> DNA
<213> Artificial Sequence

<220>
<223> Synthetic Oligonucleotide

<400> 1329
aggactccag caaccaagaa gaggaggggc caagcacctt ccctgacctg gagtctgagt 60
tccaagcagc actc 74

<210> 1330
<211> 67
<212> DNA
<213> Artificial Sequence

<220>
<223> Synthetic Oligonucleotide

<400> 1330
cggaccacca ggtcagagcc aattcgcaga gcagggaaga gtggtacctc aacacccact 60
accccctg 67

<210> 1331
<211> 67
<212> DNA
<213> Artificial Sequence

<220>
<223> Synthetic Oligonucleotide

<400> 1331
gccctccaat gtccttatca acaaggaggg ccatgtgaag atgtgtgact ttggcatcag 60
tggctac 67

<210> 1332
<211> 72
<212> DNA
<213> Artificial Sequence

<220>
<223> Synthetic Oligonucleotide

<400> 1332
gccggtcagg cacacaaggg gcccttggag cgtggactgg ttggttttgc cattttgttg 60
tgtgtatgct gc 72

<210> 1333
<211> 77
<212> DNA
<213> Artificial Sequence

<220>
<223> Synthetic Oligonucleotide

<400> 1333
ccctcaaccg gcaaatccgc gaggaggtgg cgagtgcagt gagcagctcc tacaggaatg 60
aattcagggc atggacg 77

<210> 1334
<211> 76
<212> DNA
<213> Artificial Sequence

<220>
<223> Synthetic Oligonucleotide

<400> 1334
tgagtggaaa agcctgacct atgatgaagt catcagcttt gtgccaccac cccttgacca 60
agaagagatg gagtcc 76

<210> 1335
<211> 83
<212> DNA
<213> Artificial Sequence

<220>
<223> Synthetic Oligonucleotide

<400> 1335
caacacccgt acatcaatgt ctggtatgat ccttctgaag cagaagctcc accaccaaag 60
atccctgaca agcagttaga tga 83

<210> 1336
<211> 69
<212> DNA
<213> Artificial Sequence

<220>
<223> Synthetic Oligonucleotide

<400> 1336
gaccttggaa cctttggaac ctgctgtcaa caggtcttac agggctgctt gaaccctcat 60
aggcctagg 69

<210> 1337
<211> 67
<212> DNA
<213> Artificial Sequence

<220>
<223> Synthetic Oligonucleotide

<400> 1337
cacaagctga ccttccgcga gaacgccaaa gccaagacag accacggggc ggagatcgtg 60
tacaagt 67

<210> 1338
<211> 77
<212> DNA
<213> Artificial Sequence

<220>
<223> Synthetic Oligonucleotide

<400> 1338
cagatggcca ctttgagaac attttagctg acaacagtgt gaacgaccag accaaaatcc 60
ttgtggttaa tgctgcc 77

<210> 1339
<211> 75
<212> DNA
<213> Artificial Sequence

<220>
<223> Synthetic Oligonucleotide

<400> 1339
cttcggaaac tggacatcaa aaacgaagac gatgtgaaat cgttgtctcg agtgatgatc 60
catgttttca gcgac 75

<210> 1340
<211> 75
<212> DNA
<213> Artificial Sequence

<220>
<223> Synthetic Oligonucleotide

<400> 1340
gacttttgcc cgctaccttt cattccggcg tgacaacaat gagctgttgc tcttcatact 60
gaagcagtta gtggc 75

<210> 1341
<211> 82
<212> DNA
<213> Artificial Sequence

<220>
<223> Synthetic Oligonucleotide

<400> 1341
tgatggtcct atgtgtcaca ttcatcacag gtttcatacc aacacaggct tcagcacttc 60
ctttggtgtg tttcctgtcc ca 82

<210> 1342
<211> 71
<212> DNA
<213> Artificial Sequence

<220>
<223> Synthetic Oligonucleotide

<400> 1342
cgctcagcca gatgcaatca atgccccagt cacctgctgt tataacttca ccaataggaa 60
gatctcagtg c 71

<210> 1343
<211> 69
<212> DNA
<213> Artificial Sequence

<220>
<223> Synthetic Oligonucleotide

<400> 1343
gtgaaatgaa acgcaccaca ctggacagcc ctttggggaa gctggagctg tctggttgtg 60
agcagggtc 69

<210> 1344
<211> 78
<212> DNA
<213> Artificial Sequence

<220>
<223> Synthetic Oligonucleotide

<400> 1344
ccaacgcttg ccaaatcctg acaattcaga accagctctc tgtgacccca atttgagttt 60
tgatgctgtc actaccgt 78

<210> 1345
<211> 86
<212> DNA
<213> Artificial Sequence

<220>
<223> Synthetic Oligonucleotide

<400> 1345
ccatgatgga gaggcagaca tcatgatcaa ctttggccgc tgggagcatg gcgatggata 60
cccctttgac ggtaaggacg gactcc 86

<210> 1346
<211> 67
<212> DNA
<213> Artificial Sequence

<220>
<223> Synthetic Oligonucleotide

<400> 1346
gagaaccaat ctcaccgaca ggcagctggc agaggaatac ctgtaccgct atggttacac 60
tcgggtg 67

<210> 1347
<211> 78

<212> DNA
<213> Artificial Sequence

<220>
<223> Synthetic Oligonucleotide

<400> 1347
gccatgctgg ctcagtctga gctgtgggcc acatcagcta gtggctcttc tcatgcatca 60
gttaggtggg tctgggtg 78

<210> 1348
<211> 79
<212> DNA
<213> Artificial Sequence

<220>
<223> Synthetic Oligonucleotide

<400> 1348
tcatggtgcc cgtcaatgct gtgatggcga tgaagaccaa gacgtatcag gtggcccaca 60
tgaagagcaa agacaatcg 79

<210> 1349
<211> 65
<212> DNA
<213> Artificial Sequence

<220>
<223> Synthetic Oligonucleotide

<400> 1349
aggggatgac ttggacacat ctgccattcg acatgactgc aattttgaca aagccatgca 60
gtttt 65

<210> 1350
<211> 91
<212> DNA
<213> Artificial Sequence

<220>
<223> Synthetic Oligonucleotide

<400> 1350
tcatcctggc gatctacttc ctctggcaga acctaggtcc ctctgtcctg gctggagtcg 60
ctttcatggt cttgctgatt ccactcaacg g 91

<210> 1351
<211> 82
<212> DNA
<213> Artificial Sequence

<220>
<223> Synthetic Oligonucleotide

<400> 1351
tgattaccat catggctcag attggctcct atgttcctgc agaagaagcg acaattggga 60
ttgtggatgg cattttcaca ag 82

<210> 1352
<211> 71
<212> DNA
<213> Artificial Sequence

<220>
<223> Synthetic Oligonucleotide

<400> 1352
ggccaggatc tgtggtggta caattgactc tggccttccg agaaggtacc atcaatgtcc 60
acgacgtgga g 71

<210> 1353
<211> 78
<212> DNA
<213> Artificial Sequence

<220>
<223> Synthetic Oligonucleotide

<400> 1353
gaaggaatgg gaatcagtca tgagctaatc accctggaga tcagctcccg agatgtcccg 60
gatctgactc taatagac 78

<210> 1354
<211> 74
<212> DNA
<213> Artificial Sequence

<220>
<223> Synthetic Oligonucleotide

<400> 1354
gccgagatcg ccaagatgtt gccagggagg acagacaatg ctgtgaagaa tcactggaac 60
tctaccatca aaag 74

<210> 1355
<211> 85
<212> DNA
<213> Artificial Sequence

<220>
<223> Synthetic Oligonucleotide

<400> 1355
cggtacttct cagggctaat atatacgtac tctggcctct tctgcgtggt ggtcaacccc 60
tataaacacc tgcccatcta ctcgg 85

<210> 1356
<211> 79
<212> DNA
<213> Artificial Sequence

<220>
<223> Synthetic Oligonucleotide

<400> 1356
gtgaggcagc gcgactctgg cgactggccg gccatgcctt cccgggctga ggactatgaa 60
gtgttgtaca ccattggca 79

<210> 1357
<211> 73
<212> DNA
<213> Artificial Sequence

<220>
<223> Synthetic Oligonucleotide

<400> 1357
cagaccaagg agatggacct cagcgtggtg cggctcatgt ttacagcttt tcttccggat 60
agcactggca gct 73

<210> 1358
<211> 68
<212> DNA
<213> Artificial Sequence

<220>
<223> Synthetic Oligonucleotide

<400> 1358
ctgccgggat ggcttctatg aggctgagct ctgcccggac cgctgcatcc acagtttcca 60
gaacctgg 68

<210> 1359
<211> 67
<212> DNA
<213> Artificial Sequence

<220>
<223> Synthetic Oligonucleotide

<400> 1359
cactgacacc cgcaacacca cccatggttc ggactctgtg gtttcagcca gcagagagat 60
tgcagcc 67

<210> 1360
<211> 66
<212> DNA
<213> Artificial Sequence

<220>
<223> Synthetic Oligonucleotide

<400> 1360
ctgcttcttt cccgagcttg gaacttcgtt atccgcgatg cgtttcctgg cagctacatt 60
cctgct 66

<210> 1361
<211> 73
<212> DNA
<213> Artificial Sequence

<220>
<223> Synthetic Oligonucleotide

<400> 1361
ggctgtggct gaggctgtag catctctgct ggaggtgaga cactctggga actgatttga 60
cctcgaatgc tcc 73

<210> 1362
<211> 69
<212> DNA
<213> Artificial Sequence

<220>
<223> Synthetic Oligonucleotide

<400> 1362
cggacctgaa tgtaatgcaa gaatgaacag aacaagcaaa atgaccagct gcttagtcac 60
ctggcaaag 69

<210> 1363
<211> 67
<212> DNA
<213> Artificial Sequence

<220>
<223> Synthetic Oligonucleotide

<400> 1363
caaaggaaga gcaacggaag aaacgcgagc aagaacgaaa ggagaagaaa gcaaaggttt 60
tgggaat 67

<210> 1364
<211> 65
<212> DNA
<213> Artificial Sequence

<220>
<223> Synthetic Oligonucleotide

<400> 1364
tggagactct cagggtcgaa aacggcggca gaccagcatg acagatttct accactccaa 60
acgcc 65

<210> 1365
<211> 66
<212> DNA
<213> Artificial Sequence

<220>
<223> Synthetic Oligonucleotide

<400> 1365
cggtggacca cgaagagtta acccgggact tggagaagca ctgcagagac atggaagagg 60
cgagcc 66

<210> 1366
<211> 67
<212> DNA
<213> Artificial Sequence

<220>
<223> Synthetic Oligonucleotide

<400> 1366
tcactaccag ctgacatccg gctgcctgag ggctacctgg agaagctgac cctcaatagc 60
cccatct 67

<210> 1367
<211> 66
<212> DNA
<213> Artificial Sequence

<220>
<223> Synthetic Oligonucleotide

<400> 1367
ccagctctcc ttccagctac agatcaatgt ccctgtccga gtgctggagc taagtgagag 60
ccaccc 66

<210> 1368
<211> 66

<212> DNA
<213> Artificial Sequence

<220>
<223> Synthetic Oligonucleotide

<400> 1368
gagaagcacg ccatgaaaca ggccgtcatg gaaatgatga gtcagaagat tcagcagctc 60
acagcc 66

<210> 1369
<211> 74
<212> DNA
<213> Artificial Sequence

<220>
<223> Synthetic Oligonucleotide

<400> 1369
agagccagtt gctgtagaac tcaaatctct gctgggcaag gatgttctgt tcttgaagga 60
ctgtgtaggc ccag 74

<210> 1370
<211> 71
<212> DNA
<213> Artificial Sequence

<220>
<223> Synthetic Oligonucleotide

<400> 1370
gacattgtgg taggggaagg gactcatttt ctcatcccgt gggtacagaa accaattatc 60
tttgactgcc g 71

<210> 1371
<211> 83
<212> DNA
<213> Artificial Sequence

<220>
<223> Synthetic Oligonucleotide

<400> 1371
ataccaatca ccgcacaaac ccaggctatt tgttaagtcc agtcacagcg caaagaaaca 60
tatgcggaga aaatgctagt gtg 83

<210> 1372
<211> 67
<212> DNA
<213> Artificial Sequence

<220>
<223> Synthetic Oligonucleotide

<400> 1372
ctgctcaagg acaccgtcta cacggacttc gatgggaccc gagtgtatag ccctccagag 60
tggatcc 67

<210> 1373
<211> 67
<212> DNA
<213> Artificial Sequence

<220>
<223> Synthetic Oligonucleotide

<400> 1373
tggggacatt ccctttgaga gggaccagga gattctggaa gctgagctcc acttcccagc 60
ccatgtc 67

<210> 1374
<211> 76
<212> DNA
<213> Artificial Sequence

<220>
<223> Synthetic Oligonucleotide

<400> 1374
cccatggatg ctcctctgaa gagactttcc tcattgactg ccgaggcccc atgaatcaat 60
gtctggtagc caccgg 76

<210> 1375
<211> 66
<212> DNA
<213> Artificial Sequence

<220>
<223> Synthetic Oligonucleotide

<400> 1375
ccaagttctg ggtggtggac cagacccact tctacctggg cagtgccaac atggactggc 60
gttcac 66

<210> 1376
<211> 69
<212> DNA
<213> Artificial Sequence

<220>
<223> Synthetic Oligonucleotide

<400> 1376
aatgaataca gtattcccaa gcacatcaac cccgtggccg cctccctcat ccagaagatg 60
cttcagaca 69

<210> 1377
<211> 77
<212> DNA
<213> Artificial Sequence

<220>
<223> Synthetic Oligonucleotide

<400> 1377
cttacggttt tcgtggagaa gccttggggt caatttgttg tatagctgag gttttaatta 60
caacaagaac ggctgct 77

<210> 1378
<211> 75
<212> DNA
<213> Artificial Sequence

<220>
<223> Synthetic Oligonucleotide

<400> 1378
gatgtggact gccattcaaa ccaggaagat accggatgta aatttcgagt tttgcctcag 60
ccaactaatc tcgca 75

<210> 1379
<211> 66
<212> DNA
<213> Artificial Sequence

<220>
<223> Synthetic Oligonucleotide

<400> 1379
ccacataccg tccagcctat ctactggaga acgaagaaga ggagcggaac tcccgcacat 60
gacctc 66

<210> 1380
<211> 78
<212> DNA
<213> Artificial Sequence

<220>
<223> Synthetic Oligonucleotide

<400> 1380
gcaatcatgg aacttgacga tactctaaaa tactctttct tgcagtttga cccagcacct 60
cgtagaggcg agccacat 78

<210> 1381
<211> 85
<212> DNA
<213> Artificial Sequence

<220>
<223> Synthetic Oligonucleotide

<400> 1381
gcatcaggct gtcattatgg tgtccttacc tgtgggagct gtaaggtctt ctttaagagg 60
gcaatggaag ggcagcacaa ctact 85

<210> 1382
<211> 67
<212> DNA
<213> Artificial Sequence

<220>
<223> Synthetic Oligonucleotide

<400> 1382
aggactggga cccatgaaca ttcctttggt atcagacccg aagcgcacca ttgctcagga 60
ttatggg 67

<210> 1383
<211> 69
<212> DNA
<213> Artificial Sequence

<220>
<223> Synthetic Oligonucleotide

<400> 1383
ggtgtccttc gccagatcac tgttaatgat ttgcctgtgg gacgctccgt ggatgaggct 60
ctgcggctg 69

<210> 1384
<211> 70
<212> DNA
<213> Artificial Sequence

<220>
<223> Synthetic Oligonucleotide

<400> 1384
caagcaatgc gtcatcaatg tccccagcct ctgcggaatg gatcacactg agaagagggg 60
gcggatttac 70

<210> 1385
<211> 68
<212> DNA
<213> Artificial Sequence

<220>
<223> Synthetic Oligonucleotide

<400> 1385
ctgacacttg ccgcagagaa tccctttctc acccacctca tctgcacctt ccagaccaag 60
gaccacct 68

<210> 1386
<211> 66
<212> DNA
<213> Artificial Sequence

<220>
<223> Synthetic Oligonucleotide

<400> 1386
gggttctaga cgcccctccc aagcgttcct ggccttctga actccataca gcctctacag 60
ccgtcc 66

<210> 1387
<211> 68
<212> DNA
<213> Artificial Sequence

<220>
<223> Synthetic Oligonucleotide

<400> 1387
ctccacctat gagcgtctgt ctctgtgggc ttgggatgtt aacaggagcc aaaaggaggg 60
aaagtgtg 68

<210> 1388
<211> 86
<212> DNA
<213> Artificial Sequence

<220>
<223> Synthetic Oligonucleotide

<400> 1388
gccctcccag tgtgcaaata agggctgctg tttcgacgac accgttcgtg gggtcccctg 60
gtgcttctat cctaatacca tcgacg 86

<210> 1389
<211> 81

<212> DNA
<213> Artificial Sequence

<220>
<223> Synthetic Oligonucleotide

<400> 1389
tggctaagtg aagatgacaa tcatgttgca gcaattcact gtaaagctgg aaagggacga 60
actggtgtaa tgatatgtgc a 81

<210> 1390
<211> 81
<212> DNA
<213> Artificial Sequence

<220>
<223> Synthetic Oligonucleotide

<400> 1390
cgcttgccta actcatactt tcccgttgac acttgatcca cgcagcgtgg cactgggacg 60
taagtggcgc agtctgaatg g 81

<210> 1391
<211> 74
<212> DNA
<213> Artificial Sequence

<220>
<223> Synthetic Oligonucleotide

<400> 1391
ggctactctg atctatgttg ataaggaaaa tggagaacca ggcacccgtg tggttgctaa 60
ggatgggctg aagc 74

<210> 1392
<211> 65
<212> DNA
<213> Artificial Sequence

<220>
<223> Synthetic Oligonucleotide

<400> 1392
gggacactgc gggacaagag cggttccgga gtctcaccac tgcatttttc agagacgcca 60
tgggc 65

<210> 1393
<211> 67
<212> DNA
<213> Artificial Sequence

<220>
<223> Synthetic Oligonucleotide

<400> 1393
ctgaaggacc ctacgctcgg tggcctggca cctcactttg agaagagtga gcacactggc 60
tttgcat 67

<210> 1394
<211> 78
<212> DNA
<213> Artificial Sequence

<220>
<223> Synthetic Oligonucleotide

<400> 1394
gcgacagctc ctctagttcc accatgtccg cgggcggaga cttcgggaat ccgctgagga 60
aattcaagct ggtgttcc 78

<210> 1395
<211> 71
<212> DNA
<213> Artificial Sequence

<220>
<223> Synthetic Oligonucleotide

<400> 1395
gaggagtggt gacagtctgc aaaatcaata cacaggaacc tgaggagacc ctggactttg 60
atttctgcag c 71

<210> 1396
<211> 67
<212> DNA
<213> Artificial Sequence

<220>
<223> Synthetic Oligonucleotide

<400> 1396
agctagcctc agtgacacac atgacaggtt gcactgccga cgttgtgtca acagccgtca 60
gatccgg 67

<210> 1397
<211> 73
<212> DNA
<213> Artificial Sequence

<220>
<223> Synthetic Oligonucleotide

<400> 1397
cgtcgtatgc gagagtctgt ttccaggatg cctgttagtt ctcagcacag atattctaca 60
cctcacgcct tca 73

<210> 1398
<211> 84
<212> DNA
<213> Artificial Sequence

<220>
<223> Synthetic Oligonucleotide

<400> 1398
ggtgtcagat ataaatgtgc aaatgccttc ttgctgtcct gtcggtctca gtacgttcac 60
tttatagctg ctggcaatat cgaa 84

<210> 1399
<211> 67
<212> DNA
<213> Artificial Sequence

<220>
<223> Synthetic Oligonucleotide

<400> 1399
tgtggatgct ggattgattt caccactggt gcagctgcta aatagcaaag accaggaagt 60
gctgctt 67

<210> 1400
<211> 69
<212> DNA
<213> Artificial Sequence

<220>
<223> Synthetic Oligonucleotide

<400> 1400
agtgggagac acctgacctt tctcaagctg agattgagca gaagatcaag gagtacaatg 60
cccagatca 69

<210> 1401
<211> 77
<212> DNA
<213> Artificial Sequence

<220>
<223> Synthetic Oligonucleotide

<400> 1401
cgaagccctt acaagtttcc tagttcaccc ttacggattc ctggagggaa catctatatt 60
tcacccctga agagtcc 77

<210> 1402
<211> 66
<212> DNA
<213> Artificial Sequence

<220>
<223> Synthetic Oligonucleotide

<400> 1402
cccagtgtac gaggaacaag acagaccgag atctccaacc ggacctagca actccttcct 60
cgctaa 66

<210> 1403
<211> 66
<212> DNA
<213> Artificial Sequence

<220>
<223> Synthetic Oligonucleotide

<400> 1403
gggctgggtg agaatgtgat ggagaggaag aagccggccc tggtatccat tccggaggat 60
gttgtg 66

<210> 1404
<211> 68
<212> DNA
<213> Artificial Sequence

<220>
<223> Synthetic Oligonucleotide

<400> 1404
cctacaagtc ctggggcatt ggagccccaa gcagtgttaa tcctggctac tgtgtgagcc 60
tgacctca 68

<210> 1405
<211> 67
<212> DNA
<213> Artificial Sequence

<220>
<223> Synthetic Oligonucleotide

<400> 1405
gcgaggagct ctacttgctc tgcgacaagg tgcagaaaga ggacatatca gtggtgttca 60
gcagggc 67

<210> 1406
<211> 67
<212> DNA
<213> Artificial Sequence

<220>
<223> Synthetic Oligonucleotide

<400> 1406
aagcatgaac aggacttgac catctttcca acccctgggg aagacatttg caactgactt 60
ggggagg 67

<210> 1407
<211> 68
<212> DNA
<213> Artificial Sequence

<220>
<223> Synthetic Oligonucleotide

<400> 1407
cccgttcggt ctgaggaagg ccgggacatg gcgaaccgga tcagtgcctt tggctacctt 60
gagtgctc 68

<210> 1408
<211> 74
<212> DNA
<213> Artificial Sequence

<220>
<223> Synthetic Oligonucleotide

<400> 1408
ccagacgagc gattagaagc ggcagcttgt gaggtgaatg atttggggga agaggaggag 60
gaggaagagg agga 74

<210> 1409
<211> 73
<212> DNA
<213> Artificial Sequence

<220>
<223> Synthetic Oligonucleotide

<400> 1409
tgtgcacata ggaatgagct tcagatgcag ttggccagca aagagagtga tattgagcaa 60
ttgcgtgcta aac 73

<210> 1410
<211> 74

<212> DNA
<213> Artificial Sequence

<220>
<223> Synthetic Oligonucleotide

<400> 1410
gatctggcac tgtggttcct gcatgaagac agtggctggc ggtgcctgga cgtacaatac 60
cacttccgct gtca 74

<210> 1411
<211> 75
<212> DNA
<213> Artificial Sequence

<220>
<223> Synthetic Oligonucleotide

<400> 1411
ccattctatc atcaacgggt acaaacgagt cctggccttg tctgtggaga cggattacac 60
cttcccactt gctga 75

<210> 1412
<211> 67
<212> DNA
<213> Artificial Sequence

<220>
<223> Synthetic Oligonucleotide

<400> 1412
ctgtcttcct gttggccctg acaatcatag ccagcacctg ggctctgacg cccactcact 60
acctcac 67

<210> 1413
<211> 81
<212> DNA
<213> Artificial Sequence

<220>
<223> Synthetic Oligonucleotide

<400> 1413
gctcattatg aaaaacatcc caaactttaa aatgcgaaat tattggttgg tgtgaagaaa 60
gccagacaac ttctgtttct t 81

<210> 1414
<211> 66
<212> DNA
<213> Artificial Sequence

<220>
<223> Synthetic Oligonucleotide

<400> 1414
gctctgttgt gtcctgttgc cggctctggc cttcctgtga ctgactgtga agtggcttct 60
ccgtac 66

<210> 1415
<211> 71
<212> DNA
<213> Artificial Sequence

<220>
<223> Synthetic Oligonucleotide

<400> 1415
cgaggagatg cctgtggaca ggatcctgga ggcagagctt gctgtggaac agaagagtga 60
ccagggcgtt g 71

<210> 1416
<211> 77
<212> DNA
<213> Artificial Sequence

<220>
<223> Synthetic Oligonucleotide

<400> 1416
acaccaaaat gccatctcaa atggaacacg ccatggaaac catgatgttt acatttcaca 60
aattcgctgg ggataaa 77

<210> 1417
<211> 67
<212> DNA
<213> Artificial Sequence

<220>
<223> Synthetic Oligonucleotide

<400> 1417
cttctgagcc cgtctcccgg acaggttgag gaagctgctc cagaagcgcc tcggaagggg 60
agctctc 67

<210> 1418
<211> 86
<212> DNA
<213> Artificial Sequence

<220>
<223> Synthetic Oligonucleotide

<400> 1418
cgcgagcccc tcattataca ctgggcagcc tccccacagc gcatcgagga atgcgtgctc 60
tcaggcaagg atgtcaacgg cgagtg 86

<210> 1419
<211> 70
<212> DNA
<213> Artificial Sequence

<220>
<223> Synthetic Oligonucleotide

<400> 1419
gagagagcca gtctgatcca gaaggccaag ctggcagagc aggccgaacg ctatgaggac 60
atggcagcct 70

<210> 1420
<211> 77
<212> DNA
<213> Artificial Sequence

<220>
<223> Synthetic Oligonucleotide

<400> 1420
cagtggagac cagttgggta gtggtgactg ggtacgctac aagctctgca tgtgtgctga 60
tgggacgctc ttcaagg 77

<210> 1421
<211> 73
<212> DNA
<213> Artificial Sequence

<220>
<223> Synthetic Oligonucleotide

<400> 1421
tccgcaagac acctcctgga gggcctcctg cagaaggaca ggacaaagcg gctcggggcc 60
aaggatgact tca 73

<210> 1422
<211> 71
<212> DNA
<213> Artificial Sequence

<220>
<223> Synthetic Oligonucleotide

<400> 1422
tgcattcgtt ggtttctctt atgcacctcc ttcagaagac ttatttttgt gagcagtttg 60
ccattcagaa a 71

<210> 1423
<211> 71
<212> DNA
<213> Artificial Sequence

<220>
<223> Synthetic Oligonucleotide

<400> 1423
ccaacacctt cttggcttct gggacctgtg ttcttgctga gcaccctctc cggtttgggt 60
tgggataaca g 71

<210> 1424
<211> 72
<212> DNA
<213> Artificial Sequence

<220>
<223> Synthetic Oligonucleotide

<400> 1424
agctggggtg tctgtttcat gtggaatacc tgacttcagg tcaagggatg gtatttatgc 60
tcgccttgct gt 72

<210> 1425
<211> 67
<212> DNA
<213> Artificial Sequence

<220>
<223> Synthetic Oligonucleotide

<400> 1425
gtggggagcc catcatctcg ccaagccatc acaggctctg catacacatg cactcagtgt 60
ggactgg 67

<210> 1426
<211> 64
<212> DNA
<213> Artificial Sequence

<220>
<223> Synthetic Oligonucleotide

<400> 1426
tctgccagtg ctgaattctt tgctgacatt gccctggctc ctatggaagc tgctaaggtt 60
cgaa 64

<210> 1427
<211> 67
<212> DNA
<213> Artificial Sequence

<220>
<223> Synthetic Oligonucleotide

<400> 1427
   cccaactcag gtccttggta aatcctgcaa gccaatccca gtcatgctcc ttggggtgac 60
   cctcttg 67

<210> 1428
<211> 70
<212> DNA
<213> Artificial Sequence

<220>
<223> Synthetic Oligonucleotide

<400> 1428
agatgcatac ttggaagcac agtcatatca cactgggagg caatgcaatg tggttacctg 60
gtcctaggtt 70

<210> 1429
<211> 70
<212> DNA
<213> Artificial Sequence

<220>
<223> Synthetic Oligonucleotide

<400> 1429
gcgcagaggc cagttaaagt agatcacctc ctcgaaccca ctccggttcc ccgcaaccca 60
cagctcagct 70

<210> 1430
<211> 76
<212> DNA
<213> Artificial Sequence

<220>
<223> Synthetic Oligonucleotide

<400> 1430
ggctggccaa acataagcag ctgcactgcg atgcccagtc tagaaaatct ttcagctgta 60
aatactgtga caagga 76

<210> 1431
<211> 66

<212> DNA
<213> Artificial Sequence

<220>
<223> Synthetic Oligonucleotide

<400> 1431
agtgacaaat gttggaggag cagtggtgac gggtgtgaca gcagtagccc agaagacagt 60
ggaggg 66

<210> 1432
<211> 80
<212> DNA
<213> Artificial Sequence

<220>
<223> Synthetic Oligonucleotide

<400> 1432
acccaccatg gatgtcttca agaagggctt ctccatcgcc aaggagggcg tggtgggtgc 60
ggtggaaaag accaagcagg 80

<210> 1433
<211> 70
<212> DNA
<213> Artificial Sequence

<220>
<223> Synthetic Oligonucleotide

<400> 1433
tgaagagagg catgttggag acttgggcaa tgtgactgct gacaaagatg gtgtggccga 60
tgtgtctatt 70

<210> 1434
<211> 74
<212> DNA
<213> Artificial Sequence

<220>
<223> Synthetic Oligonucleotide

<400> 1434
tgaggagtgg tattttggca agatcaccag acgggagtca gagcggttac tgctcaatgc 60
agagaacccg agag 74

<210> 1435
<211> 74
<212> DNA
<213> Artificial Sequence

<220>
<223> Synthetic Oligonucleotide

<400> 1435
atacagcacc aatggaaaga tcaccttcga cgactacatc gcctgctgcg tcaaactgag 60
ggctcttaca gaca 74

<210> 1436
<211> 81
<212> DNA
<213> Artificial Sequence

<220>
<223> Synthetic Oligonucleotide

<400> 1436
gggctcagct ttcagaagtg ctgagttggc agttttcttc tgtcaccaaa agaggtctca 60
atgtggacca gctgaacatg t 81

<210> 1437
<211> 70
<212> DNA
<213> Artificial Sequence

<220>
<223> Synthetic Oligonucleotide

<400> 1437
tcacatgcca ctttggtgtt tcataatctc ctgggagaga ttgaccagca gtatagccgc 60
ttcctgcaag 70

<210> 1438
<211> 76
<212> DNA
<213> Artificial Sequence

<220>
<223> Synthetic Oligonucleotide

<400> 1438
caagagggac tcggactgca gcagcctctg cacctctgag agcatggact atggtaccaa 60
tctctccact gacctg 76

<210> 1439
<211> 66
<212> DNA
<213> Artificial Sequence

<220>
<223> Synthetic Oligonucleotide

<400> 1439
ggactcggag acgctgtgca ggagggccgt caagatcctc aagaagaaga agttgcgaag 60
gatccc 66

<210> 1440
<211> 69
<212> DNA
<213> Artificial Sequence

<220>
<223> Synthetic Oligonucleotide

<400> 1440
catcttccag gaggaccact ctctgtggca ccctggacta cctgccccct gaaatgattg 60
aaggtcgga 69

<210> 1441
<211> 71
<212> DNA
<213> Artificial Sequence

<220>
<223> Synthetic Oligonucleotide

<400> 1441
aatacccaac gcacaaatga ccgcacgttc tctgccccgt ttcttgcccc agtgtggttt 60
gcattgtctc c 71

<210> 1442
<211> 90
<212> DNA
<213> Artificial Sequence

<220>
<223> Synthetic Oligonucleotide

<400> 1442
cctggaggct gcaacatacc tcaatcctgt cccaggccgg atcctcctga agccctttc 60
gcagcactgc tatcctccaa agccattgta 90

<210> 1443
<211> 80
<212> DNA
<213> Artificial Sequence

<220>
<223> Synthetic Oligonucleotide

<400> 1443
tgttttgatt cccgggctta ccaggtgaga agtgagggag gaagaaggca gtgtcccttt 60
tgctagagct gacagctttg 80

<210> 1444
<211> 65
<212> DNA
<213> Artificial Sequence

<220>
<223> Synthetic Oligonucleotide

<400> 1444
cacccttgga ctggaaaact cacacccggt ctggacacag aaagagaacc aacagctcat 60
caagg 65

<210> 1445
<211> 66
<212> DNA
<213> Artificial Sequence

<220>
<223> Synthetic Oligonucleotide

<400> 1445
gatcctcgcg tgagacagat caagatcaag accggcgtgg tgaagcggtt ggtcaaagaa 60
aaagtg 66

<210> 1446
<211> 68
<212> DNA
<213> Artificial Sequence

<220>
<223> Synthetic Oligonucleotide

<400> 1446
ctgttttcct ggaggactgc agtgactgcg tgctggcagt ggcctgccaa cagctccgca 60
tacacagt 68

<210> 1447
<211> 66
<212> DNA
<213> Artificial Sequence

<220>
<223> Synthetic Oligonucleotide

<400> 1447
cagccaggtg tacgagacat tgctcaccta cagtgacgtc gtgggcgcgg atgtgctgga 60
cgaggt 66

<210> 1448
<211> 66
<212> DNA
<213> Artificial Sequence

<220>
<223> Synthetic Oligonucleotide

<400> 1448
tcccgagaga ggaaagcatg atgggagcca cgaagggact gtgtatttta aatgcaggca 60
cccgac 66

<210> 1449
<211> 74
<212> DNA
<213> Artificial Sequence

<220>
<223> Synthetic Oligonucleotide

<400> 1449
cctggttgaa gcctgttaat gctttcaacg tgtggaaaac ccagcgggcc tttagcaaat 60
atgagaagtc tgca 74

<210> 1450
<211> 68
<212> DNA
<213> Artificial Sequence

<220>
<223> Synthetic Oligonucleotide

<400> 1450
ccagtgtgtg taacagggtc acaagaattc gacagccaga tgctccaaga gggtggccca 60
aggctata 68

<210> 1451
<211> 68
<212> DNA
<213> Artificial Sequence

<220>
<223> Synthetic Oligonucleotide

<400> 1451
gccaactgct ttcatttgtg agggatctga accaatacag agcagacata aaggaaatgg 60
gcctgagt 68

<210> 1452
<211> 85

<212> DNA
<213> Artificial Sequence

<220>
<223> Synthetic Oligonucleotide

<400> 1452
catccgcaaa gtgactgaag agaacaaaga gttggccaat gagctgaggc ggcctcccct 60
atgctatcac aacggagttc agtac 85

<210> 1453
<211> 84
<212> DNA
<213> Artificial Sequence

<220>
<223> Synthetic Oligonucleotide

<400> 1453
gccgggaaga ccgtaattgt ggctgcactg gatgggacct tccagaggaa gccatttggg 60
gccatcctga acctggtgcc gctg 84

<210> 1454
<211> 72
<212> DNA
<213> Artificial Sequence

<220>
<223> Synthetic Oligonucleotide

<400> 1454
aatccaaggg ggagagtgat gacttccata tggactttga ctcagctgtg gctcctcggg 60
caaaatctgt ac 72

<210> 1455
<211> 66
<212> DNA
<213> Artificial Sequence

<220>
<223> Synthetic Oligonucleotide

<400> 1455
gtgatgcctt ccctgtgggc gaggtgaaga tgctggagaa gcagacgaac ccacccgact 60
acctga 66

<210> 1456
<211> 82
<212> DNA
<213> Artificial Sequence

<220>
<223> Synthetic Oligonucleotide

<400> 1456
ctatatgcag ccagagatgt gacagccacc gtggacagcc tgccactcat cacagcctcc 60
attctcagta agaaactcgt gg 82

<210> 1457
<211> 74
<212> DNA
<213> Artificial Sequence

<220>
<223> Synthetic Oligonucleotide

<400> 1457
tgctgttgct gagtctgttg ccagtcccca gaagaccatg tctgtgttga gctgtatctg 60
tgaagccagg caag 74

<210> 1458
<211> 81
<212> DNA
<213> Artificial Sequence

<220>
<223> Synthetic Oligonucleotide

<400> 1458
ggtgtcgata ttgtcatgaa ccatcacctg caggaaacaa gtttcacaaa agaagcctac 60
aagaagtaca tcaaagatta c 81

<210> 1459
<211> 66
<212> DNA
<213> Artificial Sequence

<220>
<223> Synthetic Oligonucleotide

<400> 1459
gacgctggtc tggtgaagat gtccaggaaa ccacgagcct ccagcccatt gtccaacaac 60
caccca 66

<210> 1460
<211> 73
<212> DNA
<213> Artificial Sequence

<220>
<223> Synthetic Oligonucleotide

<400> 1460
cttcacagtg ctcctgcagt ctctctgtgt ggctgtaact tacgtgtact ttaccaacga 60
gctgaagcag atg 73

<210> 1461
<211> 65
<212> DNA
<213> Artificial Sequence

<220>
<223> Synthetic Oligonucleotide

<400> 1461
gcctcggtgt gcctttcaac atcgccagct acgccctgct cacgtacatg attgcgcaca 60
tcacg 65

<210> 1462
<211> 66
<212> DNA
<213> Artificial Sequence

<220>
<223> Synthetic Oligonucleotide

<400> 1462
ctggtcagcc ttcagggacc ctgagcaccg cctggtctct ttcctgtggc cagcccagaa 60
ctgaag 66

<210> 1463
<211> 76
<212> DNA
<213> Artificial Sequence

<220>
<223> Synthetic Oligonucleotide

<400> 1463
tgcttgtcag ttgtcaacac cttatggcca acctgcctgt ttccagcagc aacagcatca 60
aatacttgcc actcca 76

<210> 1464
<211> 68
<212> DNA
<213> Artificial Sequence

<220>
<223> Synthetic Oligonucleotide

<400> 1464
tgtcaccccg actcaacgtg agacgcaccg cccggactca ccatgcgtga atgcatctca 60
gtccacgt 68

<210> 1465
<211> 62
<212> DNA
<213> Artificial Sequence

<220>
<223> Synthetic Oligonucleotide

<400> 1465
agctcaacat gcgtgagtgt atctctatcc acgtggggca ggcaggagtc cagatcggca 60
at 62

<210> 1466
<211> 71
<212> DNA
<213> Artificial Sequence

<220>
<223> Synthetic Oligonucleotide

<400> 1466
ctcttacatc gaccgcctaa gagtcgcgct gtaagaagca acaacctctc ctcttcgtct 60
ccgccatcag c 71

<210> 1467
<211> 71
<212> DNA
<213> Artificial Sequence

<220>
<223> Synthetic Oligonucleotide

<400> 1467
gaggagggtg agttctccaa ggcccatgag gatatgactg ccctggagaa ggattacaag 60
gaggtgggca t 71

<210> 1468
<211> 71
<212> DNA
<213> Artificial Sequence

<220>
<223> Synthetic Oligonucleotide

<400> 1468
gtcccttcgc ctccttcacc gccgcagacc ccttcaagtt ctagtcatgc gtgagtgcat 60
ctccatccac g 71

<210> 1469
<211> 68
<212> DNA
<213> Artificial Sequence

<220>
<223> Synthetic Oligonucleotide

<400> 1469
cgccctacct ataccaacct caaccgcctc atcagtcaga ttgtgtcctc aatcactgct 60
tctctccg 68

<210> 1470
<211> 73
<212> DNA
<213> Artificial Sequence

<220>
<223> Synthetic Oligonucleotide

<400> 1470
cgaggacgag gcttaaaaac ttctcagatc aatcgtgcat ccttagtgaa cttctgttgt 60
cctcaagcat ggt 73

<210> 1471
<211> 70
<212> DNA
<213> Artificial Sequence

<220>
<223> Synthetic Oligonucleotide

<400> 1471
cgccctcctg cagtatttat ggcctcgtcc tcccccacct aggccacgtg tgagctgctc 60
ctgtctctgt 70

<210> 1472
<211> 84
<212> DNA
<213> Artificial Sequence

<220>
<223> Synthetic Oligonucleotide

<400> 1472
acactgactg gcatcctgct ttccagtgcc tgccagcctc cagaagagcc aggtgcctga 60
ctagtacatg gggagctaca gagc 84

<210> 1473
<211> 70

<212> DNA
<213> Artificial Sequence

<220>
<223> Synthetic Oligonucleotide

<400> 1473
gtggcctaga gccttcagtc actggggaaa gcagggaagc agtgtgaact ctttattcac 60
tcccagcctg 70

<210> 1474
<211> 73
<212> DNA
<213> Artificial Sequence

<220>
<223> Synthetic Oligonucleotide

<400> 1474
acaggcccca tgcatcctcc ctgcctcact cccctcagcc cctgccgacc ttagcttatc 60
tgggagagaa aca 73

<210> 1475
<211> 80
<212> DNA
<213> Artificial Sequence

<220>
<223> Synthetic Oligonucleotide

<400> 1475
ccctatggcc ctgaatggtg cactggttta attgtgttgg tgtcggcccc tcacaaatgc 60
agccaagtca tgtaattagt 80

<210> 1476
<211> 75
<212> DNA
<213> Artificial Sequence

<220>
<223> Synthetic Oligonucleotide

<400> 1476
agtggaatcc ttccctttcc aactctacct ccctcactca gctcctttcc cctgatcaga 60
gaaagggatc aaggg 75

<210> 1477
<211> 75
<212> DNA
<213> Artificial Sequence

<220>
<223> Synthetic Oligonucleotide

<400> 1477
ggaaggaaag aagcatggtc tactttaggt gtgcgctggg tctctggtgc tcttcactgt 60
tgcctgtcac ttttt 75

<210> 1478
<211> 67
<212> DNA
<213> Artificial Sequence

<220>
<223> Synthetic Oligonucleotide

<400> 1478
gatgccgagg gaaatcatca ccctacagtt gggccagtgc ggcaatcaga ttgggttcga 60
gttctgg 67

<210> 1479
<211> 64
<212> DNA
<213> Artificial Sequence

<220>
<223> Synthetic Oligonucleotide

<400> 1479
gcgctgcgga agatcatccc cacgctgccc tcggacaagc tgagcaagat tcagaccctc 60
aagc 64

<210> 1480
<211> 67
<212> DNA
<213> Artificial Sequence

<220>
<223> Synthetic Oligonucleotide

<400> 1480
cagtgtggag gggatggagg agcctgacat ccagtgggtg aaggatgggg ctgtggtcca 60
gaacttg 67

<210> 1481
<211> 69
<212> DNA
<213> Artificial Sequence

<220>
<223> Synthetic Oligonucleotide

<400> 1481
agttgtcgtg tgttctggat tcattccggc accaccatgt cgaaggtttc ctttaagatc 60
acgctgacg 69

<210> 1482
<211> 70
<212> DNA
<213> Artificial Sequence

<220>
<223> Synthetic Oligonucleotide

<400> 1482
gtggatgtgc cctgaaggac aagccaggcg tctacacgag agtctcacac ttcttaccct 60
ggatccgcag 70

<210> 1483
<211> 89
<212> DNA
<213> Artificial Sequence

<220>
<223> Synthetic Oligonucleotide

<400> 1483
tggcttcagg agctgaatac cctcccaggc acacacaggt gggacacaaa taagggtttt 60
ggaaccacta ttttctcatc acgacagca 89

<210> 1484
<211> 71
<212> DNA
<213> Artificial Sequence

<220>
<223> Synthetic Oligonucleotide

<400> 1484
ctgctgtctt gggtgcattg gagccttgcc ttgctgctct acctccacca tgccaagtgg 60
tcccaggctg c 71

<210> 1485
<211> 71
<212> DNA
<213> Artificial Sequence

<220>
<223> Synthetic Oligonucleotide

<400> 1485
tgacgatggc ctggagtgtg tgcccactgg gcagcaccaa gtccggatgc agatcctcat 60
gatccggtac c 71

<210> 1486
<211> 83
<212> DNA
<213> Artificial Sequence

<220>
<223> Synthetic Oligonucleotide

<400> 1486
cctcagcaag acgttatttg aaattacagt gcctctctct caaggcccca aaccagtaac 60
aatcagtttt gccaatcaca ctt 83

<210> 1487
<211> 67
<212> DNA
<213> Artificial Sequence

<220>
<223> Synthetic Oligonucleotide

<400> 1487
cggttggtga cttgtctgcc tcctgctttg ggaagactga ggcatccgtg aggcagggac 60
aagtctt 67

<210> 1488
<211> 72
<212> DNA
<213> Artificial Sequence

<220>
<223> Synthetic Oligonucleotide

<400> 1488
tgcccttaaa ggaaccaatg agtccctgga acgccagatg cgtgaaatgg aagagaactt 60
tgccgttgaa gc 72

<210> 1489
<211> 84
<212> DNA
<213> Artificial Sequence

<220>
<223> Synthetic Oligonucleotide

<400> 1489
ggttgtgctc tacgagctta tgactggctc actgccttac agccacattg gctgccgtga 60
ccagattatc tttatggtgg gccg 84

<210> 1490
<211> 68
<212> DNA
<213> Artificial Sequence

<220>
<223> Synthetic Oligonucleotide

<400> 1490
ctctccagtg tgggcaccag ccggccagaa cagatgcgag cagtccatga ctctgggagc 60
tacaccgc 68

<210> 1491
<211> 75
<212> DNA
<213> Artificial Sequence

<220>
<223> Synthetic Oligonucleotide

<400> 1491
gtatcaggac cacatgcagt acatcggaga aggcgcgaag acaggcatca aagaatgcca 60
gtatcaattc cgaca 75

<210> 1492
<211> 77
<212> DNA
<213> Artificial Sequence

<220>
<223> Synthetic Oligonucleotide

<400> 1492
gcagttggaa gacacaggaa agtatcccca aattgcagat ttatcaacgg cttttatctt 60
gaaaatagtg ccacgca 77

<210> 1493
<211> 75
<212> DNA
<213> Artificial Sequence

<220>
<223> Synthetic Oligonucleotide

<400> 1493
caggtcaggc agaggaagtc atgtgcattg catgagctaa acctatctga atgaattgat 60
ttggggcttg ttagg 75

<210> 1494
<211> 66

<212> DNA
<213> Artificial Sequence

<220>
<223> Synthetic Oligonucleotide

<400> 1494
tggtcagatg ctgctgaagt atatccttag gccgctggca tcttgcccat cccttcatgc 60
tgtgat 66

<210> 1495
<211> 66
<212> DNA
<213> Artificial Sequence

<220>
<223> Synthetic Oligonucleotide

<400> 1495
gagggagcag acagtgggta ccacgatctc cgtaaccatt tgcatgtgac ttagcaaggg 60
ctctga 66

<210> 1496
<211> 66
<212> DNA
<213> Artificial Sequence

<220>
<223> Synthetic Oligonucleotide

<400> 1496
tagaggccat caaaattggc ctcaccaagg ccctgactca gatggaggaa gcccagagga 60
aacgga 66

## Description

This application claims priority benefit of United States Provisional Application Serial Nos. 61/052,573, filed May 12, 2008, and 61/057,182 filed May 29, 2008, the entire disclosures of which are incorporated herein by reference in their entirety.

The present invention provides genes and gene sets, the expression levels of which are useful for predicting response of cancer patients to chemotherapy. The invention further concerns tests using such molecular markers, arrays and kits for use in such methods, and reports comprising the results and/or conclusions of such tests.

For many patients with cancer, treatment may include surgical resection of the tumour, hormonal therapy, and chemotherapy. A range of chemotherapy choices are available. Ideally, the choice for an individual patient takes into account both the risk of cancer recurrence and the likelihood that the patient will respond to the chemotherapy chosen.

One critical issue in treatment of breast cancer is the identification of which patients are likely to respond to a standard chemotherapy (e.g. an anthracycline and a cyclophosphamide) and which patients are less likely to respond to standard chemotherapy and should therefore be considered for more aggressive chemotherapy (e.g., a chemotherapy regimen that includes a taxane). Currently, no satisfactory tests are available for identifying patients more likely to respond to standard chemotherapy as opposed to treatment with a taxane-containing treatment regimen.

The present disclosure provides methods and compositions to facilitate prediction of the likelihood of responsiveness of cancer patients to treatment including a taxane and/or a cyclophosphamide.

The present disclosure provides methods of predicting whether a hormone receptor (HR) positive cancer patient will exhibit a beneficial response to chemotherapy, where the method involves measuring an expression level of a gene, or its expression product, in a tumour sample obtained from the patient, wherein the gene is selected from the group consisting of ABCC1, ABCC5, ABCD1, ACTB, ACTR2, AKT1, AKT2, APC, APOC1, APOE, APRT, BAK1, BAX, BBC3, BCL2L11, BCL2L13, BID, BUB1, BUB3, CAPZA1, CCT3, CD14, CDC25B, CDCA8, CHEK2, CHFR, CSNK1D, CST7, CXCR4, DDR1, DICER1, DUSP1, ECGF1, EIF4E2, ERBB4, ESR1, FAS, GADD45B, GATA3, GCLC, GDF15, GNS, HDAC6, HSPA1A, HSPA1B, HSPA9B, IL7, ILK, LAPTM4B, LILRB1, LIMK2, MAD2L1BP, MAP2K3, MAPK3, MAPRE1, MCL1, MRE11A, NEK2, NFKB1, NME6, NTSR2, PLAU, PLD3, PPP2CA, PRDX1, PRKCH, RAD1, RASSF1, RCC1, REG1A, RELA, RHOA, RHOB, RPN2, RXRA, SHC1, SIRT1, SLC1A3, SLC35B1, SRC, STK10, STMN1, TBCC, TBCD, TNFRSF10A, TOP3B, TSPAN4, TUBA3, TUBA6, TUBB, TUBB2C, UFM1, VEGF, VEGFB, VHL, ZW10, and ZWILCH; using the expression level to determine a likelihood of a beneficial response to a treatment including a taxane, wherein expression of DDR1, EIF4E2, TBCC, STK10, ZW10, BBC3, BAX, BAK1, TSPAN4, SLC1A3, SHC1, CHFR, RHOB, TUBA6, BCL2L13, MAPRE1, GADD45B, HSPA1B, FAS, TUBB, HSPA1A, MCL1, CCT3, VEGF, TUBB2C, AKT1, MAD2L1BP, RPN2, RHOA, MAP2K3, BID, APOE, ESR1, ILK, NTSR2, TOP3B, PLD3, DICER1, VHL, GCLC, RAD1, GATA3, CXCR4, NME6, UFM1, BUB3, CD14, MRE11A, CST7, APOC1, GNS, ABCC5, AKT2, APRT, PLAU, RCC1, CAPZA1, RELA, NFKB1, RASSF1, BCL2L11, CSNK1D, SRC, LIMK2, SIRT1, RXRA, ABCD1, MAPK3, DUSP1, ABCC1, PRKCH, PRDX1, TUBA3, VEGFB, LILRB1, LAPTM4B, HSPA9B, ECGF1, GDF15, ACTR2, IL7, HDAC6, CHEK2, REG1A, APC, SLC35B1, ACTB, PPP2CA, TNFRSF10A, TBCD, ERBB4, CDC25B, or STMN1 is positively correlated with increased likelihood of a beneficial response to a treatment including a taxane, and wherein expression of CDCA8, ZWILCH, NEK2, or BUB1 is negatively correlated with an increased likelihood of a beneficial response to a treatment including a taxane; and generating a report including information based on the likelihood of a beneficial response to chemotherapy including a taxane.

The methods can further involves using a gene expression level to determine a likelihood of a beneficial response to a treatment including a cyclophosphamide, wherein expression of ZW10, BAX, GADD45B, FAS, ESR1, NME6, MRE11A, AKT2, RELA, RASSF1, PRKCH, VEGFB, LILRB1, ACTR2, REG1A, or PPP2CA is positively correlated with increased likelihood of a beneficial response to a treatment including a cyclophosphamide, and wherein expression of DDR1, EIF4E2, TBCC, STK10, BBC3, BAK1, TSPAN4, SHC1, CHFR, RHOB, TUBA6, BCL2L13, MAPRE1, HSPA1, TUBB, HSPA1A, MCL1, CCT3, VEGF, TUBB2C, AKT1, MAD2L1BP, RPN2, RHOA, MAP2K3, BID, APOE, ILK, NTSR2, TOP3B, PLD3, DICER1, VHL, GCLC, RAD1, GATA3, CXCR4, UFM1, BUB3, CD14, CST7, APOC1, GNS, ABCC5, APRT, PLAU, RCC1, CAPZA1, NFKB1, BCL2L11, CSNK1D, SRC, LIMK2, SIRT1, RXRA, ABCD1, MAPK3, CDCA8, DUSP1, ABCC1, PRDX1, TUBA3, LAPTM4B, HSPA9B, ECGF1, GDF15, IL7, HDAC6, ZWILCH, CHEK2, APC, SLC35B1, NEK2, ACTB, BUB1, TNFRSF10A, TBCD, ERBB4, CDC25B, or STMN1 is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumour sample can be a formalin-fixed and paraffin-embedded (FPE) or a frozen tumour section.

The methods of the present disclosure includes methods of predicting whether a hormone receptor (HR) positive cancer patient will exhibit a beneficial response to chemotherapy, the methods involve measuring an expression level of a gene, or its expression product, in a tumour sample obtained from the patient, wherein the gene is selected from the group consisting of ABCA9, ABCC1, ABCC10, ABCC3, ABCD1, ACTB, ACTR2, ACTR3, AKT1, AKT2, APC, APEX1, APOC1, APOE, APRT, BAD, BAK1, BAX, BBC3, BCL2, BCL2L1, BCL2L11, BCL2L13, BID, BIRC3, BIRC4, BUB3, CAPZA1, CCT3, CD14, CD247, CD63, CD68, CDC25B, CHEK2, CHFR, CHGA, COL1A1, COL6A3, CRABP1, CSNK1D, CST7, CTSD, CXCR4, CYBA, CYP1B1, DDR1, DIABLO, DICER1, DUSP1, ECGF1, EIF4E2, ELP3, ERBB4, ERCC1, ESR1, FAS, FLAD1, FOS, FOXA1, FUS, FYN, GADD45B, GATA3, GBP1, GBP2, GCLC, GGPS1, GNS, GPX1, HDAC6, HRAS, HSPA1A, HSPA1B, HSPA5, HSPA9B, IGFBP2, IL2RA, IL7, ILK, KDR, KNS2, LAPTM4B, LILRB1, LIMK1, LIMK2, MAD1L1, MAD2L1BP, MAD2L2, MAP2K3, MAP4, MAPK14, MAPK3, MAPRE1, MCL1, MGC52057, MGMT, MMP11, MRE11A, MSH3, NFKB1, NME6, NPC2, NTSR2, PDGFRB, PECAM1, PIK3C2A, PLAU, PLD3, PMS1, PPP2CA, PRDX1, PRKCD, PRKCH, PTEN, PTPN21, RAB6C, RAD1, RASSF1, RB1, RBM17, RCC1, REG1A, RELA, RHOA, RHOB, RHOC, RPN2, RXRA, RXRB, SEC61A1, SGK, SHC1, SIRT1, SLC1A3, SLC35B1, SOD1, SRC, STAT1, STAT3, STK10, STK11, STMN1, TBCC, TBCD, TBCE, TFF1, TNFRSF10A, TNFRSF10B, TOP3B, TP53BP1, TSPAN4, TUBA3, TUBA6, TUBB, TUBB2C, TUBD1, UFM1, VEGF, VEGFB, VEGFC, VHL, XIST, ZW10, and ZWILCH; using the expression level to determine a likelihood of a beneficial response to a treatment including a taxane, wherein expression of DDR1, ZW10, RELA, BAX, RHOB, TSPAN4, BBC3, SHC1, CAPZA1, STK10, TBCC, EIF4E2, MCL1, RASSF1, VEGF, SLC1A3, DICER1, ILK, FAS, RAB6C, ESR1, MRE11A, APOE, BAK1, UFM1, AKT2, SIRT1, BCL2L13, ACTR2, LIMK2, HDAC6, RPN2, PLD3, RHOA, MAPK14, ECGF1, MAPRE1, HSPA1B, GATA3, PPP2CA, ABCD1, MAD2L1BP, VHL, GCLC, ACTB, BCL2L11, PRDX1, LILRB1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, PRKCH, RXRA, PLAU, CD63, CD14, RHOC, STAT1, NPC2, NME6, PDGFRB, MGMTI, GBP1, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, RBM17, TP53BP1, CD247, ABCA9, NTSR2, FOS, TNFRSF10A, MSH3, PTEN, GBP2, STK11, ERBB4, TFF1, ABCC1, IL7, CDC25B, TUBD1, BIRC4, ACTR3, SLC35B1, COL1A1, FOXA1, DUSP1, CXCR4, IL2RA, GGPS1, KNS2, RB1, BCL2L1, XIST, BIRC3, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS 1 is positively correlated with increased likelihood of a beneficial response to a treatment including a taxane, and wherein expression of CHGA, ZWILCH, or CRABP1 is negatively correlated with an increased likelihood of a beneficial response to a treatment including a taxane; and generating a report including information based on the likelihood of a beneficial response to chemotherapy including a taxane.

The methods can further involve using a gene expression level to determine a likelihood of a beneficial response to a treatment including a cyclophosphamide, wherein expression of LILRB1, PRKCH, STAT1, GBP1, CD247, IL7, IL2RA, BIRC3, or CRABP1 is positively correlated with increased likelihood of a beneficial response to a treatment including a cyclophosphamide, and wherein expression of DDR1, ZW10, RELA, BAX, RHOB, TSPAN4, BBC3, SHC1, CAPZA1, STK10, TBCC, EIF4E2, MCL1, RASSF1, VEGF, DICER1, ILK, FAS, RAB6C, ESR1, MRE11A, APOE, BAK1, UFM1, AKT2, SIRT1, BCL2L13, ACTR2, LIMK2, HDAC6, RPN2, PLD3, CHGA, RHOA, MAPK14, ECGF1, MAPRE1, HSPA1B, GATA3, PPP2CA, ABCD1, MAD2L1BP, VHL, GCLC, ACTB, BCL2L11, PRDX1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, RXRA, PLAU, CD63, CD14, RHOC, NPC2, NME6, PDGFRB, MGMTI, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, ZWILCH, RBM17, TP53BP1, ABCA9, NTSR2, FOS, TNFRSF10A, MSH3, PTEN, GBP2, STK11, ERBB4, TFF1, ABCC1, CDC25B, TUBD1, BIRC4, ACTR3, SLC35B1, COL1A1, FOXA1, DUSP1, CXCR4, GGPS1, KNS2, RB1, BCL2L1, XIST, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS1 is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumour sample can be a formalin-fixed and paraffin-embedded (FPE) or a frozen tumour section.

The methods of the present disclosure include methods of predicting whether a hormone receptor (HR) negative cancer patient will exhibit a beneficial response to chemotherapy, where the methods involve measuring an expression level of a gene, or its expression product, in a tumour sample obtained from the patient, wherein the gene is selected from the group consisting of CD247, TYMS, IGF1R, ACTG2, CCND1, CAPZA1, CHEK2, STMN1, and ZWILCH; using the expression level to determine a likelihood of a beneficial response to a treatment including a taxane, wherein expression of CD247, TYMS, IGF1R, ACTG2, CAPZA1, CHEK2, STMN1, or ZWILCH is positively correlated with increased likelihood of a beneficial response to a treatment including a taxane, and wherein expression of CCND1 is negatively correlated with an increased likelihood of a beneficial response to a treatment including a taxane; and generating a report including information based on the likelihood of a beneficial response to chemotherapy including a taxane.

The methods can further include a gene expression level to determine a likelihood of a beneficial response to a treatment including a cyclophosphamide, wherein expression of CD247, CCND1, or CAPZA1 is positively correlated with increased likelihood of a beneficial response to a treatment including a cyclophosphamide, and wherein expression of TYMS, IGF1R, ACTG2, CHEK2, STMN1, or ZWILCH is negatively correlated with an increased likelihood of a beneficial response to a treatment including a cyclophosphamide, and wherein the report includes information based on the likelihood of a beneficial response to chemotherapy including a cyclophosphamide.

The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumour sample can be a formalin-fixed and paraffin-embedded (FPE) or a frozen tumour section.

The methods of the present disclosure include methods of predicting whether a cancer patient will exhibit a beneficial response to chemotherapy, where the methods involve measuring an expression level of a gene, or its expression product, in a tumour sample obtained from the patient, wherein the gene is selected from the group consisting of ABCC1, ABCC10, ABCC5, ACTB, ACTR2, APEX1, APOC1, APRT, BAK1, BAX, BBC3, BCL2L13, BID, BUB1, BUB3, CAPZA1, CCT3, CD247, CD68, CDCA8, CENPA, CENPF, CHEK2, CHFR, CST7, CXCR4, DDR1, DICER1, EIF4E2, GADD45B, GBP1, HDAC6, HSPA1A, HSPA1B, HSPA1L, IL2RA, IL7, ILK, KALPHA1, KIF22, LILRB1, LIMK2, MAD2L1, MAPRE1, MCL1, MRE11A, NEK2, NTSR2, PHB, PLD3, RAD1, RALBP1, RHOA, RPN2, SHC1, SLC1A3, SRC, STAT1, STK10, STMN1, TBCC, TOP3B, TPX2, TSPAN4, TUBA3, TUBA6, TUBB, TUBB2C, TUBB3, TYMS, VEGF, VHL, WNT5A, ZW10, ZWILCH, and ZWINT; using the expression level to determine a likelihood of a beneficial response to a treatment including a taxane, wherein expression of SLC1A3, TBCC, EIF4E2, TUBB, TSPAN4, VHL, BAX, CD247, CAPZA1, STMN1, ABCC1, ZW10, HSPA1B, MAPRE1, PLD3, APRT, BAK1, CST7, SHC1, ZWILCH, SRC, GADD45B, LIMK2, CHEK2, RAD1, MRE11A, DDR1, STK10, LILRB1, BBC3, BUB3, TOP3B, RPN2, ILK, GBP1, TUBB3, NTSR2, BID, BCL2L13, ABCC5, HDAC6, CD68, DICER1, RHOA, CCT3, ACTR2, WNT5A, HSPA1L, APOC1, APEX1, KALPHA1, ABCC10, PHB, TUBB2C, RALBP1, MCL1, HSPA1A, IL2RA, TUBA3, ACTB, KIF22, CXCR4, STAT1, IL7, or CHFR is positively correlated with increased likelihood of a beneficial response to a treatment including a taxane, and wherein expression of CENPA, CDCA8, TPX2, NEK2, TYMS, ZWINT, VEGF, BUB1, MAD2L1, or CENPF is negatively correlated with an increased likelihood of a beneficial response to a treatment including a taxane; and generating a report including information based on the likelihood of a beneficial response to chemotherapy including a taxane.

The methods can further include using a gene expression level to determine a likelihood of a beneficial response to a treatment including a cyclophosphamide, wherein expression of SLC1A3, TSPAN4, BAX, CD247, CAPZA1, ZW10, CST7, SHC1, GADD45B, MRE11A, STK10, LILRB1, BBC3, BUB3, ILK, GBP1, BCL2L13, CD68, DICER1, RHOA, ACTR2, WNT5A, HSPA1L, APEX1, MCL1, IL2RA, ACTB, STAT1, IL7, or CHFR is positively correlated with increased likelihood of a beneficial response to a treatment including a cyclophosphamide, and wherein expression of TBCC, EIF4E2, TUBB, VHL, STMN1, ABCC1, HSPA1B, MAPRE1, APRT, BAK1, TUBA6, ZWILCH, SRC, LIMK2, CENPA, CHEK2, RAD1, DDR1, CDCA8, TOP3B, RPN2, TUBB3, NTSR2, BID, TPX2, ABCC5, HDAC6, NEK2, TYMS, CCT3, ZWINT, KALPHA1, ABCC10, PHB, TUBB2C, RALBP1, VEGF, HSPA1A, BUB1, MAD2L1, CENPF, TUBA3, KIF22, or CXCR4 is negatively correlated with an increased likelihood of a beneficial response to a treatment including a cyclophosphamide, and wherein the report includes information based on the likelihood of a beneficial response to chemotherapy including a cyclophosphamide.
The chemotherapy can include an anthracycline. The anthracycline can be doxorubicin. Where the chemotherapy is a taxane, the taxane can be docetaxel.

The methods can accomplish measuring of the gene expression level by quantitative PCR. The methods can accomplish measuring of the gene expression level by detection of an intron-based sequence of an RNA transcript of the gene, wherein the expression of which correlates with the expression of a corresponding exon sequence.

The tumour sample can be a formalin-fixed and paraffin-embedded (FPE) or a frozen tumour section.

The present disclosure also provides kits containing one or more (1) extraction buffer/reagents and protocol; (2) reverse transcription buffer/reagents and protocol; and (3) qPCR buffer/reagents and protocol, suitable for performing the method disclosed herein. Also contemplated are arrays having bound polynucleotides that specifically hybridize to one or more genes used in the methods disclosed herein, as well as arrays having bound one or more antibodies that specifically bind a polypeptides expressed by a gene used in the methods disclosed herein.

Various aspects and embodiments will be apparent from the following discussion, including the Examples. Such additional embodiments, without limitation, include any and all of the ESR1 gene combinations discussed and/or specifically listed in Example 2.

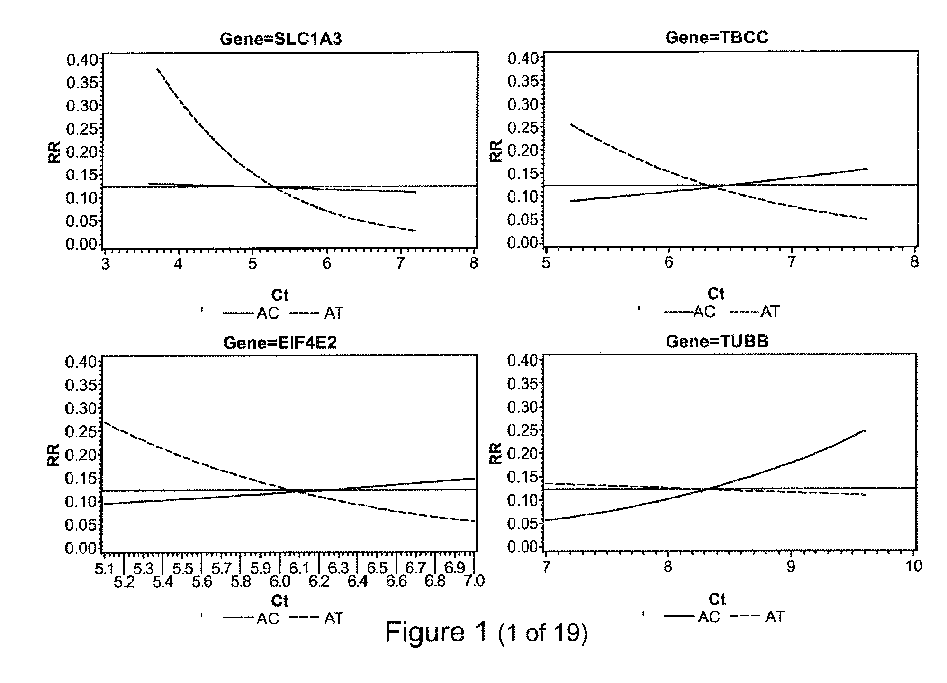

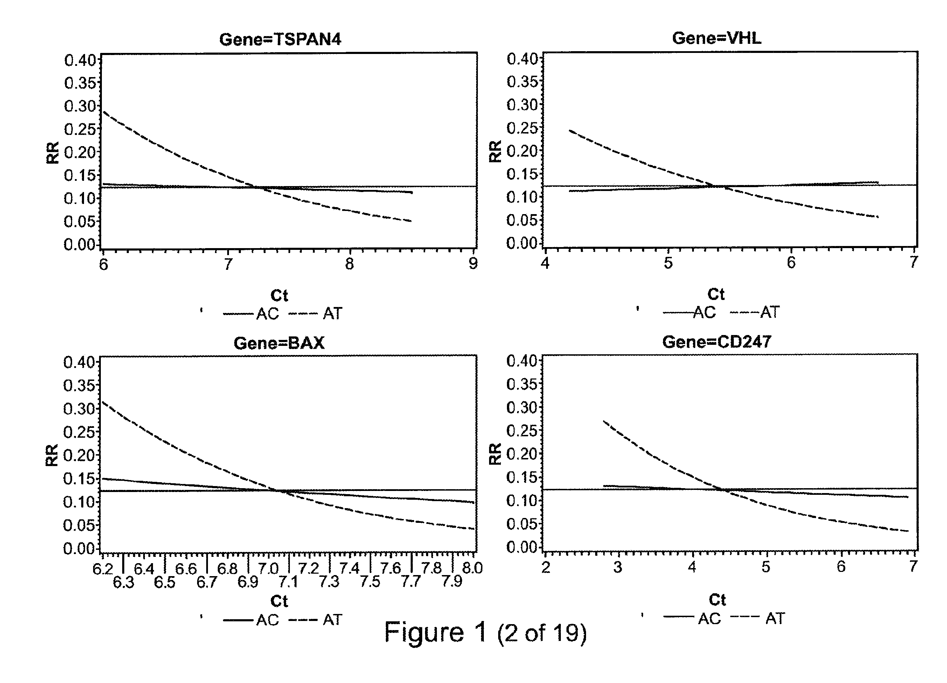

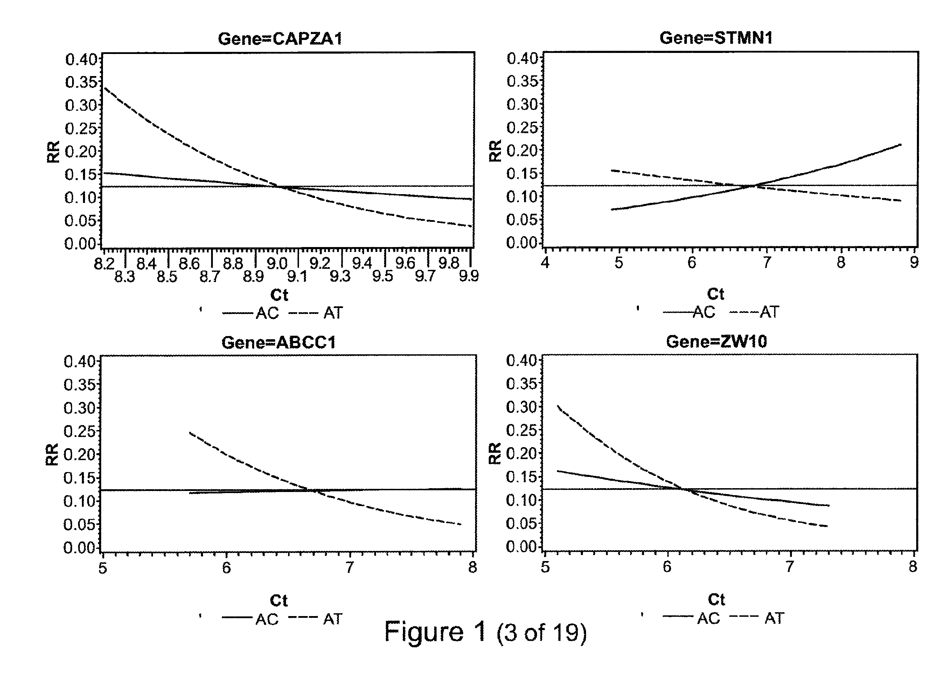

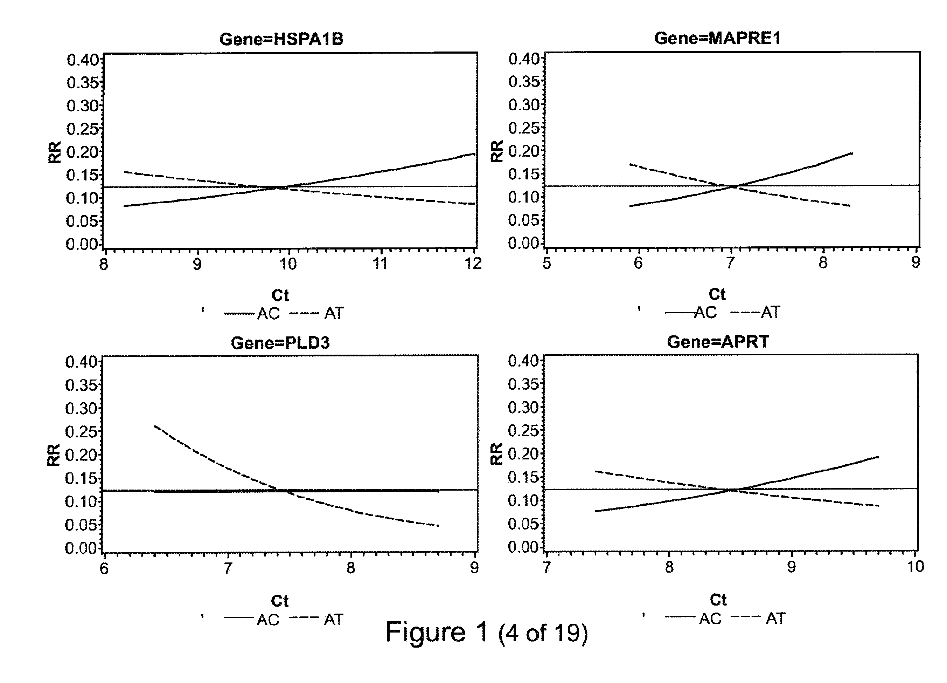

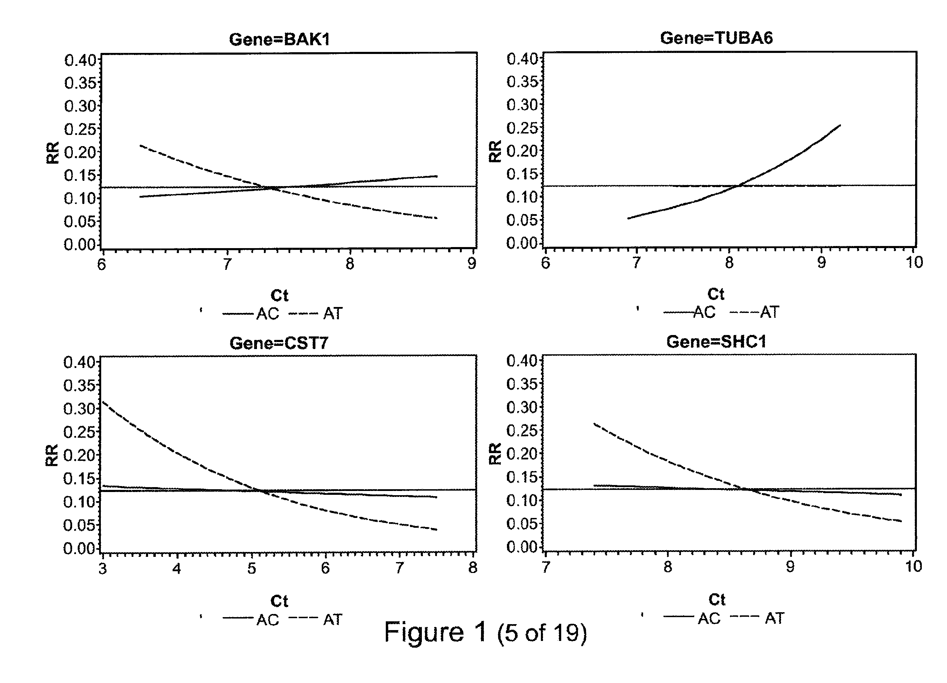

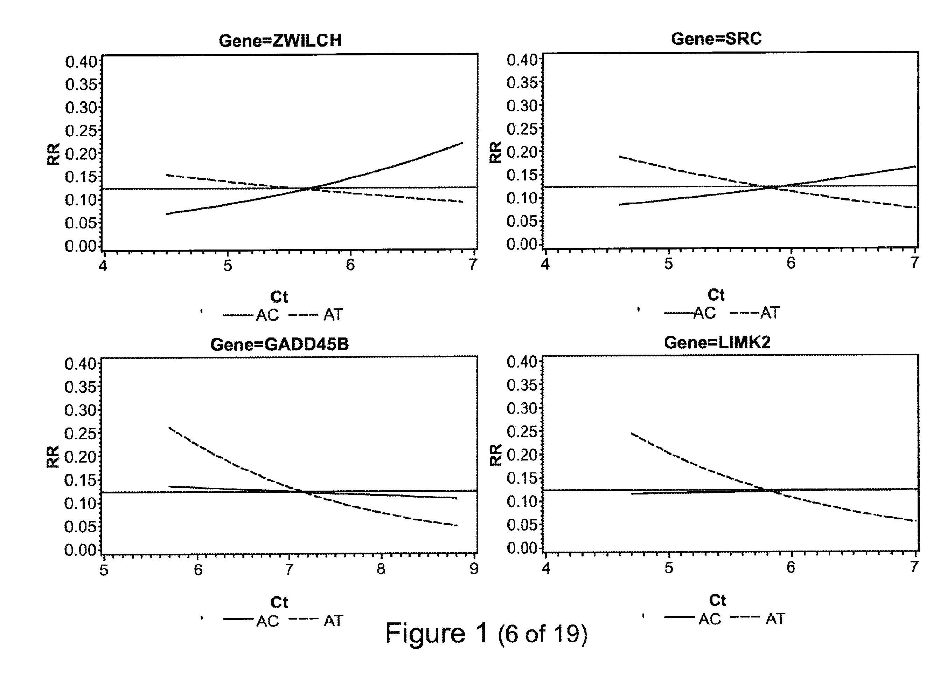

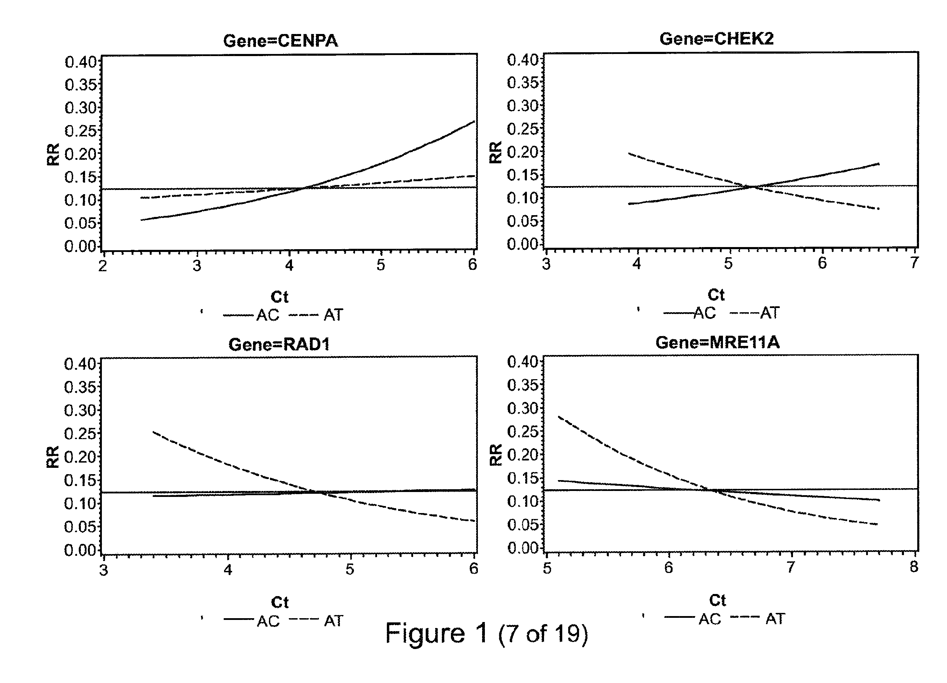

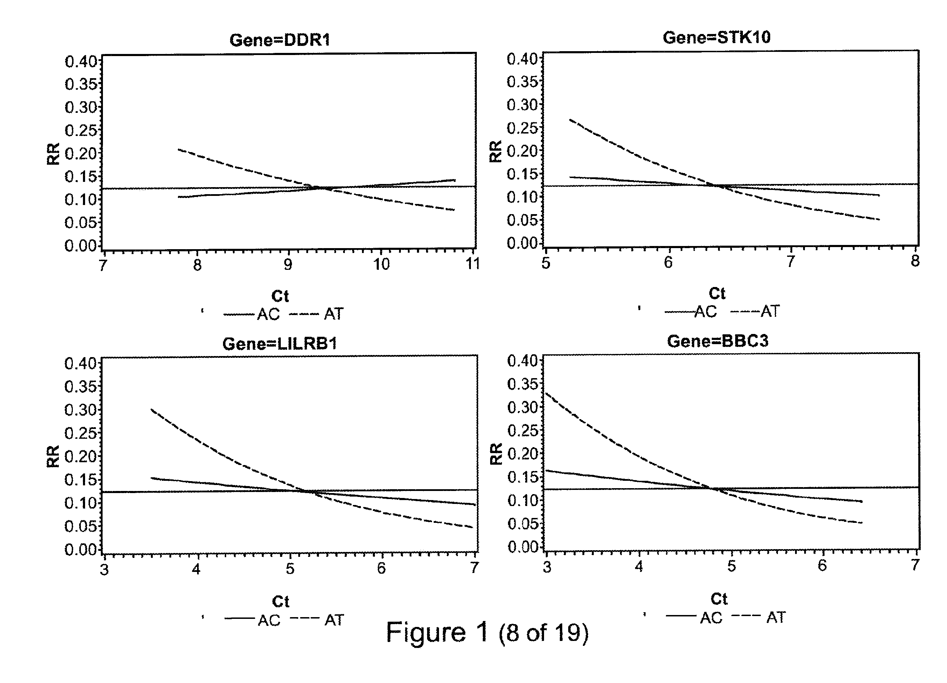

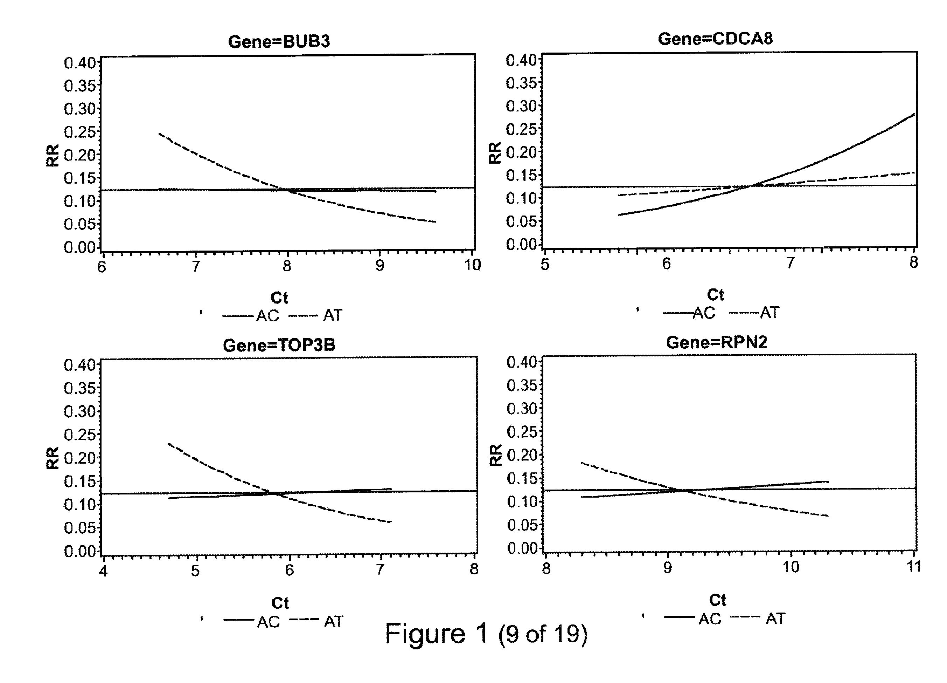

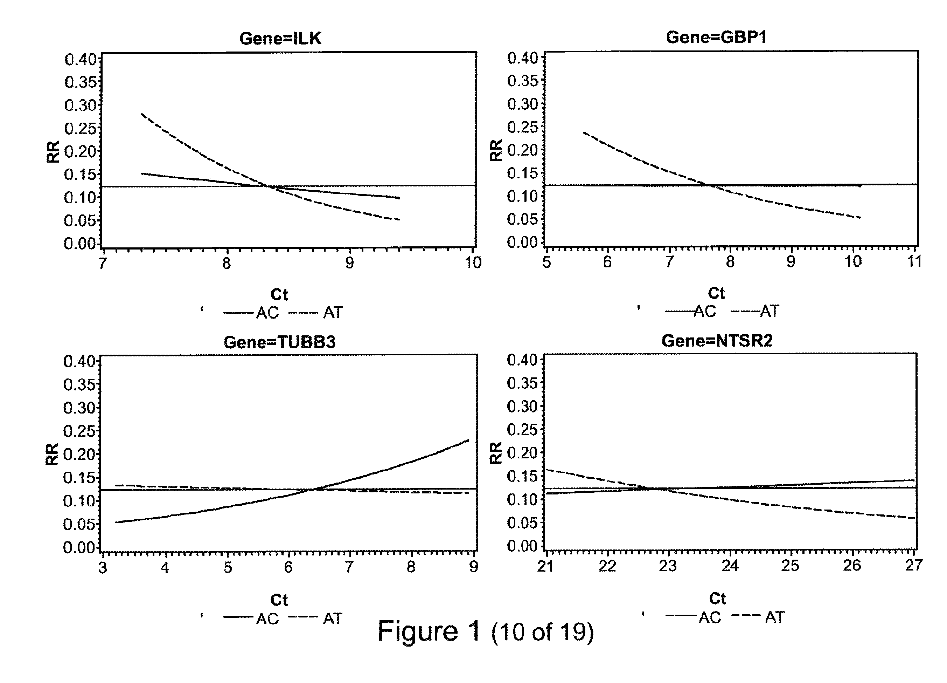

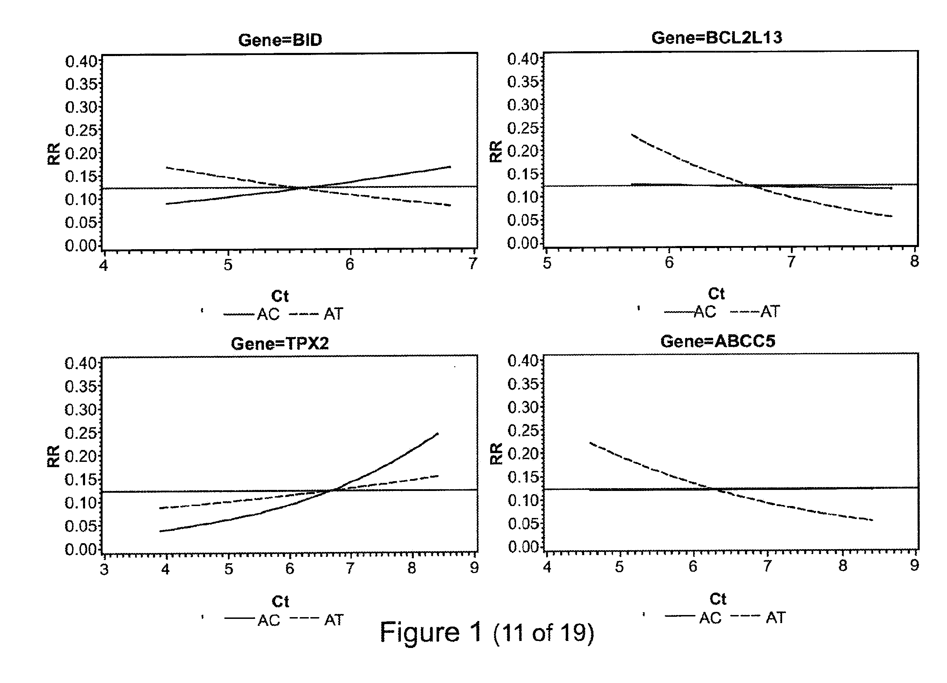

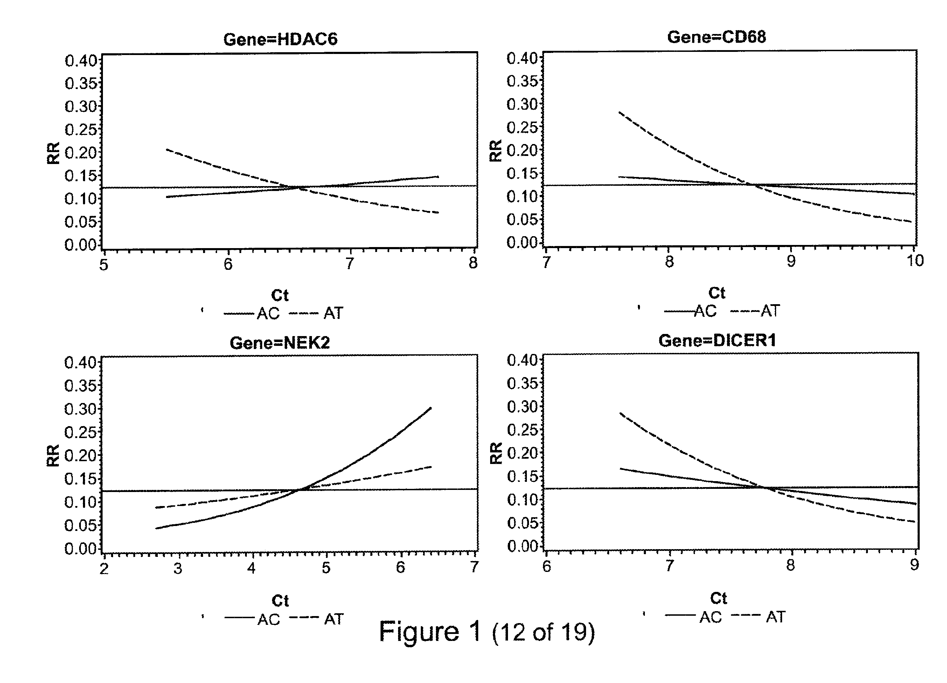

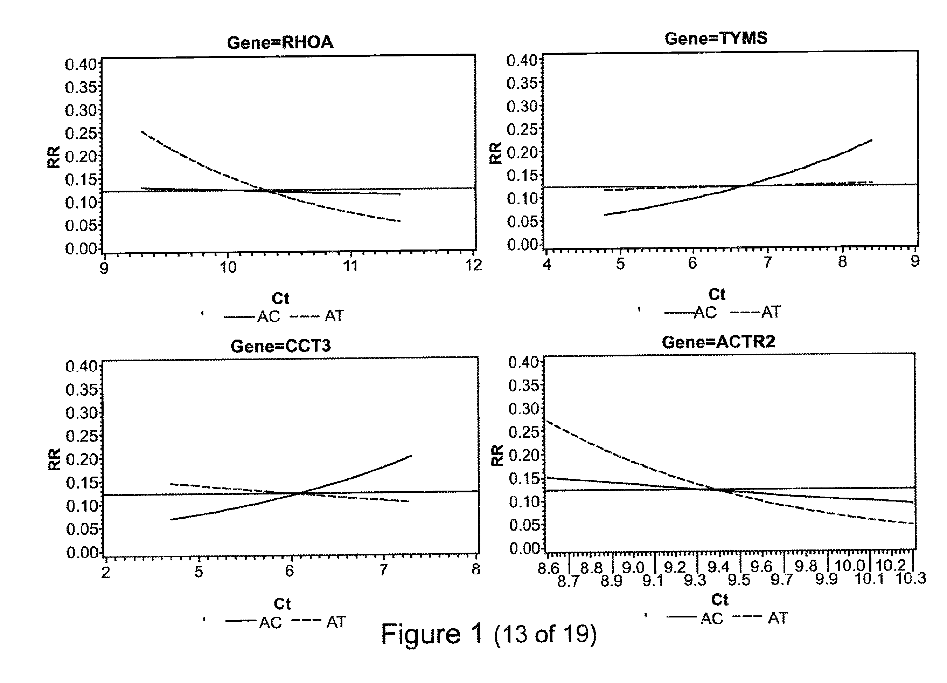

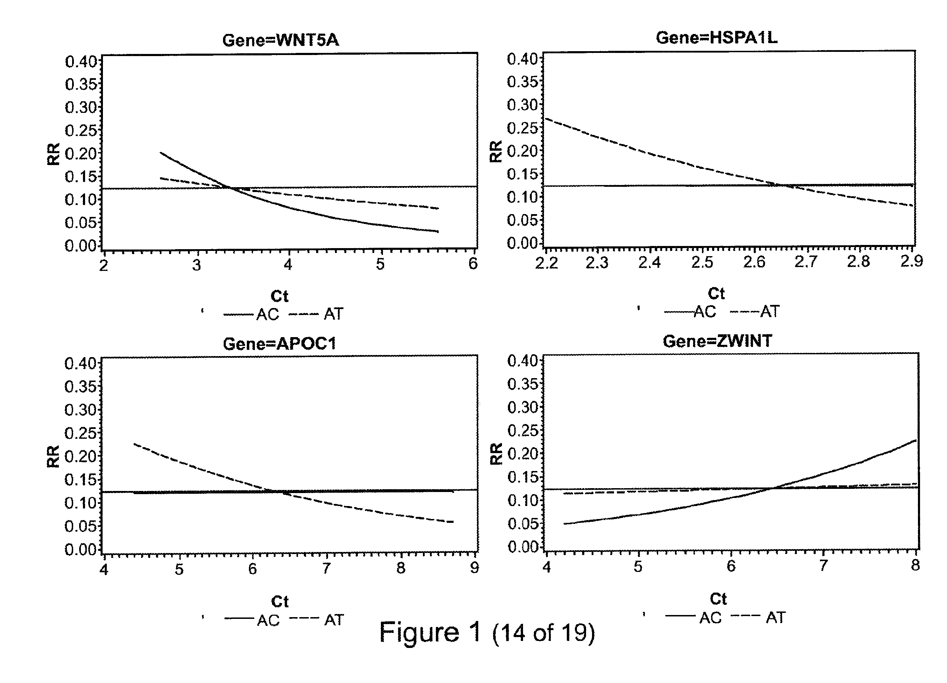

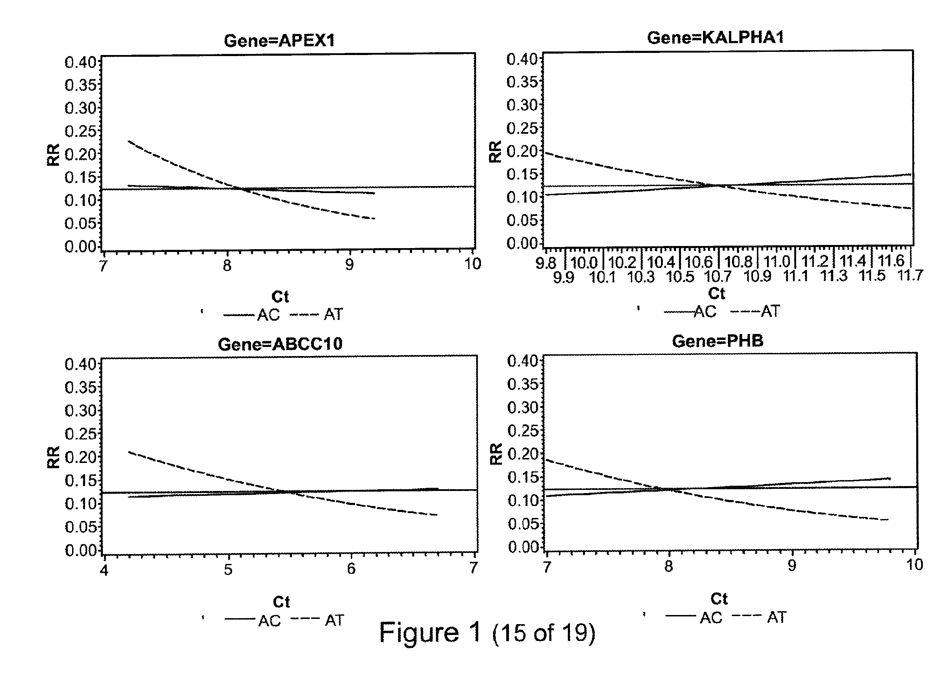

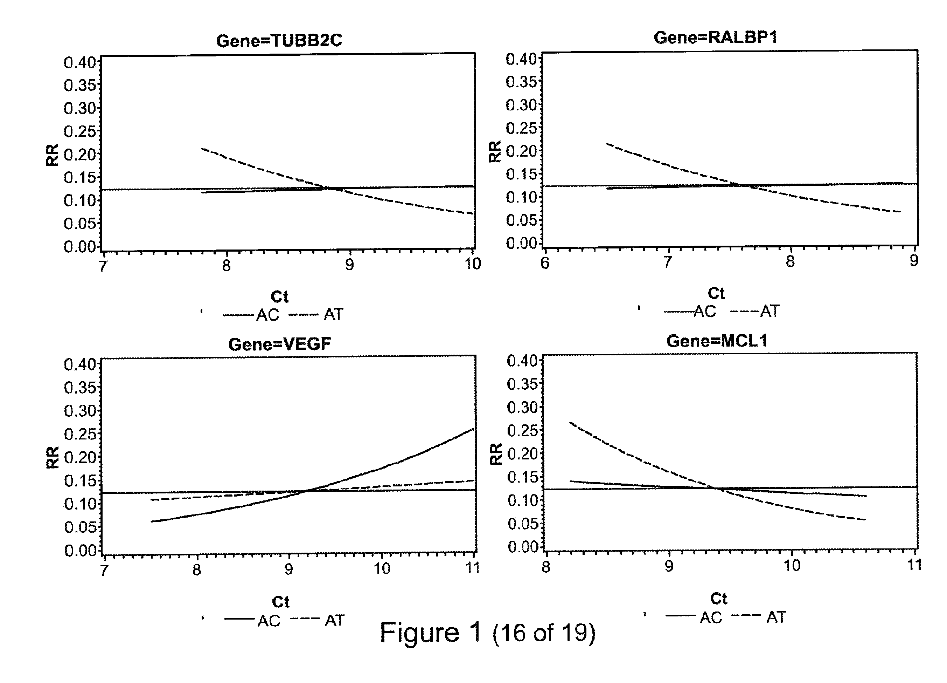

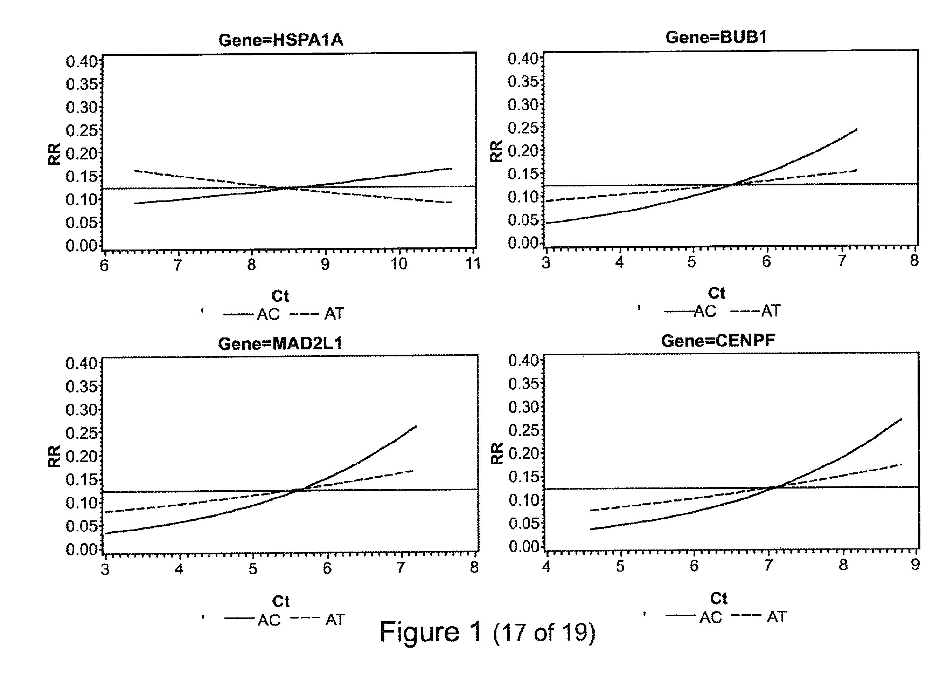

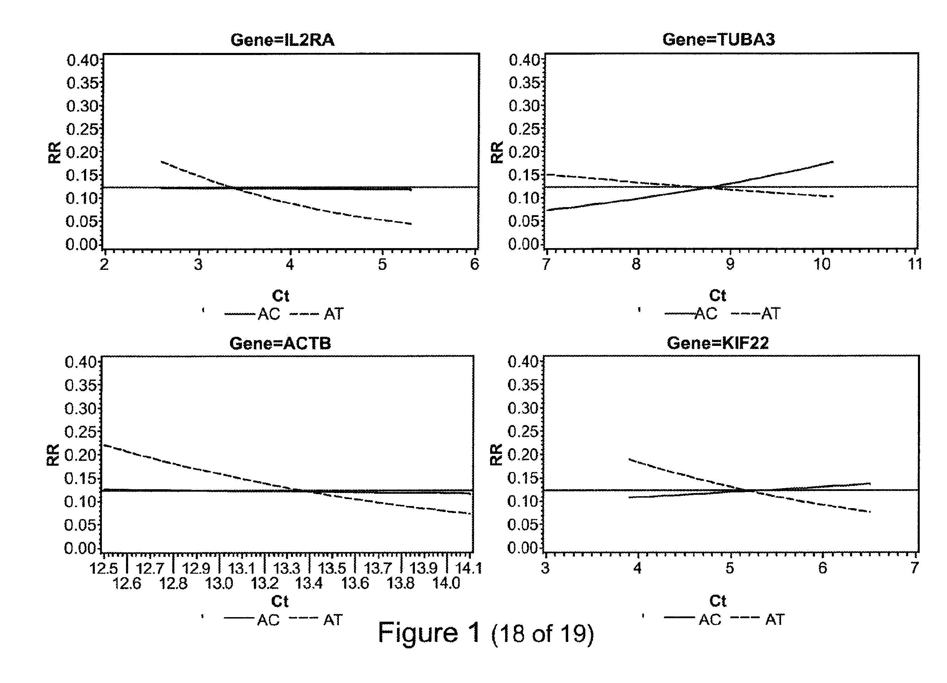

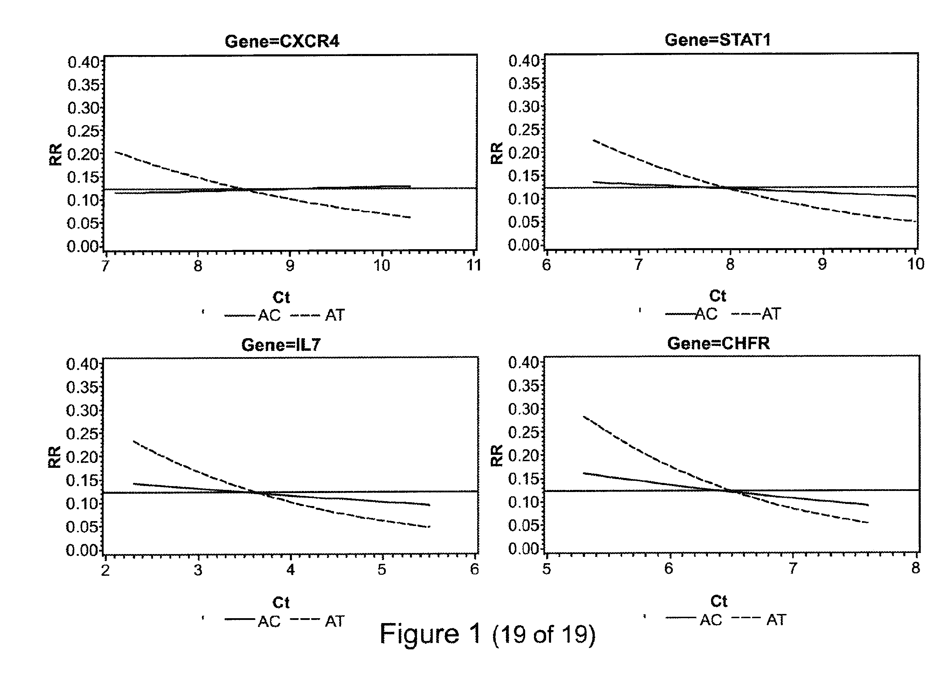

Figure 1 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line). A horizontal dashed line in each graph represents the overall (i.e., not gene expression-specific) 5-year RR in the study population who were randomized to treatment with either AC or AT. In Figure 1 the patients were included without regard to hormone receptor expression status of the tumour.

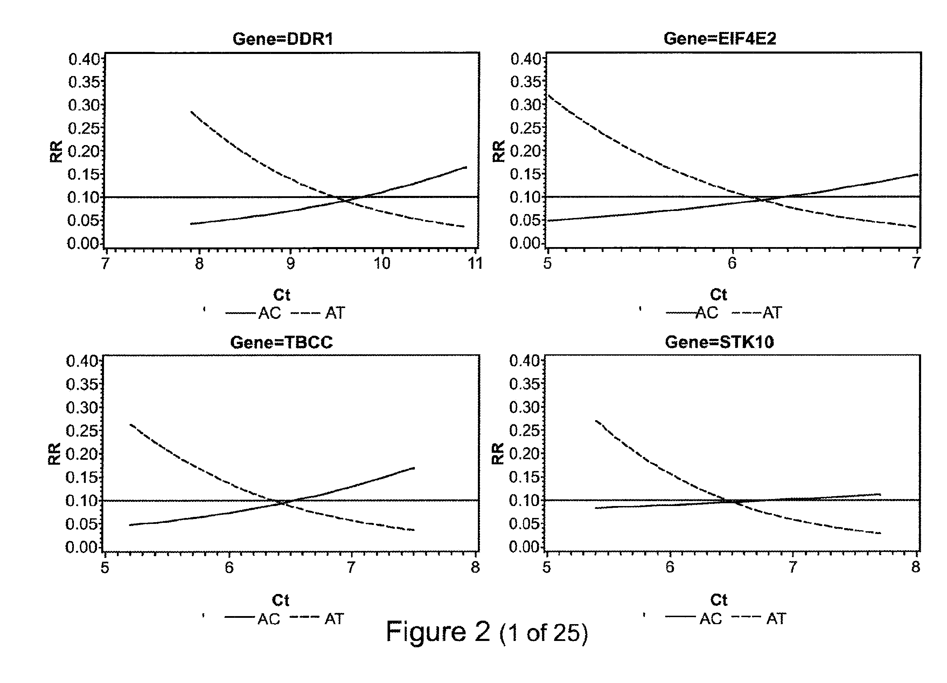

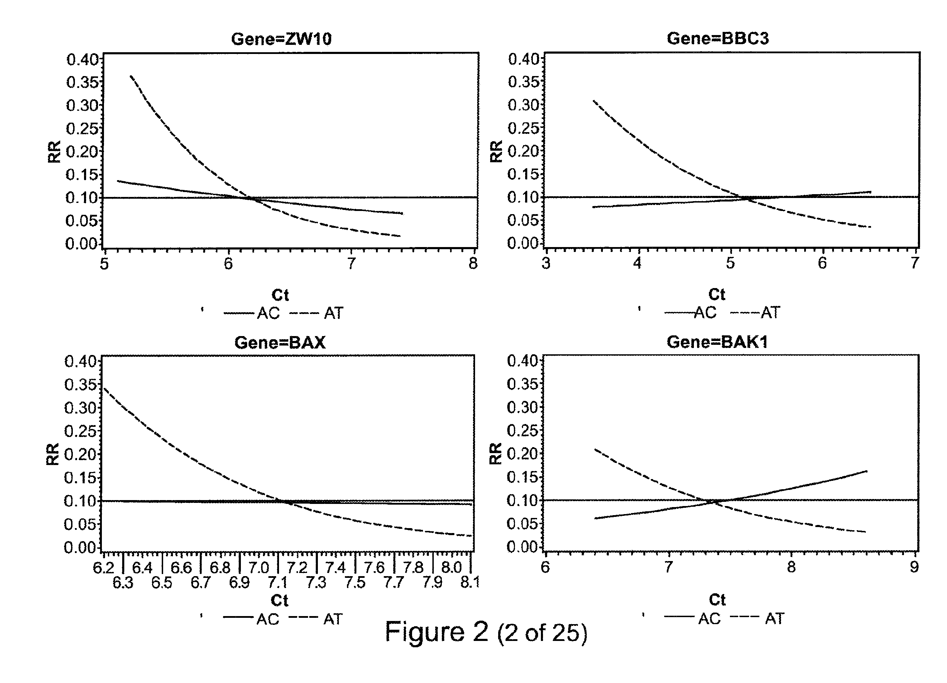

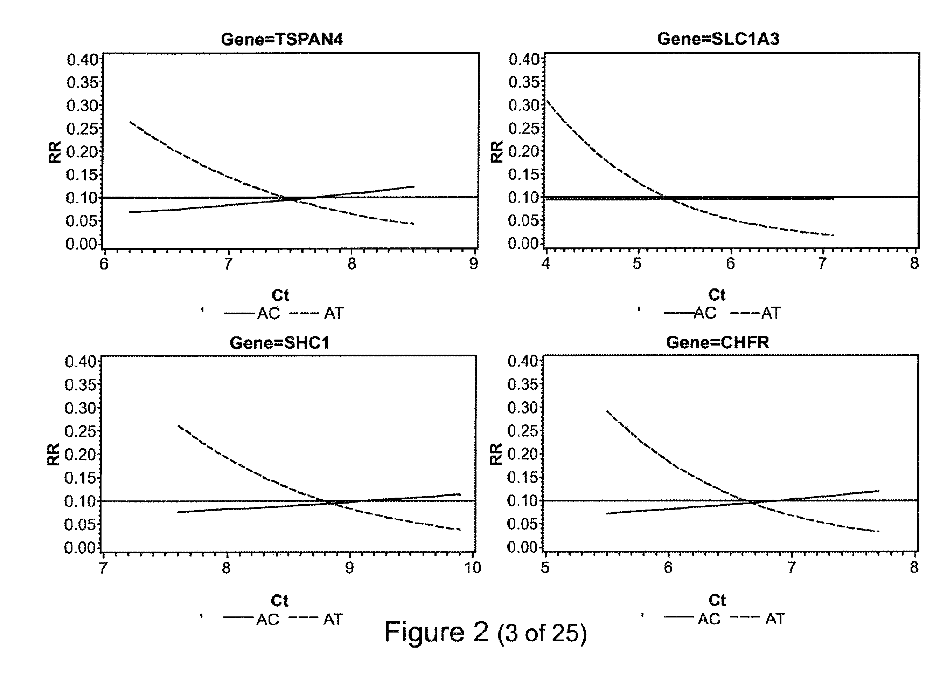

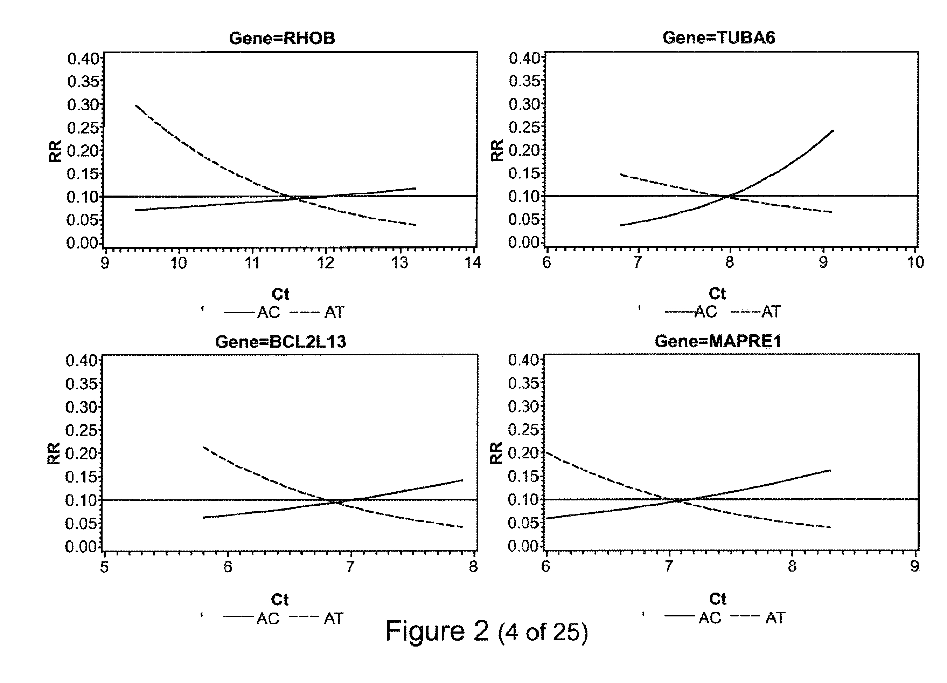

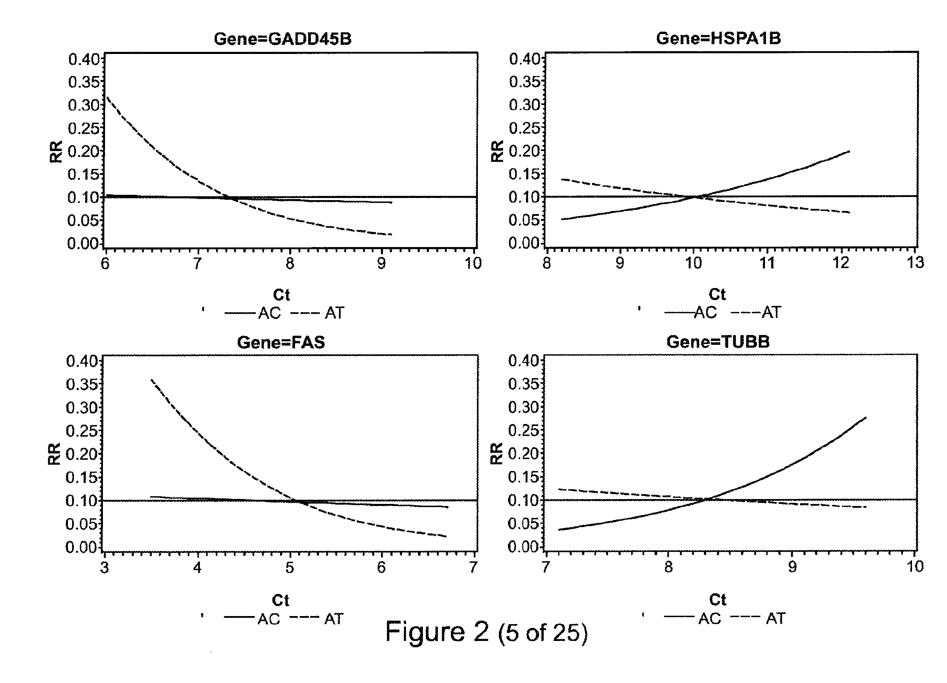

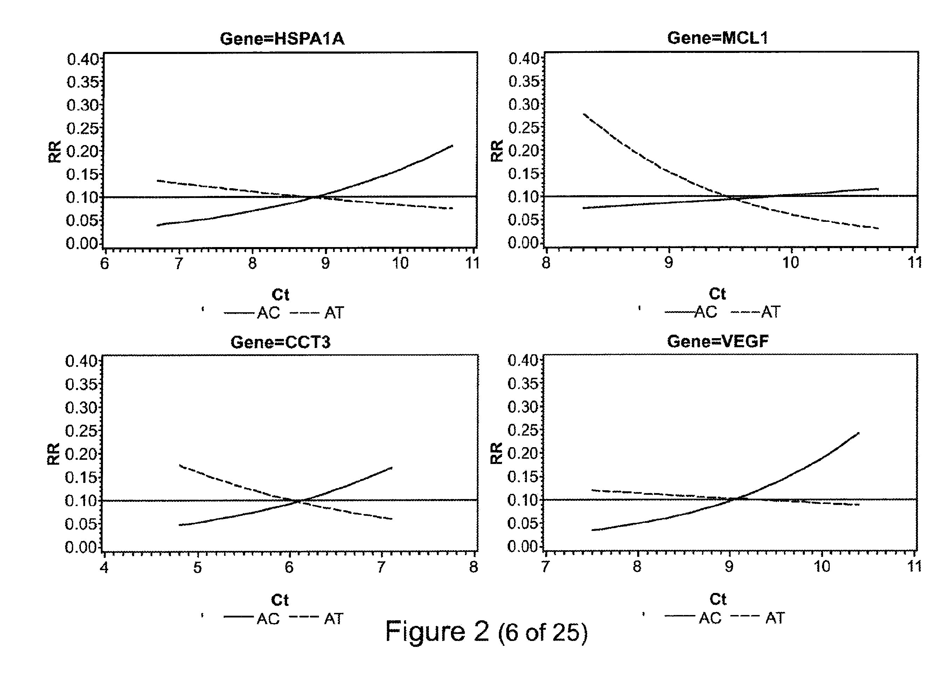

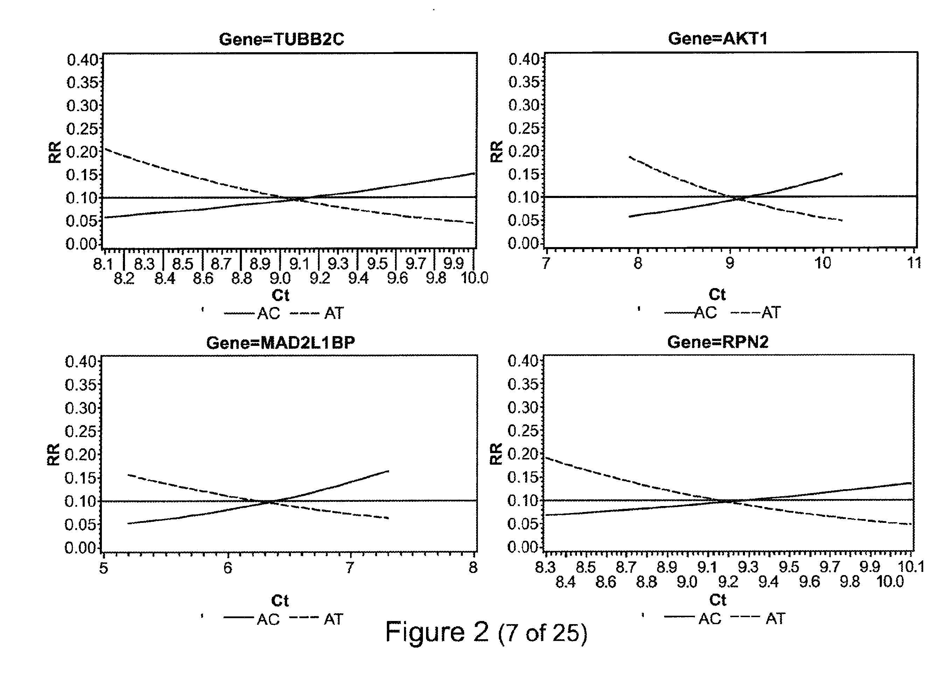

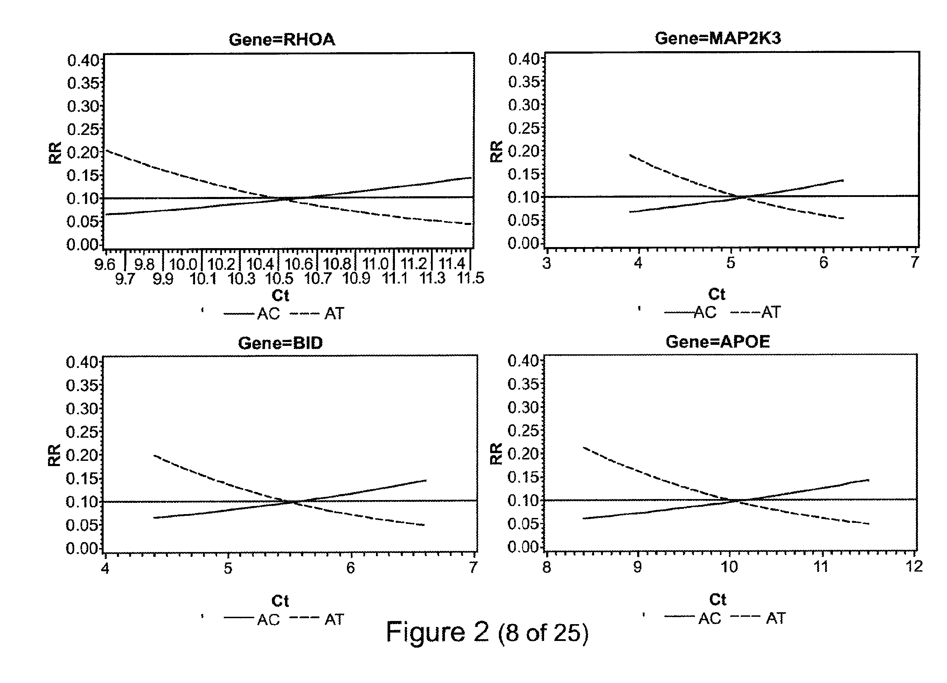

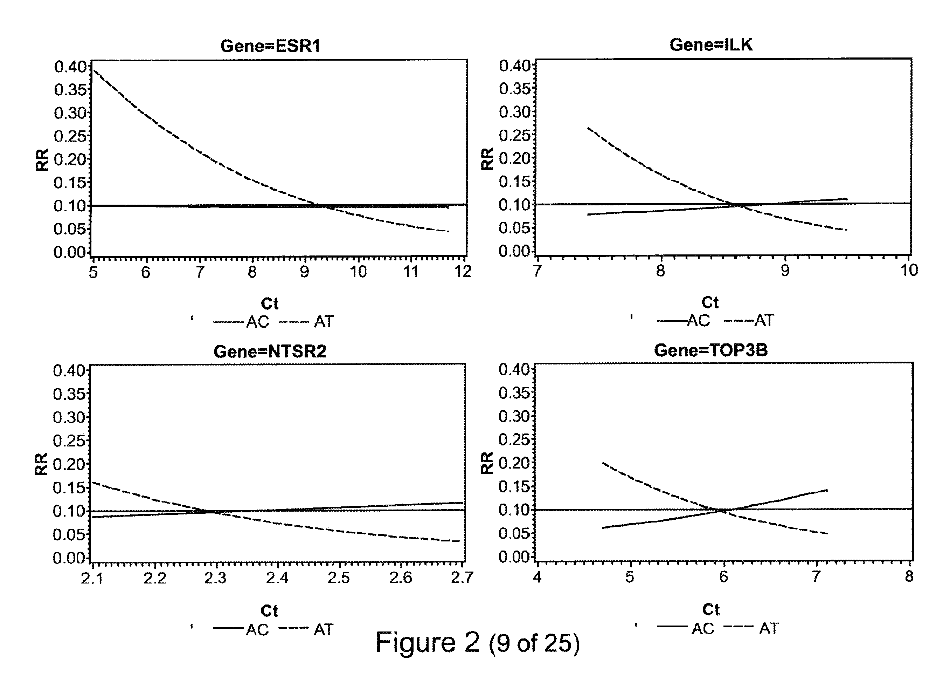

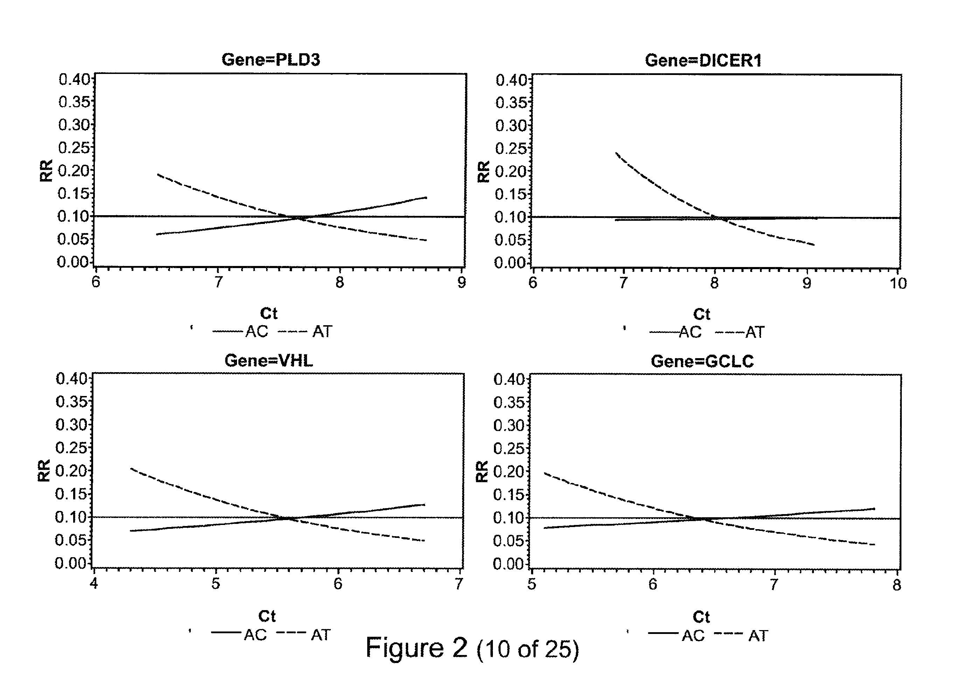

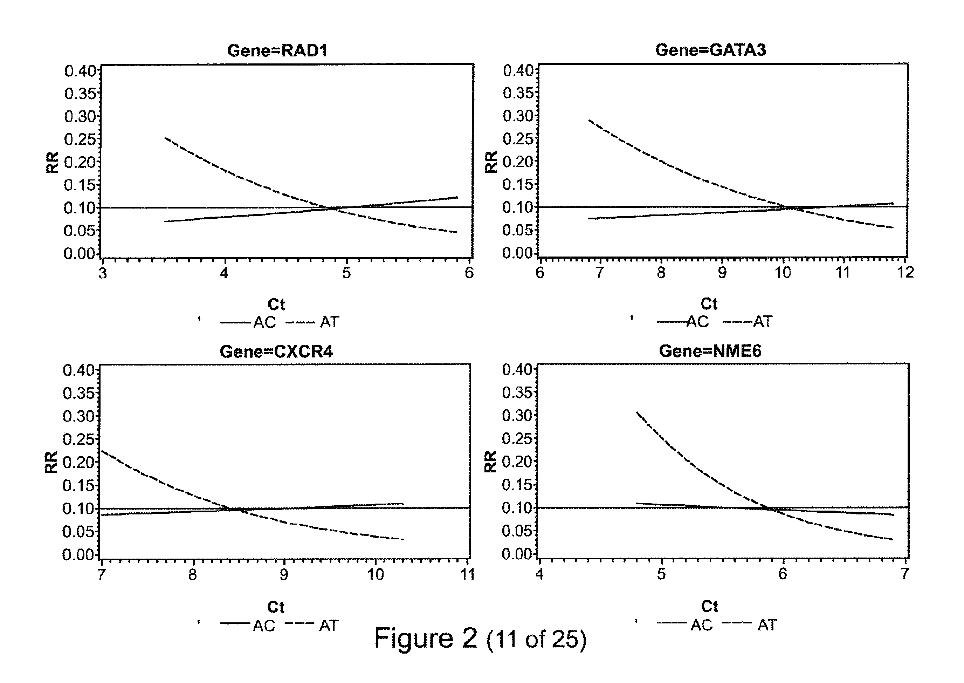

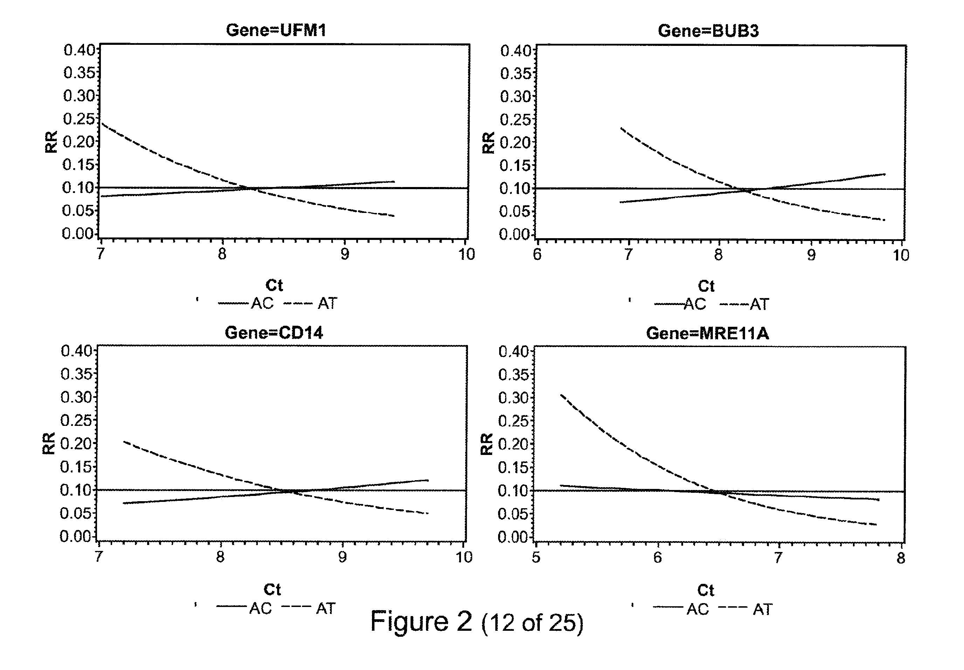

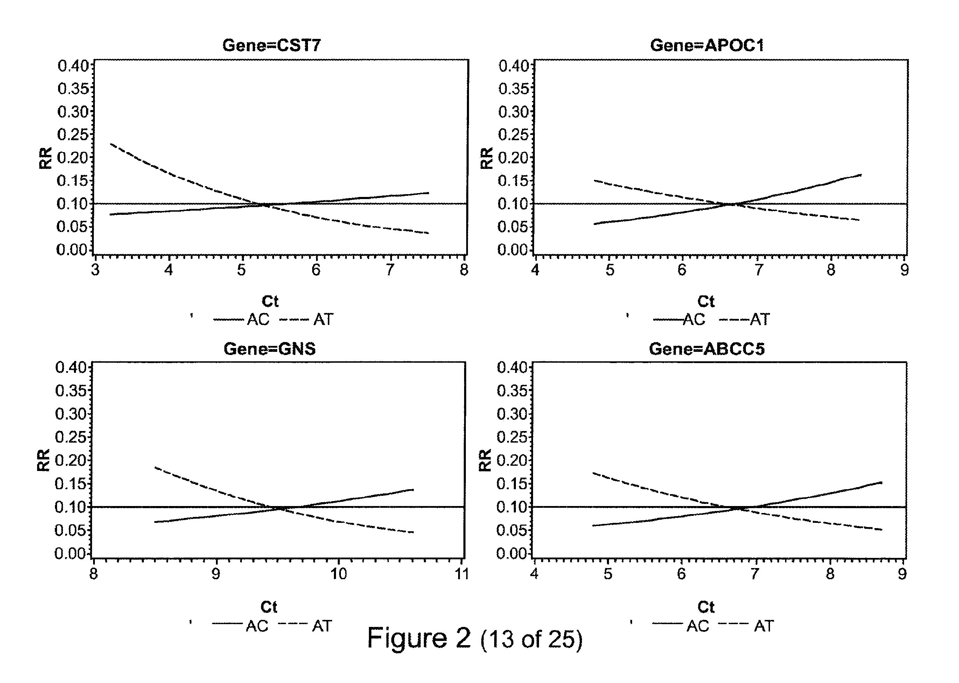

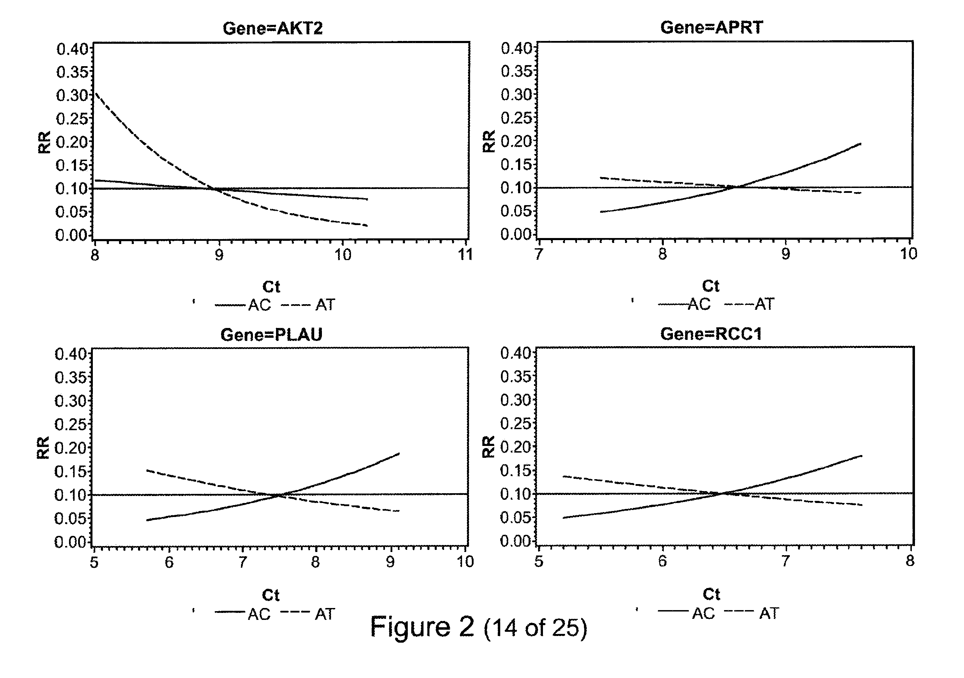

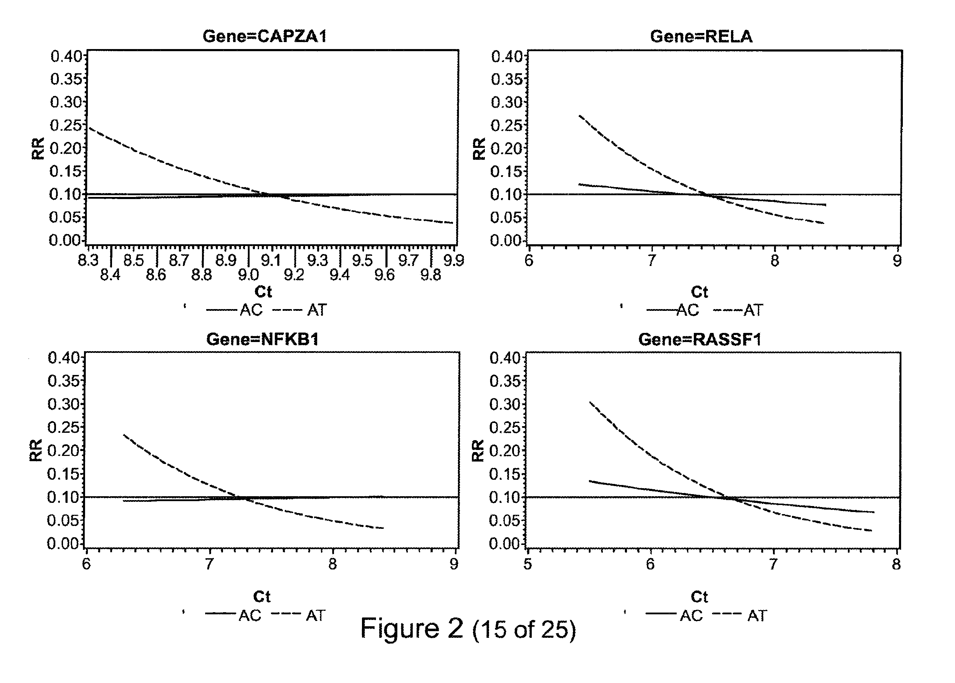

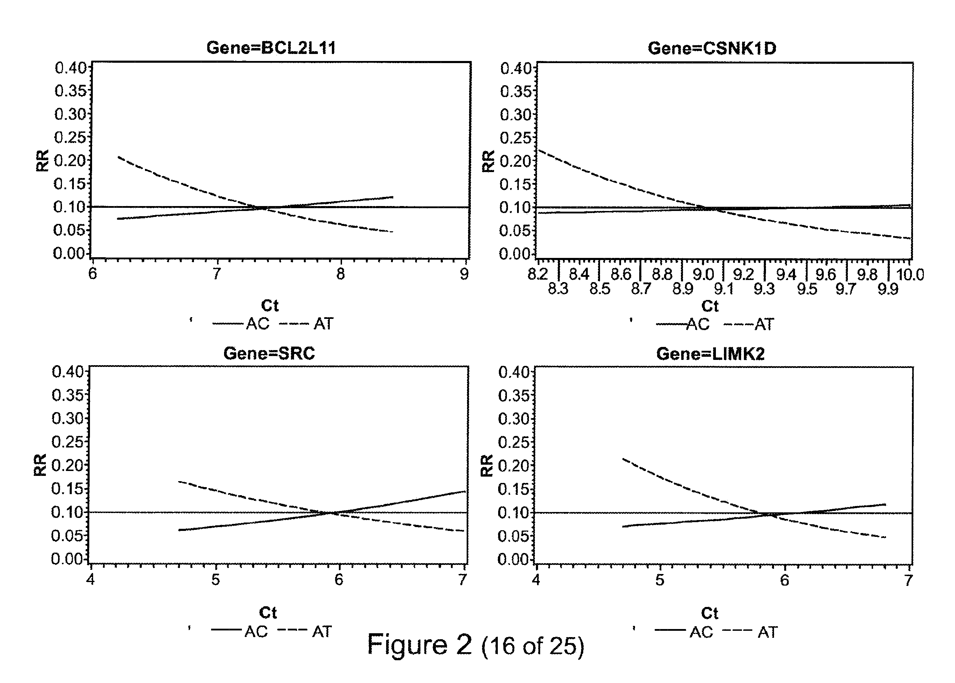

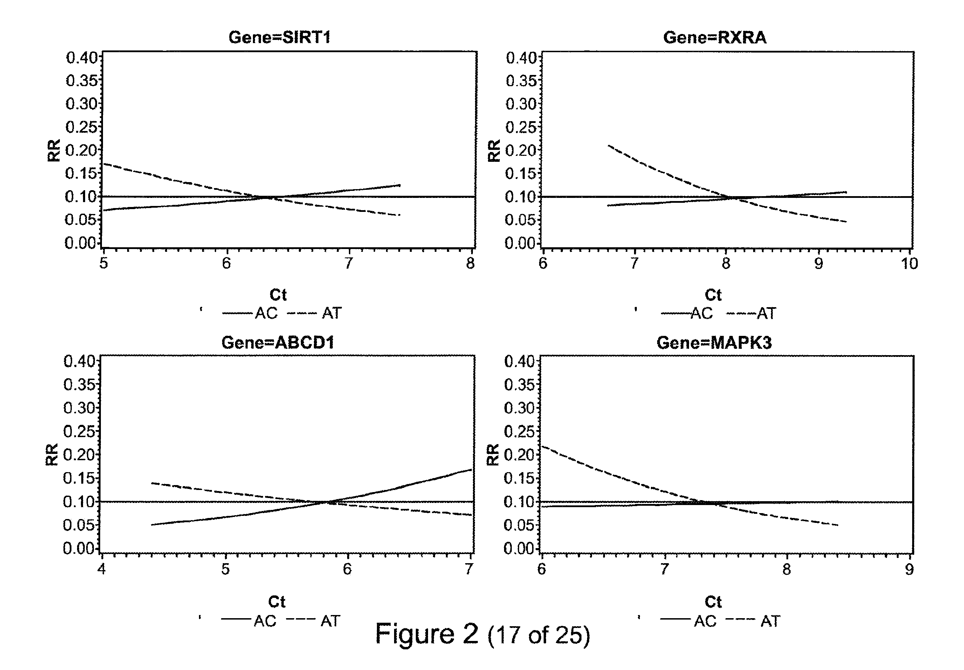

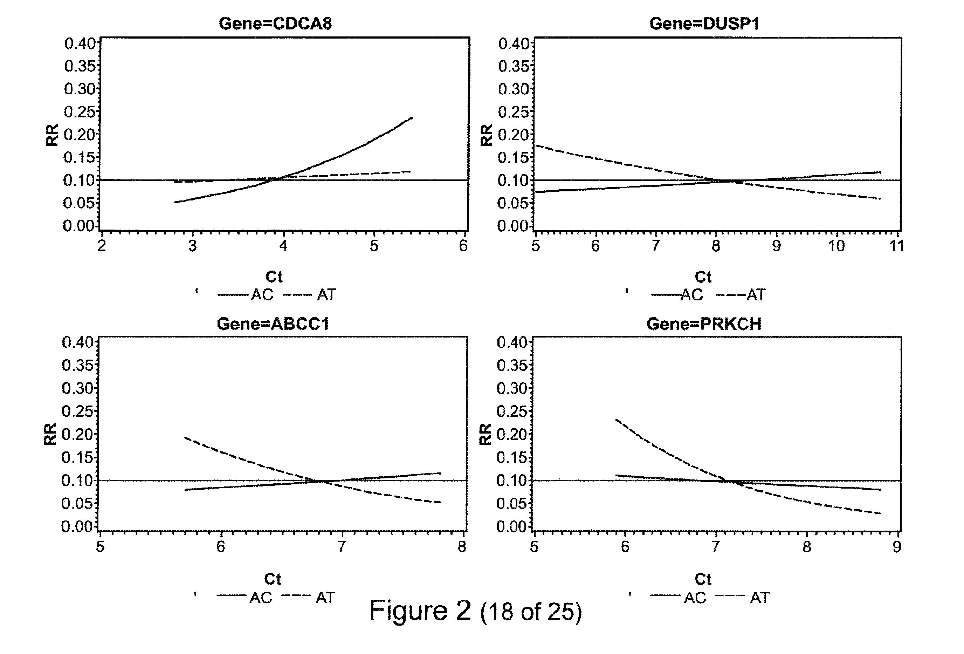

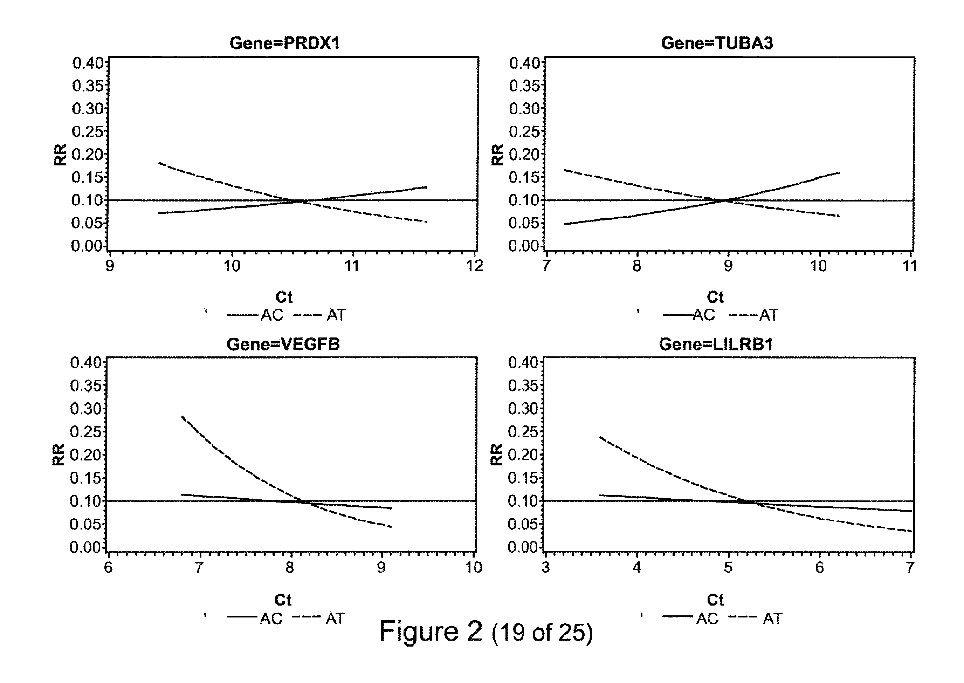

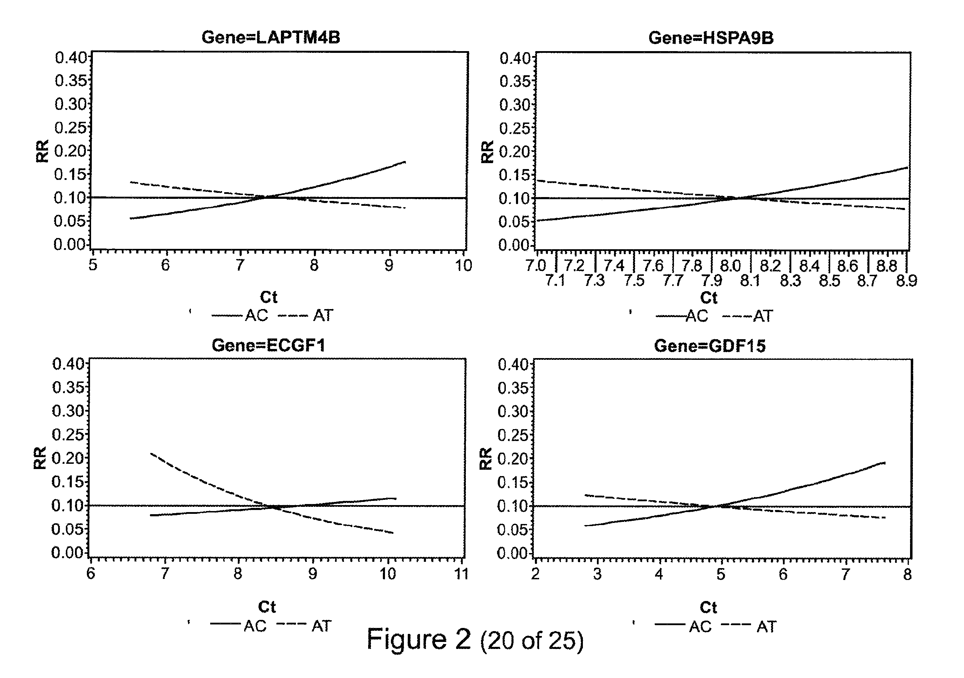

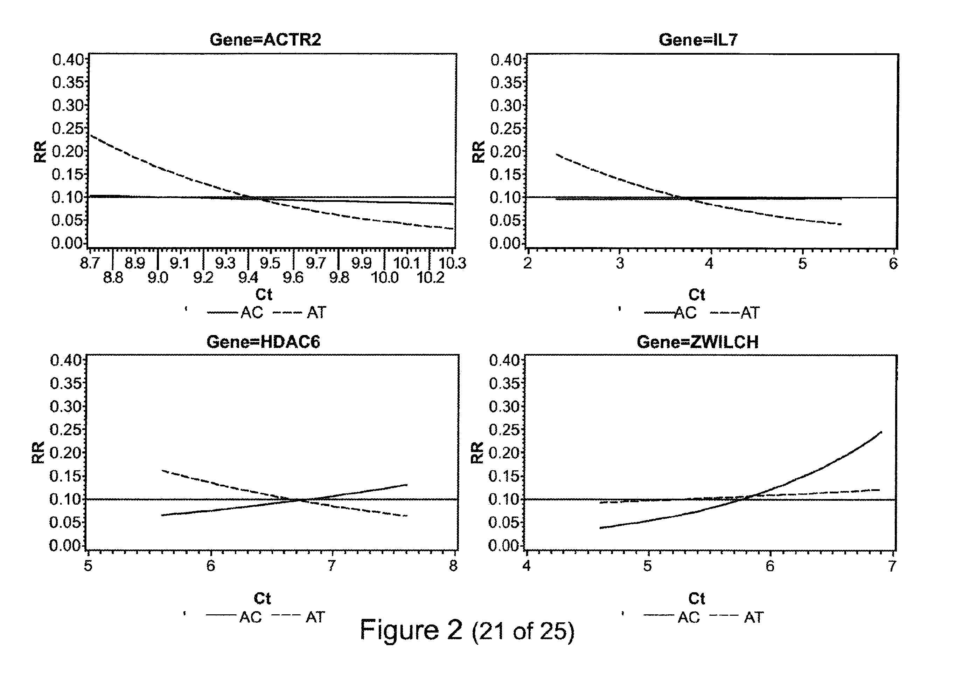

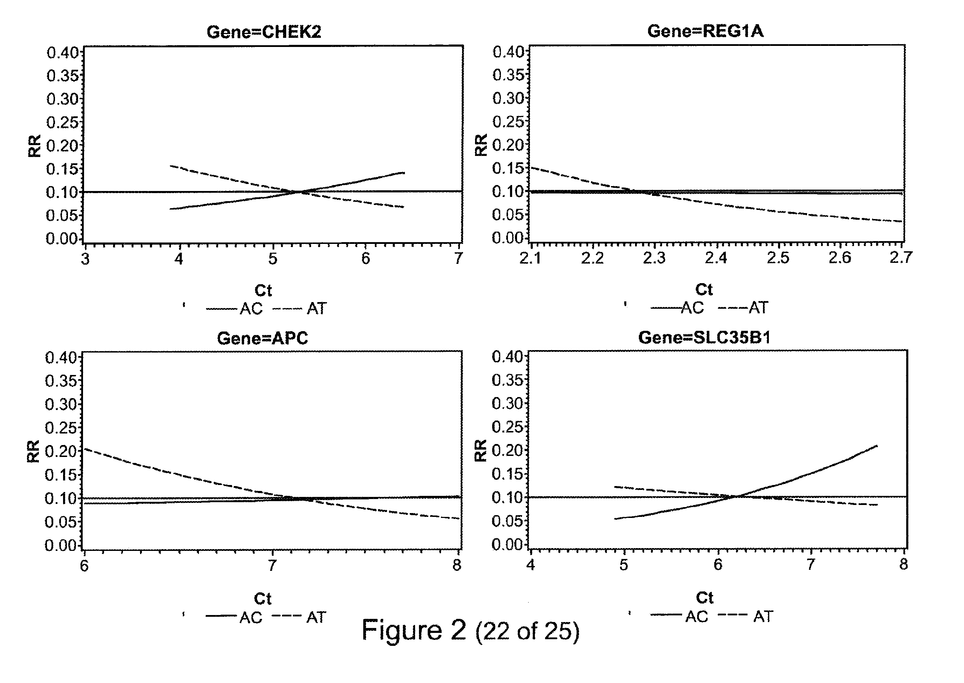

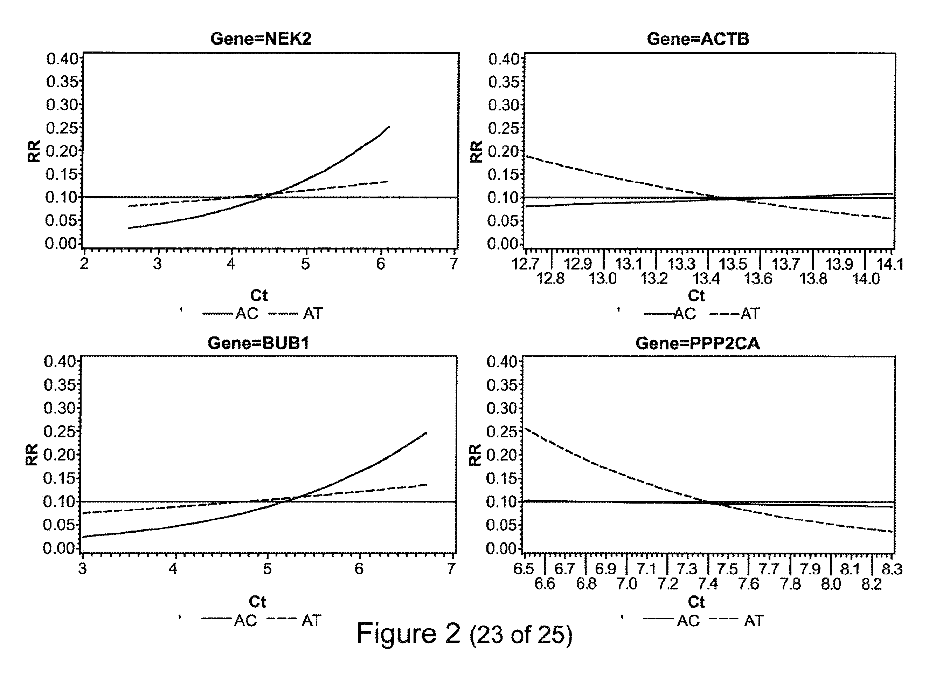

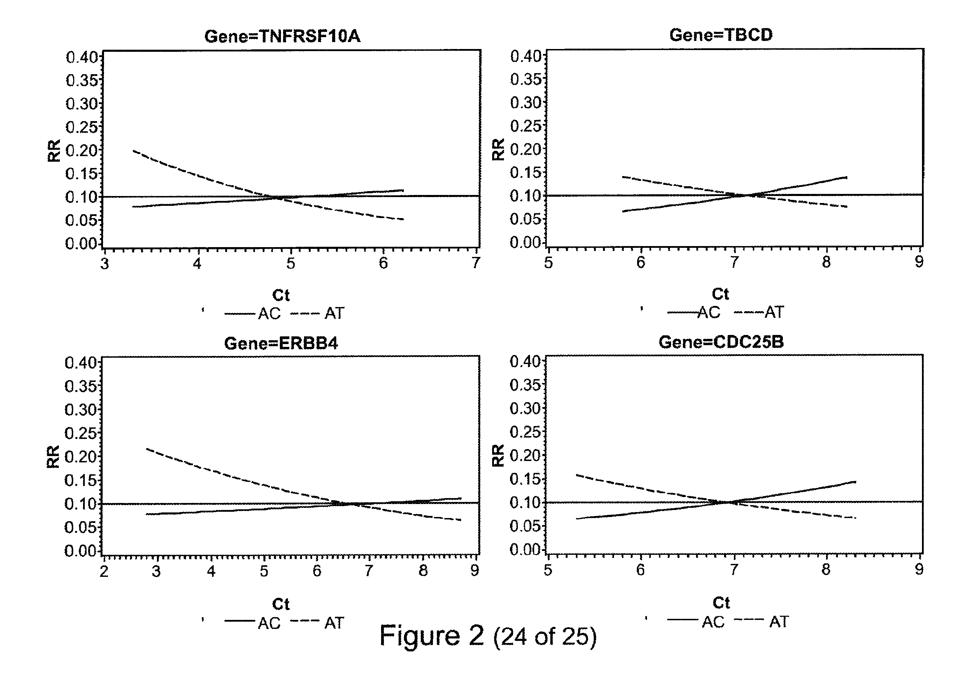

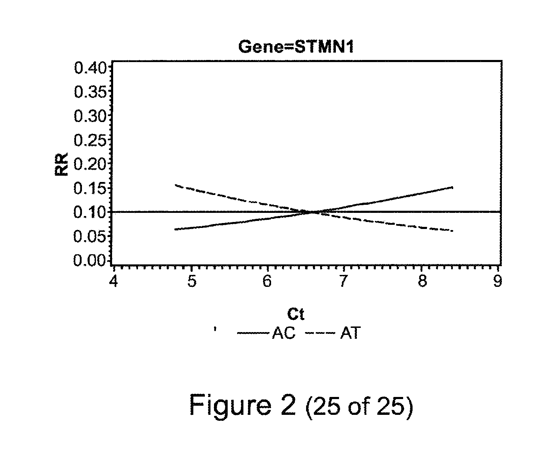

Figure 2 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer. A horizontal dashed line in each graph represents the overall (i.e., not gene expression-specific) 5-year RR in patients in the study population having HR+ breast cancer who were randomized to treatment with either AC or AT.

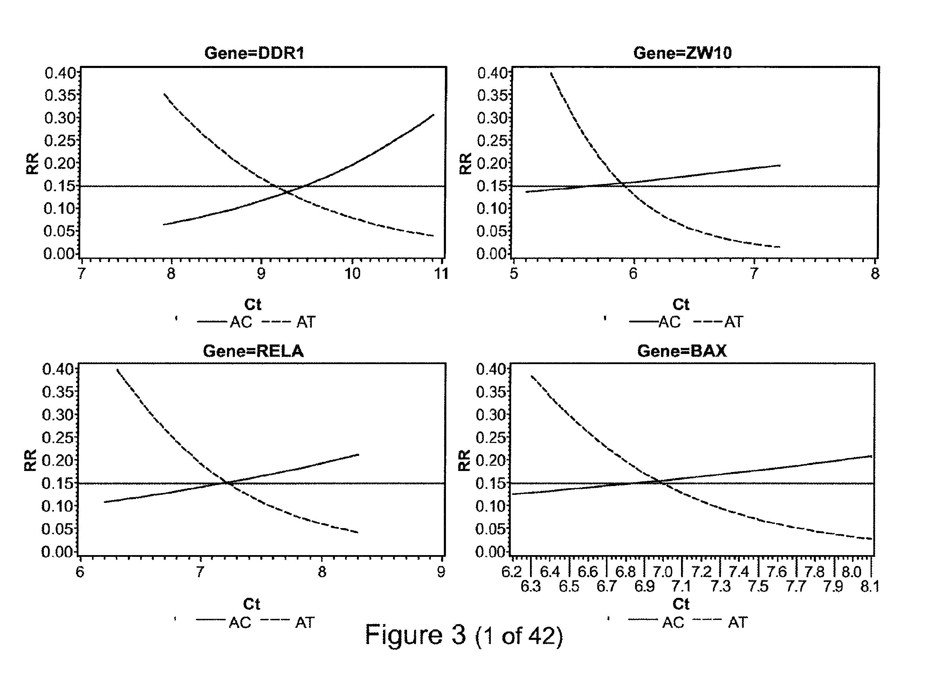

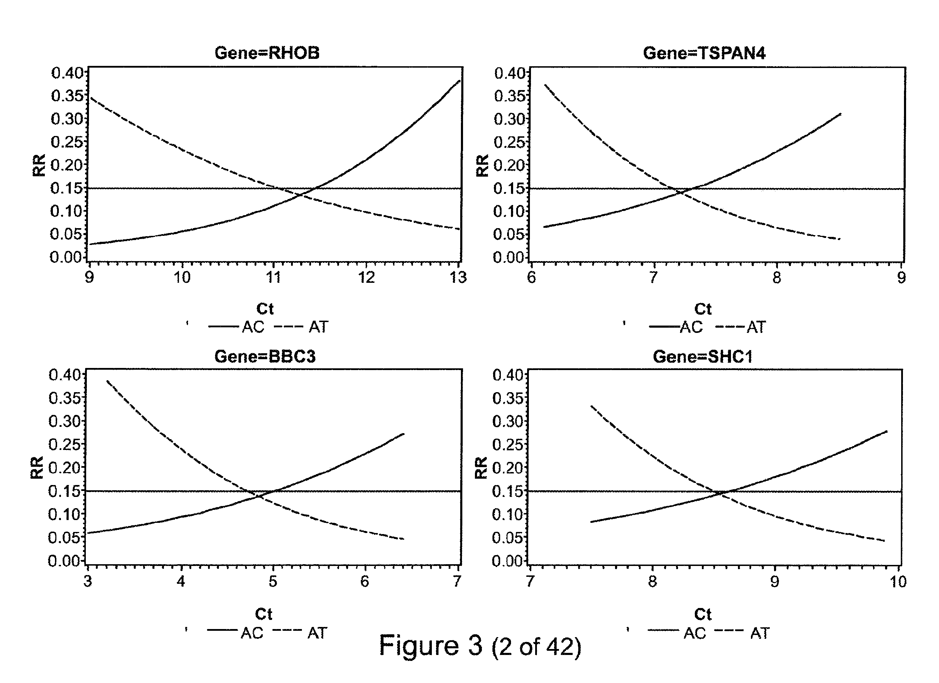

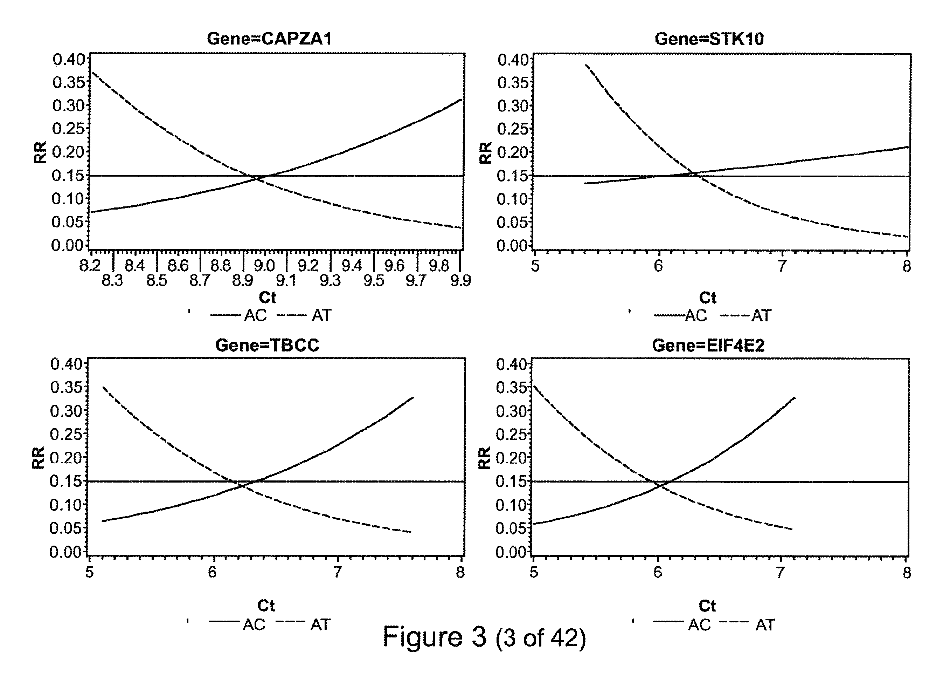

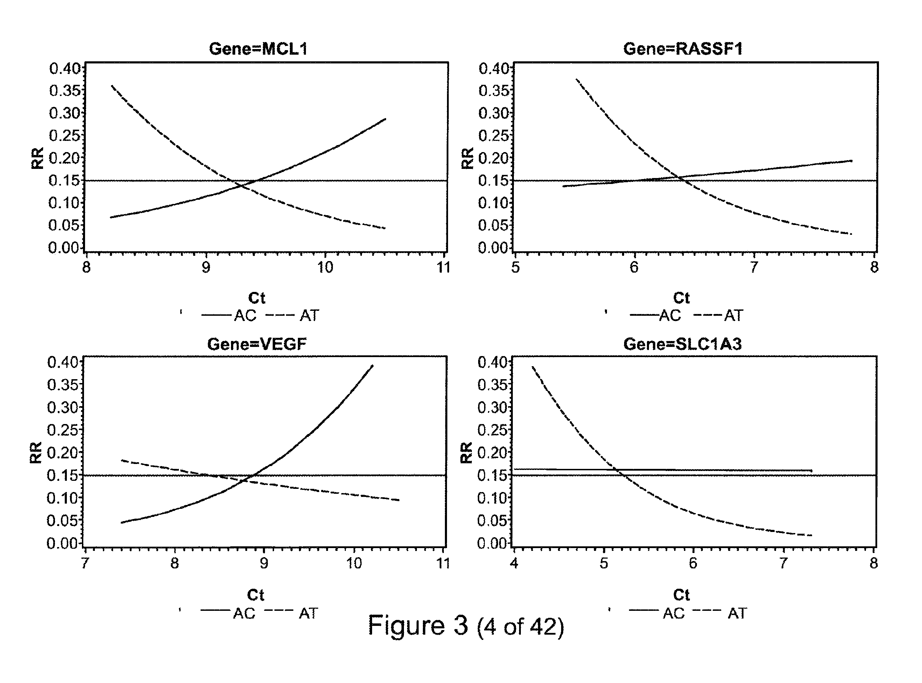

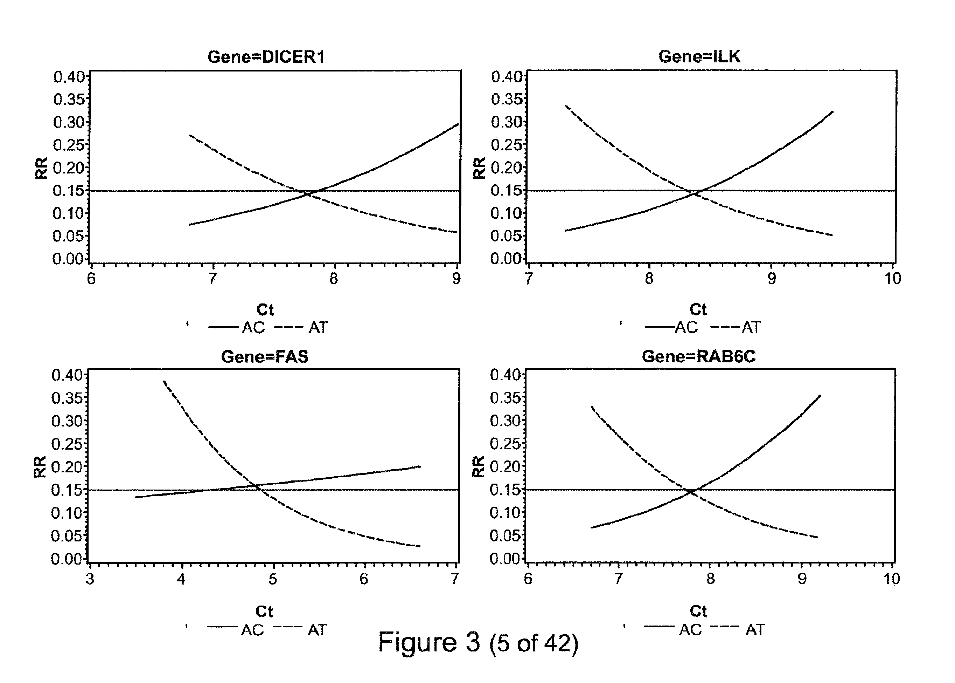

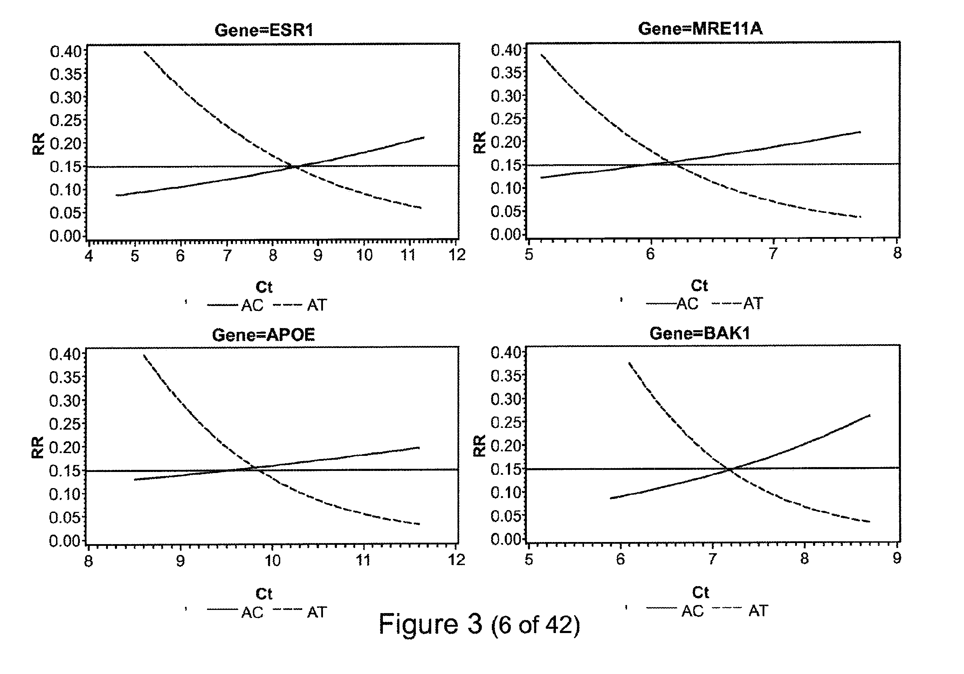

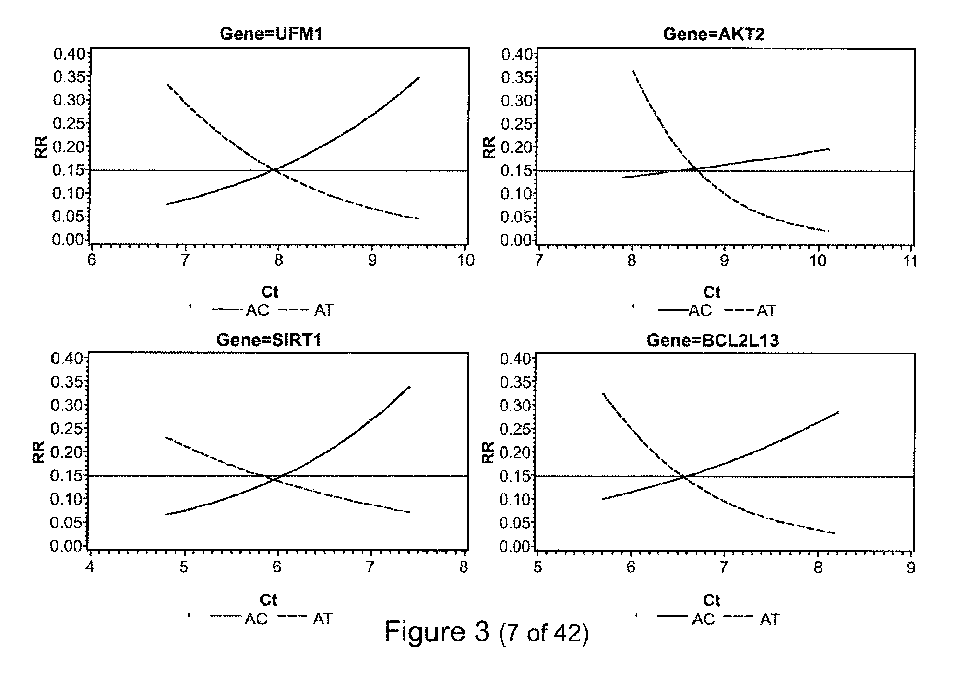

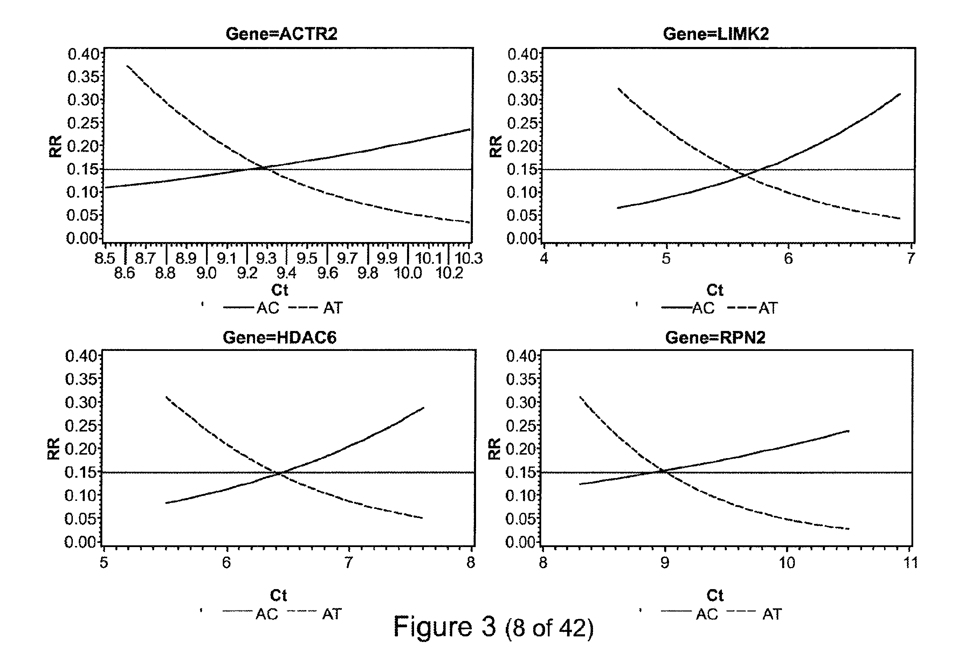

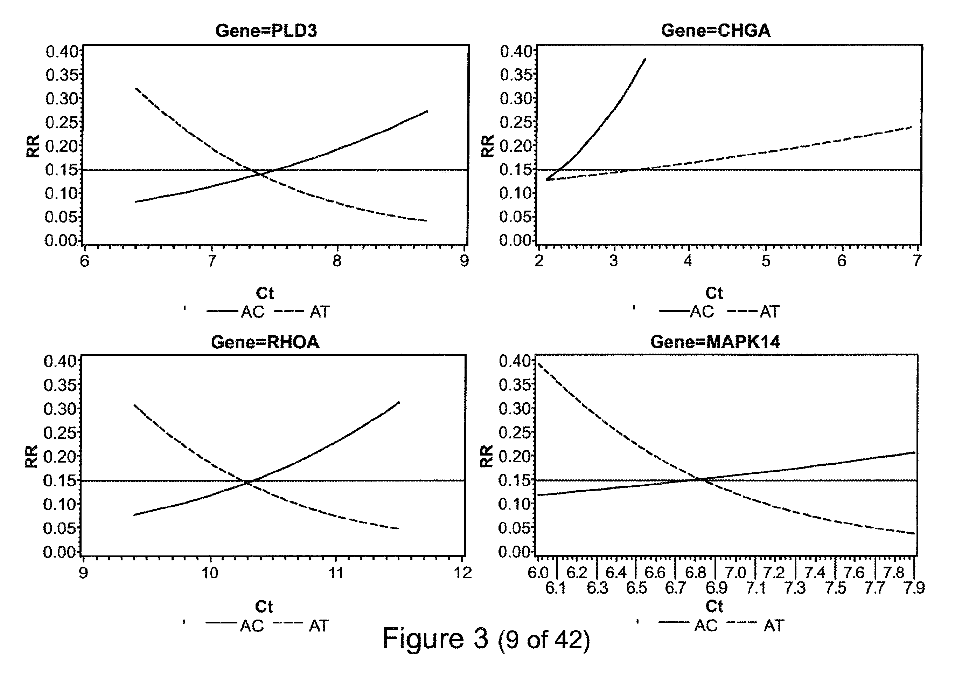

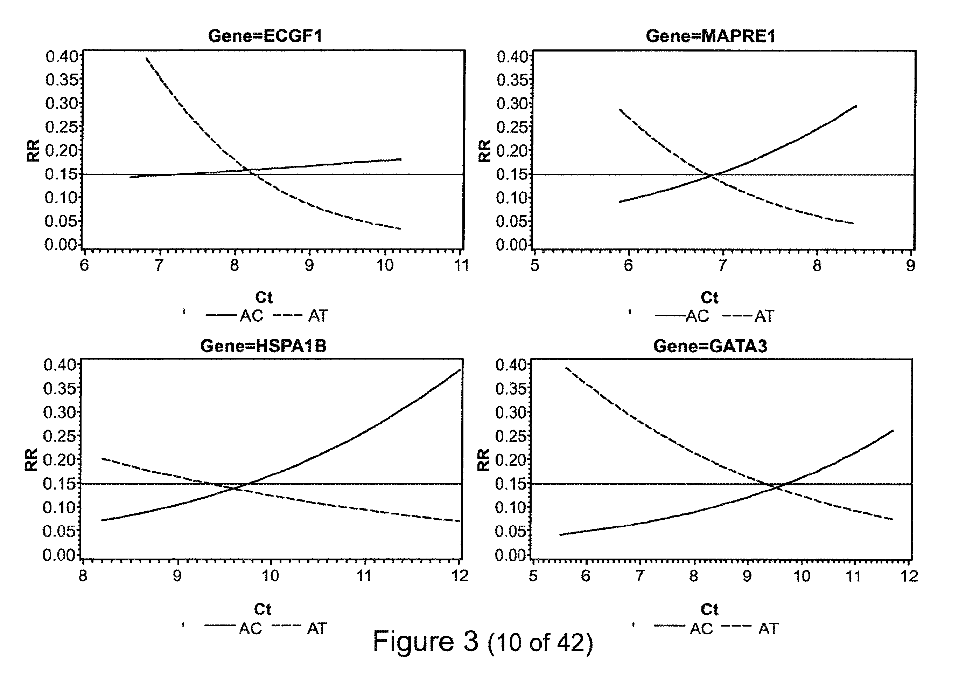

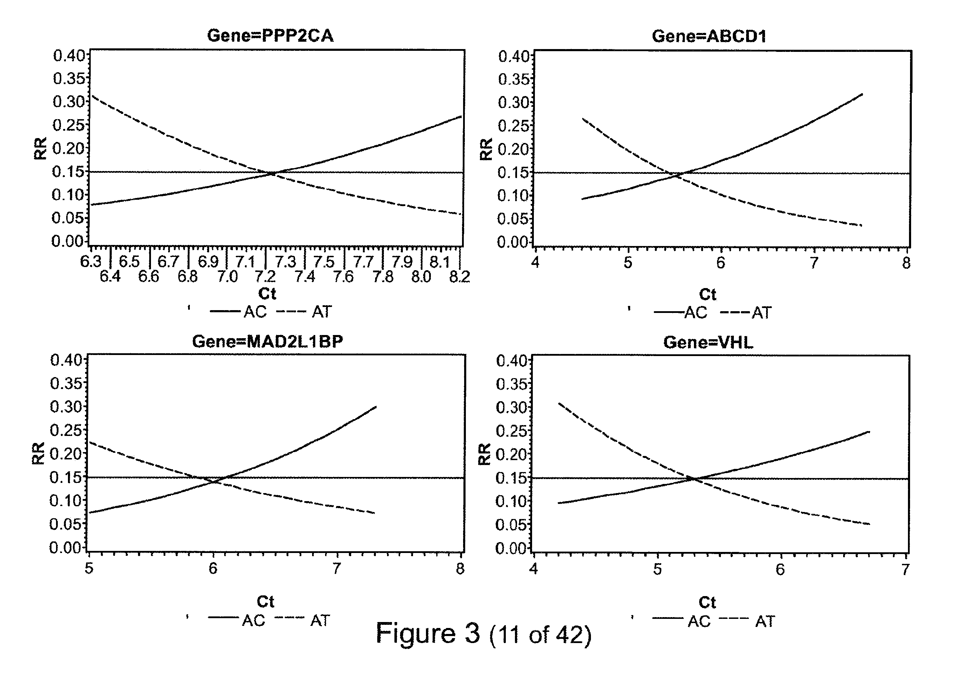

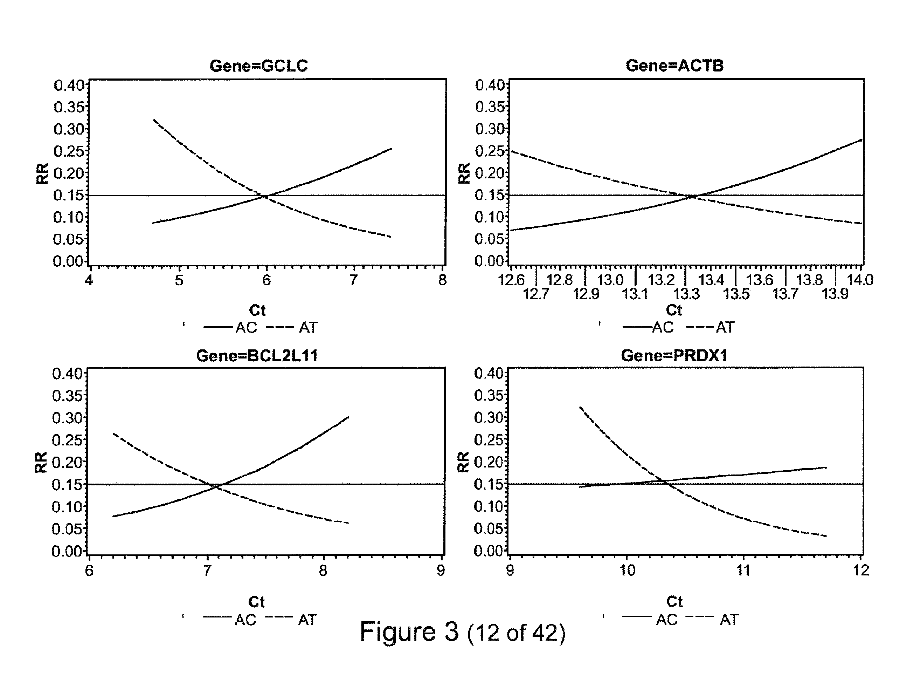

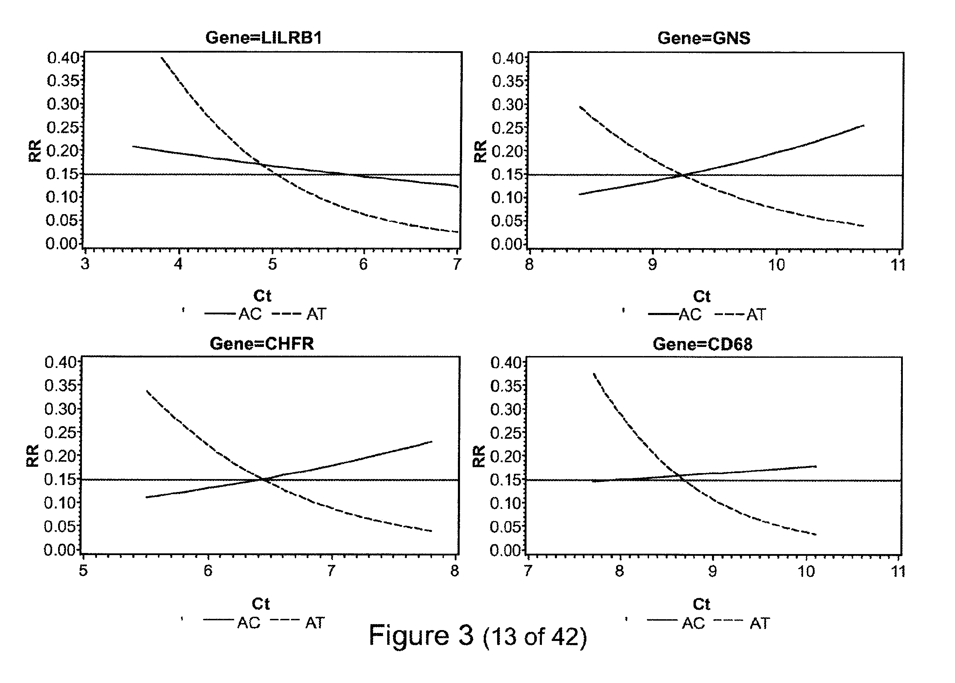

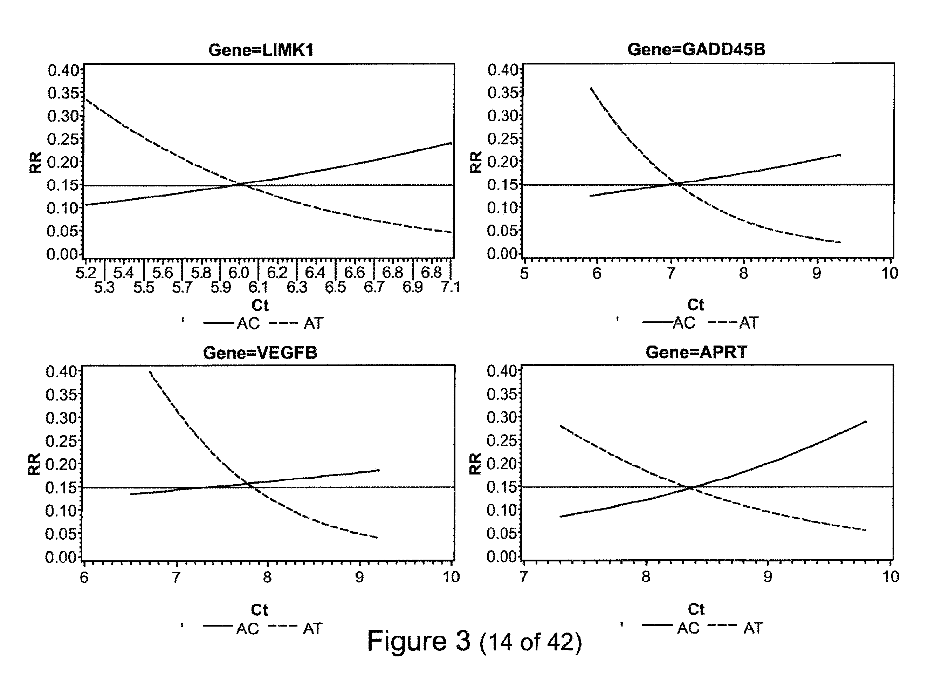

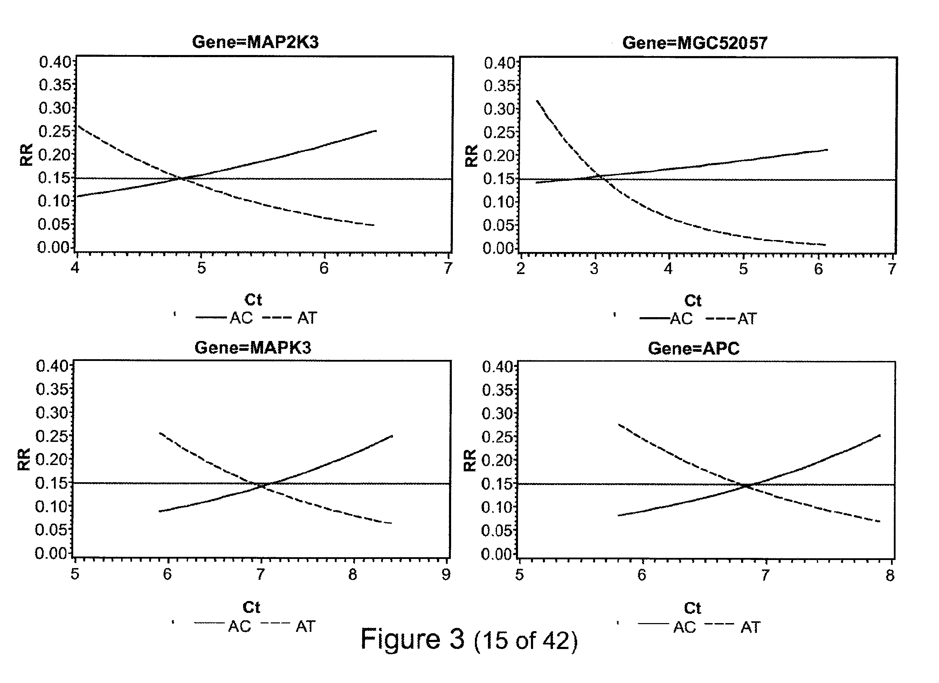

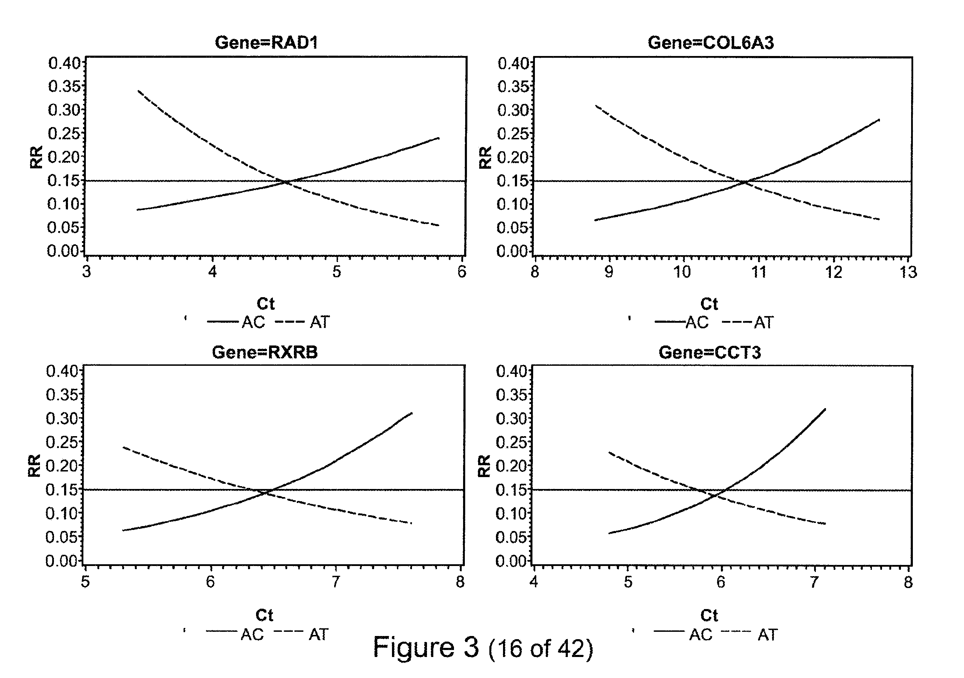

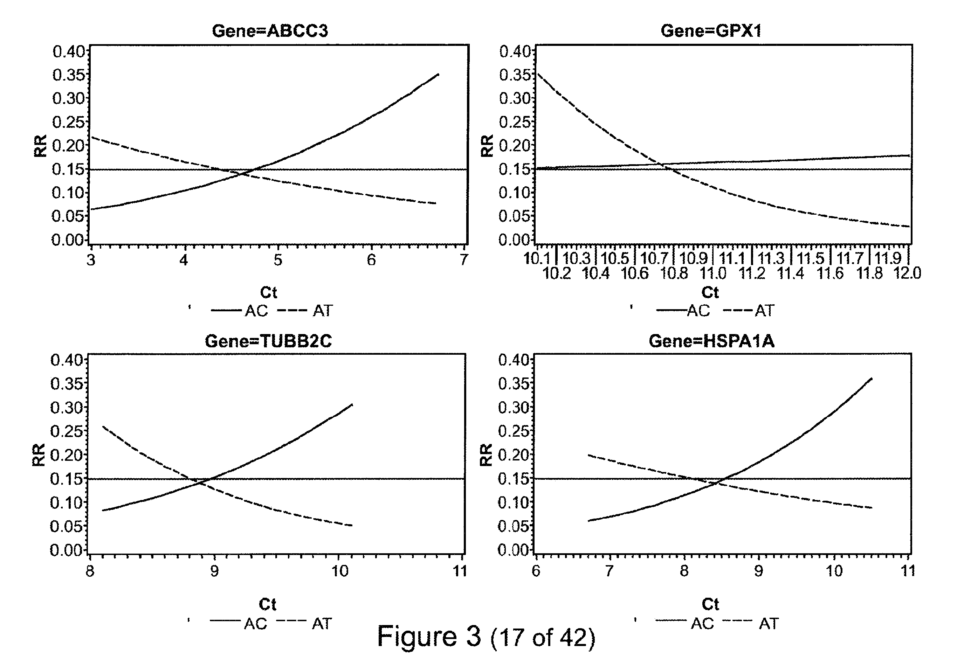

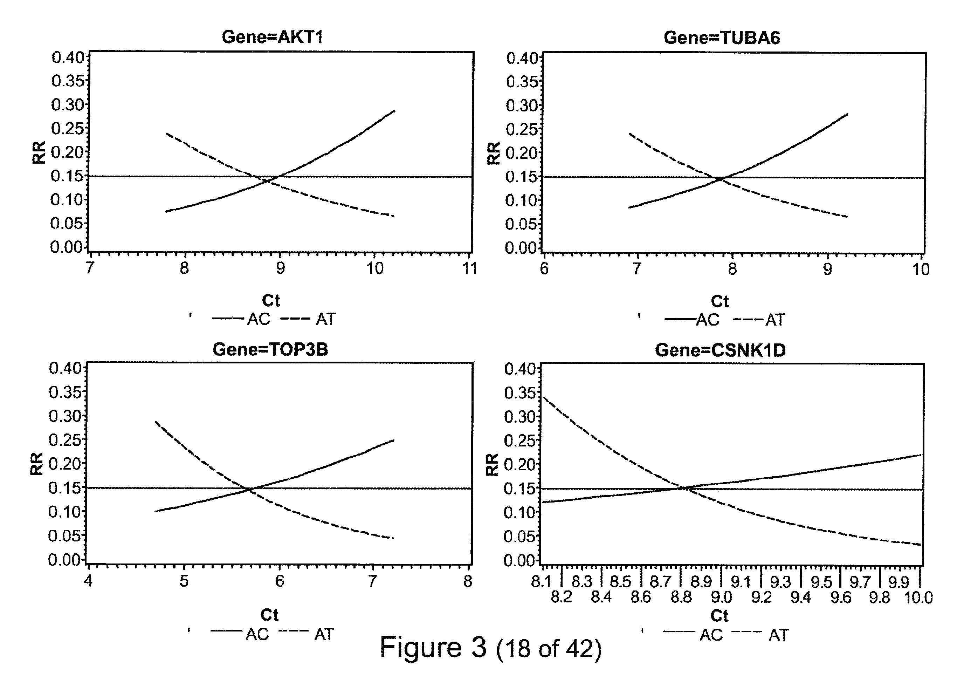

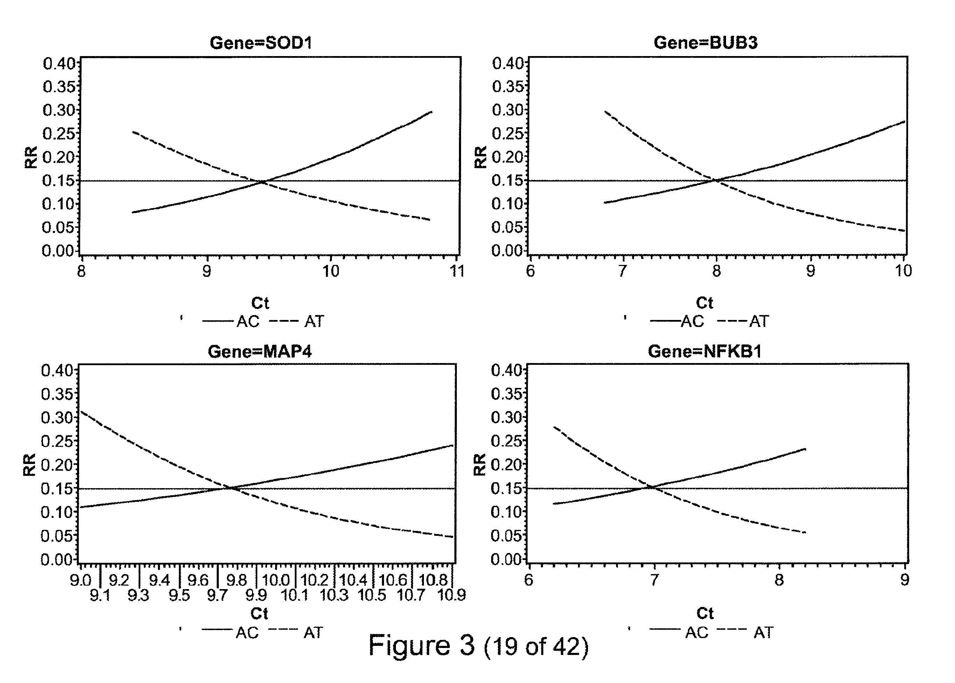

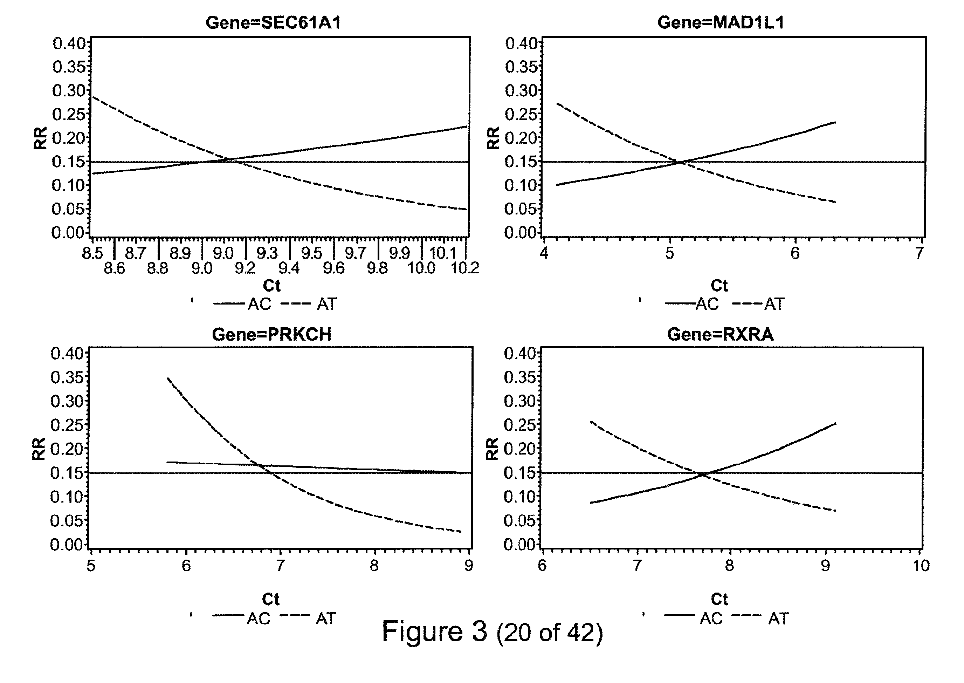

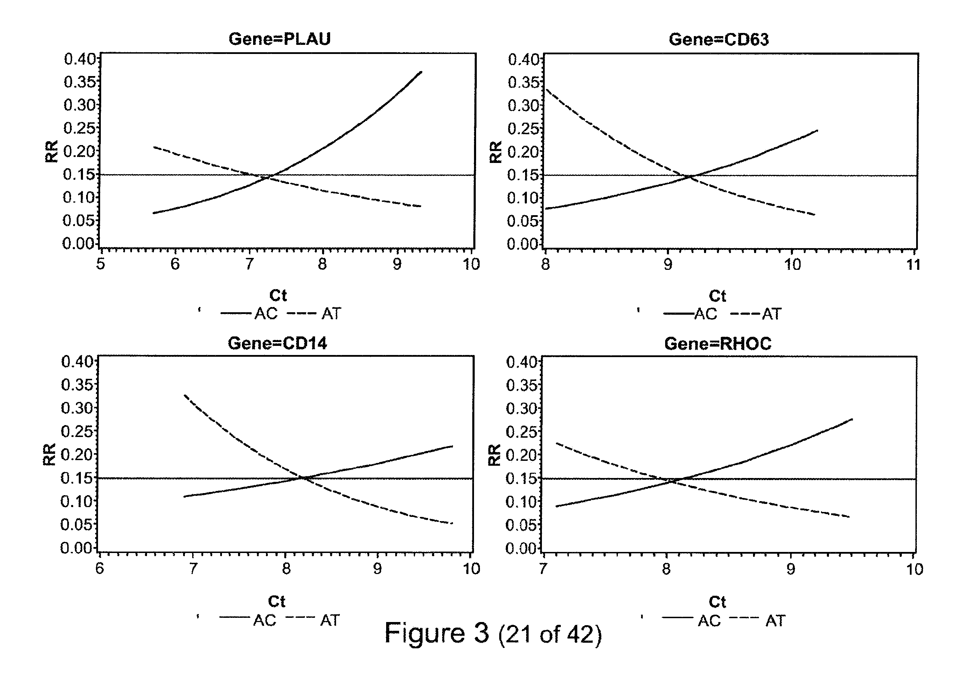

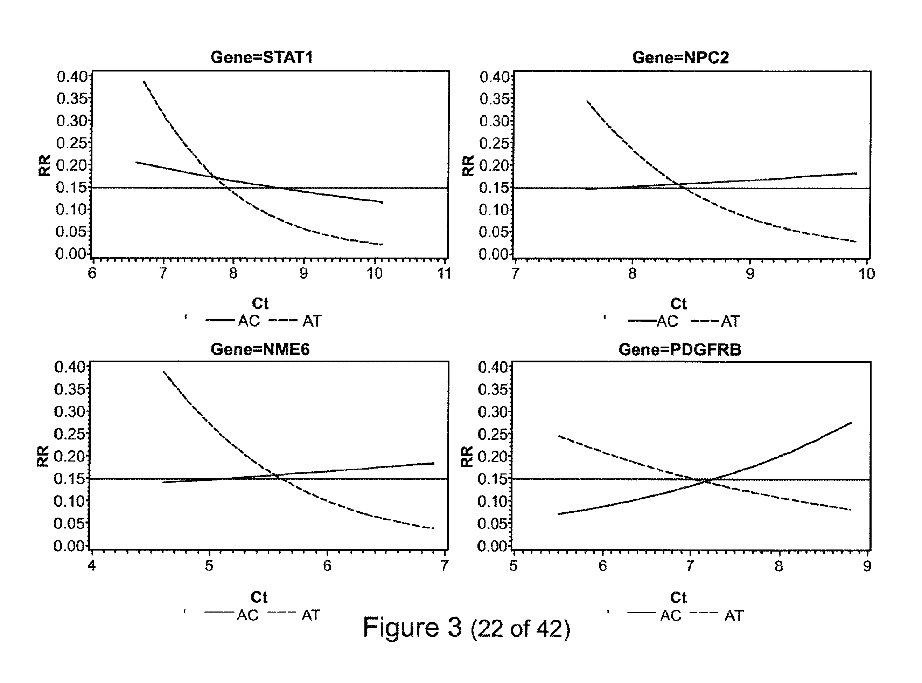

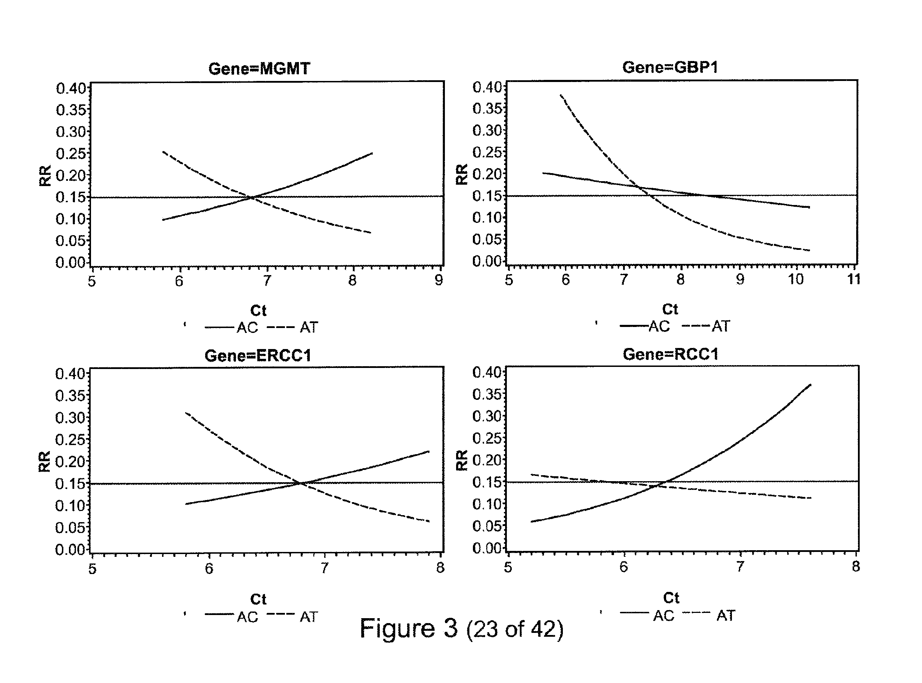

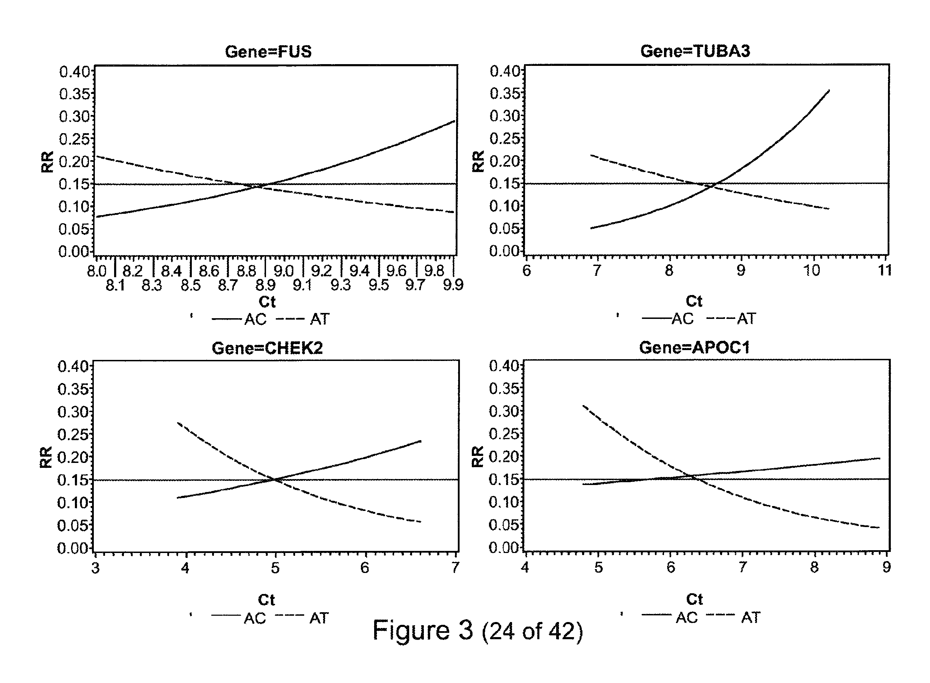

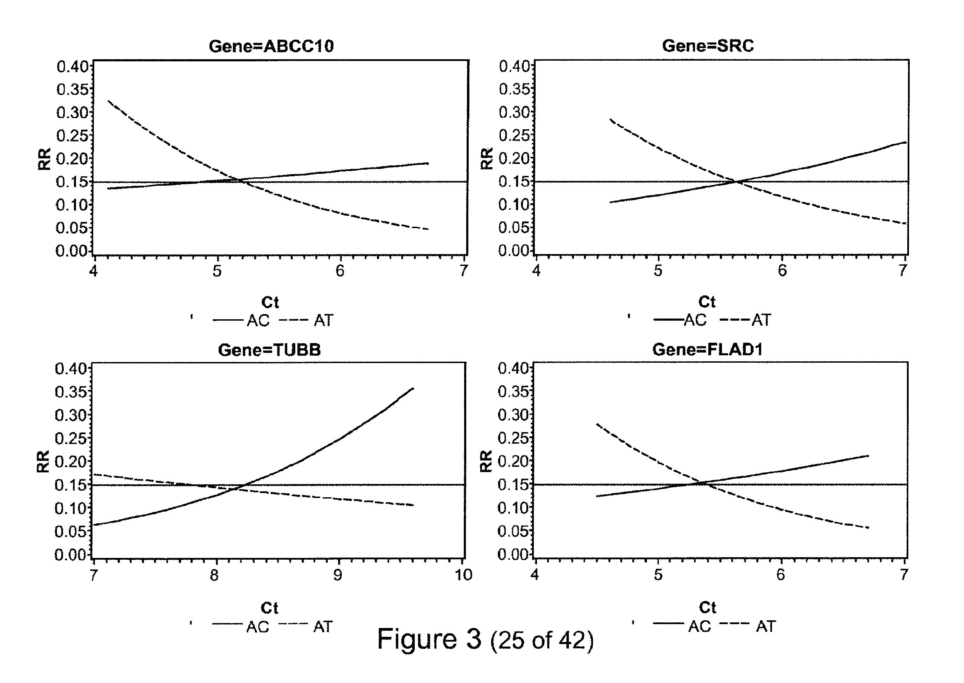

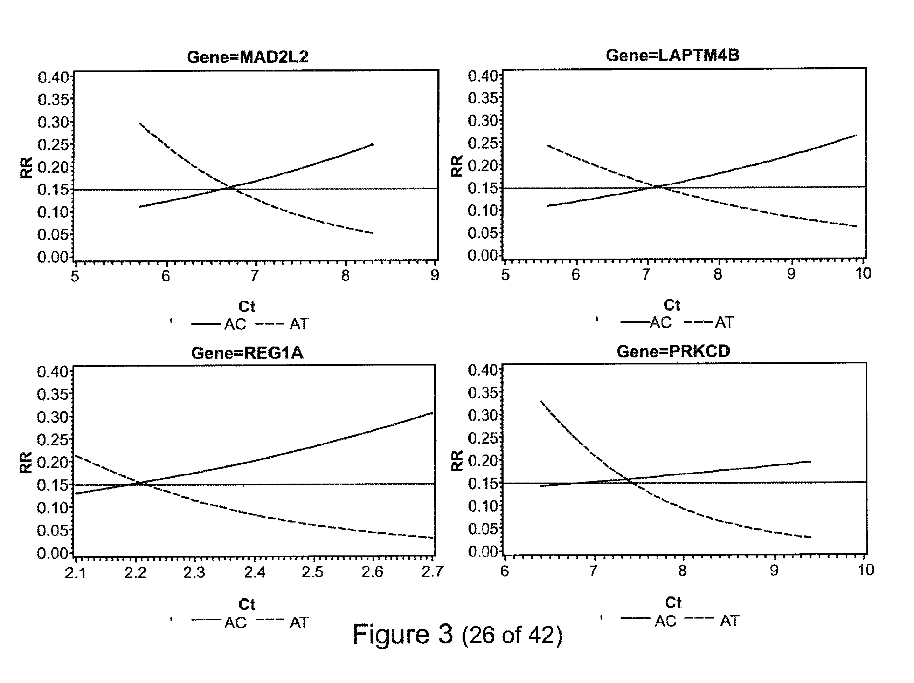

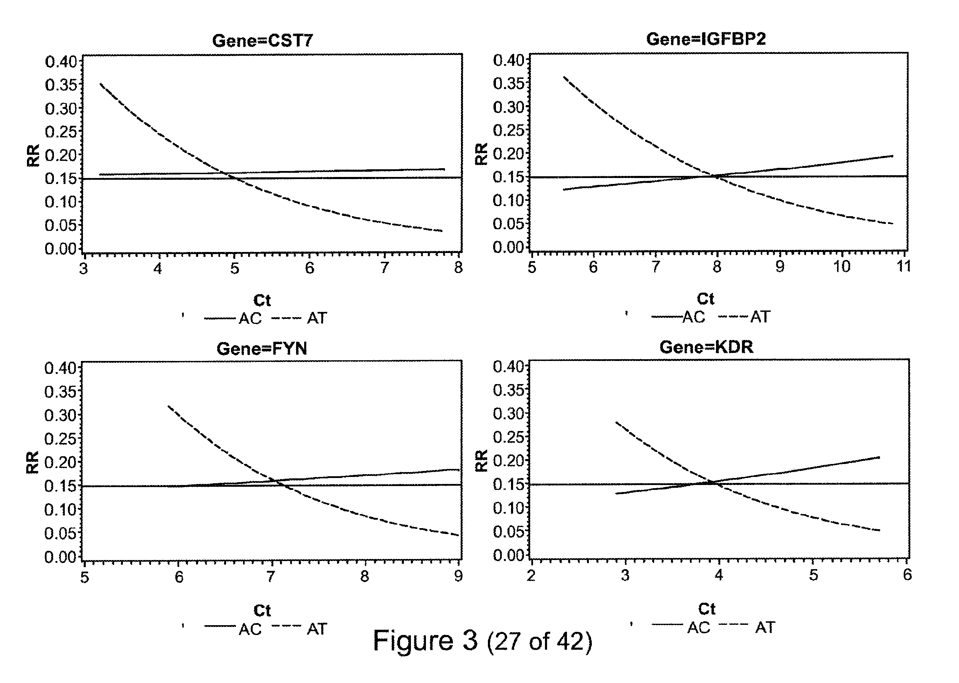

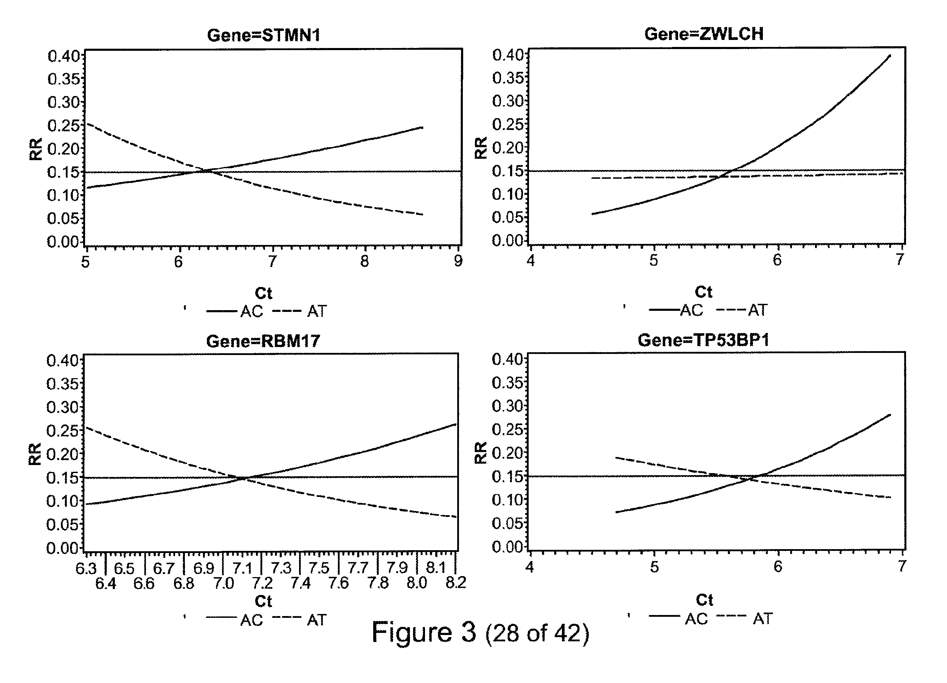

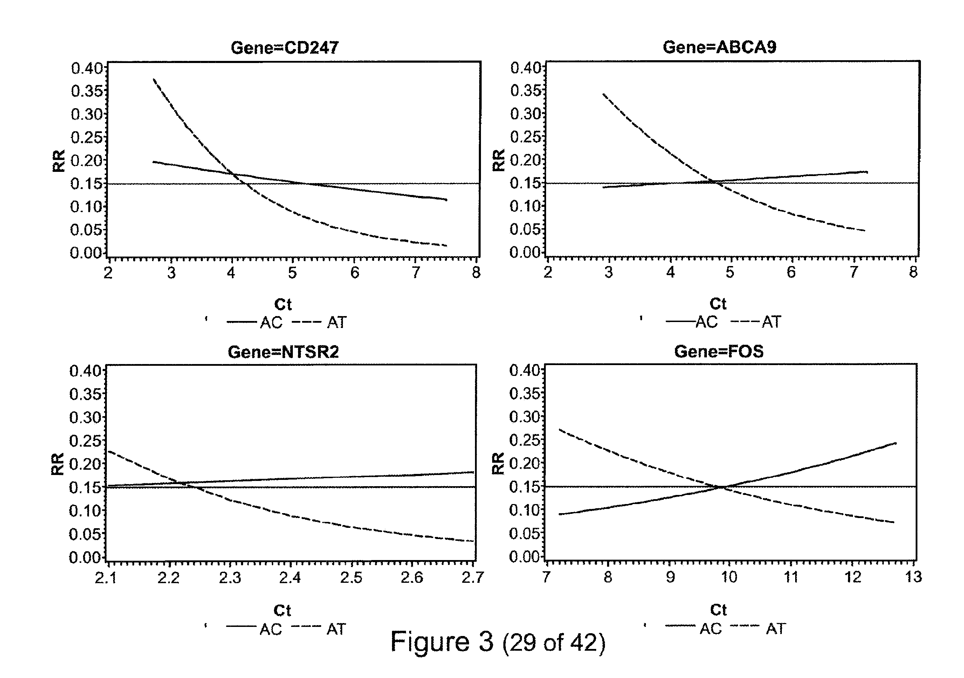

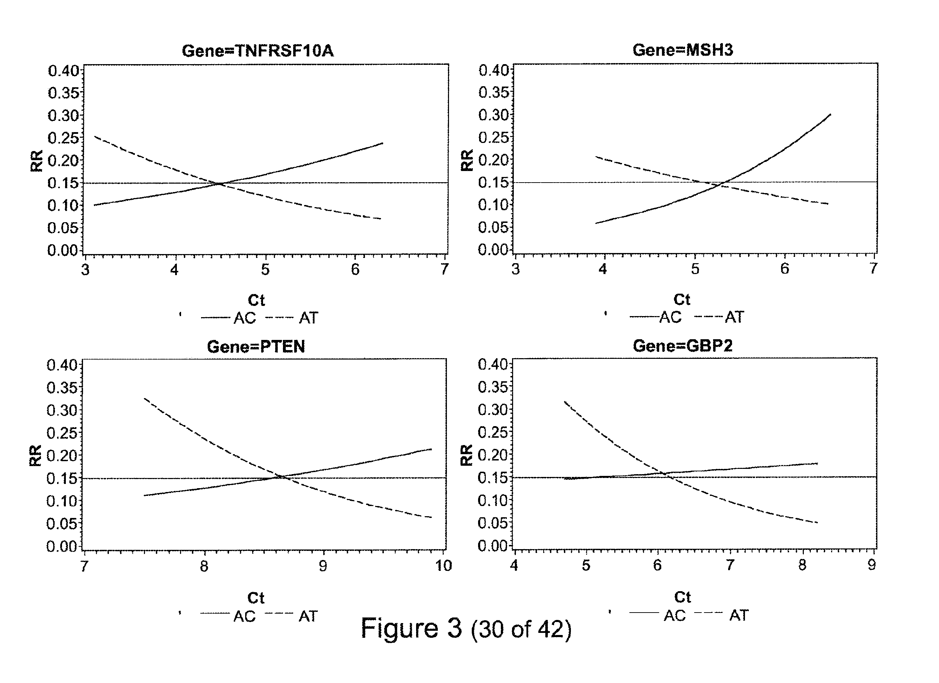

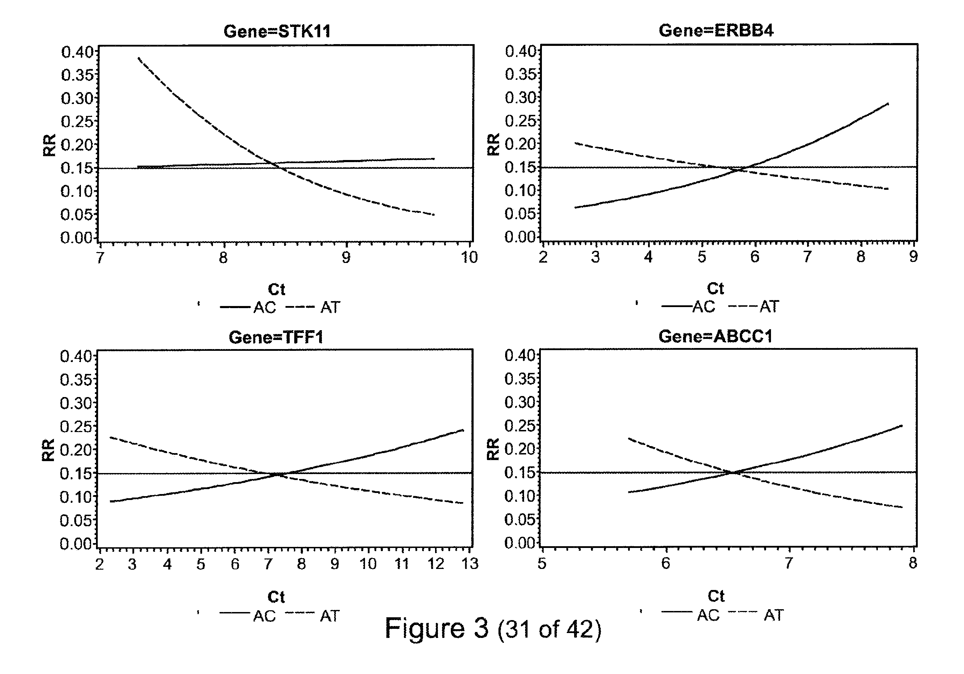

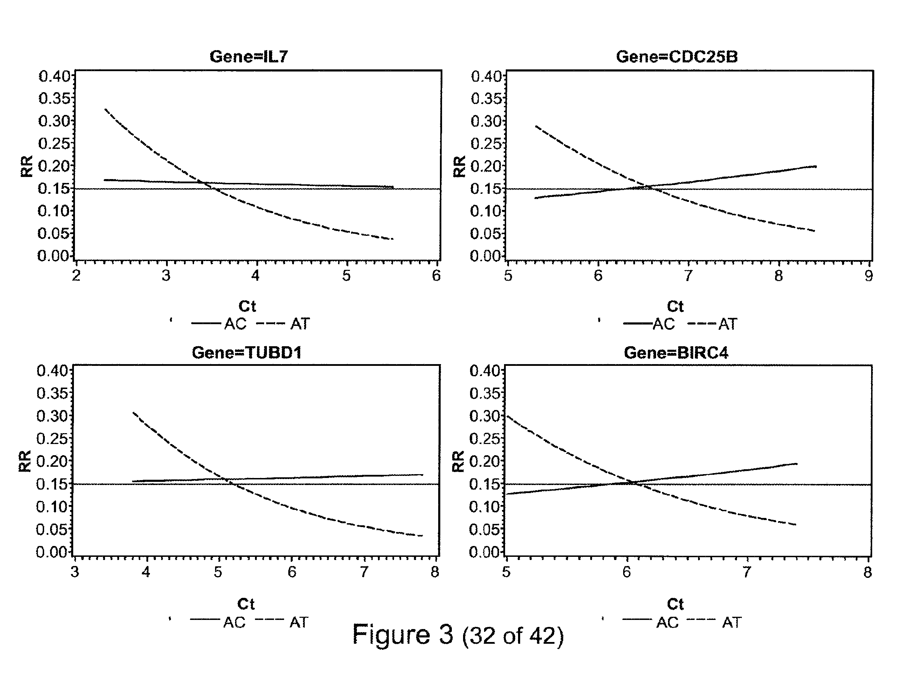

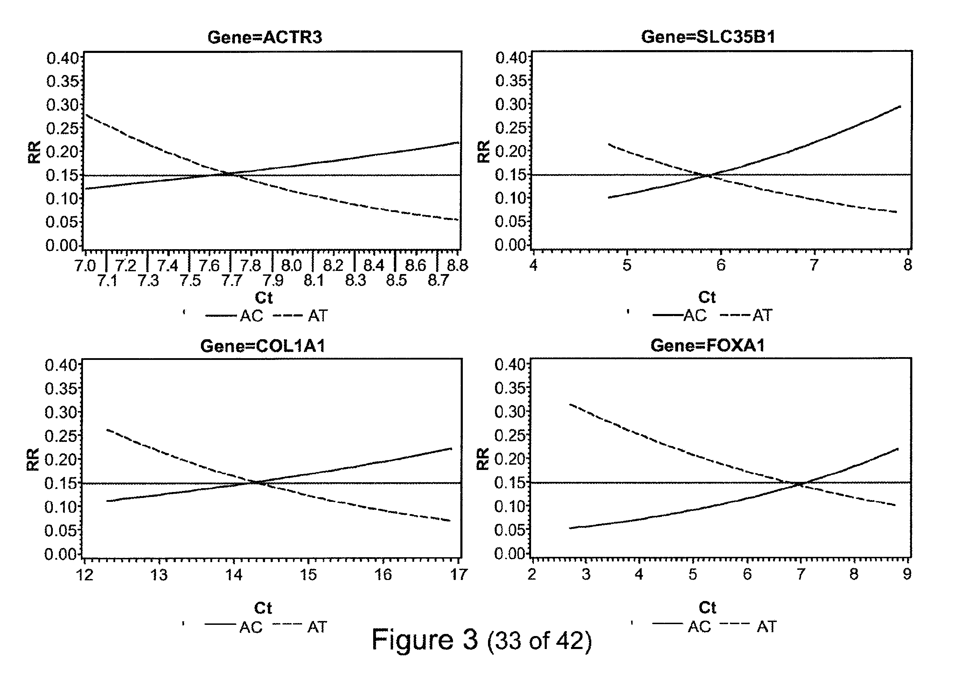

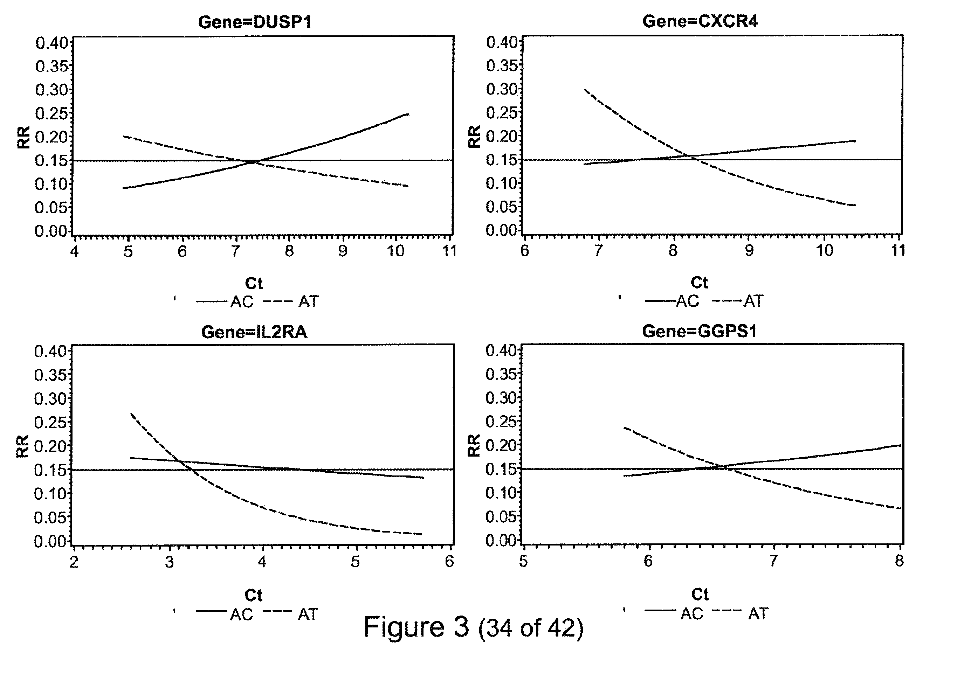

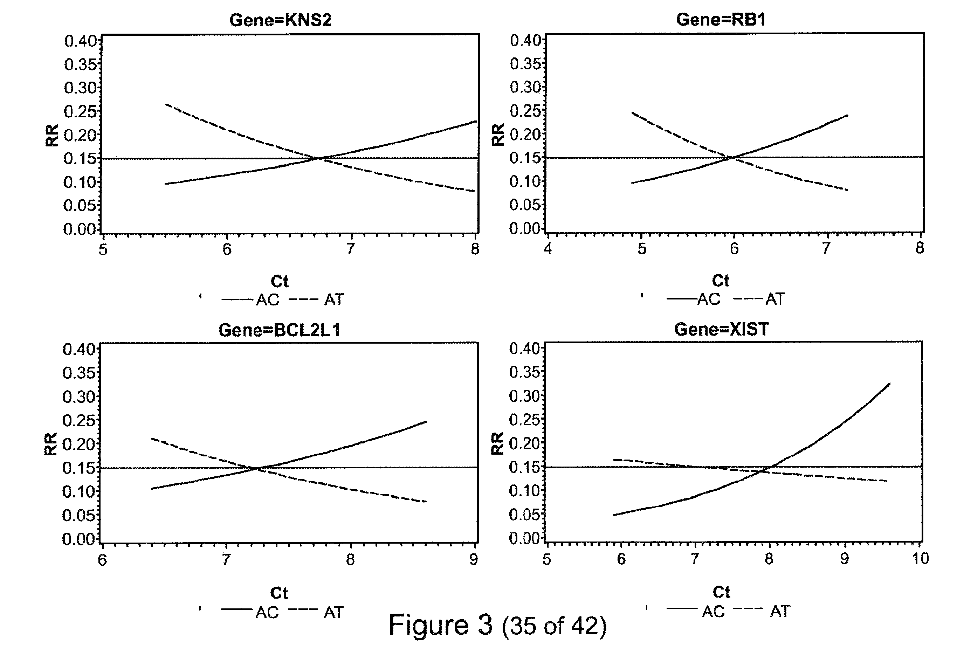

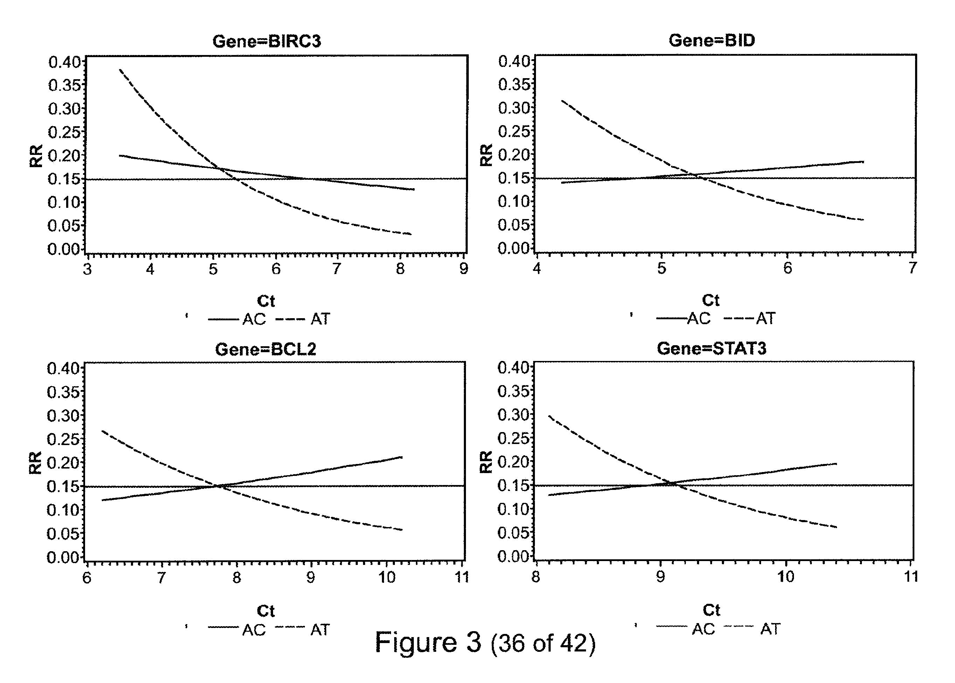

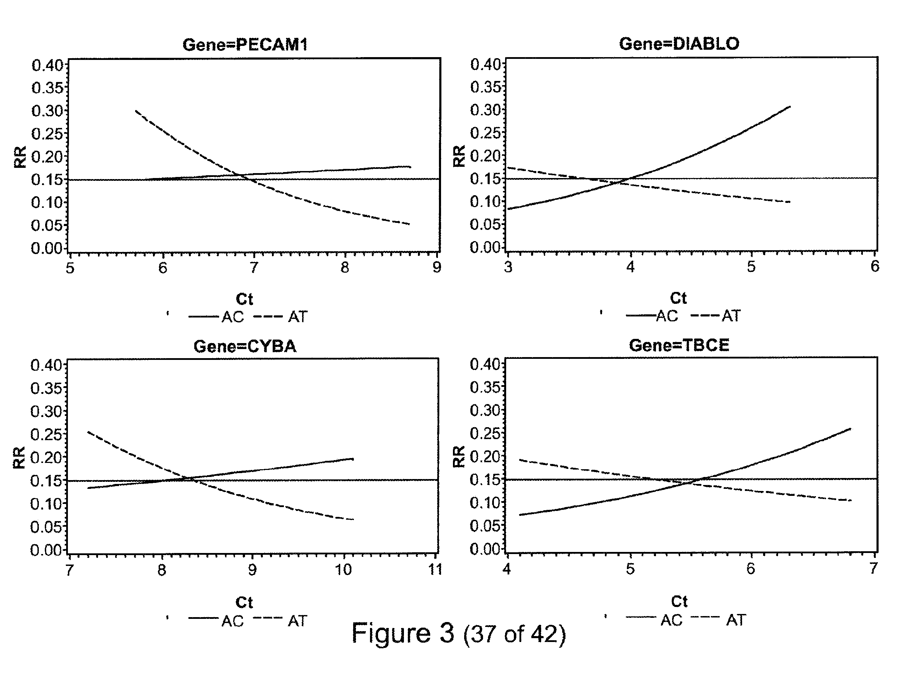

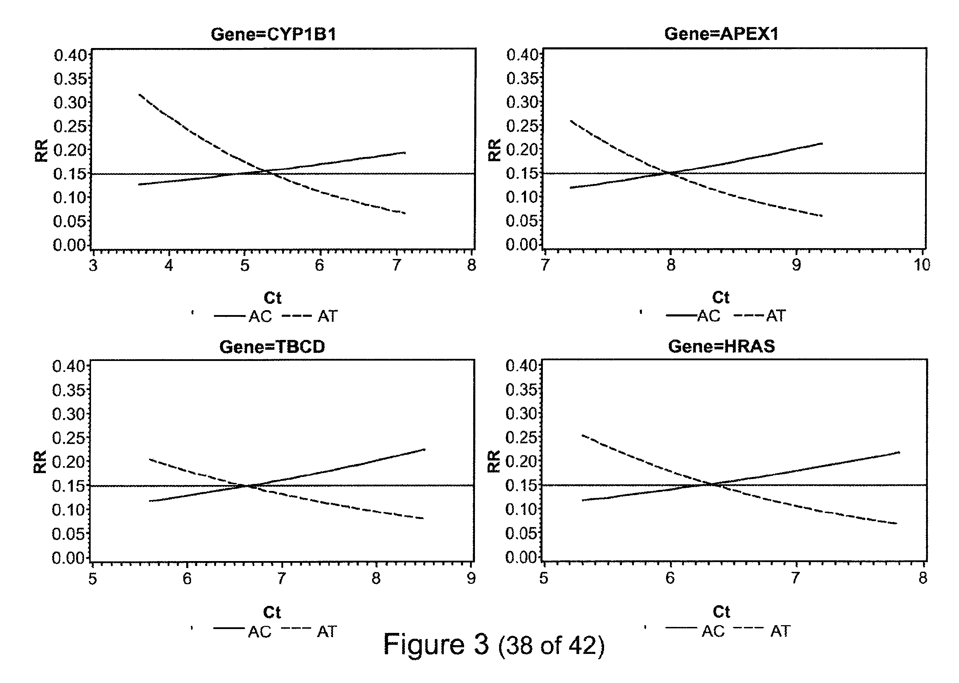

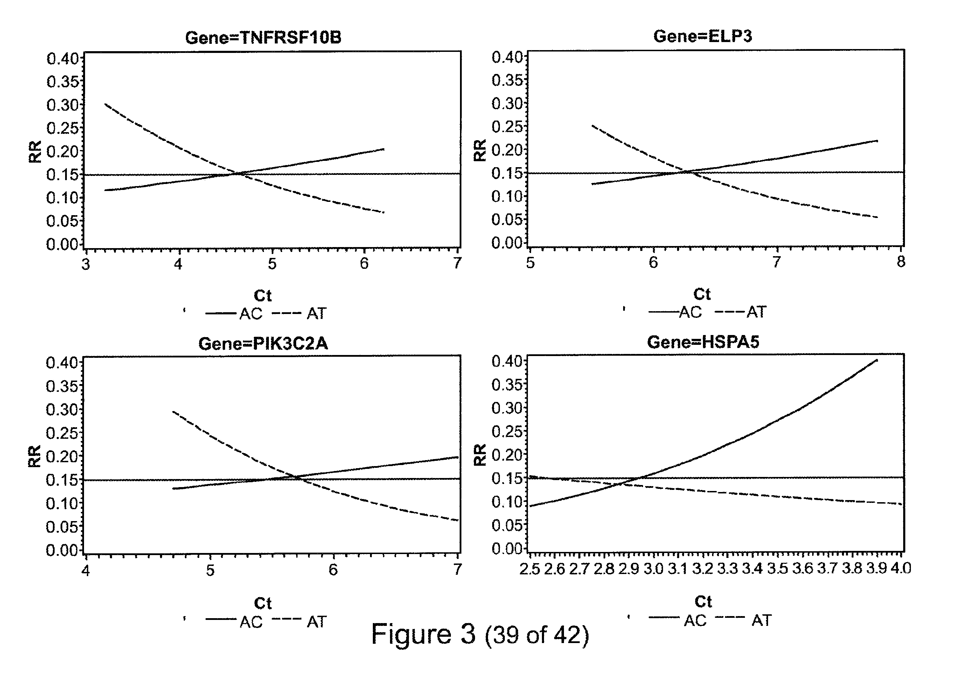

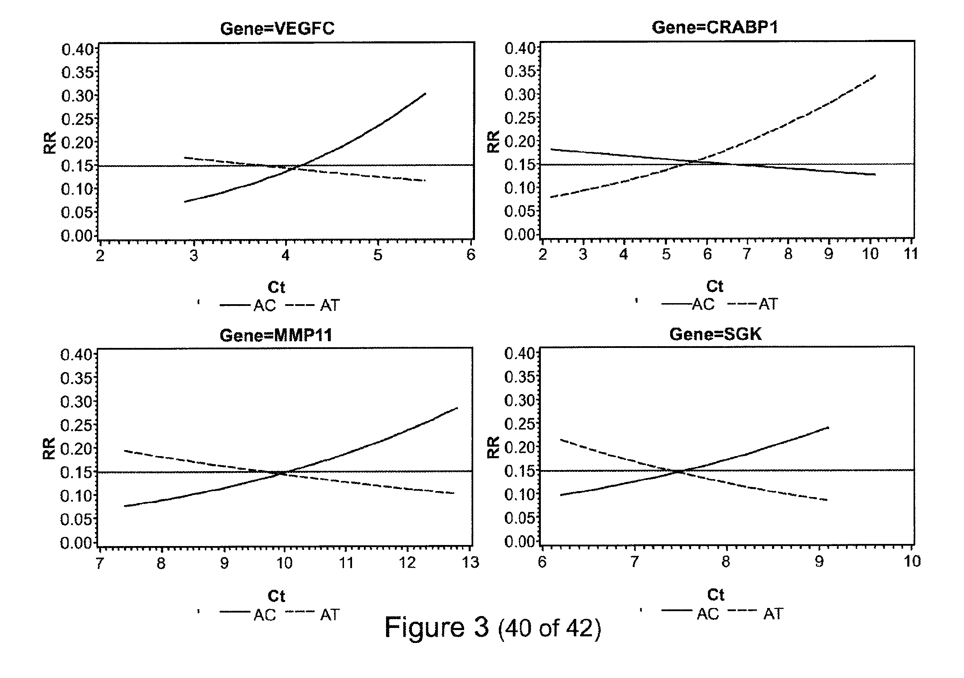

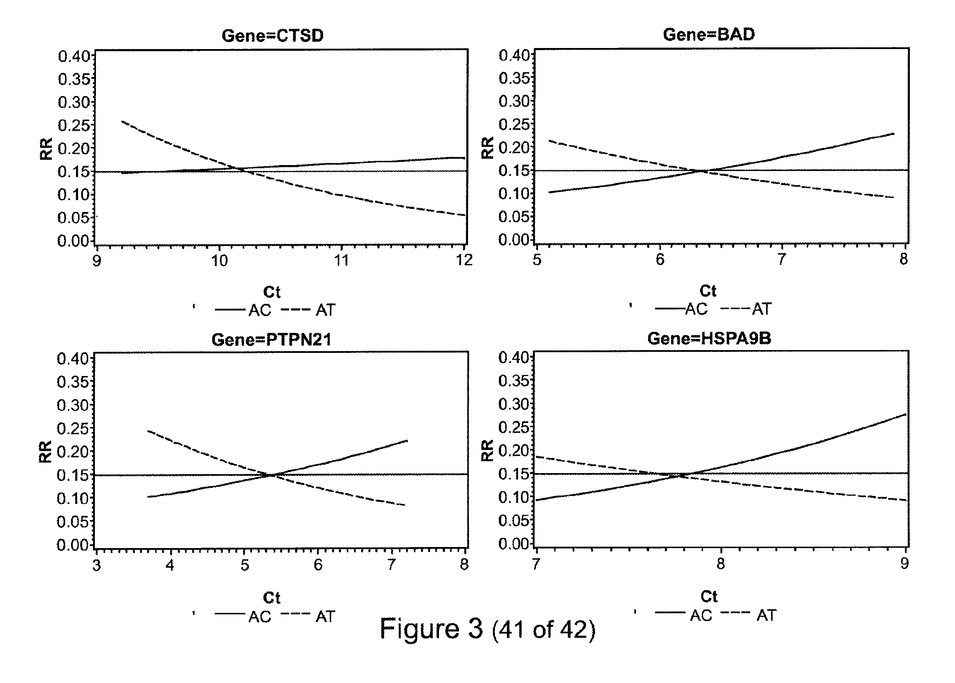

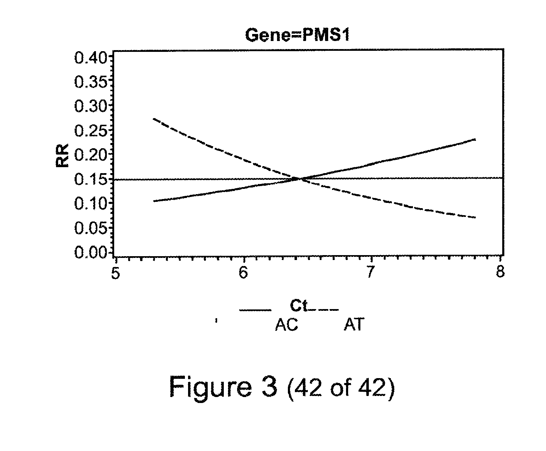

Figure 3 is a set of graphs showing the relationship between normalized expression (represented by "Cₜ") of the indicated gene (gene name provided at top of each graph) and 5-year recurrence rate (RR) of breast cancer in a treatment group receiving anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor positive (HR⁺) breast cancer and an Oncotype Dx Recurrence Score of greater than 18. A horizontal dashed line in each graph represents the overall (i.e., not gene expression-specific) 5-year RR in patients in the study having HR+ breast cancer and an Oncotype Dx Recurrence Score greater than 18 who were randomized to treatment with either AC or AT.

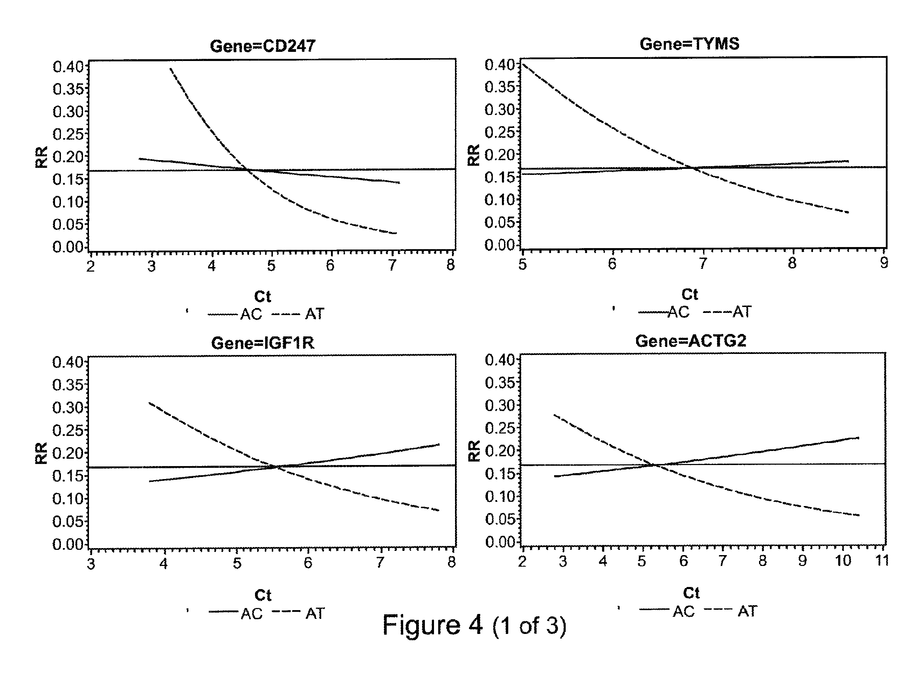

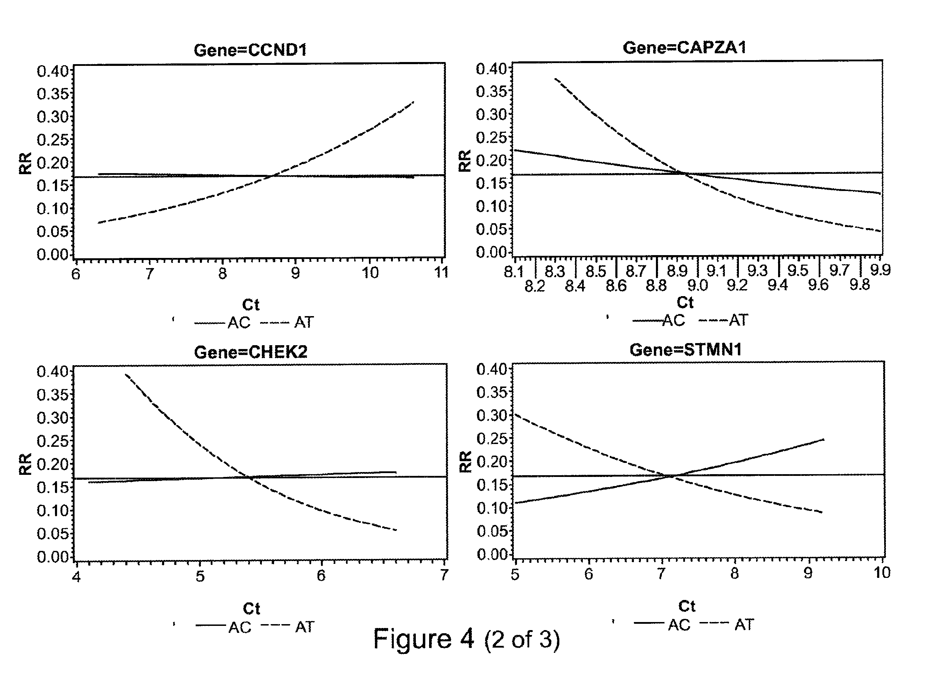

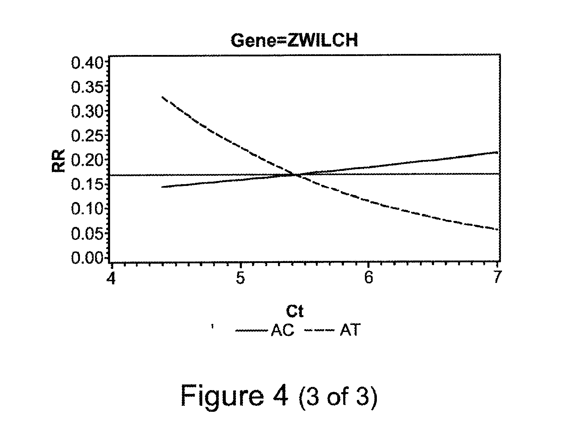

Figure 4 is a set of graphs showing the relationship between normalized expression (represented by "Cₜ") of the indicated gene (gene name provided at top of each graph) and 5-year recurrence rate (RR) of breast cancer in a treatment group receiving an anthracycline and a cyclophosphamide (AC prediction curve; smooth line) and the relationship between expression of the indicated gene and RR in a treatment group receiving anthracycline and a taxane (AT prediction curve; hatched line), where the patients in the treatment groups had hormone receptor negative (HR⁻) breast. A horizontal dashed line in each graph represents the overall (i.e., not gene expression-specific) 5-year RR in patients in the study having HR⁻ breast cancer who were randomized to treatment with either AC or AT

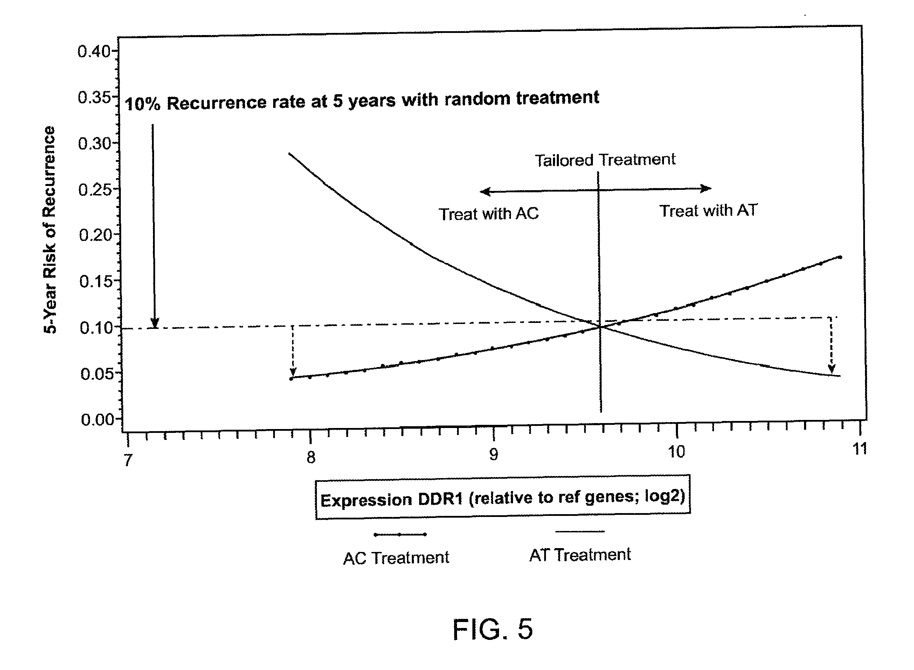

Figure 5 is a graph illustrating the impact of using DDR1 to select HR-positive patients for treatment with AC vs AT. The dotted line depicts the relationship between normalized expression of DDR1 and the 5-year recurrence rate (RR) of breast cancer in the AC treatment group (the AC prediction curve, also referred to as the cyclophosphamide benefit (CB) curve); the solid line depicts the relationship between normalized expression of DDR1 and the 5-year recurrence rate (RR) of breast cancer in the AT treatment group (the AT prediction curve, also referred to as the taxane benefit (TB) curve. Expression is provided on the x-axis as a normalized DDR1 expression level (relative to reference genes; log2). The y-axis provides the risk of cancer recurrence at 5 years.

The following Appendices and Tables are provided in the specification just prior to the claims.
Appendix 1. RT-PCR probe and primer sequences
Appendix 2. RT-PCR amplicon sequences
Table 1.Differential Markers of Response Identified in Breast Cancer Patients, All Patients.
Table 2. Differential Markers of Response Identified in Breast Cancer Patients, HR-Positive Patients
Table 3. Differential Markers of Response Identified in Breast Cancer Patients, HR-Positive Patients, RS > 18
Table 4. Differential Markers of Response Identified in Breast Cancer Patients, HR-Negative Patients.
Table 5. Additional genes involved in NFκB signaling

### DEFINITIONS

Unless defined otherwise, technical and scientific terms used herein have the same meaning as commonly understood by one of ordinary skill in the art to which this invention belongs. See, e.g., Singleton P and Sainsbury D., Dictionary of Microbiology and Molecular Biology 3rd ed., J. Wiley & Sons, Chichester, New York, 2001.

As used herein, the term "anthracycline" refers to a class of antineoplastic antibiotics that are typically derived by *Streptomyces* bacteria (e.g., *Streptomyces peucetius* or *Streptomyces coeruleorubidus*)*.* Although the precise mechanism of action is unknown, anthracyclines are believed to derive their chemotherapeutic activity, at least in part, from their ability to damage DNA by intercalation, metal ion chelation, and the generation of free radicals and can inhibit enzyme activity critical to DNA function. Examples of anthracyclines include daunorubicin, doxorubicin, epirubicin, idarubicin, amrubicin, pirarubicin, valrubicin, zorubicin, caminomycin, detorubicin, esorubicin, marcellomycin, quelamycin, rodorubicin, and aclarubicin, as well as pharmaceutically active salts, acids or derivatives of any of these.

As used herein, the term "taxane" refers to a family of antimitotic/antimicrotubule agents that inhibit cancer cell growth by stopping cell division. Examples of taxanes include paclitaxel, docetaxel, larotaxel, ortataxel, tesetaxel and other related diterpene compounds that have chemotherapeutic activity as well as pharmaceutically active salts, acids or derivatives of any of these. Paclitaxel was originally derived from the Pacific yew tree. Related diterpenes are produced by plants of the genus *Taxus* (yews) and synthetic or semi-synthetic taxanes with chemotherapeutic activity have also been synthesized, e.g., docetaxel, and are encompassed in the term taxane.

As used herein, the term "cyclophosphasmide" refers to a cytotoxic alkylating agent of the nitrogen mustard group, including the chemotherapeutic compound *N,N-*bis(2-chloroethyl)-1,3,2-oxazaphosphinan-2-amine 2-oxide (also known as cytophosphane). It is a highly toxic, immunosuppressive, antineoplastic drug, used in the treatment of Hodgkin's disease, lymphoma, and certain other forms of cancer, such as leukemia and breast cancer.

A "taxane-containing treatment" (also referred to as "taxane-containing regimen" or "taxane-containing treatment regimen") or "cyclophosphamide-containing treatment" (also referred to as "cyclophosphamide-containing regimen" or "cyclophosphamide-containing treatment regimen") is meant to encompass therapies in which a taxane or a cyclophosphamide, respectively, is administered alone or in combination with another therapeutic regimen (e.g., another chemotherapy (e.g., anthracycline), or both). Thus, a taxane-containing treatment can include, for example, administration a taxane in combination with anthracyline, with anthracyline and cyclosphophamide, and the like.

The term "in combination with" such as when used in reference to a therapeutic regimen, refers to administration or two or more therapies over the course of a treatment regimen, where the therapies may be administered together or separately, and, where used in reference to drugs, may be administered in the same or different formulations, by the same or different routes, and in the same or different dosage form type.

The term "prognosis" is used herein to refer to the prediction of the likelihood of cancer-attributable death or progression, including recurrence, of a neoplastic disease, such as breast cancer, in a patient. The concept of prognosis is used in the context of the minimal standard of care. For example, in the context of early stage, ER+ invasive breast care, the minimal standard of care could be surgery plus adjuvant hormonal therapy.

The term "prediction" is used herein to refer to a likelihood that a patient will have a particular clinical outcome following administration of a treatment regimen, e.g., a chemotherapeutic regimen. Clinical benefit may be measured, for example, in terms of clinical outcomes such as disease recurrence, tumour shrinkage, and/or disease progression.

The term "patient" or "subject" as used herein refers to a human patient.
The term "long-term" survival is used herein to refer to survival for at least 3 years, more preferably for at least 8 years, most preferably for at least 10 years following surgery or other treatment.

The term "tumour," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth.

The term "breast cancer" is used herein to include all forms and stages of breast cancer, including, without limitation, locally advanced breast cancer, invasive breast cancer, and metastatic breast cancer.

A "tumour sample" as used herein is a sample derived from, or containing tumour cells from, a patient's tumour. Examples of tumour samples herein include, but are not limited to, tumour biopsies, circulating tumour cells, circulating plasma proteins, ascitic fluid, primary cell cultures or cell lines derived from tumours or exhibiting tumour-like properties, as well as preserved tumour samples, such as formalin-fixed, paraffin-embedded tumour samples.
The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

As used herein, the term "expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The terms "threshold" or "thresholding" refer to a procedure used to account for non-linear relationships between gene expression measurements and clinical response as well as to further reduce variation in reported patient scores. When thresholding is applied, all measurements below or above a threshold are set to that threshold value. Non-linear relationship between gene expression and outcome could be examined using smoothers or cubic splines to model gene expression in Cox PH regression on recurrence free interval or logistic regression on recurrence status. Variation in reported patient scores could be examined as a function of variability in gene expression at the limit of quantitation and/or detection for a particular gene.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA.

Unless indicated otherwise, each gene name used herein corresponds to the Official Symbol assigned to the gene and provided by Entrez Gene (URL: http://www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure, such as for example between the expression level of a gene and the likelihood of beneficial response to treatment with a drug. For example, the increased expression level of a gene may be positively correlated (positively associated) with an increased likelihood of good clinical outcome for the patient, such as an increased likelihood of long-term survival without recurrence of the cancer and/or beneficial response to a chemotherapy, and the like. Such a positive correlation may be demonstrated statistically in various ways, e.g. by a low hazard ratio. In another example, the increased expression level of a gene may be negatively correlated (negatively associated) with an increased likelihood of good clinical outcome for the patient. In that case, for example, the patient may have a decreased likelihood of long-term survival without recurrence of the cancer and/or beneficial response to a chemotherapy, and the like. Such a negative correlation indicates that the patient likely has a poor prognosis or will respond poorly to a chemotherapy, and this may be demonstrated statistically in various ways, e.g., a high hazard ratio.

A "positive clinical outcome" and "beneficial response" can be assessed using any endpoint indicating a benefit to the patient, including, without limitation, (1) inhibition, to some extent, of tumour growth, including slowing down and complete growth arrest; (2) reduction in the number of tumour cells; (3) reduction in tumour size; (4) inhibition (i.e., reduction, slowing down or complete stopping) of tumour cell infiltration into adjacent peripheral organs and/or tissues; (5) inhibition of metastasis; (6) enhancement of anti-tumour immune response, possibly resulting in regression or rejection of the tumour; (7) relief, to some extent, of one or more symptoms associated with the tumour; (8) increase in the length of survival following treatment; and/or (9) decreased mortality at a given point of time following treatment. Positive clinical response may also be expressed in terms of various measures of clinical outcome. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

The term "risk classification" means a level of risk (or likelihood) that a subject will experience a particular clinical outcome. A subject may be classified into a risk group or classified at a level of risk based on the methods of the present disclosure, e.g. high, medium, or low risk. A "risk group" is a group of subjects or individuals with a similar level of risk for a particular clinical outcome.

The term "normalized expression" with regard to a gene or an RNA transcript or other expression product (e.g., protein) is used to refer to the level of the transcript (or fragmented RNA) determined by normalization to the level of reference mRNAs, which might be all measured transcripts in the specimen or a particular reference set of mRNAs. A gene exhibits "increased expression" or "increased normalized expression" in a subpopulation of subjects when the normalized expression level of an RNA transcript (or its gene product) is higher in one clinically relevant subpopulation of patients (e.g., patients who are responsive to chemotherapy treatment) than in a related subpopulation (e.g., patients who are not responsive to said chemotherapy). In the context of an analysis of a normalized expression level of a gene in tissue obtained from an individual subject, a gene is exhibits "increased expression" when the normalized expression level of the gene trends toward or more closely approximates the normalized expression level characteristic of such a clinically relevant subpopulation of patients. Thus, for example, when the gene analyzed is a gene that shows increased expression in responsive subjects as compared to nonresponsive subjects, then if the expression level of the gene in the patient sample trends toward a level of expression characteristic of a responsive subject, then the gene expression level supports a determination that the individual patient is likely to be a responder. Similarly, where the gene analyzed is a gene that is increased in expression in nonresponsive patients as compared to responsive patients, then if the expression level of the gene in the patient sample trends toward a level of expression characteristic of a nonresponsive subject, then the gene expression level supports a determination that the individual patient will be nonresponsive. Thus normalized expression of a given gene as disclosed herein can be described as being positively correlated with an increased likelihood of positive clinical response to chemotherapy or as being positively correlated with a decreased likelihood of a positive clinical response to chemotherapy.

The term "recurrence score" or "RS" refers to an algorithm-based indicator useful in determining the likelihood of an event of interest, such as a likelihood of cancer recurrence and/or the likelihood that a patient will respond to a treatment modality as may be assessed by cancer recurrence following therapy with the treatment modality.

The term "hormone receptor positive (HR+) tumour" means a tumour expressing either estrogen receptor (ER+) or progesterone receptor (PR+) above a certain threshold as determined by standard methods, including immunohistochemical staining of nuclei and polymerase chain reaction (PCR) in a biological sample obtained from a patient. The term "hormone receptor negative (HR-) tumour" means a tumour that does not express either estrogen receptor (ER-) or progesterone receptor (PR-) above a certain threshold. The threshold may be measured, for example, using an Allred score or gene expression. See, e.g., J. Harvey, et al., J Clin Oncol 17:1474-1481 (1999); S. Badve, et al., J Clin Oncol 26(15):2473-2481 (2008).
"Overall survival (OS)" refers to the patient remaining alive for a defined period of time, such as 1 year, 5 years, etc, e.g., from the time of diagnosis or treatment.

"Progression-free survival (PFS)" refers to the patient remaining alive, without the cancer getting worse.

"Neoadjuvant therapy" is adjunctive or adjuvant therapy given prior to the primary (main) therapy. Neoadjuvant therapy includes, for example, chemotherapy, radiation therapy, and hormone therapy. Thus, chemotherapy may be administered prior to surgery to shrink the tumour, so that surgery can be more effective, or, in the case of previously unoperable tumours, possible.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50 °C; (2) employ during hybridization a denaturing agent, such as formamide, for example, 50% (v/v) formamide with 0.1 % bovine serum albumin/0.1% Ficoll/0.1% polyvinylpyrrolidone/50 mM sodium phosphate buffer at pH 6.5 with 750 mM sodium chloride, 75 mM sodium citrate at 42 °C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 µg/ml), 0.1% SDS, and 10% dextran sulfate at 42 °C, with washes at 42 °C in 0.2 x SSC (sodium chloride/sodium citrate) and 50% formamide at 55 °C, followed by a high-stringency wash consisting of 0.1 x SSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37 °C. in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50 °C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

Herein, numerical ranges or amounts prefaced by the term "about" expressly include the exact range or exact numerical amount.

### GENERAL DESCRIPTION

The disclosed methods are useful to facilitate treatment decisions by providing an assessment of the likelihood of clinical benefit to a treatment that includes a taxane, a treatment that includes a cyclophosphamide, or both. Because taxanes and cyclophosphamide have different mechanisms of action, it is possible that tumours of certain patients exhibit molecular pathology that makes them more likely to respond to one drug type than the other. For example, the methods disclosed herein can be used to facilitate treatment decisions by providing an assessment of the likelihood of clinical benefit to an anthracycline-based treatment that includes a taxane, an anthracycline-based treatment that includes a cyclophosphamide, or an anthracycline-based treatment that includes both a cyclophosphamide and a taxane. Accordingly, such predictive methods are useful to facilitate chemotherapy treatment decisions that are tailored to individual patients. For example, the methods disclosed herein can be used to assess whether there is clinical benefit to addition of a taxane to a chemotherapeutic regimen.
Genes for which expression is correlated either positively or negatively with increased likelihood of response to a treatment that includes a taxane, a treatment that includes a cyclophosphamide, or both are provided in Figures 1-4 and Tables 1-4.

The relationships between expression level of a marker gene of the present disclosure and a positive or negative correlation with likelihood of recurrence of cancer (e.g., breast cancer) following treatment with a taxane-containing regimen or a cyclophosphamide-containing regimen are exemplified in Figures 1-4. The hatched line in each graph represents the relationship between expression of the gene in patients treated with a taxane-containing regimen (e.g., anthracycline plus a taxane) and the 5-year recurrence rate (RR) of cancer (the taxane benefit (TB) prediction curve). The TB prediction line thus represents the correlation of expression of the gene and the likelihood of clinical benefit of a taxane in a treatment regimen. The smooth line in each graph represents the relationship between expression of the gene in patients treated with a cyclophosphamide-containing regimen (e.g., anthracycline plus cyclophosphamide) and the 5-year recurrence rate (RR) of cancer (the cyclophosphamide benefit (CB) prediction curve). The CB prediction curve thus represents the correlation of expression of the gene and the likelihood of clinical benefit of a cyclophosphamide in a treatment regimen. Because the patients in the study also received an anthracycline, the TB prediction curve and CB prediction curve can also be considered an anthracycline plus a taxane (AT) benefit prediction curve and an anthracycline plus a cyclophosphamide (AC) benefit prediction curve, respectively.

Each of the graphs in Figures 1-4 include a horizontal dashed line that represents the overall (i.e., not gene expression-specific) recurrence rate at 5-years in the relevant population who were randomized to treatment with AC or AT. The difference between the TB and CB prediction curves and this horizontal line depicts the extent to which clinical benefit may be improved by a gene expression-guided treatment decision.

Other characteristics of the tumour can be taken into account when assessing likelihood of taxane and/or cyclophosphamide benefit by analysis of expression level of a marker gene disclosed herein. For example, hormone receptor expression status (e.g., ER⁺, ER⁻, PR⁺, PR⁻) can be assessed for the tumour sample, and taken into consideration when evaluating expression levels of the marker gene, e.g., the expression level is compared to expression level correlations to TB and/or CB in a population sharing the same characteristics. For example, Figure 1 provides TB (AT) and CB (AC) prediction curves in all patients in the study discussed in the Examples below without regard to hormone expression status or likelihood of cancer recurrence as predicted by the Oncotype DX RS. Figure 2 provides TB (AT) and CB (AC) prediction curves in hormone receptor positive patients. Figure 3 provides TB (AT) and CB (AC) prediction curves in hormone receptor positive patients having an Oncotype DX RS score of about 18 or greater, which indicates a significant risk of cancer recurrence within 10 years following surgery and tamoxifen therapy. Figure 4 provides TB (AT) and CB (AC) prediction curves in hormone receptor negative patients.

The prediction curves can be used to assess information provided by an expression level of a marker gene disclosed herein and in turn facilitate a treatment decision with respect to selection of a taxane-containing and/or a cyclophosphamide-containing regimen. For example, where a gene exhibits an expression level having a TB (AT) prediction curve having a negative slope as exemplified in Figures 1-4, then increasing normalized expression levels of the gene are positively correlated with a likelihood of clinical benefit of including a taxane in the treatment regimen (since patients who exhibited this expression pattern of the particular gene had lower recurrence rates following a taxane-containing regimen). Conversely, where a gene exhibits an expression level having a TB (AT) prediction curve having a positive slope as exemplified in Figures 1-4, then increasing normalized expression levels of the gene are negatively correlated with a likelihood of clinical benefit of including a taxane in the treatment regimen. Similarly, where a gene exhibits an expression level having a CB (AC) prediction curve having a negative slope as exemplified in Figures 1-4, then increasing normalized expression levels of the gene are positively correlated with a likelihood of clinical benefit of including a cyclophosphamide in the treatment regimen (since patients who exhibited this expression pattern of the particular gene had lower recurrence rates following cyclophosphamide-containing regimen). Conversely, where a gene exhibits an expression level having a CB (AC) prediction curve having a positive slope as exemplified in Figures 1-4, then increasing normalized expression levels of the gene are negatively correlated with a likelihood of clinical benefit of including a cyclophosphamide in the treatment regimen.

Accordingly, the expression levels of the marker genes can be used to facilitate a decision as to whether a taxane should be included or excluded in a treatment regimen, and to facilitate a decision as to whether a cyclophosphamide should be included or excluded in a treatment regimen. The marker genes can be used to facilitate selection of a treatment regimen that includes, a taxane and/or a cyclophosphamide, or neither a taxane nor a cyclophosphamide.

In some instances the marker gene expression level may suggest clinical benefit for both a taxane and a cyclophosphamide, e.g., where increasing expression levels are associated with a recurrence risk below a selected recurrence risk. For example, as illustrated in Fig. 2 for the gene ZW10, increased expression of ZW10 in HR-positive cancer patients is associated with increased likelihood of clinical benefit for both a taxane and for a cyclophosphamide. In addition, because the magnitudes of the slopes are significantly different, patients with increased expression of ZW10 are predicted to have lower risks of recurrence if treated with AT instead of AC, and patients with decreased expression of ZW10 are predicted to have lower risks of recurrence if treated with AC instead of AT. Thus, the marker genes that are associated with TB (AT) and CT (AC) prediction curves that differ in slope can facilitate a decision in selecting between a taxane-containing regimen and a cyclophosphamide-containing regimen, even where there may be clinical benefit with either or both treatment regimen.

The methods of the present disclosure also can facilitate selection between a taxane-containing regimen and a cyclophosphamide-containing regimen (e.g., between and AT and AC therapy). For example, where the curves in Figures 1-4 have significantly different slopes in the Cox regression model and the TB (AT) and CB (AC) prediction curves cross, expression levels of the marker gene can be used to assess the likelihood the patient will respond to a taxane-containing regimen (e.g., AT) or to a cyclophosphamide-containing regimen (e.g., AC).

For example, Fig. 5 illustrates a plot of the 5-year risk of relapse versus gene expression, presented for an exemplary gene, DDR1. As illustrated in Fig. 5, the expression level of DDR1 can be used to facilitate selection of therapy where treatment with a cyclophosphamide is favored over treatment with a taxane at lower expression levels of DDR1, with a "switch" of the relative clinical benefit of these therapies occurring at a point where the recurrence risk associated with taxane treatment is lower than that associated with cyclophosphamide treatment, thus favoring a treatment regimen including a taxane over a cyclophosphamide.

There are many types of systemic treatment regimens available for patients diagnosed with cancer. For example, the table below lists various chemotherapeutic and hormonal therapies for breast cancer.

**Single Agents Useful in Breast Cancer**

| GENERIC NAME | COMMON TRADE NAME | CLASS |
|---|---|---|
| Cyclophosphamide (C) | Cytoxan® | Nitrogen mustards |
| Doxorubicin | Adriamycin® | Anthracyclines |
| Epirubicin | Pharmorubicin® | Anthracyclines |
| Fluorouracil | | Pyrimidine analogs |
| Methotrexate | Rheumatrex® | Folic acid analogs |
| Paclitaxel | Taxol® | Taxanes (T) |
| Docetaxel | Taxotere® | Taxanes (T) |
| Capecitabine | Xeloda® | Pyrimidine analogs |
| Trastuzumab | Herceptin® | Monoclonal Antibodies |
| Bevacizumab | Avastin® | Monoclonal Antibodies |

| Combinations Useful in Breast Cancer | | |
|---|---|---|
| CAF | Cyclophosphamide, Adriamycin, Fluorouracil | US |
| CMF | Cyclophosphamide, Methotrexate, Fluorouracil | US |
| AC | Adriamycin, Cyclophosphamide | US |
| AT | Adriamycin, Taxane | US |
| ACT | Adriamycin, Cyclophosphamide, Taxane | US |
| TAC | Taxane, Adriamycin, Cyclophosphamide | US |
| TC | Taxane, Cyclophosphamide | US |
| | Fluorouracil, Epirubicin, Cyclophosphamide | Europe |

### GENE EXPRESSION PROFILING

The practice of the methods and compositions of the present disclosure will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

Methods of gene expression profiling include methods based on hybridization analysis of polynucleotides, methods based on sequencing of polynucleotides, and proteomics- based methods. Exemplary methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription PCT (RT-PCR) (Weis et al., Trends in Genetics 8:263-264 (1992)). Antibodies may be employed that can recognize sequence-specific duplexes, including DNA duplexes, RNA duplexes, and DNA-RNA hybrid duplexes or DNA-protein duplexes. Representative methods for nucleic acid sequencing analysis include Serial Analysis of Gene Expression (SAGE), and Digital Gene Expression (DGE).

Representative methods of gene expression profiling are disclosed, for example, in U.S. Patent Nos. 7,056,674 and 7,081,340, and in U.S. Patent Publication Nos. 20020095585; 20050095634; 20050260646; and 20060008809. Representative scientific publications including methods of gene expression profiling, including data analysis, include Gianni et al., J Clin Oncol. 2005 Oct 10;23(29):7265-77; Paik et al., NEngl JMed. 2004 Dec 30;351(27):2817-26; and Cronin et al., Am JPathol. 2004 Jan;164(1):35-42. The disclosures of these patent and scientific publications are expressly incorporated by reference herein.

### Reverse Transcriptase PCR (RT-PCR)

Typically, mRNA is isolated from a test sample. The starting material is typically total RNA isolated from a human tumour, usually from a primary tumour. Optionally, normal tissues from the same patient can be used as an internal control. mRNA can be extracted from a tissue sample, e.g., from a sample that is fresh, frozen (e.g. fresh frozen), or paraffin-embedded and fixed (e.g. formalin-fixed).

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumour can be isolated, for example, by cesium chloride density gradient centrifugation.

The sample containing the RNA is then subjected to reverse transcription to produce cDNA from the RNA template, followed by exponential amplification in a PCR reaction. The two most commonly used reverse transcriptase enzymes are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

PCR-based methods use a thermostable DNA-dependent DNA polymerase, such as a Taq DNA polymerase. For example, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction product. A third oligonucleotide, or probe, can be designed to facilitate detection of a nucleotide sequence of the amplicon located between the hybridization sites the two PCR primers. The probe can be detectably labeled, e.g., with a reporter dye, and can further be provided with both a fluorescent dye, and a quencher fluorescent dye, as in a Taqman® probe configuration. Where a Taqman® probe is used, during the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data. TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as a threshold cycle ("Cₜ"). Fluorescence values are recorded during every cycle and represent the amount of product amplified to that point in the amplification reaction. The threshold cycle (Cₜ) is generally described as the point when the fluorescent signal is first recorded as statistically significant.

It is desirable to correct for (normalize away) both differences in the amount of RNA assayed and variability in the quality of the RNA used. Therefore, the assay typically measures, and expression analysis of a marker gene incorporates analysis of, the expression of certain reference genes (or "normalizing genes"), including well known housekeeping genes, such as GAPDH. Alternatively, normalization can be based on the mean or median signal (Ct) of all of the assayed genes or a large subset thereof (often referred to as a "global normalization" approach). On a gene-by-gene basis, measured normalized amount of a patient tumour mRNA may be compared to the amount found in a colon cancer tissue reference set. See M. Cronin, et al., Am. Soc. Investigative Pathology 164:35-42 (2004).

Gene expression measurements can be normalized relative to the mean of one or more (e.g., 2, 3, 4, 5, or more) reference genes. Reference-normalized expression measurements can range from 0 to 15, where a one unit increase generally reflects a 2-fold increase in RNA quantity.

RT- PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for use in the methods of the present disclosure use fixed, paraffin-embedded tissues as the RNA source mRNA isolation, purification, primer extension and amplification can be preformed according to methods available in the art. (see, e.g., Godfrey et al. J. Molec. Diagnostics 2: 84-91 (2000); Specht et al., Am. J. Pathol. 158: 419-29 (2001)). Briefly, a representative process starts with cutting about 10 µm thick sections of paraffin-embedded tumour tissue samples. The RNA is then extracted, and protein and DNA depleted from the RNA-containing sample. After analysis of the RNA concentration, RNA is reverse transcribed using gene specific primers followed by RT-PCR to provide for cDNA amplification products.

### Design of Intron-Based PCR Primers and Probes

PCR primers and probes can be designed based upon exon or intron sequences present in the mRNA transcript of the gene of interest. Primer/probe design can be performed using publicly available software, such as the DNA BLAT software developed by Kent, W.J., Genome Res. 12(4):656-64 (2002), or by the BLAST software including its variations.

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz et al. (eds.) Bioinformatics Methods and Protocols: Methods in Molecular Biology. Humana Press, Totowa, NJ, pp 365-386).
Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 °C, e.g. about 50 to 70 °C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

### Quantitative PCR for Gene Expression Analysis

Per VanGuilder et al., BioTechniques 44: 619 (2008), quantitative PCR (qPCR) now represents the method of choice for analyzing gene expression of numerous genes in anywhere from a small number to thousands of samples. For investigators studying gene expression, there is a multitiered technological approach depending on the number of genes and samples being examined. Gene expression microarrays are still the preferred method for large-scale (e.g., whole-genome) discovery experiments. Due to the logistics, sensitivity, and costs of whole-genome micorarrays, there is also a niche for focused microarrays that allow for analysis of a smaller number of genes in a larger number of samples. Nonetheless, for validation of microarray discovery, reverse-transcription quantitative PCR (RT-qPCR) remains the gold standard. The current maturation of real-time qPCR with fluorescent probes allows for rapid and easy confirmation of microarray results in a large number of samples. Often, a whole-genome discovery experiment is not required, as the gene or pathway of interest is already known. In that case, the data collection can begin with qPCR. Finally, qPCR has also shown great utility in biomarker monitoring. In this scenario, previously developed identified targets can be assayed in very large numbers of samples (1000s).

Data Analysis. Analysis of real-time qPCR data has also reached a mature stage of development. Analyses can be either of absolute levels (i.e., numbers of copies of a specific RNA per sample) or relative levels (i.e., sample 1 has twice as much mRNA of a specific gene as sample 2). By far, the majority of analyses use relative quantitation as this is easier to measure and is of primary interest to researchers examining disease states. For absolute quantitation, an RNA standard curve of the gene of interest is required in order to calculate the number of copies. In this case, a serial dilution of a known amount (number of copies) of pure RNA is diluted and subjected to amplification. Like a protein assay, the unknown signal is compared with the curve so as to extrapolate the starting concentration.

The most common method for relative quantitation is the 2^{-ΔΔCT} method. This method relies on two assumptions. The first is that the reaction is occurring with 100% efficiency; in other words, with each cycle of PCR, the amount of product doubles. This can be ascertained through simple experiments as described in the scientific literature. This assumption is also one of the reasons for using a low cycle number when the reaction is still in the exponential phase. In the initial exponential phase of PCR, substrates are not limiting and there is no degradation of products. In practice, this requires setting the crossing threshold or cycle threshold (Cₜ) at the earliest cycle possible. The Cₜ is the number of cycles that it takes each reaction to reach an arbitrary amount of fluorescence. The second assumption of the 2^{-ΔΔCT} method is that there is a gene (or genes) that is expressed at a constant level between the samples. This endogenous control will be used to correct for any difference in sample loading.

Once the Cₜ value is collected for each reaction, it can be used to generate a relative expression level. One 2^{-ΔΔCT} method is now described. In this example, there are two samples (Control and Treated) and we have measured the levels of (i) a gene of interest (Target Gene (TG)) and (ii) an endogenous control gene (Control Gene (CG)). For each sample, the difference in Cₜ values for the gene of interest and the endogenous control is calculated (the ΔCₜ). Next, subtraction of the control-condition Δ Cₜ from the treated-condition ΔCₜ yields the ΔΔCₜ. The negative value of this subtraction, the -ΔΔCₜ, is used as the exponent of 2 in the equation and represents the difference in "corrected" number of cycles to threshold. The exponent conversion comes from the fact that the reaction doubles the amount of product per cycle. For example, if the control sample ΔCₜ is 2 and the treated sample ΔCₜ is 4, computing the 2^{-ΔΔCT} (which becomes 2 ⁻⁽⁴⁻²⁾) yields 0.25. This value is often referred to as the RQ, or relative quantity value. This means that the level of the gene of interest in the treated sample is only 25% of the level of that gene in the control sample. This becomes evident because the treated sample took two more cycles of PCR to reach the same amount of product as the control sample and therefore there was less of that cDNA to begin with in the treated sample. The 2^{-ΔΔCT} method is the most common quantitation strategy, but it should be noted that there are other valid methods for analyzing qPCR Cₜ values. Several investigators have proposed alternative analysis methods.

### MassARRAY® System

In MassARRAY-based methods, such as the exemplary method developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix- assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### Other PCR-based Methods

Further PCR-based techniques that can find use in the methods disclosed herein include, for example, BeadArray® technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression® (BADGE), using the commercially available Luminex 100 LabMAP® system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003).

### Microarrays

Expression levels of a gene of interest can also be assessed using the microarray technique. In this method, polynucleotide sequences of interest (including cDNAs and oligonucleotides) are arrayed on a substrate. The arrayed sequences are then contacted under conditions suitable for specific hybridization with detectably labeled cDNA generated from mRNA of a test sample. As in the RT-PCR method, the source of mRNA typically is total RNA isolated from a tumour sample, and optionally from normal tissue of the same patient as an internal control or cell lines. mRNA can be extracted, for example, from frozen or archived paraffin-embedded and fixed (e.g. formalin-fixed) tissue samples.

For example, PCR amplified inserts of cDNA clones of a gene to be assayed are applied to a substrate in a dense array. Usually at least 10,000 nucleotide sequences are applied to the substrate. For example, the microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After washing under stringent conditions to remove non-specifically bound probes, the chip is scanned by confocal laser microscopy or by another detection method, such as a CCD camera. Quantitation of hybridization of each arrayed element allows for assessment of corresponding mRNA abundance.

With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et at, Proc. Natl. Acad. Sci. USA 93(2):106-149 (1996)). Microarray analysis can be performed by commercially available equipment, following manufacturer's protocols, such as by using the Affymetrix GenChip® technology.

### Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### Isolating RNA from Body Fluids

Methods of isolating RNA for expression analysis from tissue (e.g., breast tissue), blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### Immunonological methods

Immunological methods (e.g., immunohistochemistry methods)are also suitable for detecting the expression levels of genes and applied to the method disclosed herein. Antibodies (e.g., monoclonal antibodies) that specifically bind a gene product of a gene of interest can be used in such methods. The antibodies can be detected by direct labeling of the antibodies themselves, for example, with radioactive labels, fluorescent labels, haptene labels such as, biotin, or an enzyme such as horse radish peroxidase or alkaline phosphatase. Alternatively, unlabeled primary antibody can be used in conjunction with a labeled secondary antibody specific for the primary antibody. Immunological methods protocols and kits are well known in the art and are commercially available.

### Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N- terminal sequencing, and (3) analysis of the data using bioinformatics.

### GENERAL DESCRIPTION OF EXEMPLARY PROTOCOL

The steps of a representative protocol for profiling gene expression using fixed, paraffin-embedded tissues as the RNA source, including mRNA isolation, purification, primer extension and amplification are provided in various published journal articles. (See, e.g., T.E. Godfrey et al,. J. Molec. Diagnostics 2: 84-91 (2000); K. Specht et al., Am. J. Pathol. 158: 419-29 (2001), M. Cronin, et al., Am J Pathol 164:35-42 (2004)). Briefly, a representative process starts with cutting a tissue sample section (e.g. about 10 µm thick sections of a paraffin-embedded tumour tissue sample). The RNA is then extracted, and protein and DNA are removed. After analysis of the RNA concentration, RNA repair is performed if desired. The sample can then be subjected to analysis, e.g., by reverse transcribed using gene specific promoters followed by RT-PCR.

### KITS

The materials for use in the methods of the present disclosure are suited for preparation of kits produced in accordance with well known procedures. The present disclosure thus provides kits comprising agents, which may include gene-specific or gene-selective probes and/or primers, for quantitating the expression of the disclosed genes for predicting clinical outcome or response to treatment. Such kits may optionally contain reagents for the extraction of RNA from tumour samples, in particular fixed paraffin-embedded tissue samples and/or reagents for RNA amplification. In addition, the kits may optionally comprise the reagent(s) with an identifying description or label or instructions relating to their use in the methods of the present disclosure. The kits may comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present disclosure (e.g., appropriate length poly(T) or random primers linked to a promoter reactive with the RNA polymerase). Mathematical algorithms used to estimate or quantify prognostic and/or predictive information are also properly potential components of kits.

The methods provided by the present disclosure may also be automated in whole or in part.

### REPORTS

The methods of the present disclosure are suited for the preparation of reports summarizing the predictions resulting from the methods of the present disclosure. A "report," as described herein, is an electronic or tangible document which includes report elements that provide information of interest relating to a likelihood assessment and its results. A subject report includes at least a likelihood assessment, e.g., an indication as to the likelihood that a cancer patient will exhibit a beneficial clinical response to a treatment regimen of interest. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patients tumour tissue. The report may include a prediction that said subject has an increased likelihood of response to treatment with a particular chemotherapy or the report may include a prediction that the subject has a decreased likelihood of response to the chemotherapy. The report may include a recommendation for treatment modality such as surgery alone or surgery in combination with chemotherapy. The report may be presented in electronic format or on paper.

Thus, in some embodiments, the methods of the present disclosure further includes generating a report that includes information regarding the patient's likelihood of response to chemotherapy, particularly a therapy including cyclophophamide and/or a taxane. For example, the methods disclosed herein can further include a step of generating or outputting a report providing the results of a subject response likelihood assessment, which report can be provided in the form of an electronic medium (e.g., an electronic display on a computer monitor), or in the form of a tangible medium (e.g., a report printed on paper or other tangible medium).

A report that includes information regarding the likelihood that a patient will respond to treatment with chemotherapy, particularly a including cyclophophamide and/or a taxane, is provided to a user. An assessment as to the likelihood that a cancer patient will respond to treatment with chemotherapy, or predicted comparative response to two therapy options, is referred to below as a "response likelihood assessment" or, simply, "likelihood assessment." A person or entity who prepares a report ("report generator") will also perform the likelihood assessment. The report generator may also perform one or more of sample gathering, sample processing, and data generation, e.g., the report generator may also perform one or more of: a) sample gathering; b) sample processing; c) measuring a level of an indicator response gene product(s); d) measuring a level of a reference gene product(s); and e) determining a normalized level of a response indicator gene product(s). Alternatively, an entity other than the report generator can perform one or more sample gathering, sample processing, and data generation.

For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a response indicator gene product(s); level of a reference gene product(s); normalized level of a response indicator gene product(s)) for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the invention, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### COMPUTER-BASED SYSTEMS AND METHODS

The methods and systems described herein can be implemented in numerous ways. In one embodiment of particular interest, the methods involve use of a communications infrastructure, for example the internet. Several embodiments of the invention are discussed below. It is also to be understood that the present invention may be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "score," where the score is transmitted as processed or partially processed responses to the reviewer's computing environment in the form of test code for subsequent execution of one or more algorithms to provide a results and/or generate a report in the reviewer's computing environment. The score can be a numerical score (representative of a numerical value) or a non-numerical score representative of a numerical value or range of numerical values (e.g., "A' representative of a 90-95% likelihood of an outcome; "high" representative of a greater than 50% chance of response (or some other selected threshold of likelihood); "low" representative of a less than 50% chance of response (or some other selected threshold of likelihood); and the like.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a response indicator gene product(s); level of a reference gene product(s); normalized level of a response indicator gene product(s)); and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also referred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different servers.

Other computing arrangements for the client and server(s), including processing on a single machine such as a mainframe, a collection of machines, or other suitable configuration are contemplated. In general, the client and server machines work together to accomplish the processing of the present invention.

Where used, the database(s) is usually connected to the database server component and can be any device which will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CD-ROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program which provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the invention can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present disclosure may also be practiced such that a limited number of additional genes that are co-expressed with the disclosed genes, for example as evidenced by high Pearson correlation coefficients, are included in a prognostic and/or predictive test in addition to and/or in place of disclosed genes.

Having described exemplary embodiments of the invention, the same will be more readily understood through reference to the following Examples, which are provided by way of illustration, and are not intended to limit the invention in any way. All citations throughout the disclosure are hereby expressly incorporated by reference.

### EXAMPLES

The following examples are offered by way of illustration and not by way of limitation. The disclosures of all citations in the specification are expressly incorporated herein by reference.

### EXAMPLE 1: IDENTIFICATION OF DIFFERENTIAL MARKERS OF RESPONSE IN BREAST CANCER PATIENTS

The data from intergroup trial E2197 (Goldstein L, O'Neill A, Sparano J, et al. E2197: phase III AT (doxorubicin/docetaxel) vs. AC (doxorubicin/cyclophosphamide) in the adjuvant treatment of node positive and high risk node negative breast cancer. Proc Am Soc Clin Oncol. 2005; 23:7s. [Abstract 512]) was used to evaluate the relative efficacy of adjuvant treatment of breast cancer patients with an anthracycline (doxorubicin) + a taxane (AT) compared to an anthracycline (doxorubicin) + cyclophosphamide(AC). The trial compared 4 cycles of a standard doxorubicin-cyclophosphamide (AC) combination given every 3 weeks with 4 cycles of doxorubicin plus docetaxel (AT) in patients with 0-3 positive lymph nodes. The trial was powered to detect a 25% reduction in the disease-free survival (DFS) hazard rate (from an anticipated 5-year DFS of 78% for the AC arm to 83% for the AT arm). Tamoxifen (20 mg daily for 5 years) was recommended for hormone receptor-positive patients following completion of chemotherapy, although approximately 40% of patients eventually took an aromatase inhibitor at some point before or after 5 years. The treatment arms were well balanced with regard to median age (51 years), proportion of lymph node-negative disease (65%), and estrogen receptor (ER)-positive disease (64%).

When single genes by treatment (taxane (T) vs cyclophosphamide (C); or AT vs AC) interactions were evaluated, large numbers of genes with significant interaction effects were observed, in all subjects analyzed; in hormone receptor (HR) positive subjects; in HR positive, Oncotype DX Recurrence Score@ value > about 18 subjects; and in HR negative subjects. Most of these interactions are in the same "direction", i.e., higher expression is associated with greater T benefit and/or less C benefit. Where Oncotype DX Recurrence Score® (RS) was used, the RS was calculated according to the algorithm described in Paik et al., N Engl J Med. 2004 Dec 30;351(27):2817-26 and in U.S. application publication No. 20050048542, published March 3, 2005, the entire disclosures of which are expressly incorporated by reference herein.
The predictive utility of PR protein expression was evaluated by immunohistochemistry in a central lab and quantitative RNA expression by RT-PCR for 371 genes (including the 21-gene Recurrence Score [RS]) in a representative sample of 734 patients who received at least 3-4 treatment cycles.

### Methods

*Patient Selection:* All recurrences with available tissue and randomly selected patients without recurrence were identified by an ECOG statistician (ratio 3.5 without recurrence to 1 with recurrence).
*Central Immunohistochemistry (IHC) for ER and PR:* IHC was performed on two 1.0-mm tissue microarrays (TMAs), using 4 µm sections, DakoCytomation EnVision+ System®
(Dako Corporation, Carpinteria, CA), and standard methodology using anti-ER antibody (clone 1D5, dilution 1:100) and anti-PR antibody 636 (1:200).
TMAs were reviewed centrally and scored by two pathologists who were blinded to outcomes and local laboratory ER/PR status.

Scoring was performed using the Allred method (see, e. g. Harvey JM, Clark GM, Osborne CK et al. J Clin Oncol 1999;17:1474-1481) scoring the proportion of positive cells (scored on a 0-5 scale) and staining intensity (scored on a 0-3 scale); proportion and intensity scores were added to yield Allred Score of 0 or 2 through 8 with Allred scores > 2 considered positive.

*Genes and RT-PCR analysis:* Candidate genes were selected to represent multiple biological processes. Quantitative RT-PCR analysis was performed by methods known in the art. For each gene, the appropriate mRNA reference sequence (REFSEQ) accession number was identified and the consensus sequence was accessed through the NCBI Entrez nucleotide database. Appendix 1. Besides the REFSEQ, RT-PCR probe and primer sequences are provided in Appendix 1. Sequences for the amplicons that result from the use of these primer sets are listed in Appendix 2.

*Statistical methods:* Single Gene by Treatment Interaction Analysis. The objective of this evaluation was to identify genes whose expression, treated as a continuous variable, is differentially associated with the risk of relapse between patients treated with AC versus those treated with AT. A gene expression by treatment interaction model was employed for this purpose and statistical analyses were performed by using Cox Regression models (SAS version 9.1.3). The Cox regression model that was employed for these analyses includes terms for the main effect of treatment, the main effect of gene expression, and the interaction of treatment and gene expression. This model enables prediction of the association between gene expression and the risk of recurrence for patients treated with AC, and of the association between gene expression and the risk of recurrence for patients treated with AT. The point at which these two curves cross is the level of gene expression at which the predicted risk of recurrence is identical if the patient is treated with AC or with AT. This crossover point is easily calculated from the parameter estimates from this model as the negative of the estimated treatment effect, divided by the estimate of the interaction effect.

All hypothesis tests were reported using two-sided p-values, and p-values of < 0.05 was considered statistically significant. Relapse-Free Interval was defined as the time from study entry to the first evidence of breast cancer relapse, defined as invasive breast cancer in local, regional or distant sites, including the ipsilateral breast, but excluding new primary breast cancers in the opposite breast. Follow-up for relapse was censored at the time of death without relapse, new primary cancer in the opposite breast, or at the time of the patient was last evaluated for relapse.

The variance of the partial likelihood estimators was estimated with a weighted estimate. See R. Gray, Lifetime Data Anal. 15(1):24-40 (2009); K. Chen K, S-H Lo, Biometrika 86:755-764 (1999).

Individual genes by treatment interactions were tested in Cox models for relapse-free interval (RFI) for the HR+ and HR- patients combined and separately. Since there is little chemotherapy benefit for RS<18, the HR+, RS>18 subset was also analyzed. The interaction between gene expression and treatment for genes could be depicted graphically. As example we present treatment group-specific plots of the 5-year risk of relapse versus DDR1 gene expression.

Supervised principal components (SPC) was used to combine genes into a multigene predictor of differential treatment benefit, and was evaluated via cross-validation (CV). Pre-validation (PV) inference (Tibshirani and Efron, Stat Appl Genet and Mol Biol 2002;1:Articlel. Epub 2002 Aug 22), based on 20 replicates of 5 fold cross-validation, was used to estimate and test (via permutations) the utility of the SPC predictors.

### Results

Tables 1-4 include an Estimated Coefficient for each response indicator gene listed in the tables in all subjects analyzed (Table 1); in HR+ subjects (Table 2); in HR⁺ subjects having an Oncotype DX Recurrence Score® value greater than about 18 (Table 3); and in HR negative subjects (Table 4). Figures 1-4 represent graphically the results for each gene summarized in Tables 1-4, respectively. Each graph of Figures 1-4 shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). Each of the graphs in Figures 1-4 are presented with 5-year risk of recurrence on the y-axis and normalized expression (Cₜ) on the x-axis, where increasing normalized Cₜ values indicate increasing expression levels.

The Estimated Coefficient referred to in Tables 1-4 is a reflection of the difference between the slopes in the Cox regression model of the AC prediction curve and the AT prediction curve. The magnitude of the Estimated Coefficient is related to the difference between the slopes of the AC prediction curve and the AT prediction curve; the sign of the Estimated Coefficient is an indication of which treatment (AT or AC) becomes the favored treatment as expression of the gene increases. For example, in Table 1, the Estimated Coefficient for SLC1A3 is -0.7577. The magnitude (absolute value = 0.7577) is related to the difference between the slopes of the AC prediction curve and the AT prediction curve (shown in the first panel of Figure 1) for SLC1A3 in this population (all patients, i.e. not stratified by hormone receptor status or by RS). The negative sign indicates that higher expression levels of SLC1A3 favor treatment with AT while lower expression levels of SLC1A3 favor treatment with AC.

The p-value given in Table 1 is a measure of the statistical significance of the difference between the slope of the AC prediction curve and the slope of the AT prediction curve in the Cox regression model, i.e. the probability that the observed difference in slopes is due to chance. Smaller p-values indicate greater statistical significance.

### Analysis of gene expression in all patients in study population (irrespective of HR status and Oncotype Dx® RS score)

Table 1 shows a list of 76 genes whose normalized expression level is differentially associated with response to AT vs. AC treatment in all patients. When the estimated coefficient is <0, high expression of that gene is indicative that AT treatment is more effective than AC treatment; low gene expression of that gene is indicative that AC treatment is more effective than AT treatment. When the estimated coefficient is >0, high expression of that gene is indicative that AC treatment is more effective than AT treatment; low expression of that gene is indicative that AT treatment is more effective than AC treatment.

As noted above, Figure 1 shows a graph for each gene in Table 1. Each graph shows a smooth line representing the model-predicted relationship between expression of the gene and 5-year recurrence rate (RR) in an AC treatment group (the AC prediction curve) and a hatched line representing the model-predicted relationship between gene expression and RR in an AT treatment group (the AT prediction curve). For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line, represents the 12.3% recurrence rate at 5-year RR in all patients analyzed (i.e., without regard to HR status or Oncotype Dx RS).

The first panel of Figure 1, for example, shows the AC-prediction curve and the AT prediction curve for SLC1A3. The curves have significantly different slopes in the Cox regression model and the lines cross, resulting in the ability to discriminate, based on the expression level of SLC1A3, patients who are more likely to respond to AT (or to AC). For SLC1A3, patients with higher expression levels are more likely to respond to AT than AC, while patients with lower expression levels are more likely to respond to AC than AT.

### Analysis of gene expression in HR+ patients in study population

Table 2 shows a list of 97 genes having a normalized expression level that is differently correlated with response to AT vs. AC in hormone receptor (HR)-positive patients (without regard to Oncotype Dx RS value). When the estimated coefficient is <0, high expression of that gene is indicative that AT treatment is more effective than AC treatment; low expression of that gene is indicative that AC treatment is more effective than AT treatment. When the estimated coefficient is >0, high expression of that gene is indicative than AC treatment is more effective than AT treatment; low expression of that gene is indicative that AT treatment is more effective than AC treatment.

The data summarized in Table 2 are provided in graph form for each gene in Figure 2. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 10.0% recurrence rate at 5-year RR in HR-positive patients.

### Analysis of gene expression in HR+ patients in the study population having an Oncotype Dx RS of about 18 or greater

Table 3 shows a list of 165 genes whose normalized expression level is differentially associated with response to AT vs. AC in HR-positive patients having a Recurrence Score (RS) > 18. These patients have an increased likelihood of cancer recurrence. When the estimated coefficient is <0, high expression of that gene is indicative that AT treatment is more effective than AC treatment; low expression of that gene is indicative that AC treatment is more effective than AT treatment. When the estimated coefficient is > 0, high expression of that gene is indicative that AC treatment is more effective than AT treatment; low expression of that gene is indicative that AT treatment is more effective than AC treatment.

The data summarized in Table 3 are provided in graph form for each gene in Figure 3. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 14.9% recurrence rate at 5-year RR in the HR-positive patient group having an Oncotype Dx RS of about 18 or greater.

### Analysis of gene expression in HR- patients in study population

Table 4 shows a list of 9 genes whose normalized expression level is differentially associated with response to AT vs. AC treatment in HR-negative patients. The data summarized in Table 4 is provided in graph form for each gene in Figure 4. For each gene, the AC prediction curve and the AT prediction curve have statistically significant different slopes in the Cox regression model, indicating that AC or AT can be chosen as a favored treatment based, at least in part, on the expression of the gene. The graph for each gene also shows, as a horizontal dashed line represents the 16.9% recurrence rate at 5-year RR in the HR-negative patient group.

### Discussion

PR Analysis. There was a weak benefit for AT in PR-negative (AT vs AC hazard ratio [RR]=0.75; p=0.06) and AC in PR-positive disease (RR=1.37; p=0.05) by central immunhistochemistry (Allred score > 2 positive) but not when genomic PR was evaluated by RT-PCR (>5.5 units positive).

RS and Genes Analyzed. Table 1 illustrates genes that can be used as markers of benefit of taxane therapy irrespective of hormone receptor expression status, and facilitate selection of AC vs AT therapy. (Table 1). Several genes strongly predicted taxane benefit when assessed in the context of AT vs AC therapy in the HR-positive subset (Table 2), and especially in the HR-positive, Oncotype Dx RS > 18 subset (Table 3).

Nine genes were identified for which gene expression can be used as markers of benefit of taxane therapy in hormone receptor (HR)-negative breast cancer, and could be used to assess AT vs. AC benefit in the hormone receptor (HR)-negative patients (Table 4).

Of the genes listed in Table 1, SLC1A3 (glial high affinity glutamase transporter 3) is a member of a large family of solute transport proteins, located within the multiple sclerosis locus on 5p.

Of the genes identified in the HR-positive subset (Table 2), DDR1 (discoidin domain receptor 1) is a transmembrane receptor TK the aberrant expression and signaling of which has been linked to accelerated matrix degradation and remodeling, including tumour invasion. Collagen-induced DDR1 activation is believed to be involved in normal mammary cell adhesion, and may distinguish between invasive ductal carcinoma (IDC) and invasive lobular carcinoma (ILC), and further may induce cyclooxygenase-2 and promoter chemoresistance through the NF-κB pathway. EIF4E2 (human transcription initiation factor 4) is an mRNA cap-binding protein.

When differential response markers in HR-positive, RS > 18 patients (Table 3) are ranked in ascending order by p-value, DDR1, RELA, ZW10, and RhoB are four of the top five genes. RELA is an NF-κB subunit, which plays a role in inflammation, innate immunity, cancer and anti-apoptosis. This gene has also been associated with chemoresistance, and may be necessary for IL-6 induction, which is involved in immune cell homeostasis. ZW10 is a kinetochore protein involved in mitotic spindle formation. It is part of the ROD-ZW10-Zwilch complex, and binds tubulin. RhoB is a low molecular weight GPTase belonging to the RAS superfamily. The Rho protein is pivotal in regulation of actin cytoskeleton. RhoB acts as tumour suppressor gene and inhibits tumour growth and metastases in vitro and in vivo, and activates NF-κB. KO mice for RhoB show increased sensitivity to chemical carcinogenesis and resistance to radiation and cytotoxic induced apoptosis.

DDR1, RELA and RhoB are key elements in the NFκB signaling pathway. Based on these findings, it is expected that other genes in the NFκB pathway are likely to be differentially associated with response to AT vs. AC treatment in HR-positive patients at high risk for cancer recurrence, and such can be used as differential response markers for AT vs. AC treatment. Some additional genes that are known to be involved in NFκB signaling are shown in Table 5.

In the HR-negative subset, CD247 exhibited a correlation of expression with AT vs. AC therapy (p-value < 0.01) and exhibited a strong correlation indicating that expression was positively correlated with increased likelihood of benefit of treatment including a taxane (Fig. 4). The estimated coefficient <0 indicates that high gene expression favors AT treatment, while low gene expression favors AC treatment (see also Fig. 4). CD247, also known as T cell receptor zeta (TCRzeta) functions as an amplification module of the TCR signaling cascade. This gene is downregulated in many chronic infectious and inflammatory processes, such as systemic lupus erythematosus (SLE).

Fig. 5 illustrates an exemplary treatment group-specific plot of the 5-year risk of relapse versus gene expression presented for an exemplary gene, DDR1.

### EXAMPLE 2:ESR1 GENE COMBINATIONS

Using the differential response markers identified in Table 2, supervised principle component analysis was carried out in HR+ RS>18 patients treated with AT vs AC according the methods of Bair E, Hastie T, Paul D, Tibshirani R. Prediction by supervised principal components. J. Amer. Stat. Assoc. 101:119-137, 2006.

Principal Components can be used in regression problems for dimensionality reduction in a data set by keeping the most important principal components and ignoring the other ones. Supervised principal components (Bair et al. *supra*) is similar to conventional principal components analysis except that it uses a subset of the predictors (i.e. individual genes) that are selected based on their association with relapse-free interval (assessed using Cox regression). In the present example, only the first component was utilized to obtain a score from a weighted combination of genes.

In this patient group, the most heavily weighted gene by supervised principle components analysis was ESR1, indicating that ESR1 is particularly useful when used in combinations with any of the other genes listed in Table 3 in predicting differential response to taxane vs. cyclophosphamide in HR+ high recurrence risk patients. Exemplary combinations of genes include, without limitation:
DDR1 + ESR1, ZW10 + ESR1, RELA + ESR1, BAX + ESR1, RHOB + ESR1,
TSPAN4 + ESR1, BBC3 + ESR1, SHC1 + ESR1, CAPZA1 + ESR1, STK10 + ESR1,
TBCC + ESR1, EIF4E2 + ESR1, MCL1 + ESR1, RASSF1 + ESR1, VEGF + ESR1,
SLC1A3 + ESR1, DICER1 + ESR1, ILK + ESR1, FAS + ESR1, RAB6C + ESR1,
ESR1 + ESR1, MRE11A + ESR1, APOE + ESR1, BAK1 +ESR1, UFM1 + ESR1,
AKT2 + ESR1, SIRT1 + ESR1, BCL2L13 + ESR1, ACTR2 + ESR1, LIMK2 + ESR1,
HDAC6 + ESR1, RPN2 + ESR1, PLD3 + ESR1, CHGA + ESR1, RHOA + ESR1,
MAPK14 + ESR1, ECGF1 + ESR1, MAPRE1 + ESR1, HSPA1B + ESR1, GATA3 + ESR1, PPP2CA + ESR1, ABCD1 + ESR1, MAD2L1BP + ESR1, VHL + ESR1,
GCLC + ESR1, ACTB + ESR1, BCL2L11 + ESR1, PRDX1 + ESR1, LILRB1 + ESR1, GNS + ESR1, CHFR + ESR1, CD68 + ESR1, LIMK1 + ESR1, GADD45B + ESR1, VEGFB + ESR1, APRT + ESR1, MAP2K3 + ESR1, MGC52057 + ESR1,
MAPK3 + ESR1, APC + ESR1, RAD1 + ESR1, COL6A3 + ESR1, RXRB + ESR1,
CCT3 + ESR1, ABCC3 + ESR1, GPX1 + ESR1, TUBB2C + ESR1, HSPA1A + ESR1,
AKT1 + ESR1, TUBA6 + ESR1, TOP3B + ESR1, CSNK1D + ESR1, SOD1 + ESR1,
BUB3 + ESR1, MAP4 + ESR1, NFKB1 + ESR1, SEC61A1 + ESR1, MAD1L1 + ESR1, PRKCH + ESR1, RXRA + ESR1, PLAU + ESR1, CD63 + ESR1, CD14 + ESR1, RHOC + ESR1, STAT1 + ESR1, NPC2 + ESR1, NME6 + ESR1, PDGFRB + ESR1, MGMT + ESR1, GBP1 + ESR1, ERCC1 + ESR1, RCC1 + ESR1, FUS + ESR1,
TUBA3 + ESR1, CHEK2 + ESR1, APOC1 + ESR1, ABCC10 + ESR1, SRC + ESR1,
TUBB + ESR1, FLAD1 + ESR1, MAD2L2 + ESR1, LAPTM4B + ESR1, REG1A + ESR1, PRKCD + ESR1, CST7 + ESR1, IGFBP2 + ESR1, FYN + ESR1, KDR + ESR1, STMN1 + ESR1, ZWILCH + ESR1, RBM17 + ESR1, TP53BP1 + ESR1,
CD247 + ESR1, ABCA9 + ESR1, NTSR2 + ESR1, FOS + ESR1, TNFRSF10A + ESR1, MSH3 + ESR1, PTEN + ESR1, GBP2 + ESR1, STK11 + ESR1, ERBB4 + ESR1, TFF1 + ESR1, ABCC1 + ESR1, IL7 + ESR1, CDC25B + ESR1, TUBD1 + ESR1, BIRC4 + ESR1, ACTR3 + ESR1, SLC35B1 + ESR1, COL1A1 + ESR1,
FOXA1 + ESR1, DUSP1 + ESR1, CXCR4 + ESR1, IL2RA + ESR1, GGPS1 + ESR1,
KNS2 + ESR1, RB1 + ESR1, BCL2L1 + ESR1, XIST + ESR1, BIRC3 + ESR1, BID + ESR1, BCL2 + ESR1, STAT3 + ESR1, PECAM1 + ESR1, DIABLO + ESR1, CYBA + ESR1, TBCE + ESR1, CYP1B1 + ESR1, APEX1 + ESR1, TBCD + ESR1, HRAS + ESR1, TNFRSF10B + ESR1, ELP3 + ESR1, PIK3C2A + ESR1, HSPA5 + ESR1,
VEGFC + ESR1, CRABP1 + ESR1, MMP11 + ESR1, SGK + ESR1, CTSD + ESR1,
BAD + ESR1, PTPN21 + ESR1, HSPA9B + ESR1, and PMS1 + ESR1

Any combination of two or more genes from Table 3, said combination not comprising ESR1 is also expected to be useful in predicting differential response to taxane vs. cyclophosphamide in HR+ high recurrence risk patients.

Similarly it is expected that ESR1 is particularly useful when used in combinations with any of the other genes listed in Table 2 in predicting differential response to taxane vs. cyclophosphamide in HR+ patients. Exemplary combinations of genes include:
DDR1 + ESR1, EIF4E2 + ESR1, TBCC + ESR1, STK10 + ESR1, ZW10 + ESR1,
BBC3 + ESR1, BAX + ESR1, BAK1 + ESR1, TSPAN4 + ESR1, SLC1A3 + ESR1,
SHC1 + ESR1, CHFR + ESR1, RHOB + ESR1, TUBA6 + ESR1, BCL2L13 + ESR1,
MAPRE1 + ESR1, GADD45B + ESR1, HSPA1B + ESR1, FAS + ESR1, TUBB + ESR1, HSPA1A + ESR1, MCL1 + ESR1, CCT3 + ESR1, VEGF + ESR1, TUBB2C + ESR1, AKT1 + ESR1, MAD2L1BP + ESR1, RPN2 + ESR1, RHOA + ESR1,
MAP2K3 + ESR1, BID + ESR1, APOE + ESR1, ESR1 + ESR1, ILK + ESR1, NTSR2 + ESR1, TOP3B + ESR1, PLD3 + ESR1, DICER1 + ESR1, VHL + ESR1, GCLC + ESR1, RAD1 + ESR1, GATA3 + ESR1, CXCR4 + ESR1, NME6 + ESR1, UFM1 + ESR1, BUB3 + ESR1, CD14 + ESR1, MRE11A + ESR1, CST7 + ESR1, APOC1 + ESR1, GNS + ESR1, ABCC5 + ESR1, AKT2 + ESR1, APRT + ESR1, PLAU + ESR1,
RCC1 + ESR1, CAPZA1 + ESR1, RELA + ESR1, NFKB1 + ESR1, RASSF1 + ESR1,
BCL2L11 + ESR1, CSNK1D + ESR1, SRC + ESR1, LIMK2 + ESR1, SIRT1 + ESR1,
RXRA + ESR1, ABCD1 + ESR1, MAPK3 + ESR1, CDCA8 + ESR1, DUSP1 + ESR1,
ABCC1 + ESR1, PRKCH + ESR1, PRDX1 + ESR1, TUBA3 + ESR1, VEGFB + ESR1, LILRB1 + ESR1, LAPTM4B + ESR1, HSPA9B + ESR1, ECGF1 + ESR1,
GDF15 + ESR1, ACTR2 + ESR1, IL7 + ESR1, HDAC6 + ESR1, ZWILCH + ESR1,
CHEK2 + ESR1, REG1A + ESR1, APC + ESR1, SLC35B1 + ESR1, NEK2 + ESR1,
ACTB + ESR1, BUB1 + ESR1, PPP2CA + ESR1, TNFRSF10A + ESR1, TBCD + ESR1, ERBB4 + ESR1, CDC25B + ESR1, and STMN1+ ESR1.

A combination of two or more genes from Table 2, said combination not comprising ESR1 is also expected to be useful in predicting differential response to taxane vs. cyclophosphamide in HR+ patients at high recurrence risk for cancer.

### EXAMPLE 3:GENES OF THE NFκB PATHWAY

When the differential response markers in HR-positive, RS > 18 patients are ranked in ascending order of p-value, three of the top five revealed genes are DDR1, RELA and RHOB. The RELA gene encodes one of the principle subunits of the NFκB transcription factor. Therefore, it is notable that both the DDR1 gene and the RHOB gene stimulate the NFκB signaling pathway. These results indicate that additional genes that stimulate the activity of the NFκB pathway, given in Table 5, also predict increased likelihood of response to AT vs. AC chemotherapy.

### EXAMPLE 4: GENE EXPRESSION PROFILING PROTOCOL

Breast tumour formalin-fixed and paraffin-embedded (FPE) blocks or frozen tumour sections are provided. Fixed tissues are incubated for 5 to 10 hours in 10% neutral-buffered formalin before being alcohol-dehydrated and embedded in paraffin.

RNA is extracted from three 10-µm FPE sections per each patient case. Paraffin is removed by xylene extraction followed by ethanol wash. RNA is isolated from sectioned tissue blocks using the MasterPure Purification kit (Epicenter, Madison, WI); a DNase I treatment step is included. RNA is extracted from frozen samples using Trizol reagent according to the supplier's instructions (Invitrogen Life Technologies, Carlsbad, CA). Residual genomic DNA contamination is assayed by a TaqMan® (Applied Biosystems, Foster City, CA) quantitative PCR assay (no RT control) for ß-actin DNA. Samples with measurable residual genomic DNA are resubjected to DNase I treatment, and assayed again for DNA contamination. TaqMan is a registered trademark of Roche Molecular Systems.

RNA is quantitated using the RiboGreen® fluorescence method (Molecular Probes, Eugene, OR), and RNA size is analyzed by microcapillary electrophoresis using an Agilent 2100 Bioanalyzer (Agilent Technologies, Palo Alto, CA).

Reverse transcription (RT) is performed using a SuperScript® First-Strand Synthesis kit for RT-PCR (Invitrogen Corp., Carlsbad, CA). Total FPE RNA and pooled gene-specific primers are present at 10 to 50 ng/µl and 100 nmol/L (each), respectively.

TaqMan reactions are performed in 384-well plates according to instructions of the manufacturer, using Applied Biosystems Prism 7900HT TaqMan instruments. Expression of each gene is measured either in duplicate 5-µl reactions using cDNA synthesized from 1 ng of total RNA per reaction well, or in single reactions using cDNA synthesized from 2 ng of total RNA. Final primer and probe concentrations are 0.9 µmol/L (each primer) and 0.2 µmol/L, respectively. PCR cycling is performed as follows: 95°C for 10 minutes for one cycle, 95°C for 20 seconds, and 60°C for 45 seconds for 40 cycles. To verify that the RT-PCR signals derives from RNA rather than genomic DNA, for each gene tested a control identical to the test assay but omitting the RT reaction (no RT control) is included. The threshold cycle for a given amplification curve during RT-PCR occurs at the point the fluorescent signal from probe cleavage grows beyond a specified fluorescence threshold setting. Test samples with greater initial template exceed the threshold value at earlier amplification cycle numbers than those with lower initial template quantities.

For normalization of extraneous effects, cycle threshold (C_{T}) measurements obtained by RT-PCR were normalized relative to the mean expression of a set of five reference genes: ATP5E, PGK1, UBB, VDAC2, and GPX1. A one unit increase in reference normalized expression measurements generally reflects a 2-fold increase in RNA quantity.

While the present invention has been described with reference to what are considered to be the specific embodiments, it is to be understood that the invention is not limited to such embodiments. To the contrary, the invention is intended to cover various modifications and equivalents included within the spirit and scope of the appended claims.

The disclosures of EP 13159677.7, from which the present application is divided, of EP 12195318.6 (the grandparent application) and of EP 09747388.8 (the great-grandparent application) are incorporated herein by reference.

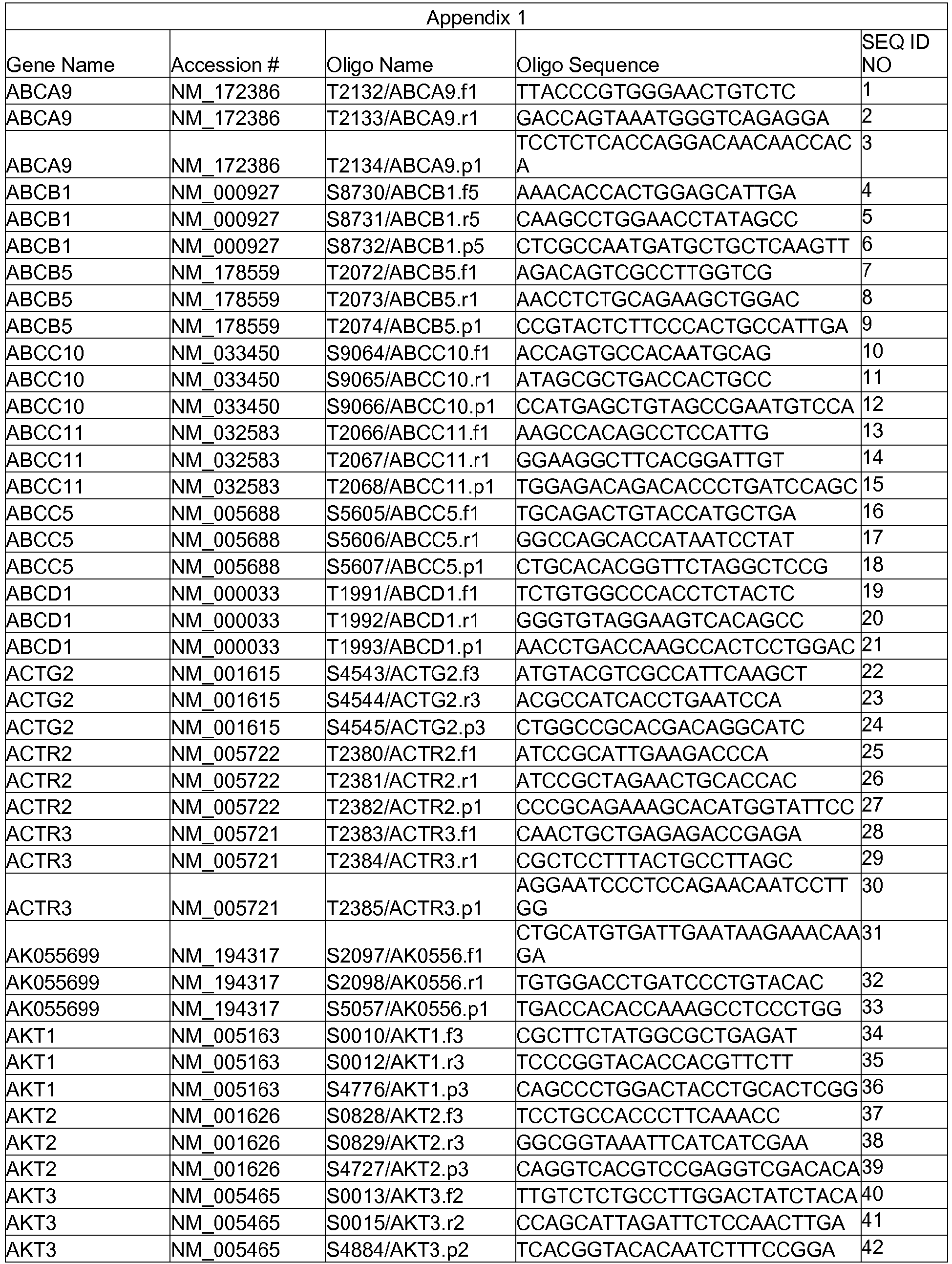

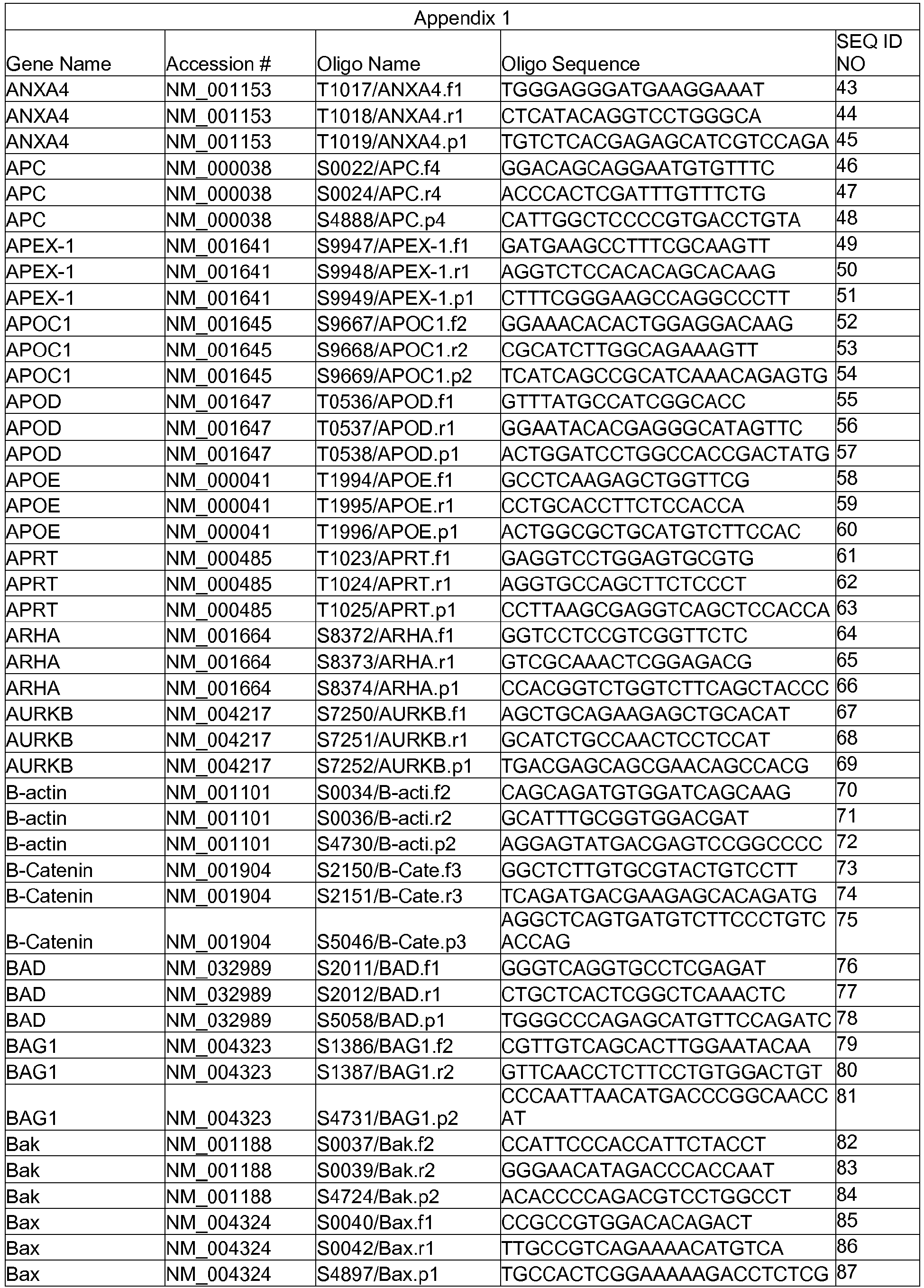

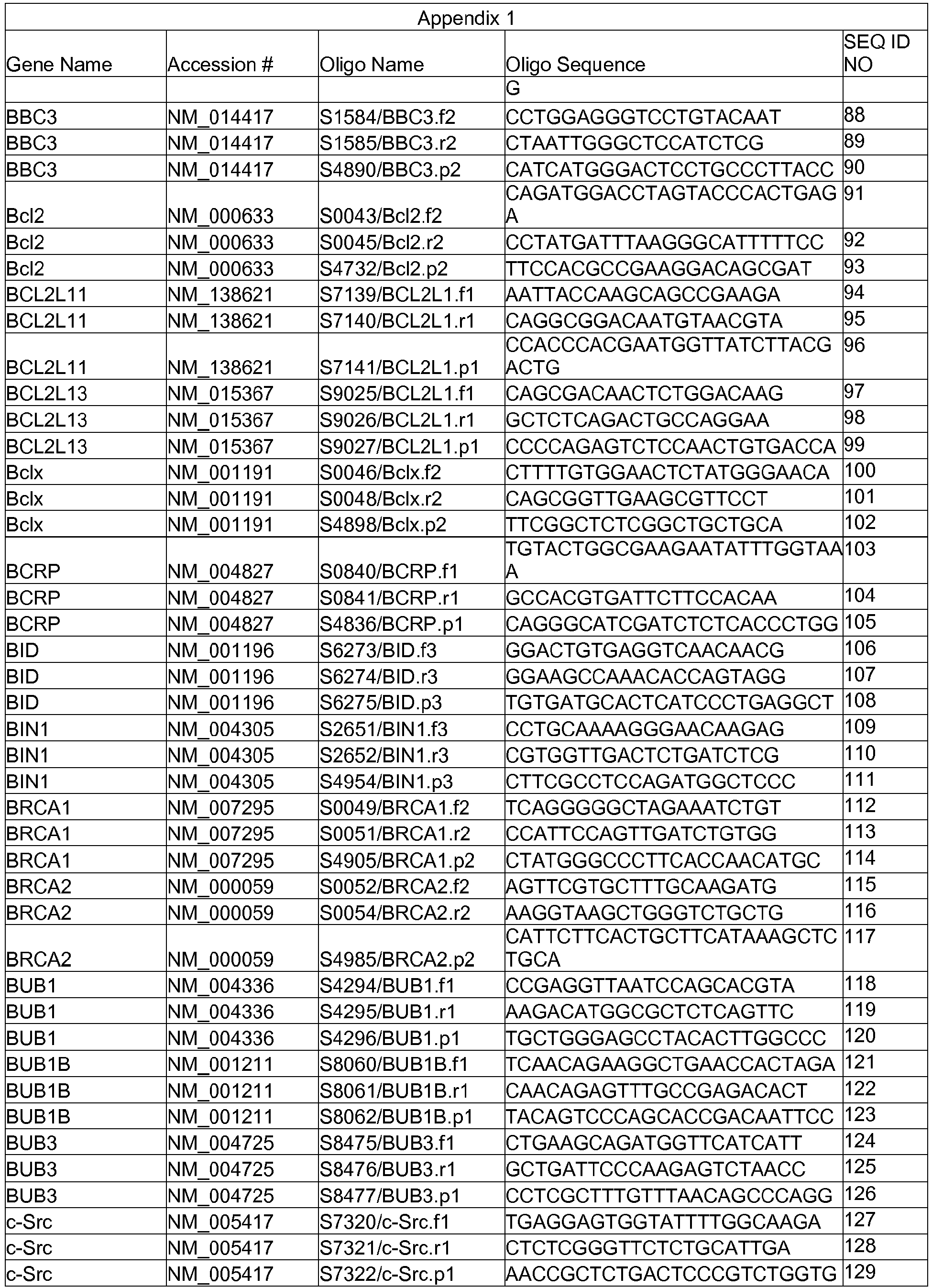

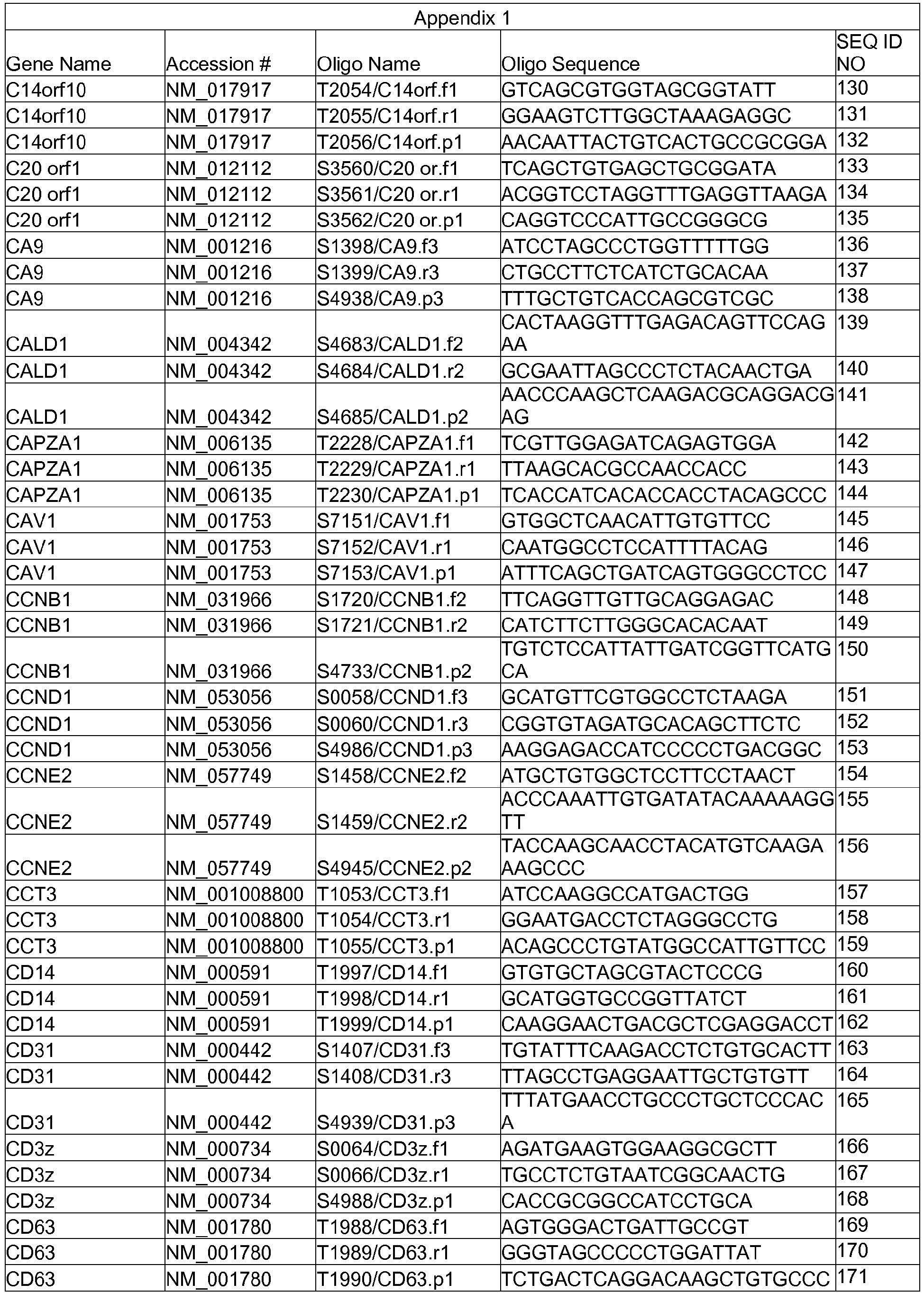

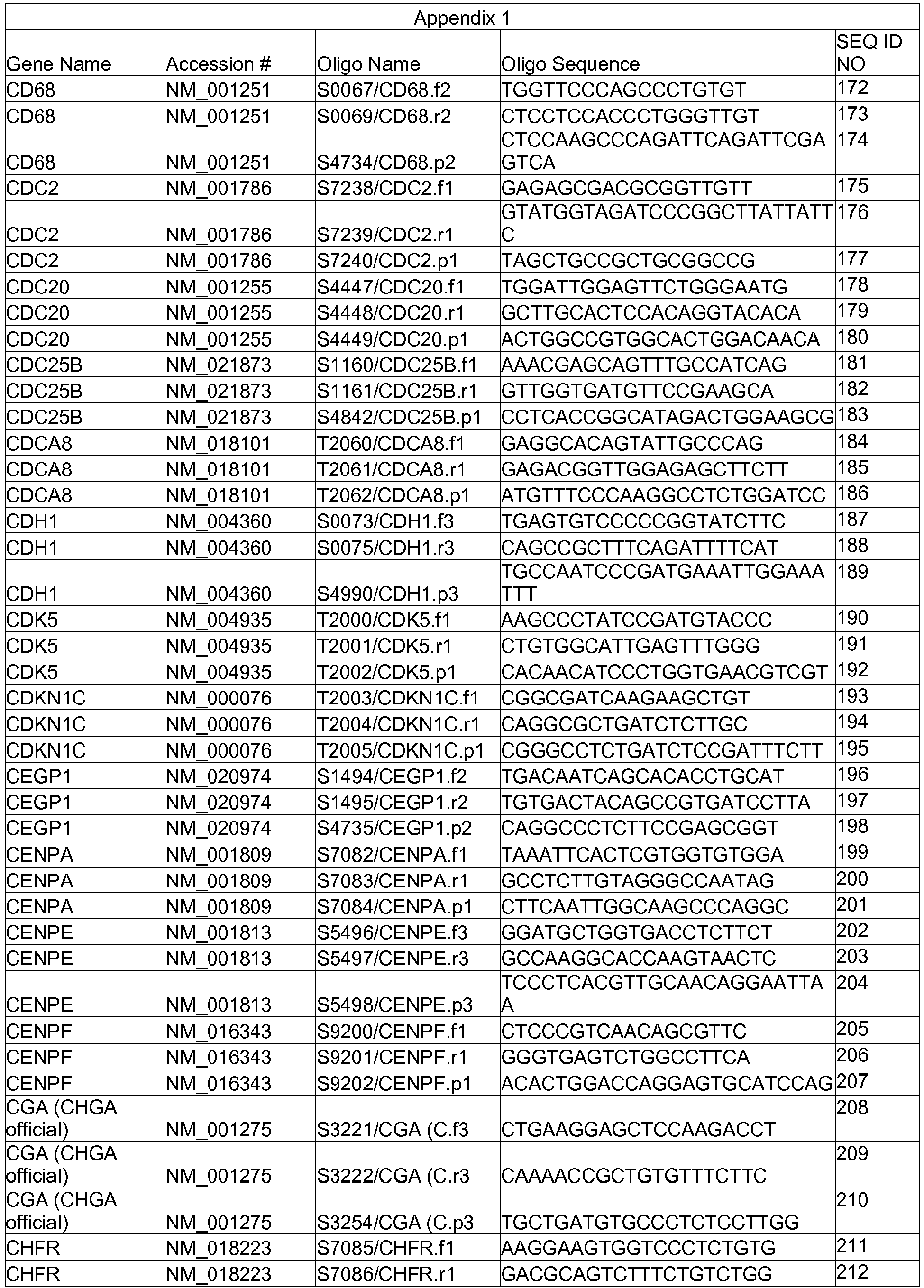

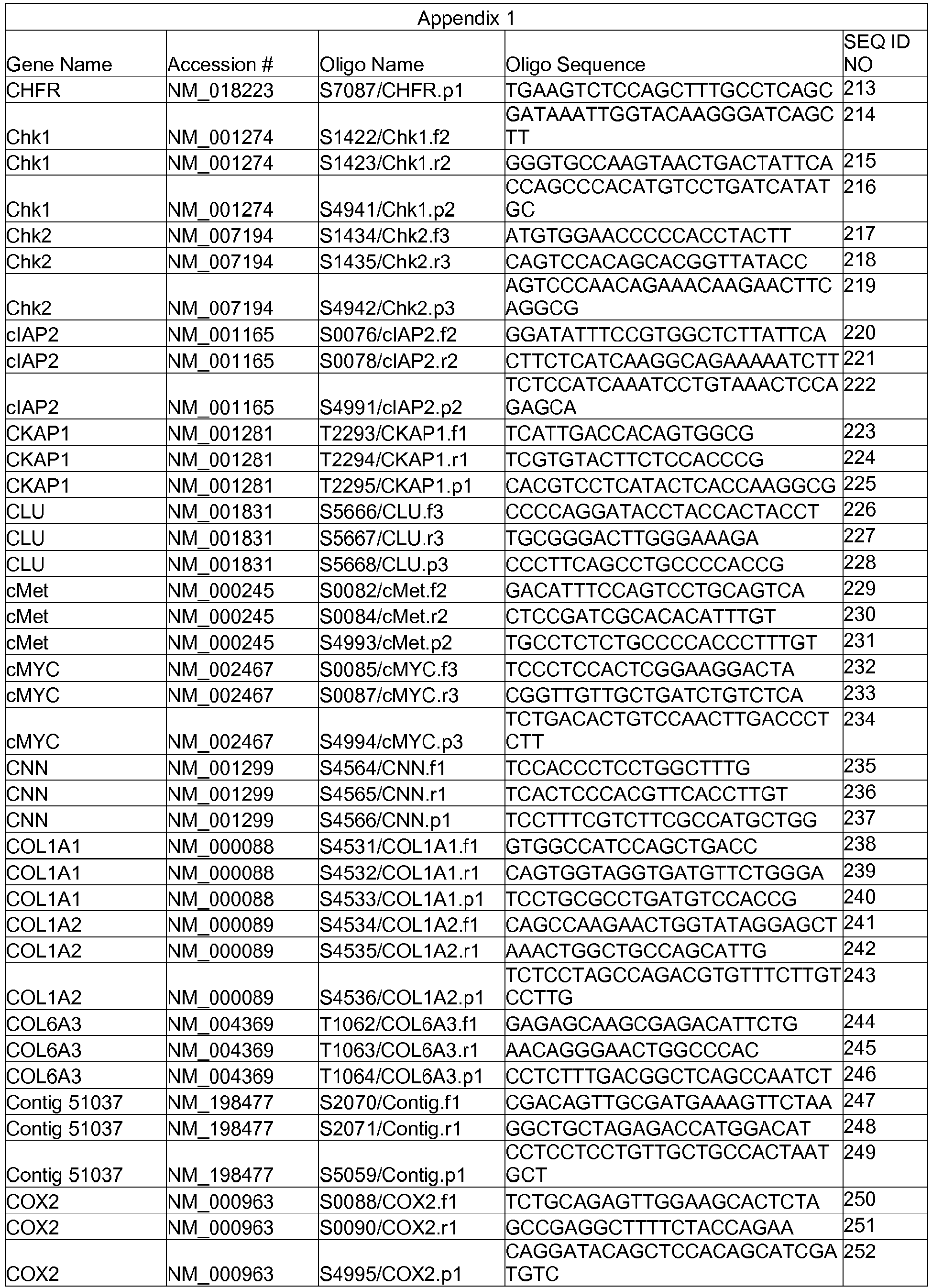

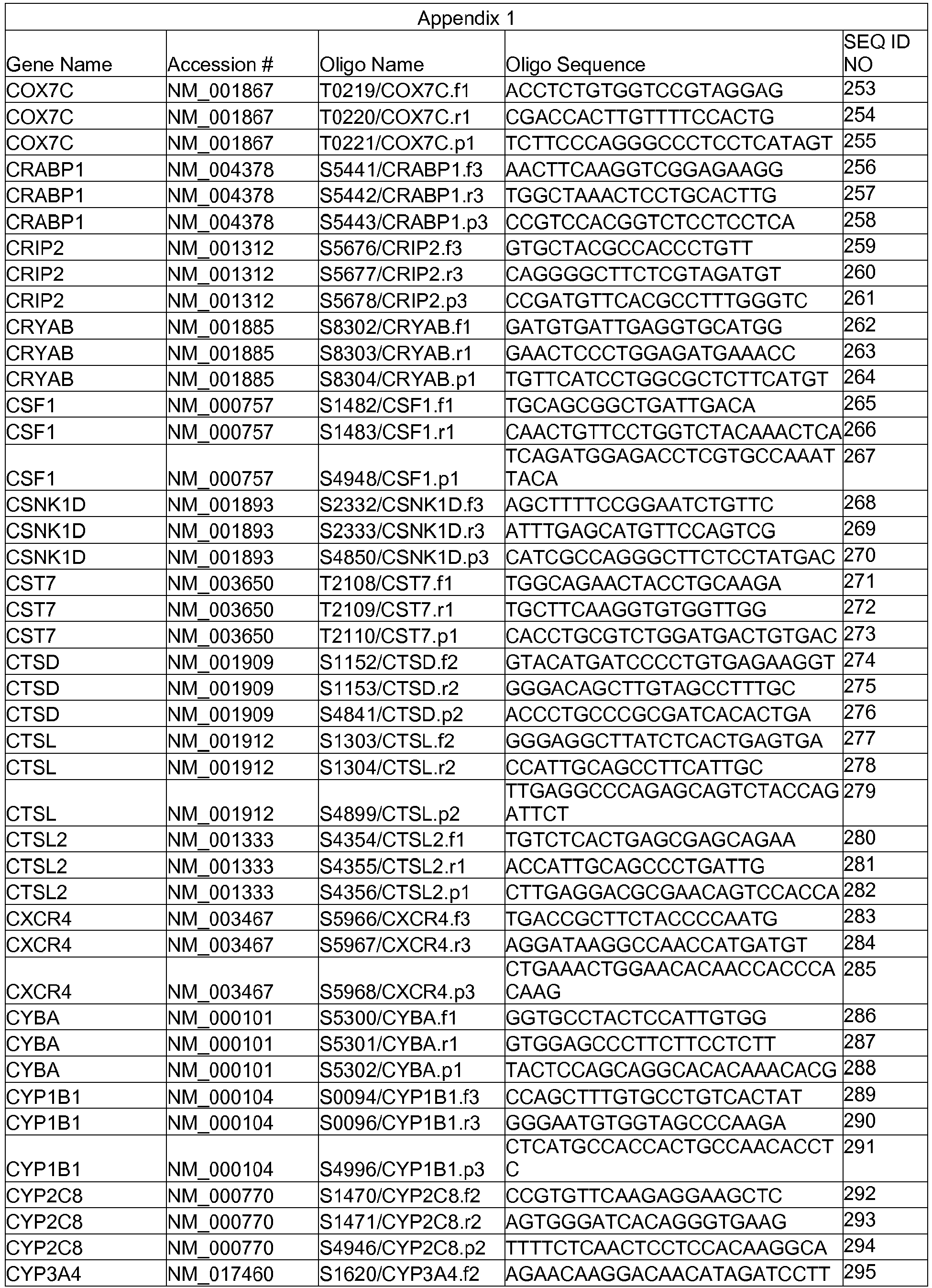

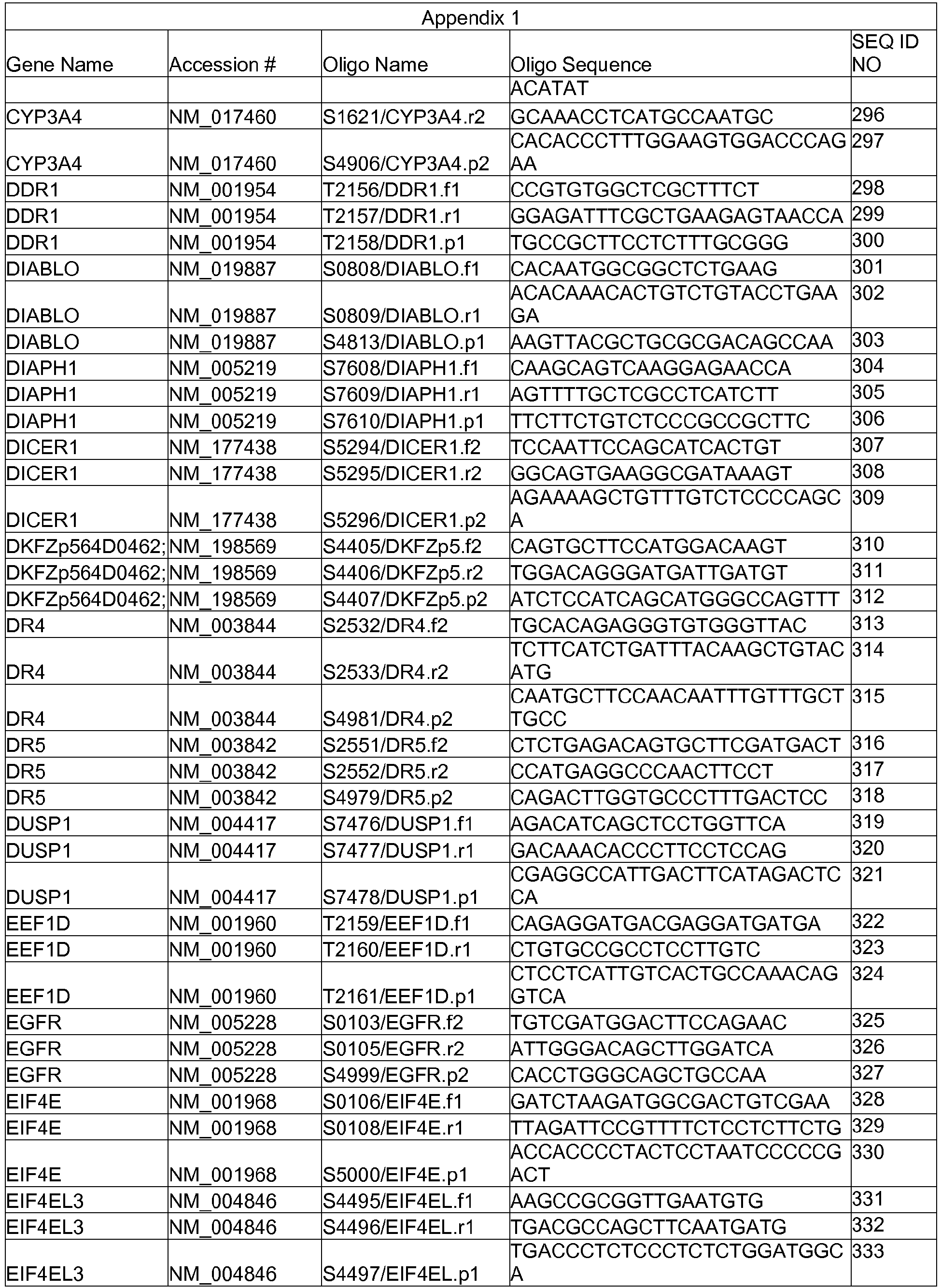

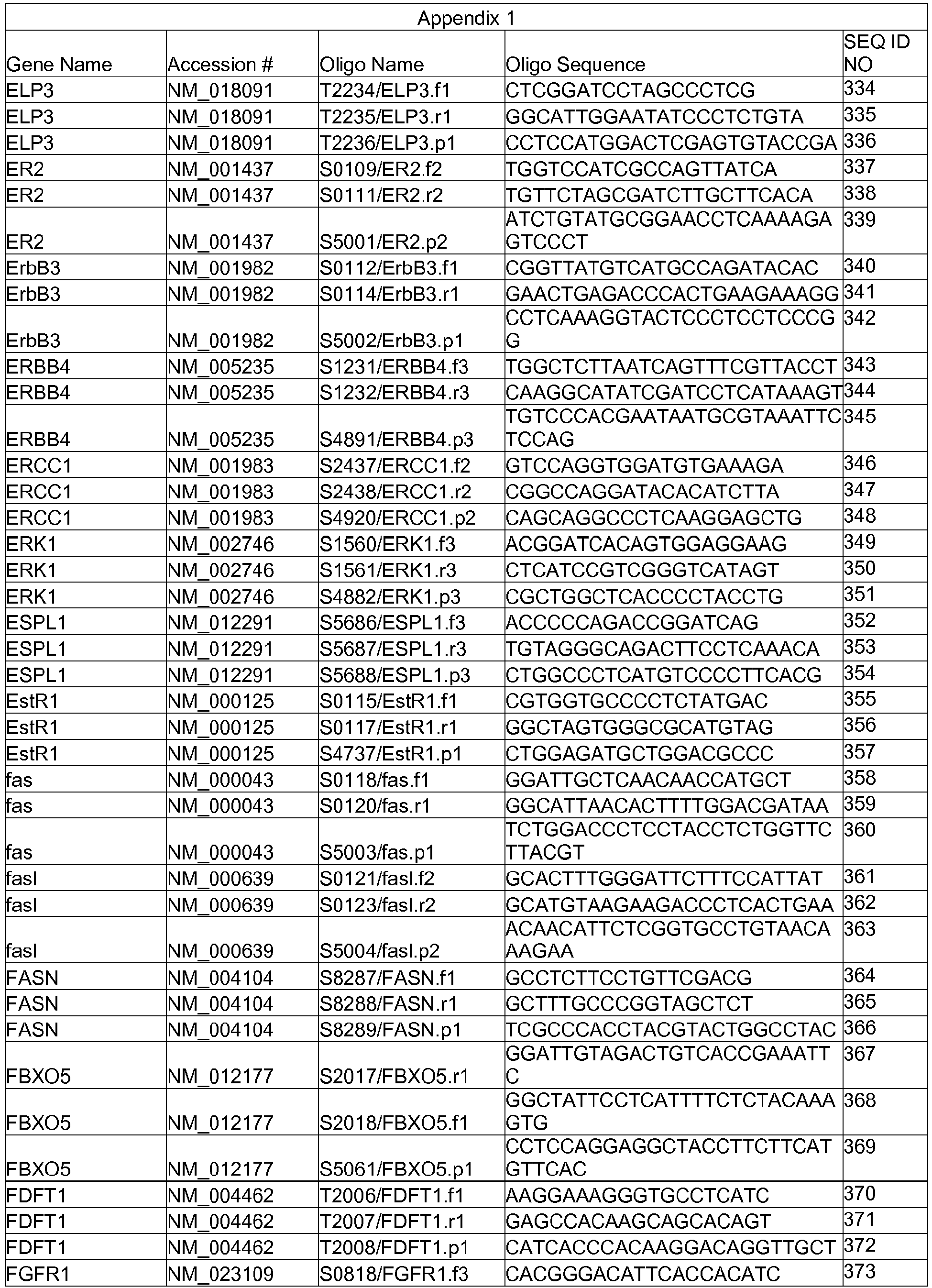

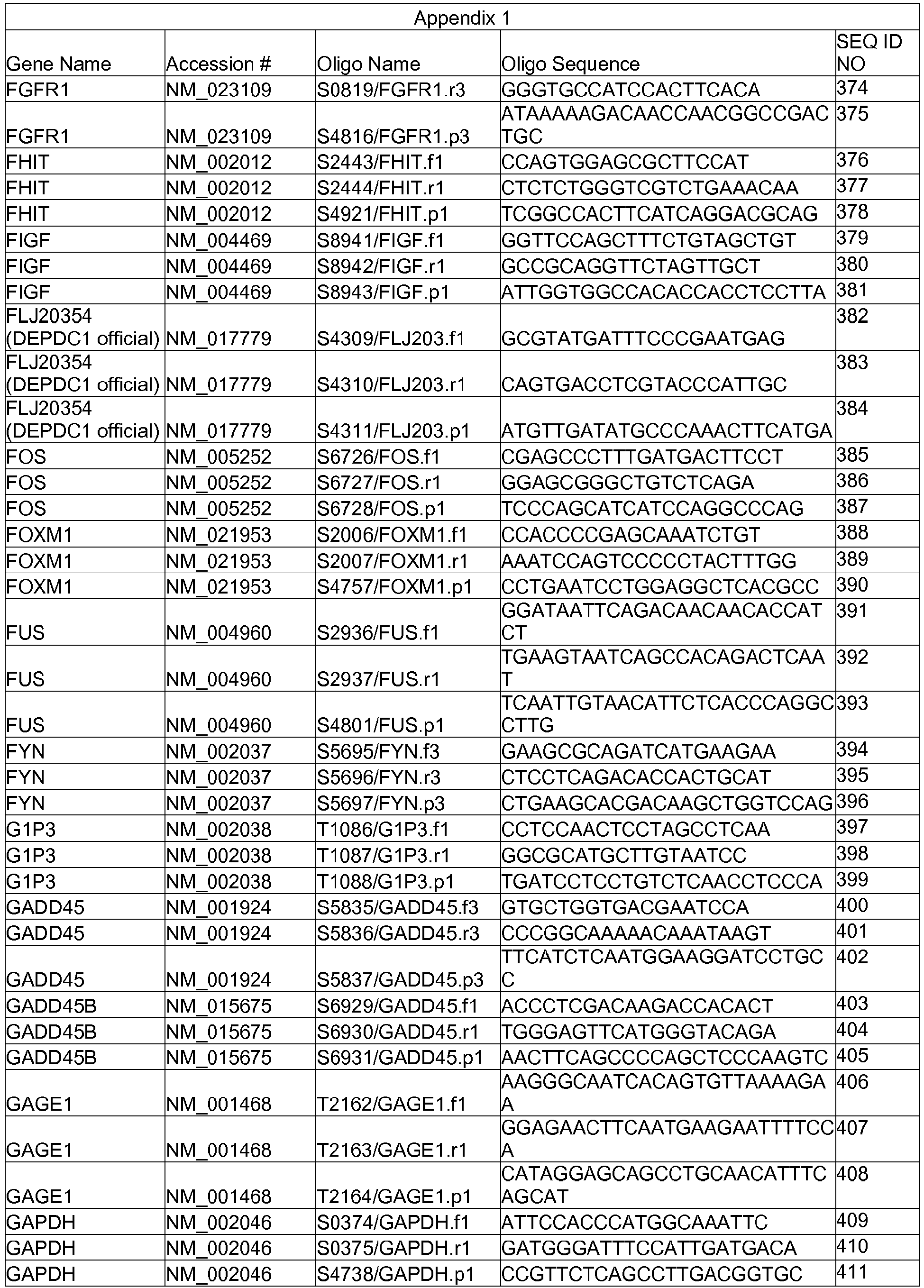

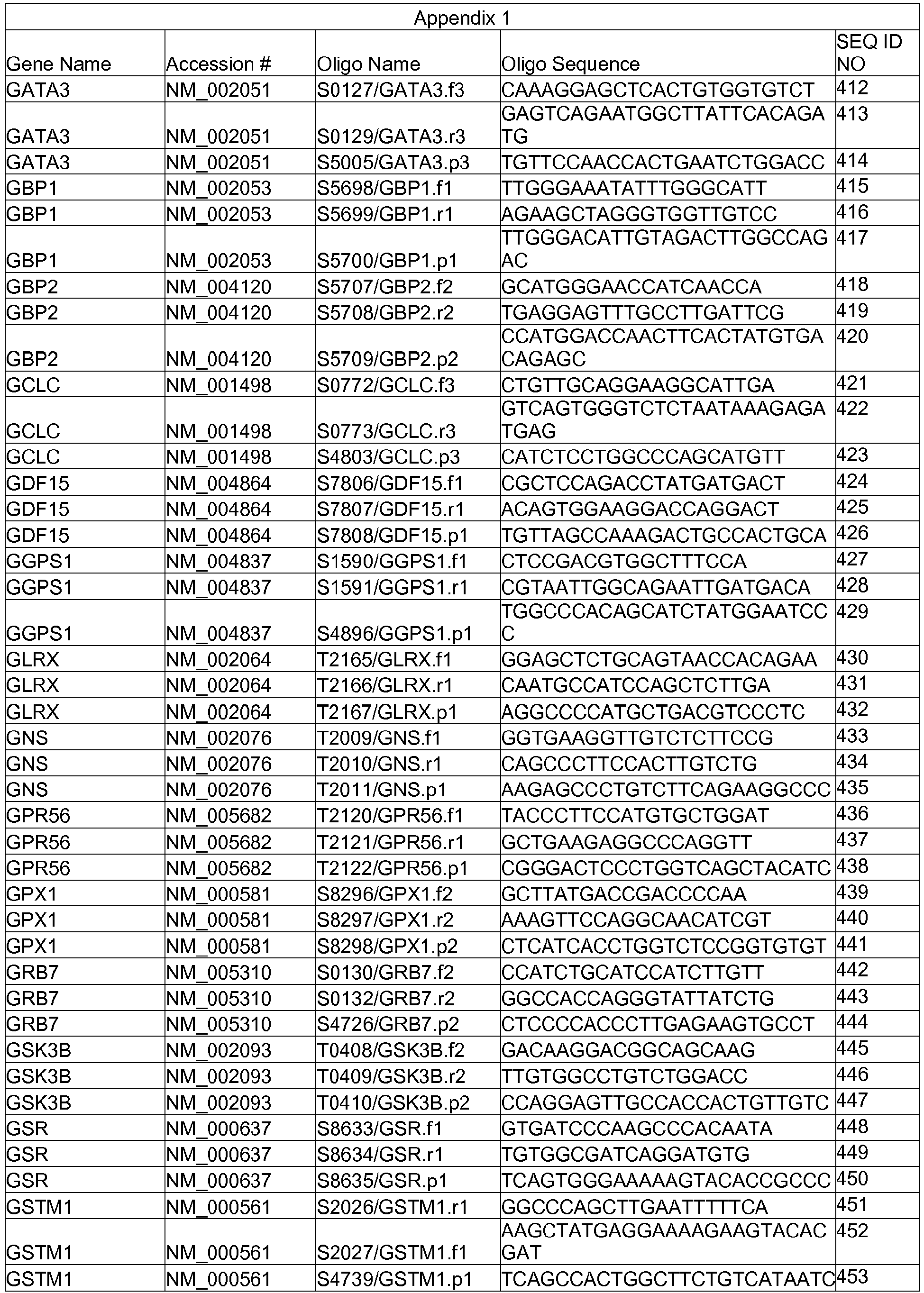

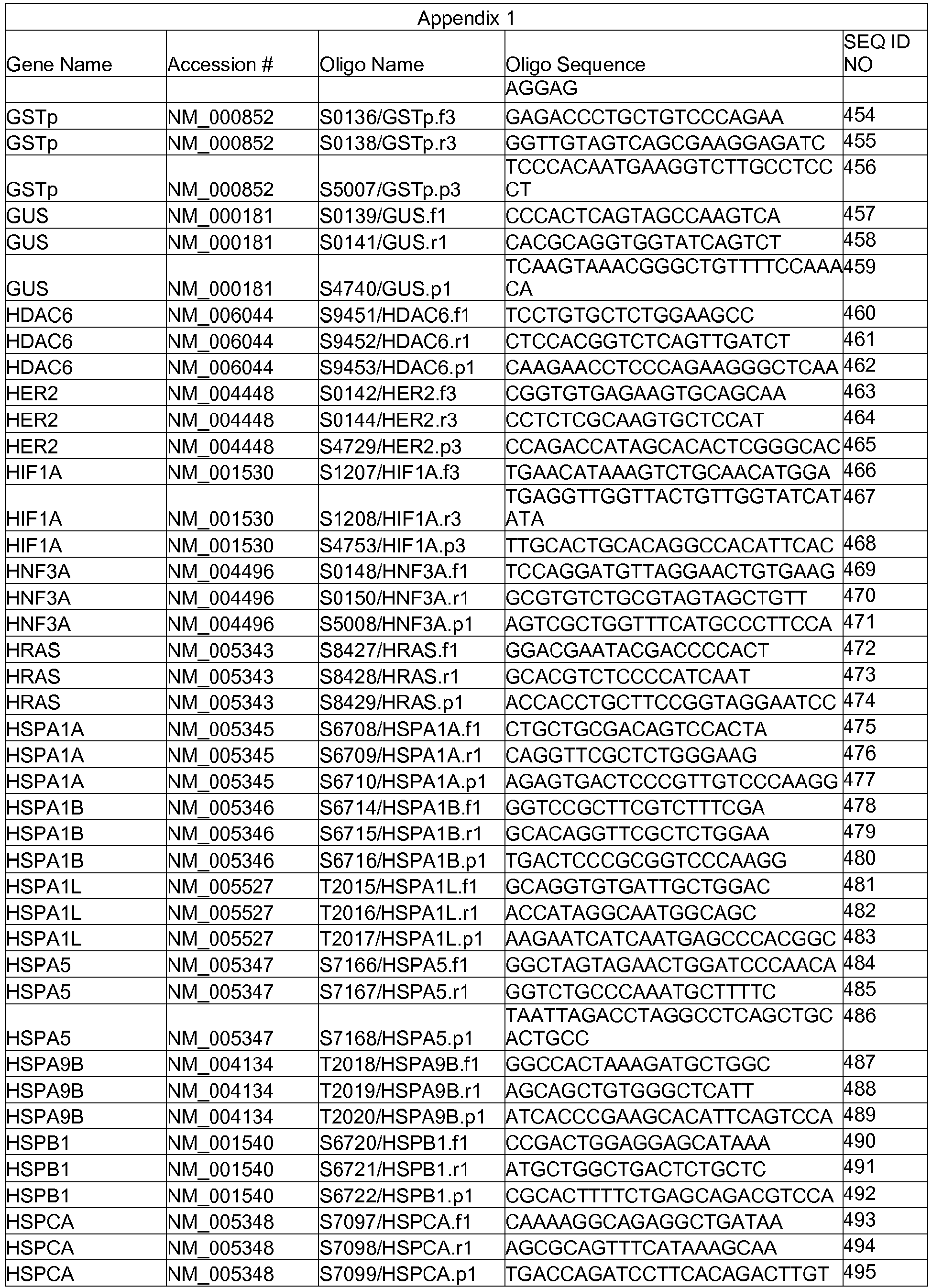

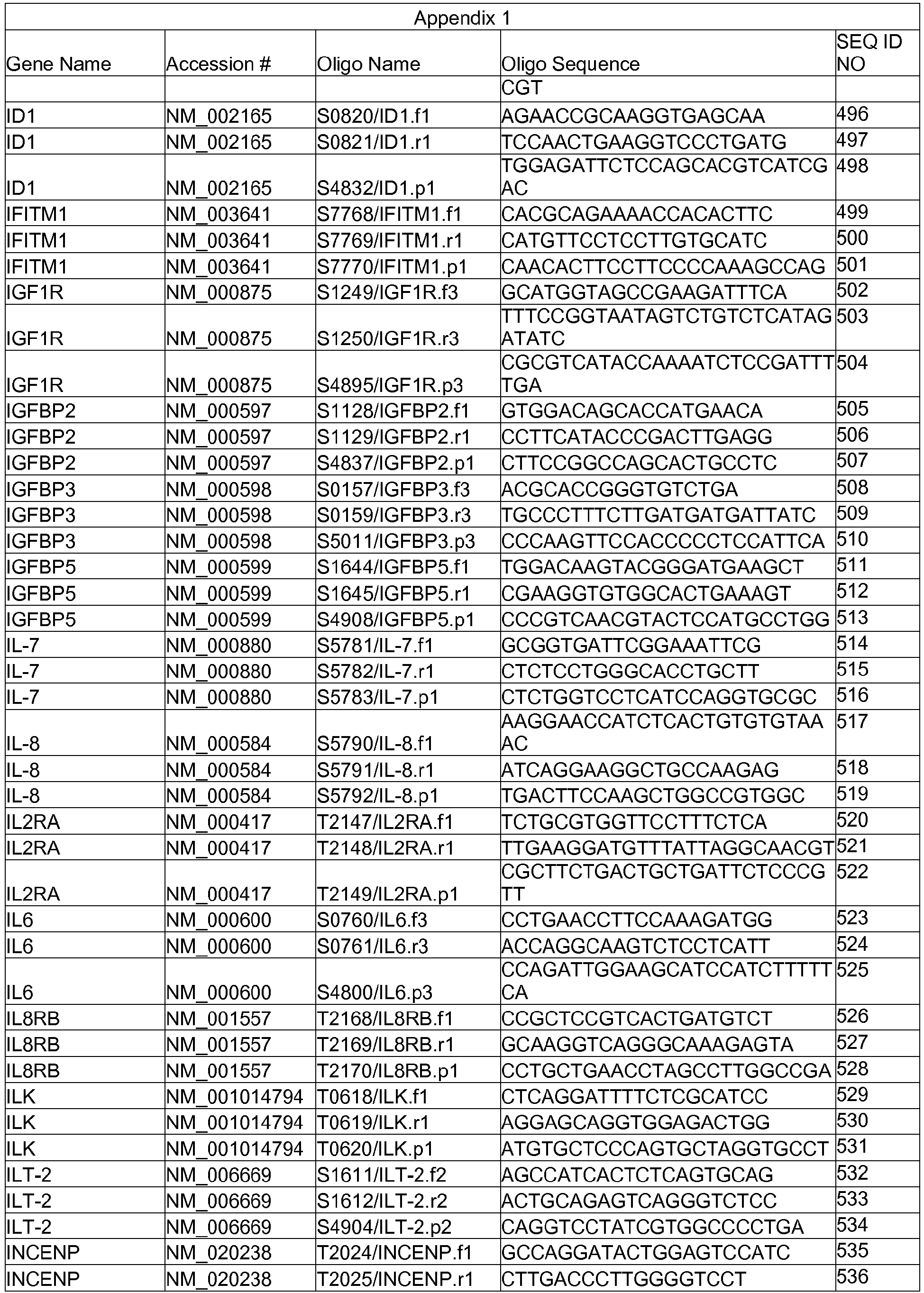

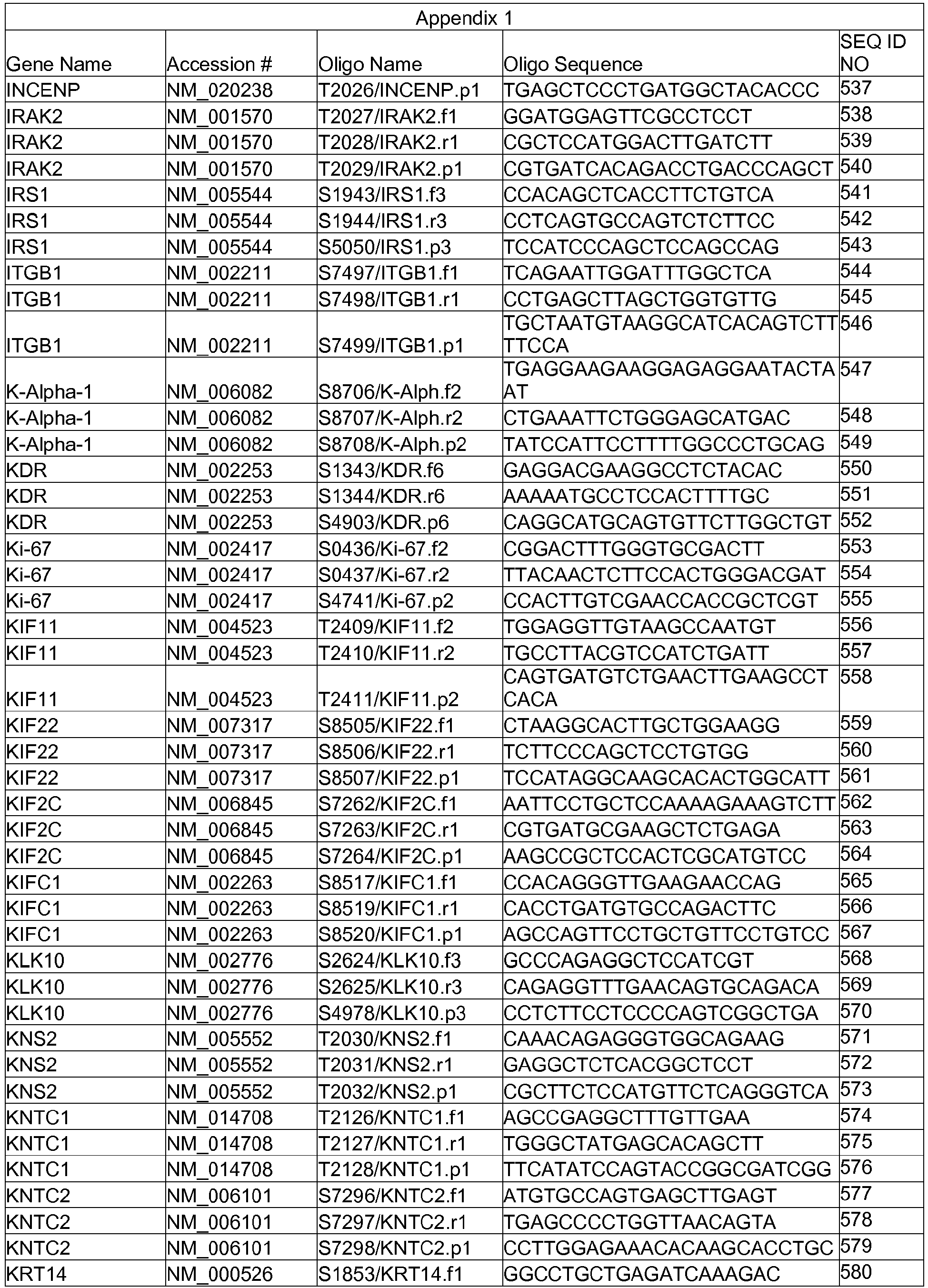

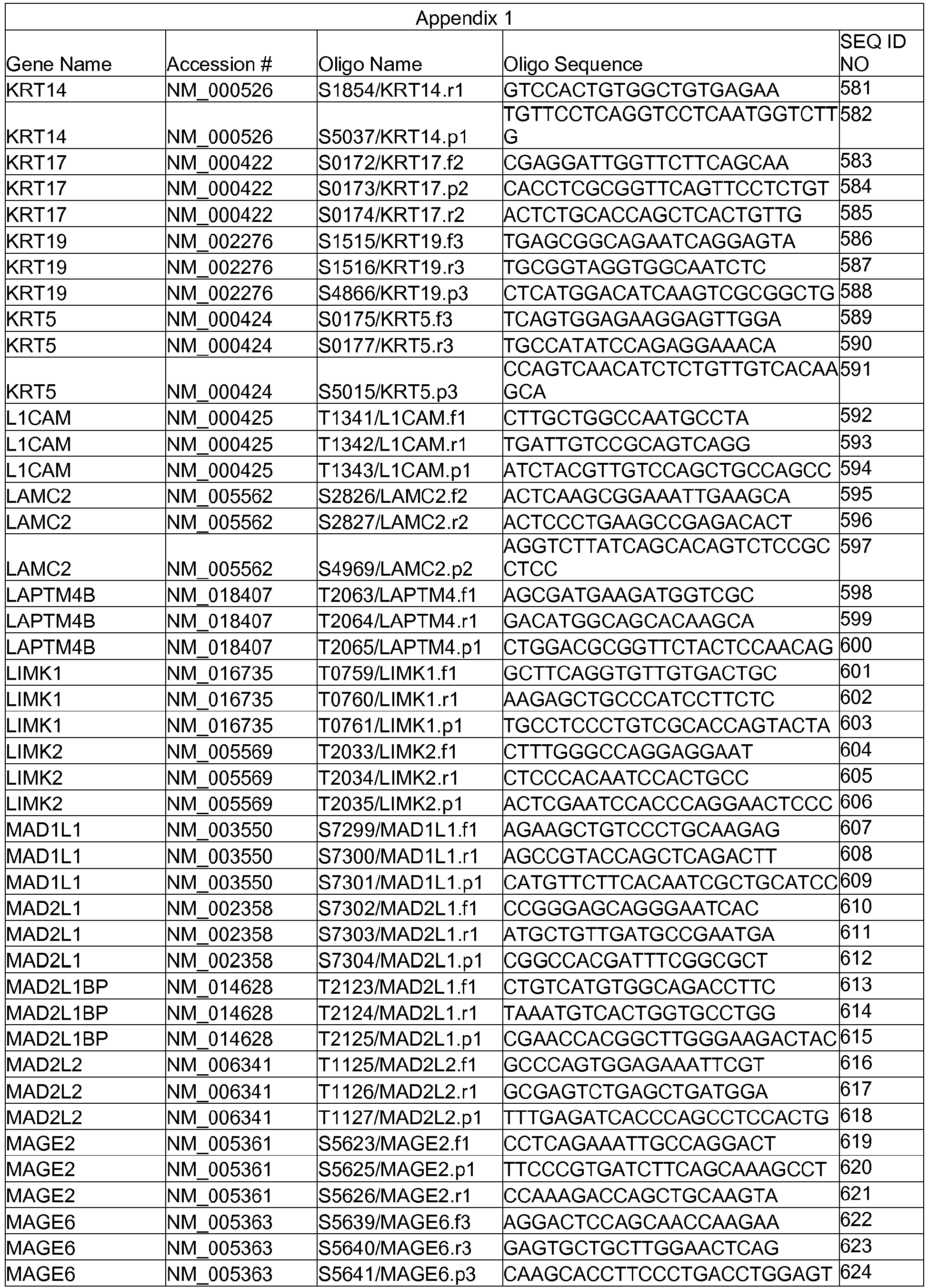

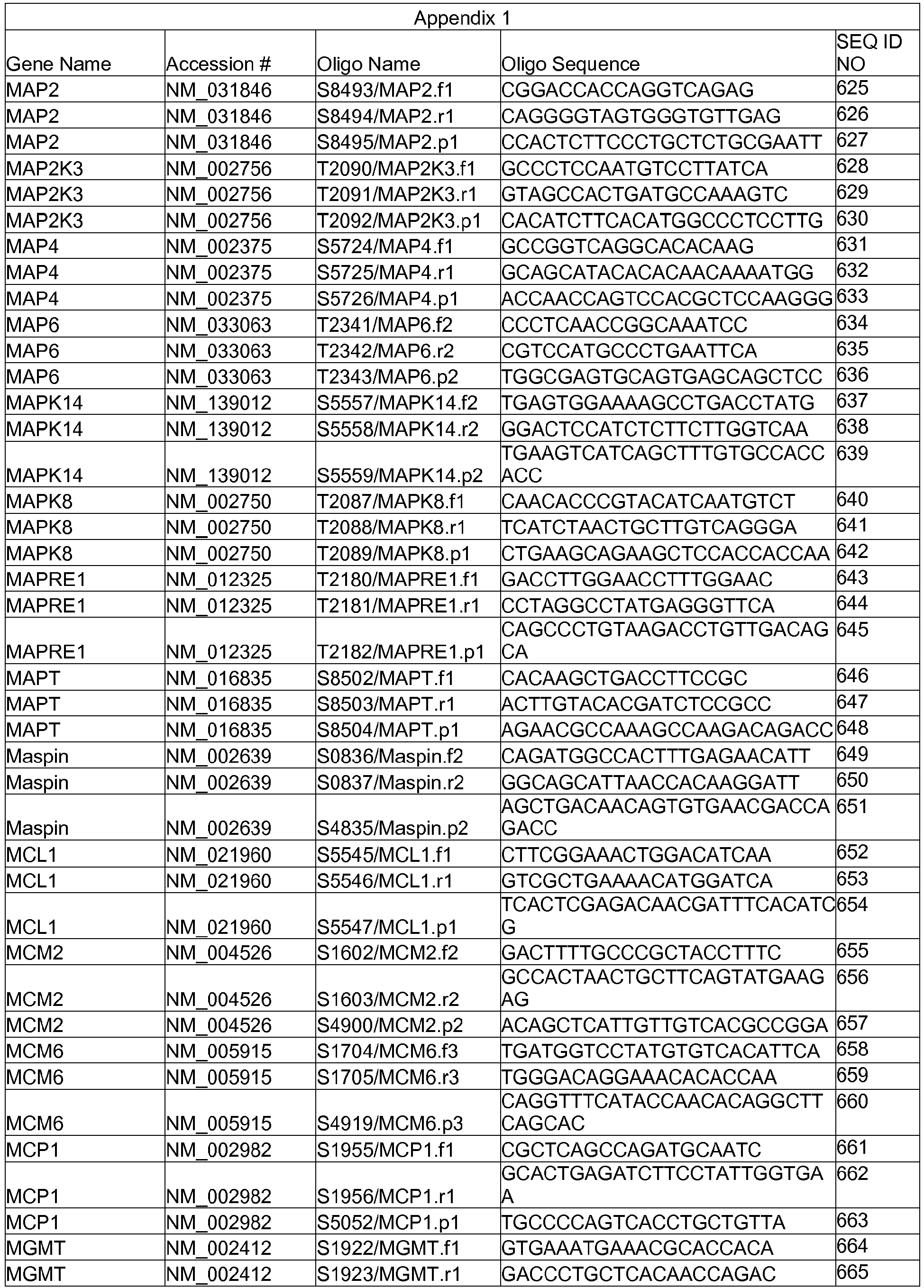

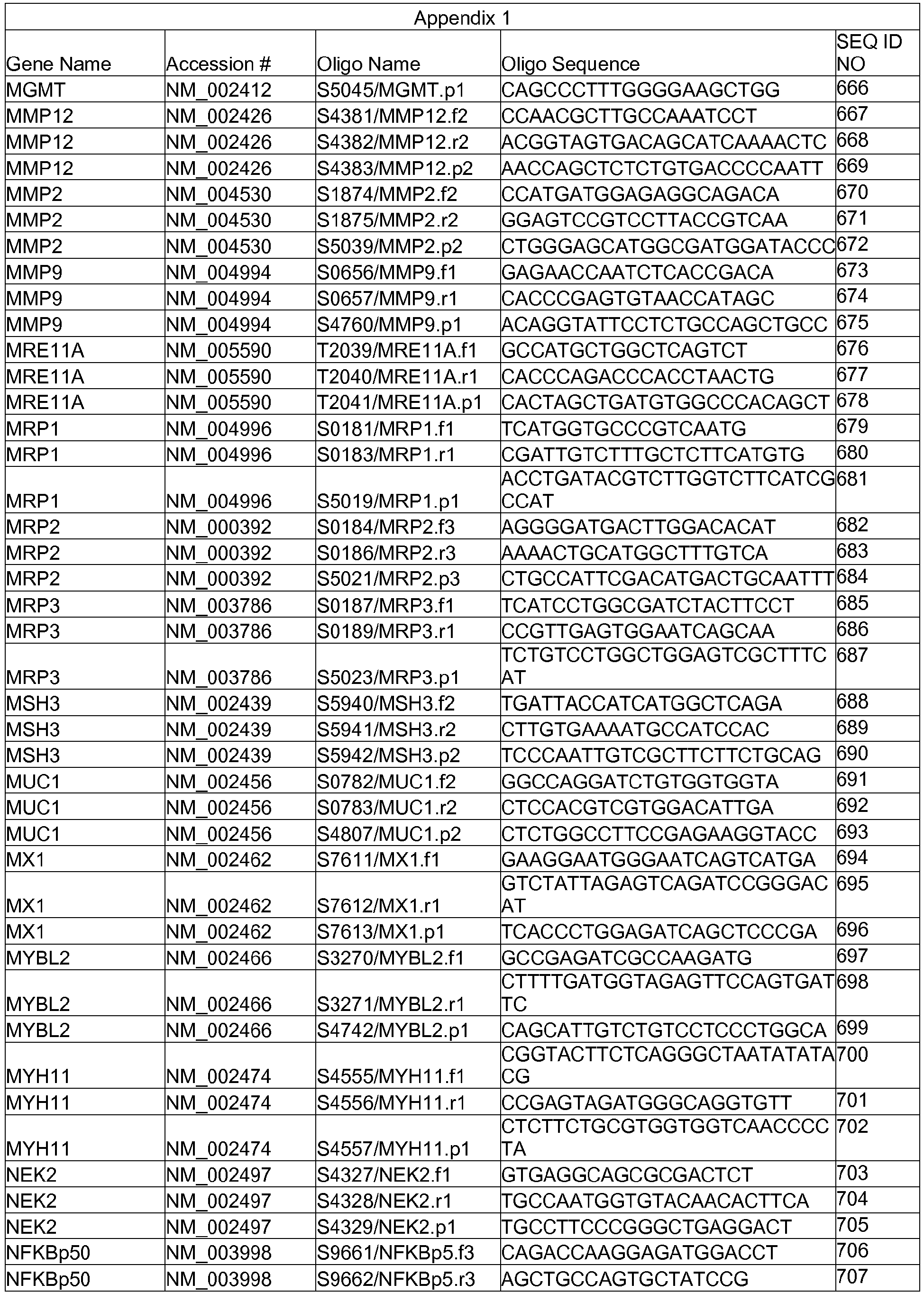

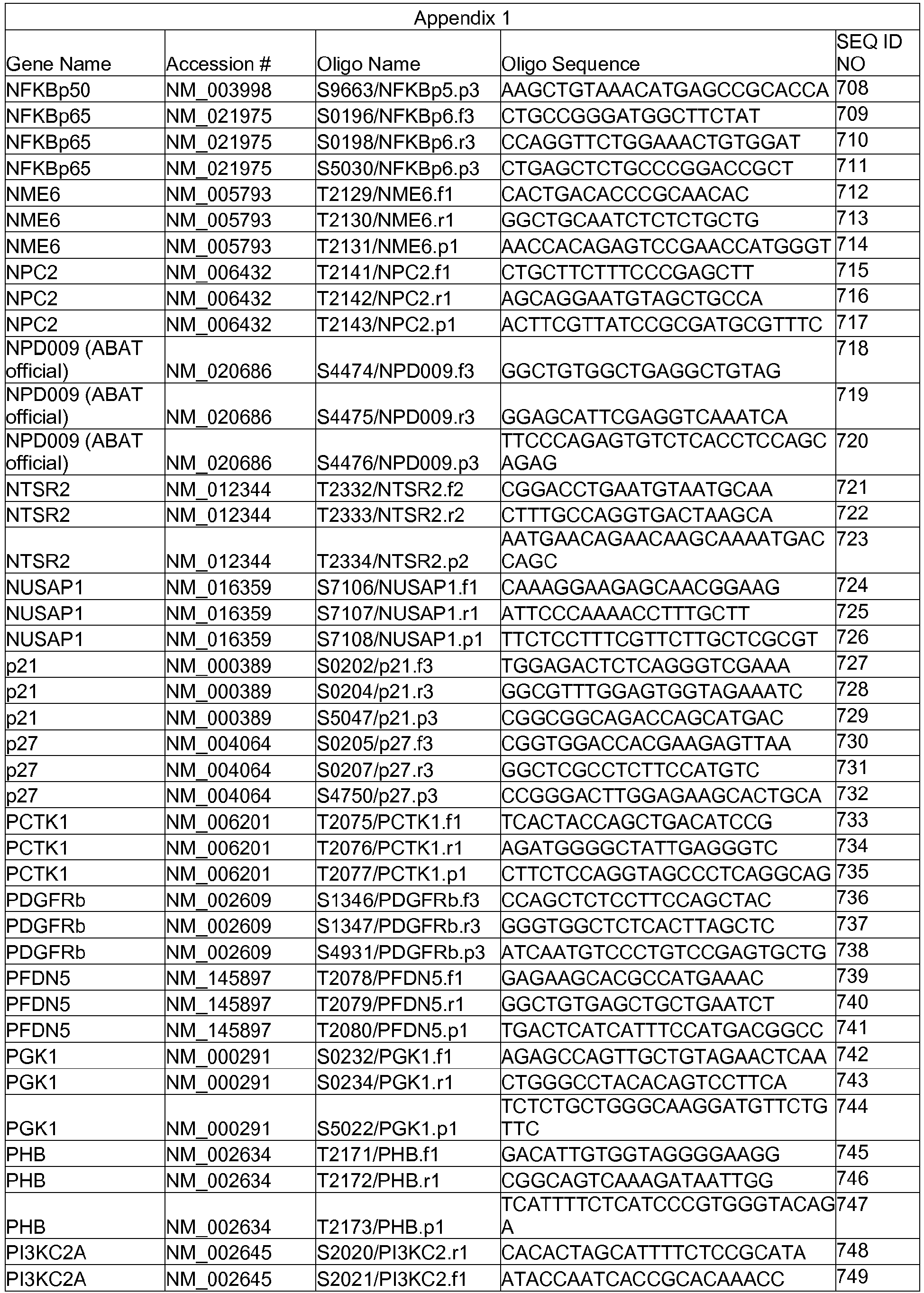

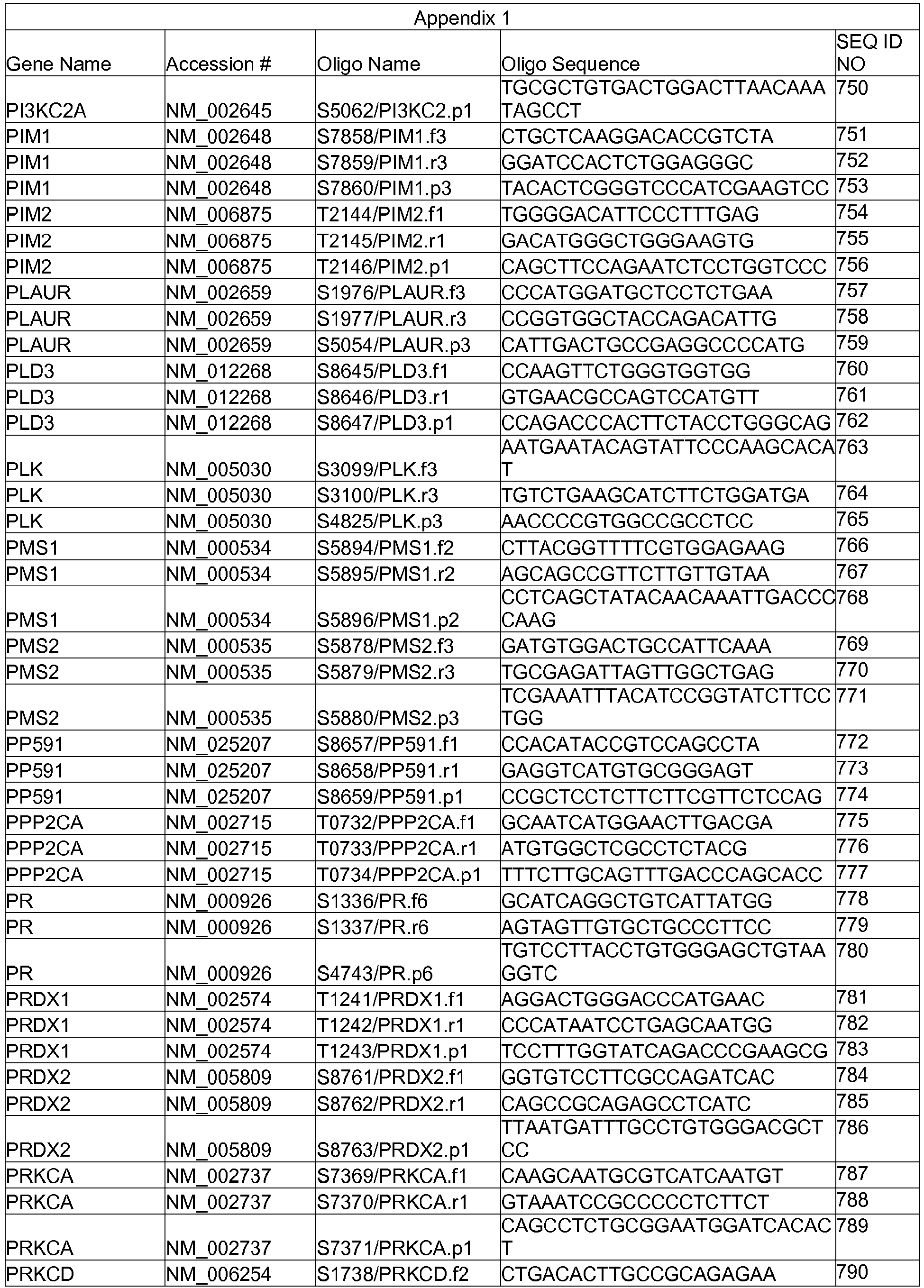

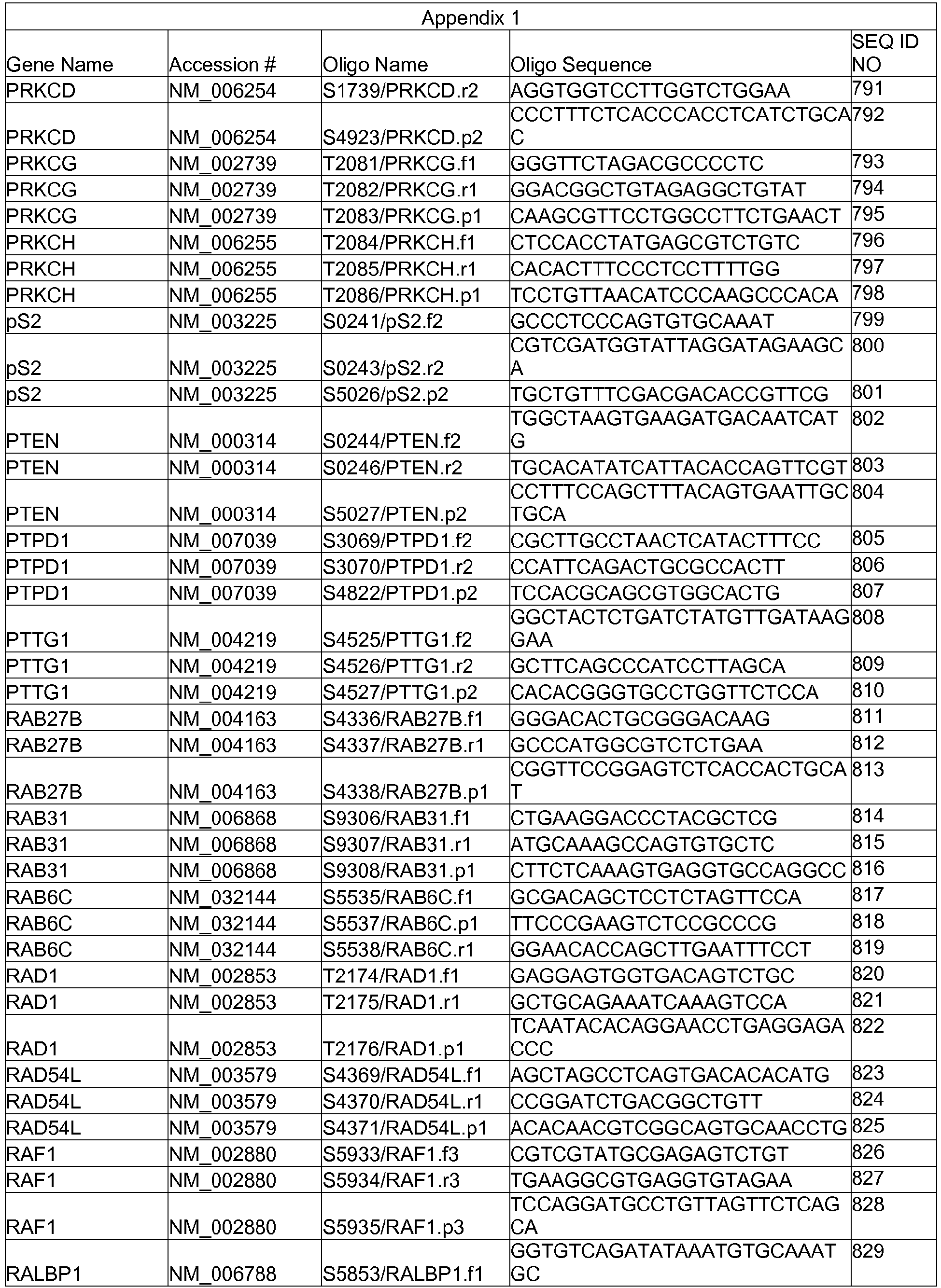

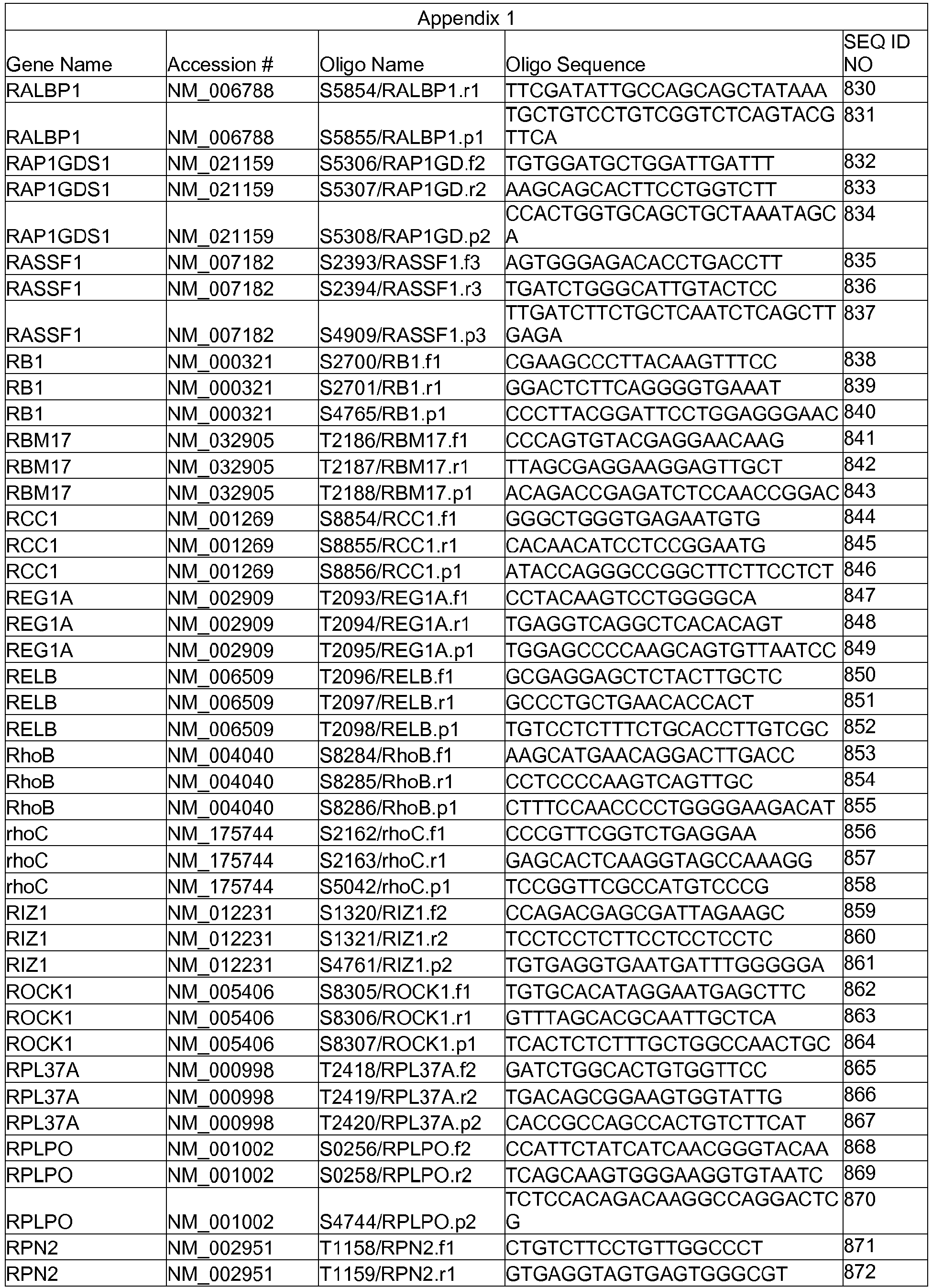

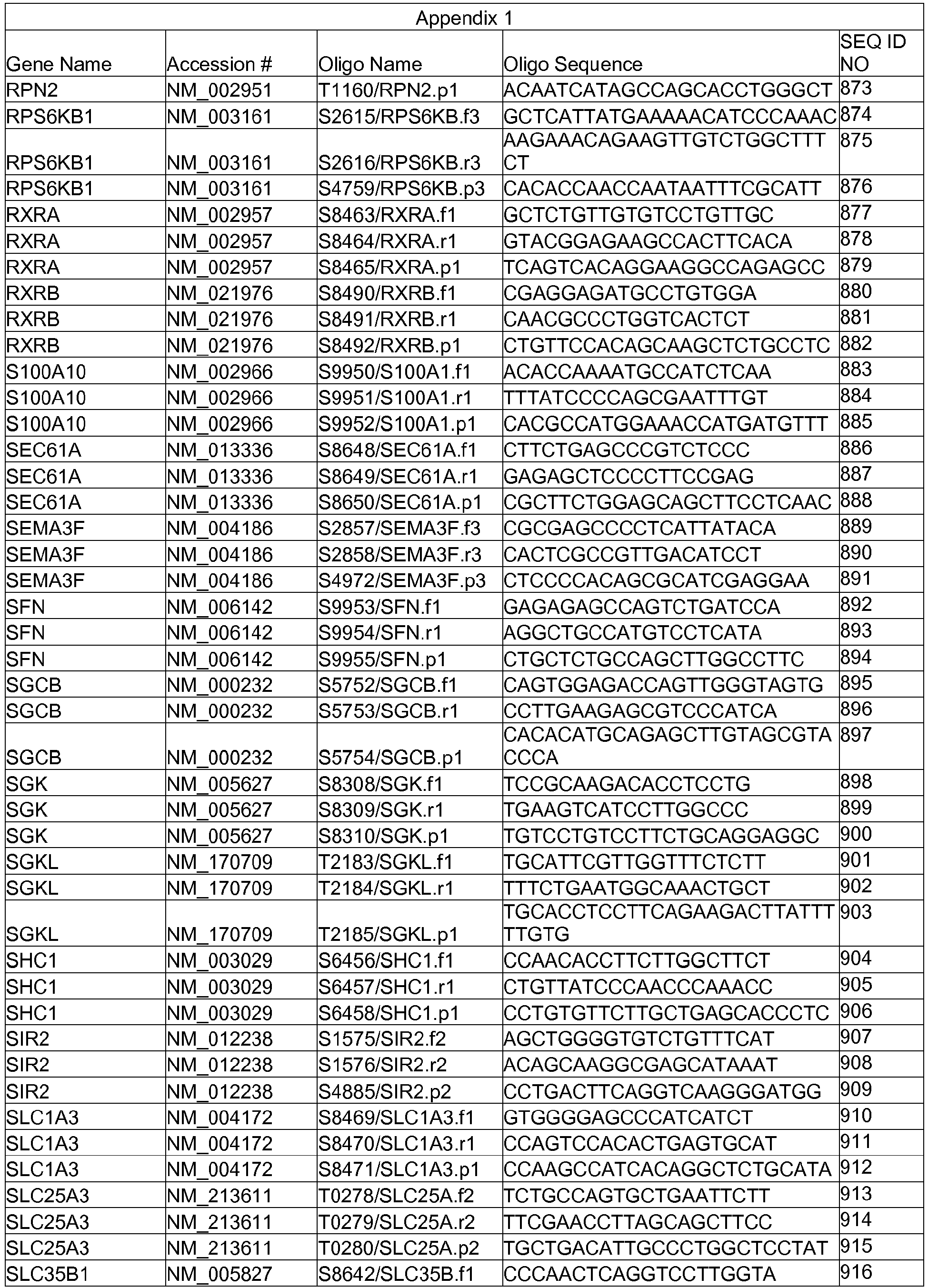

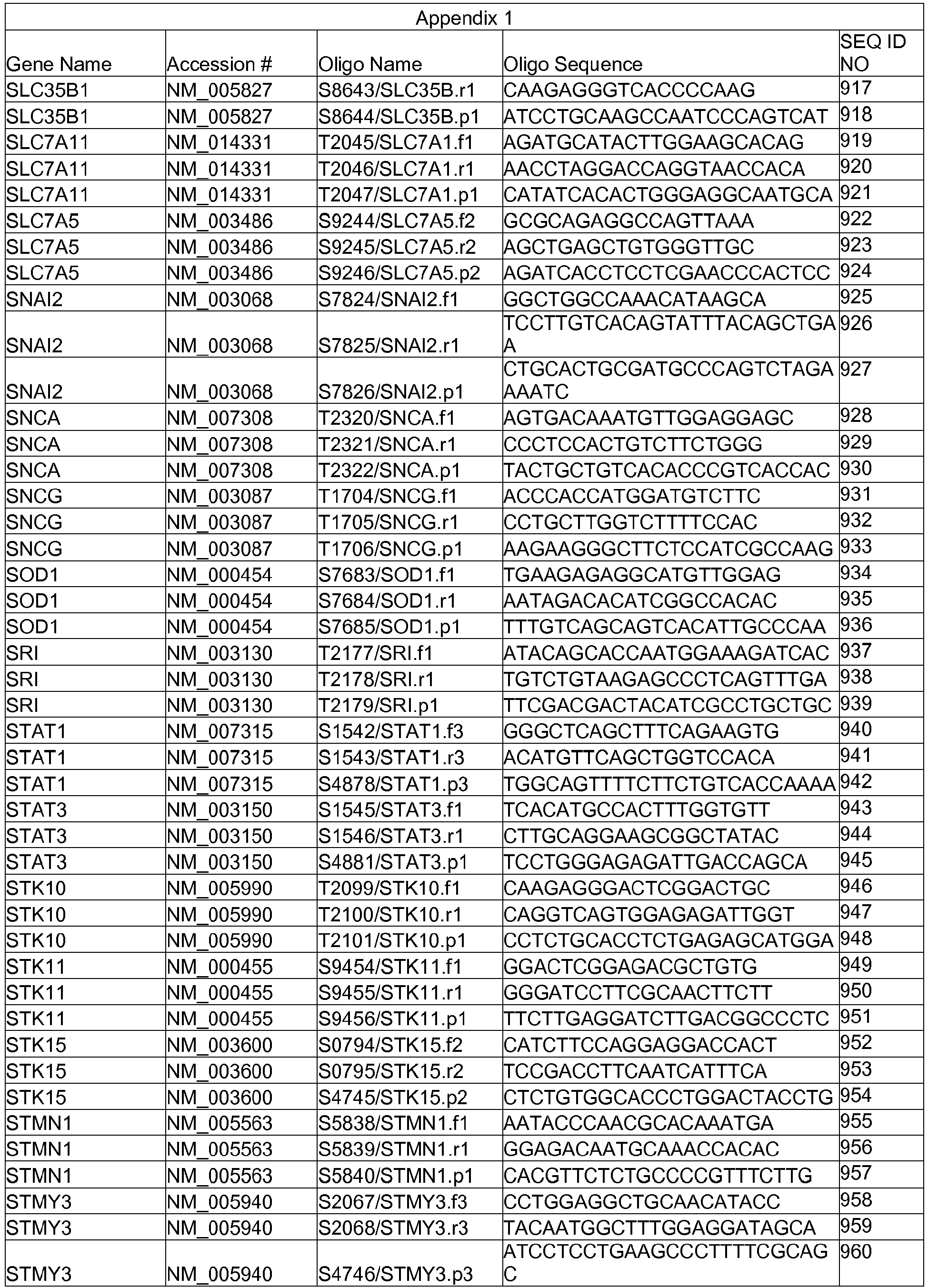

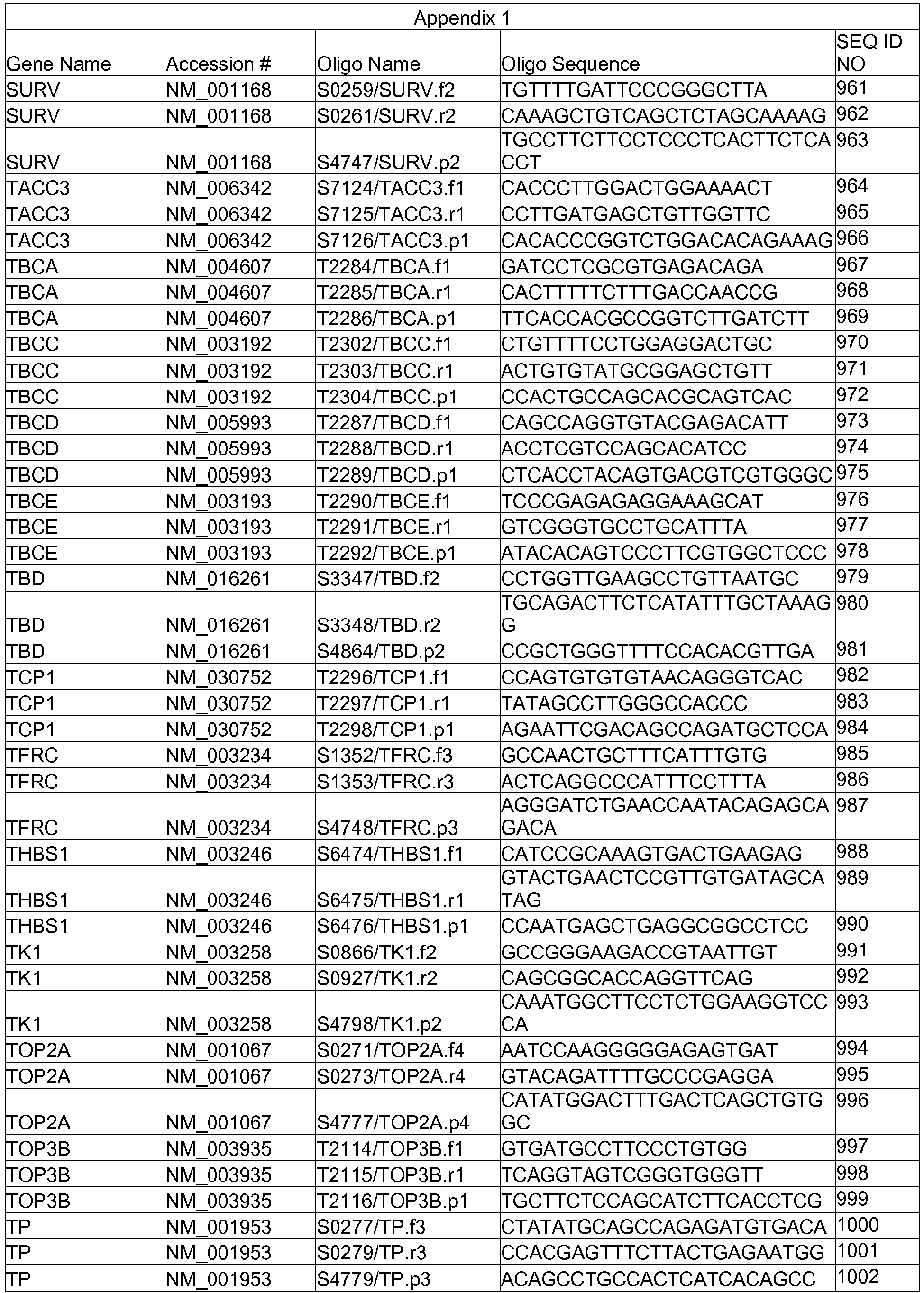

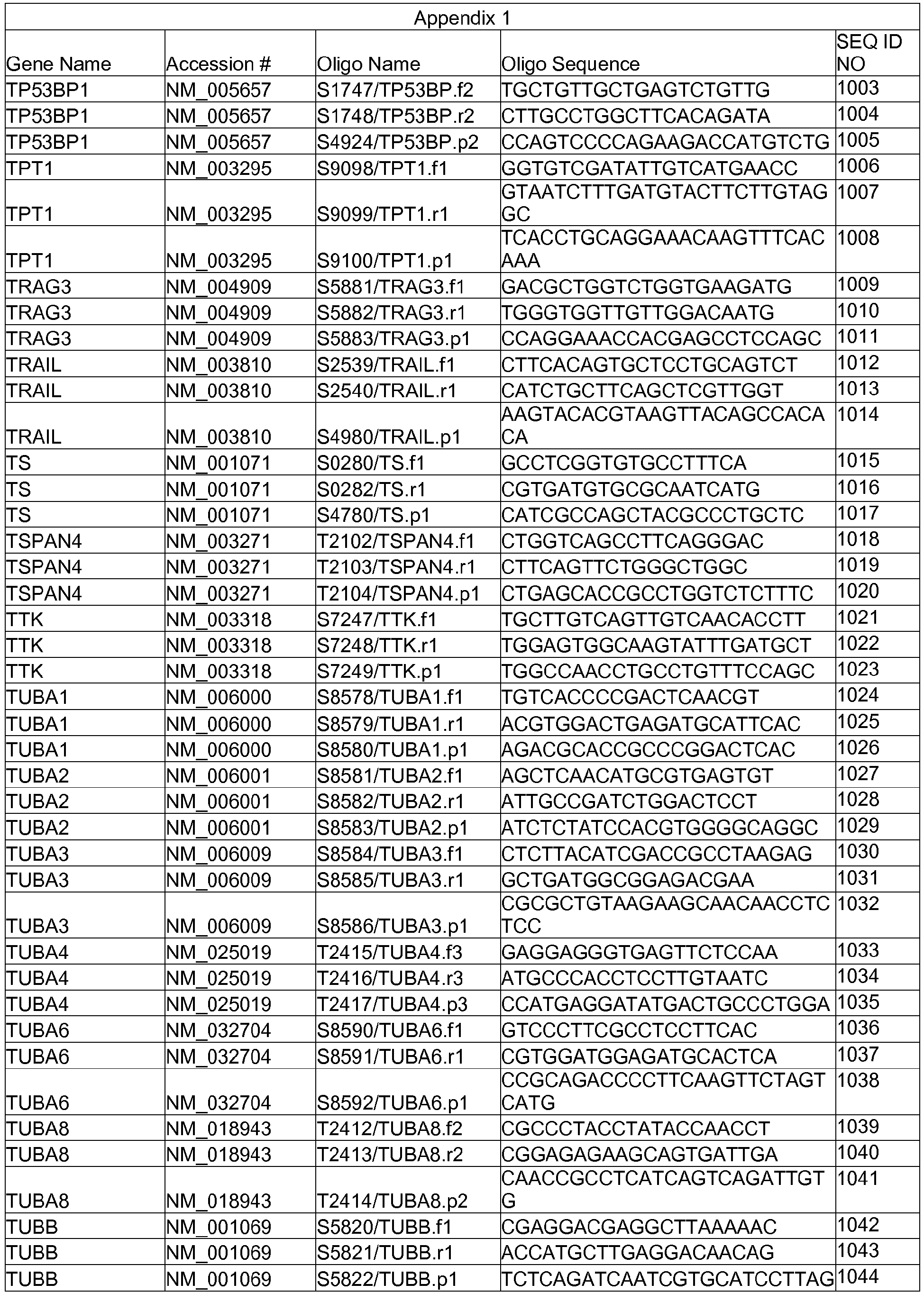

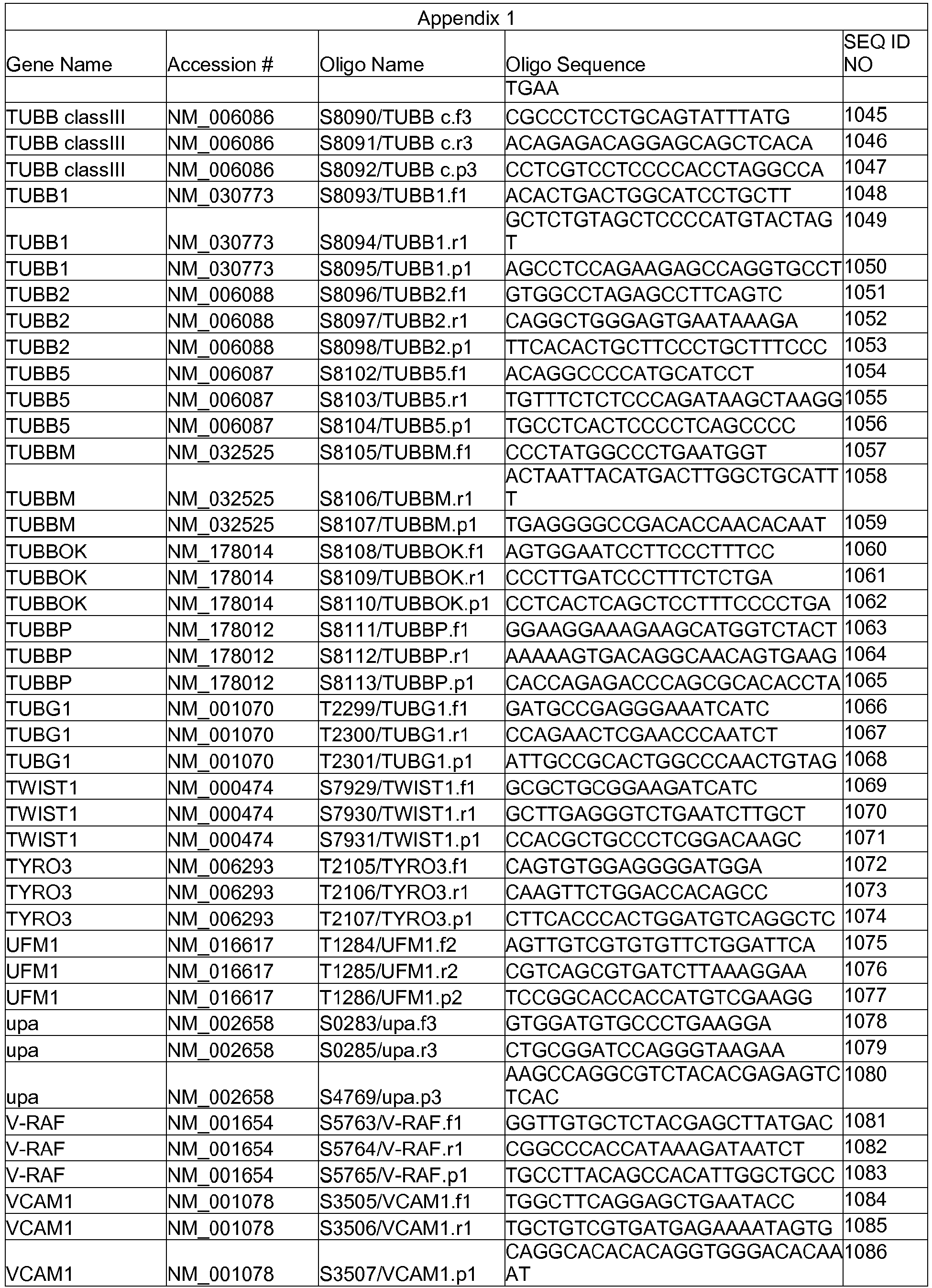

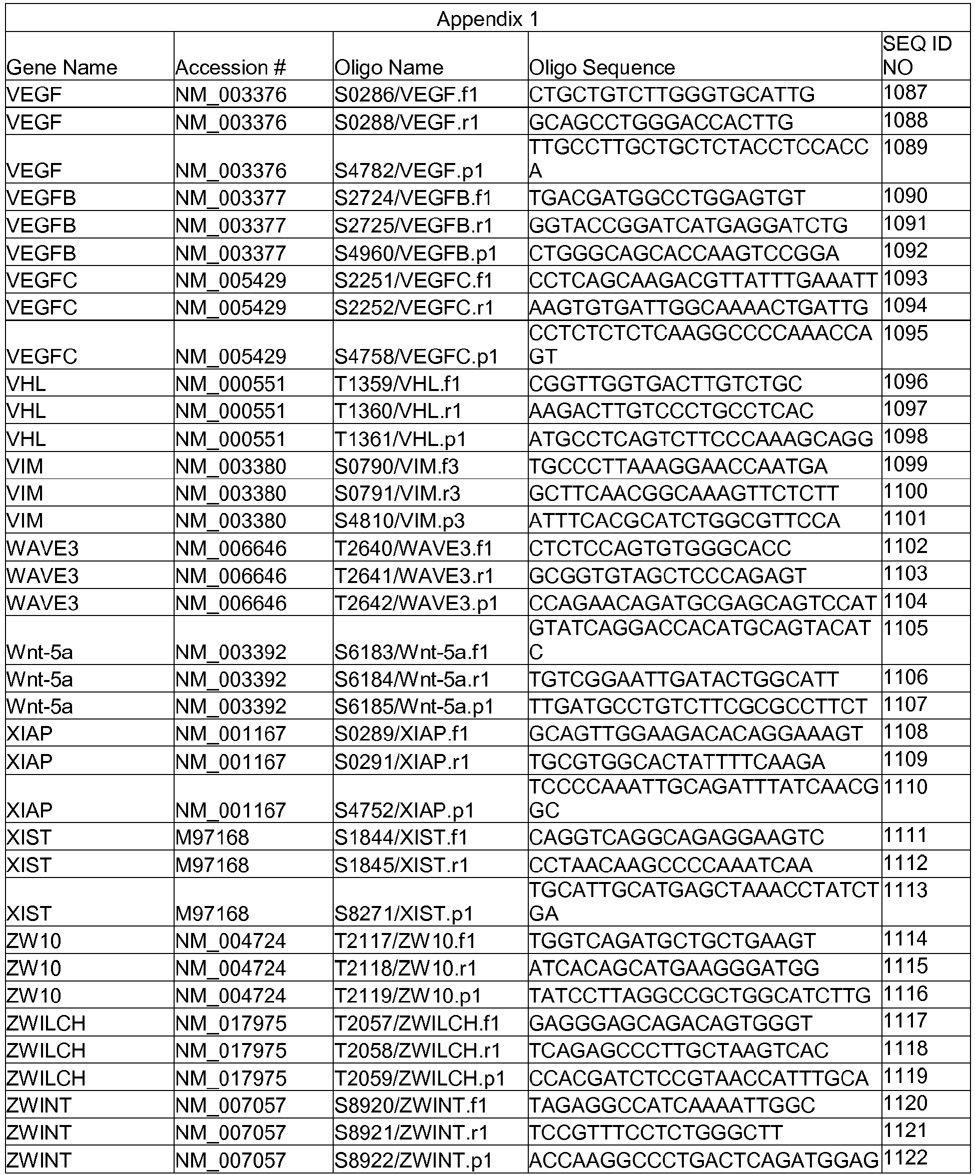

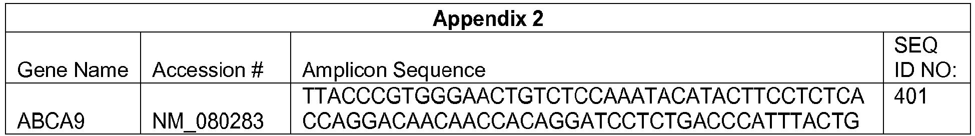

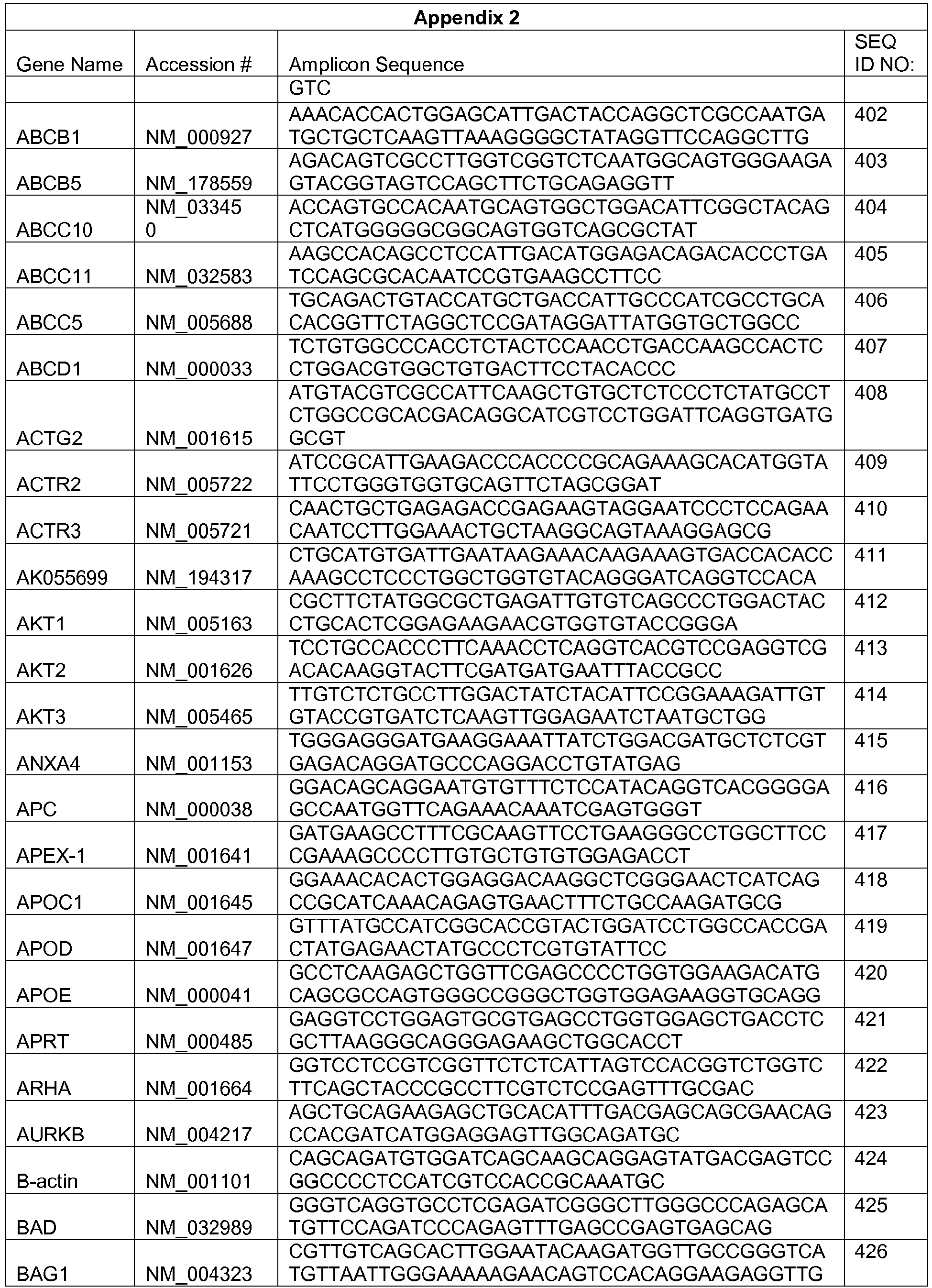

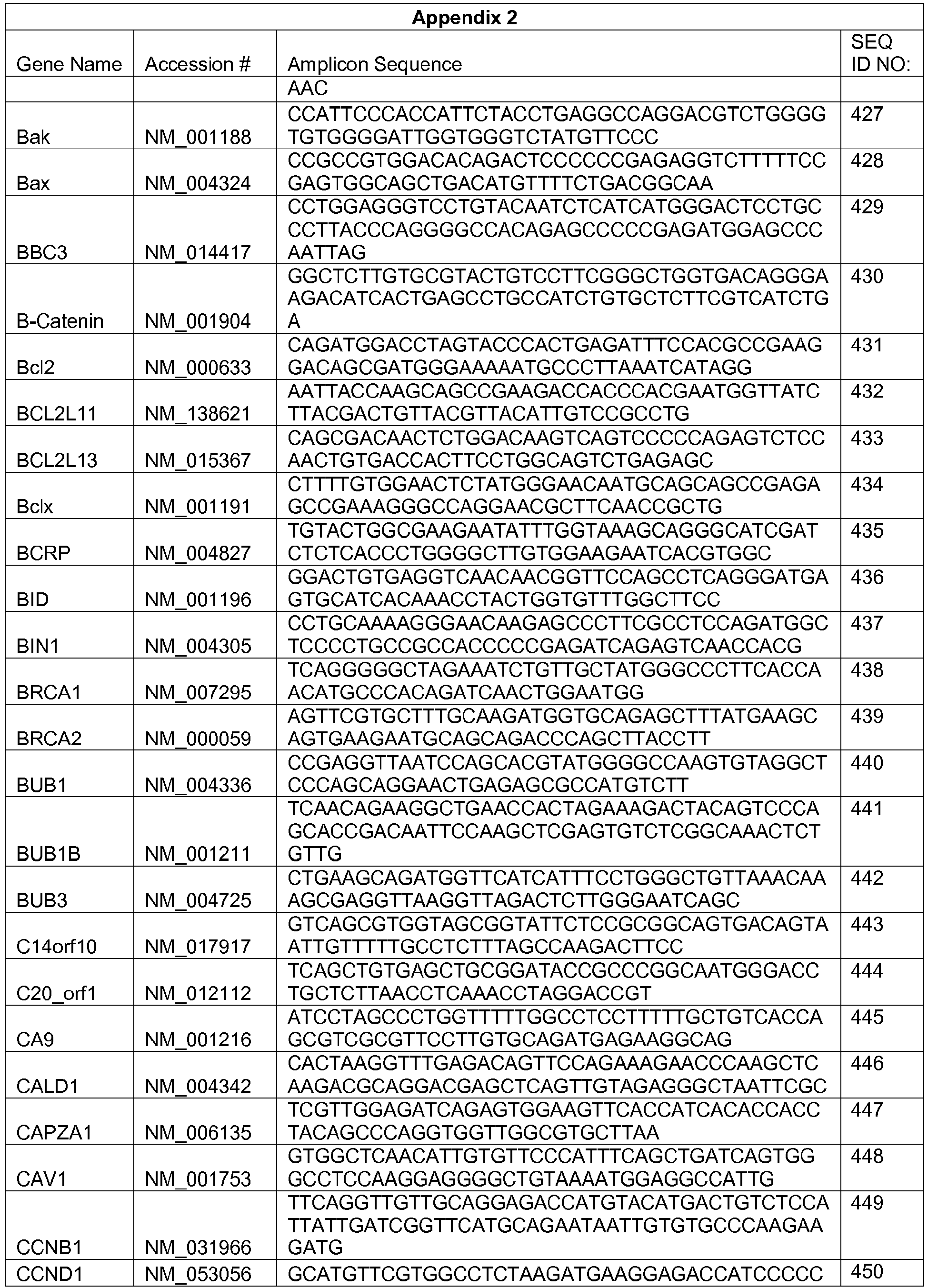

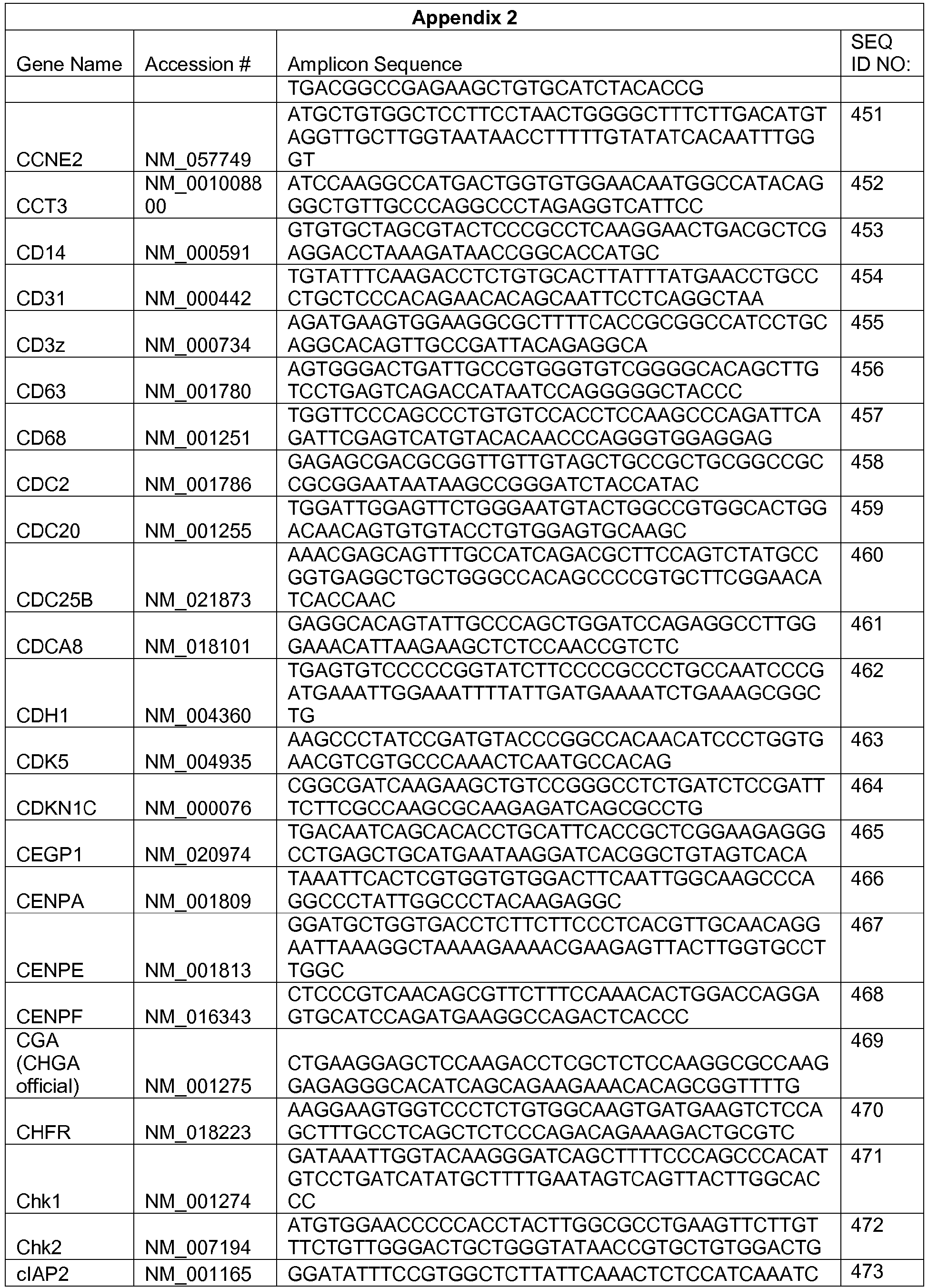

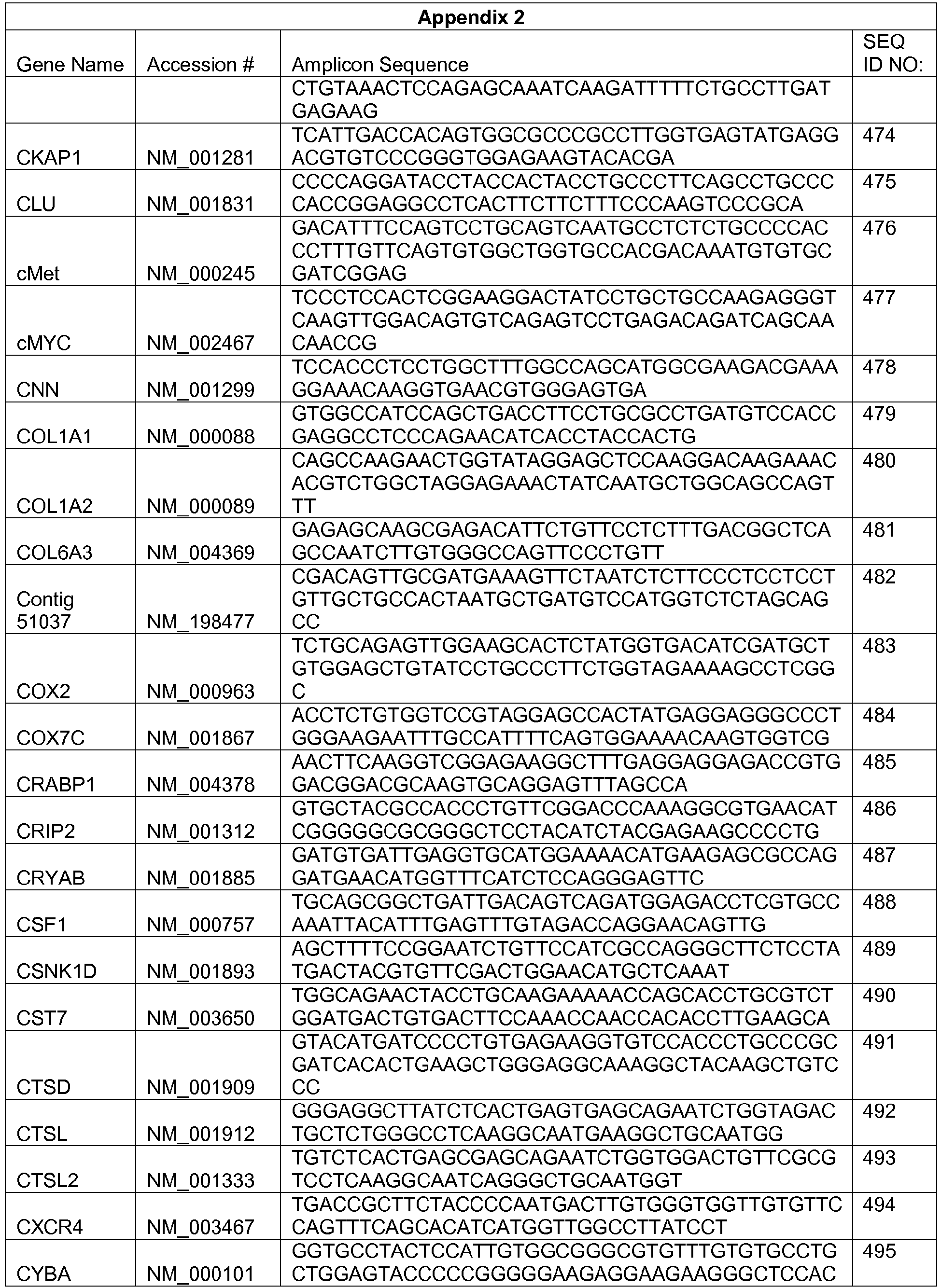

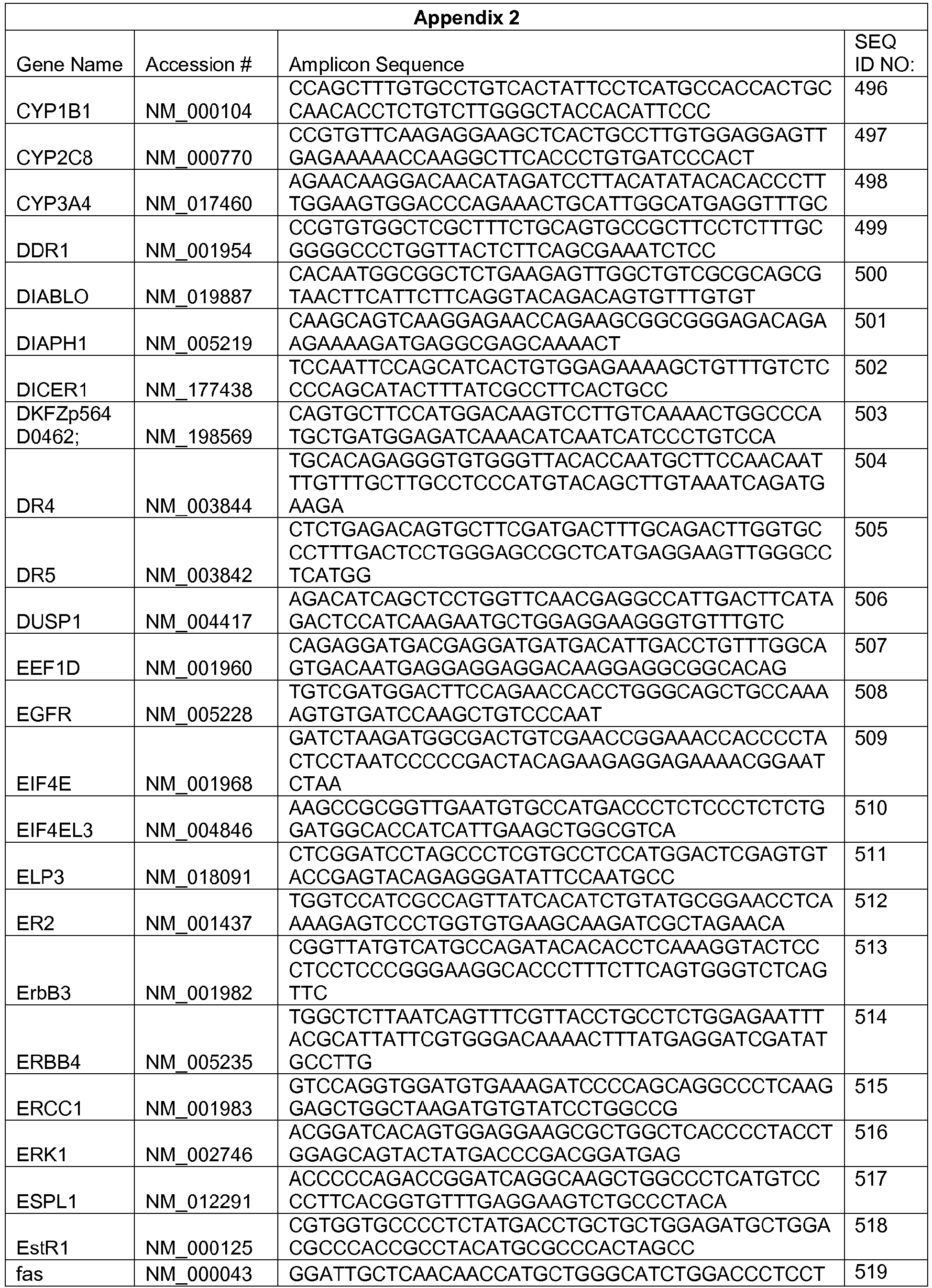

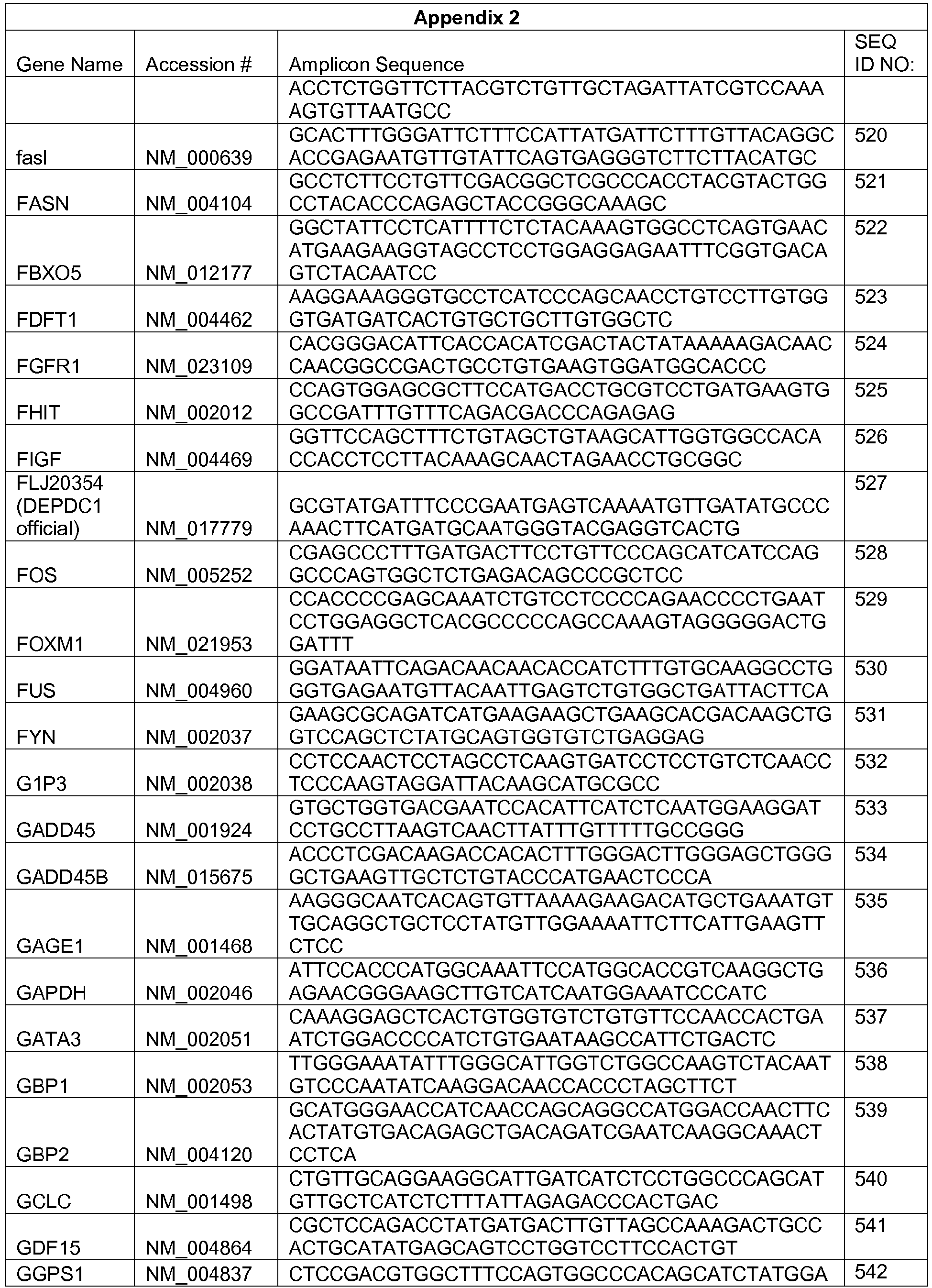

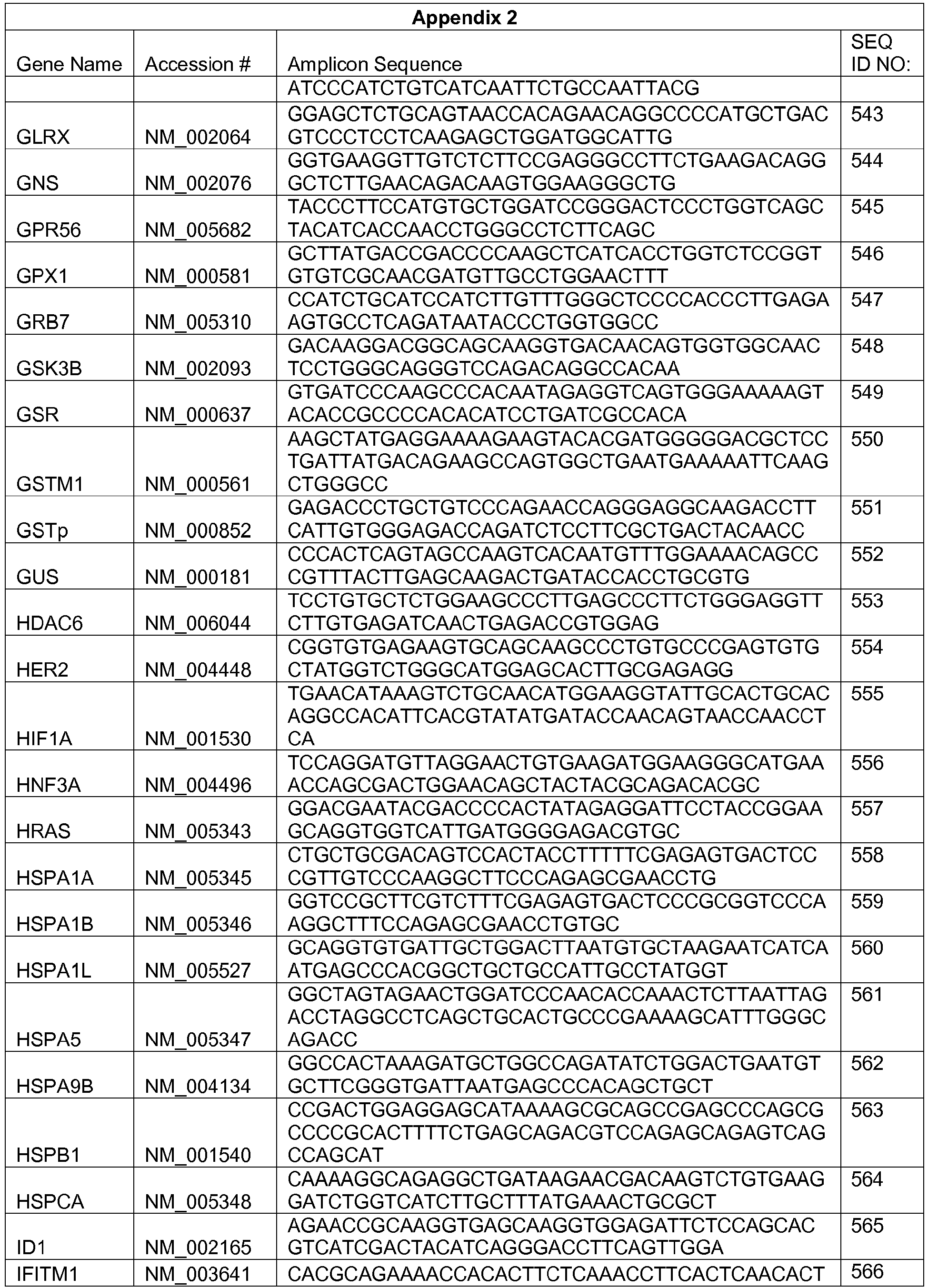

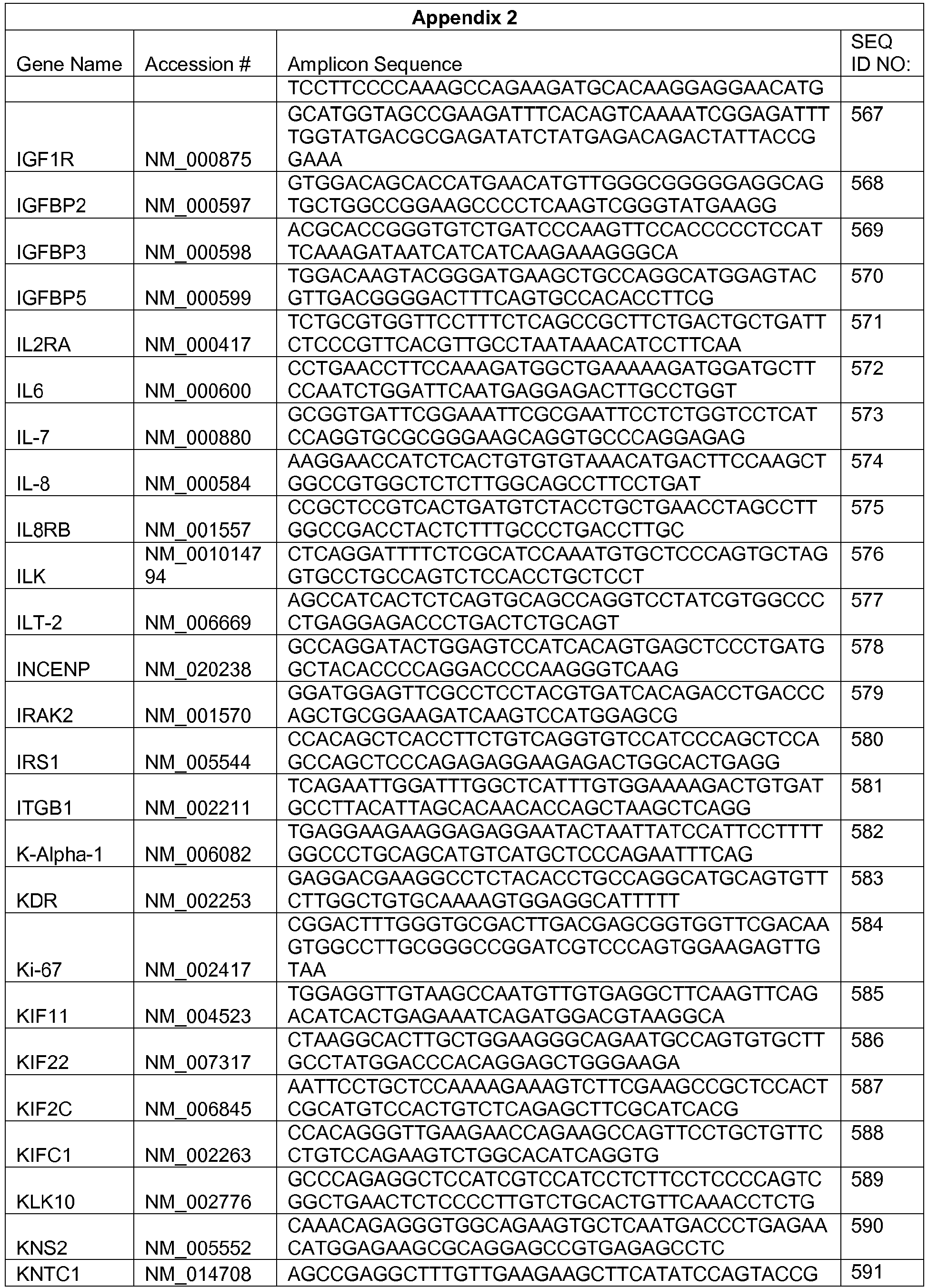

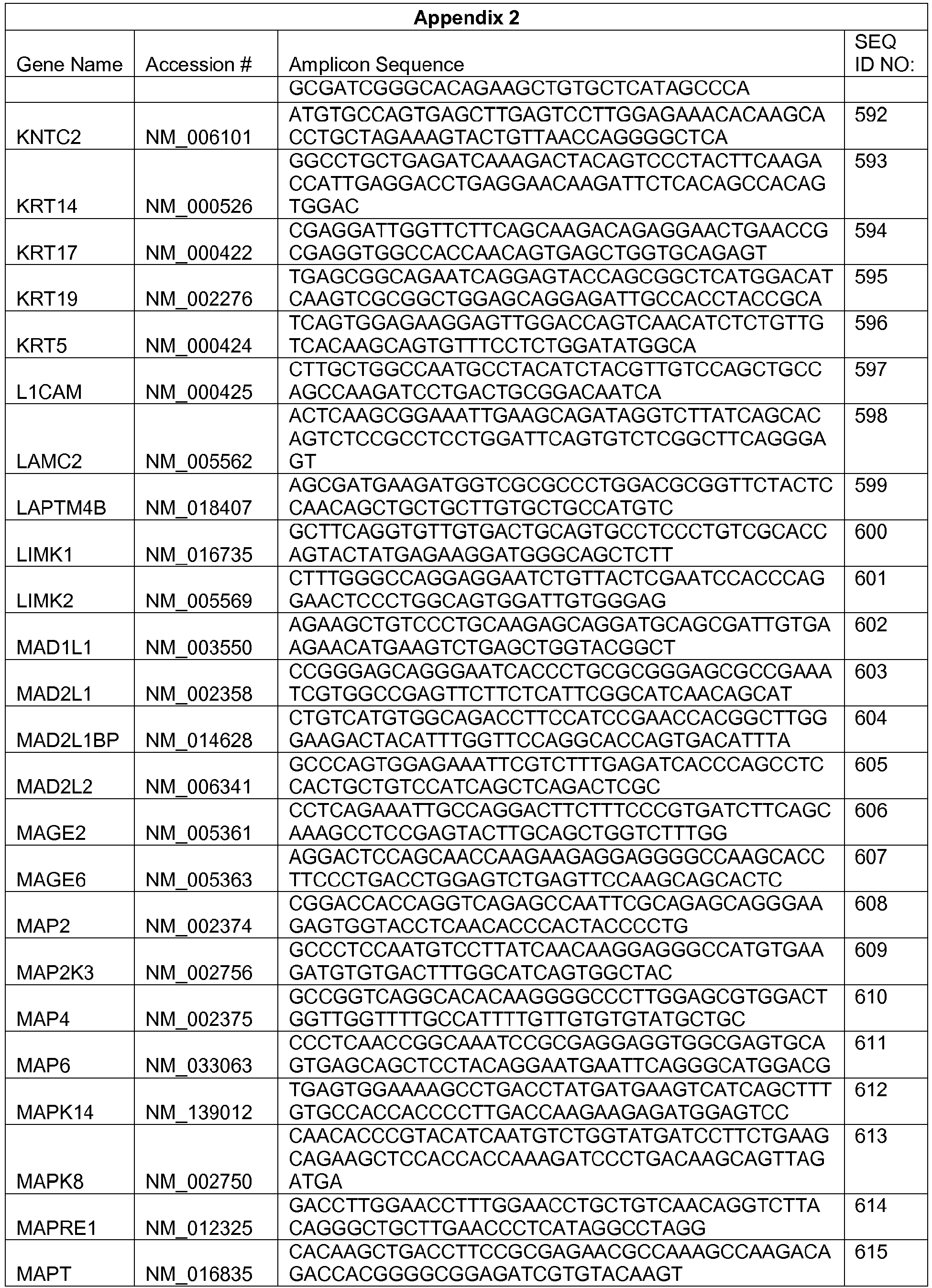

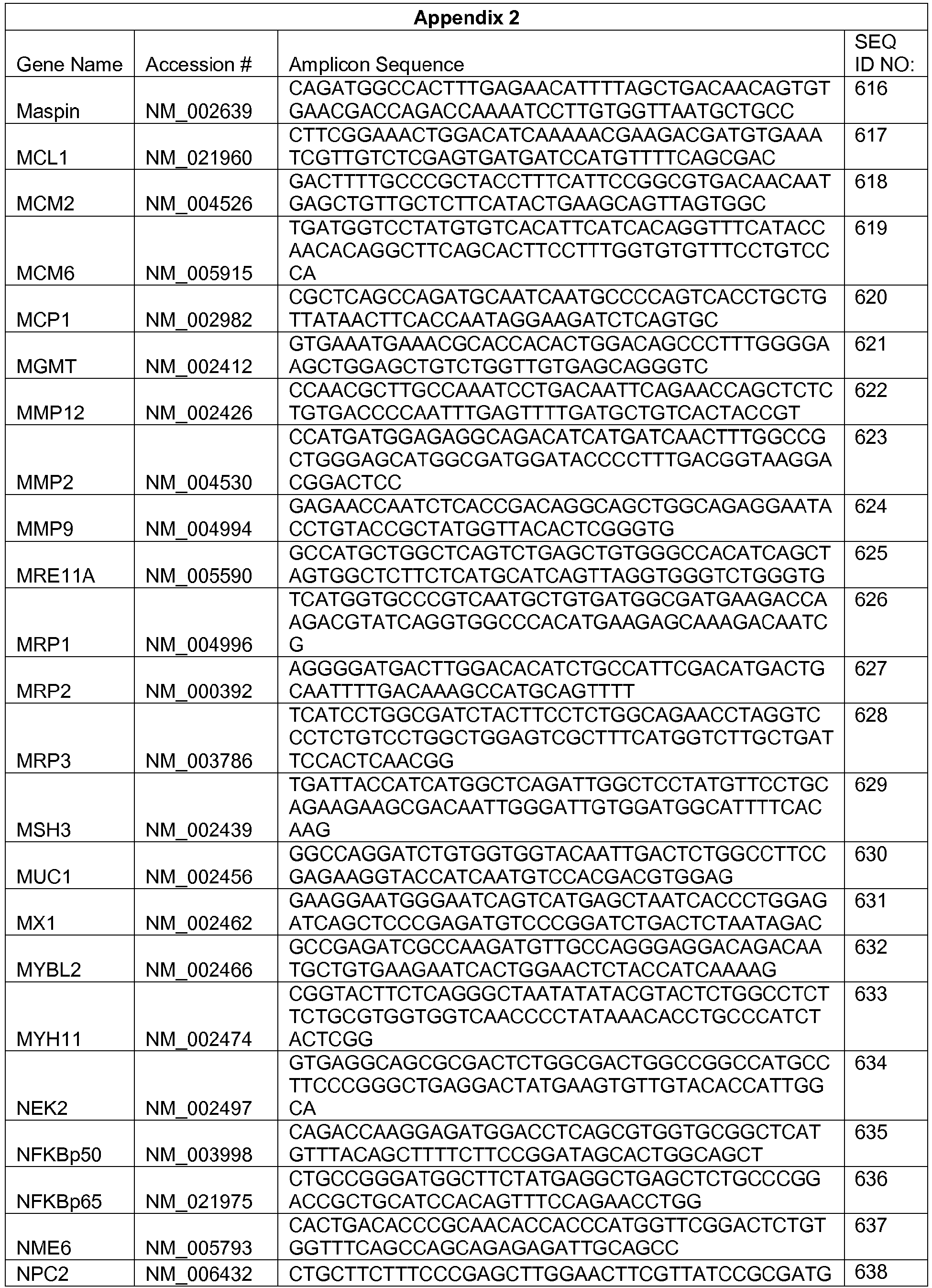

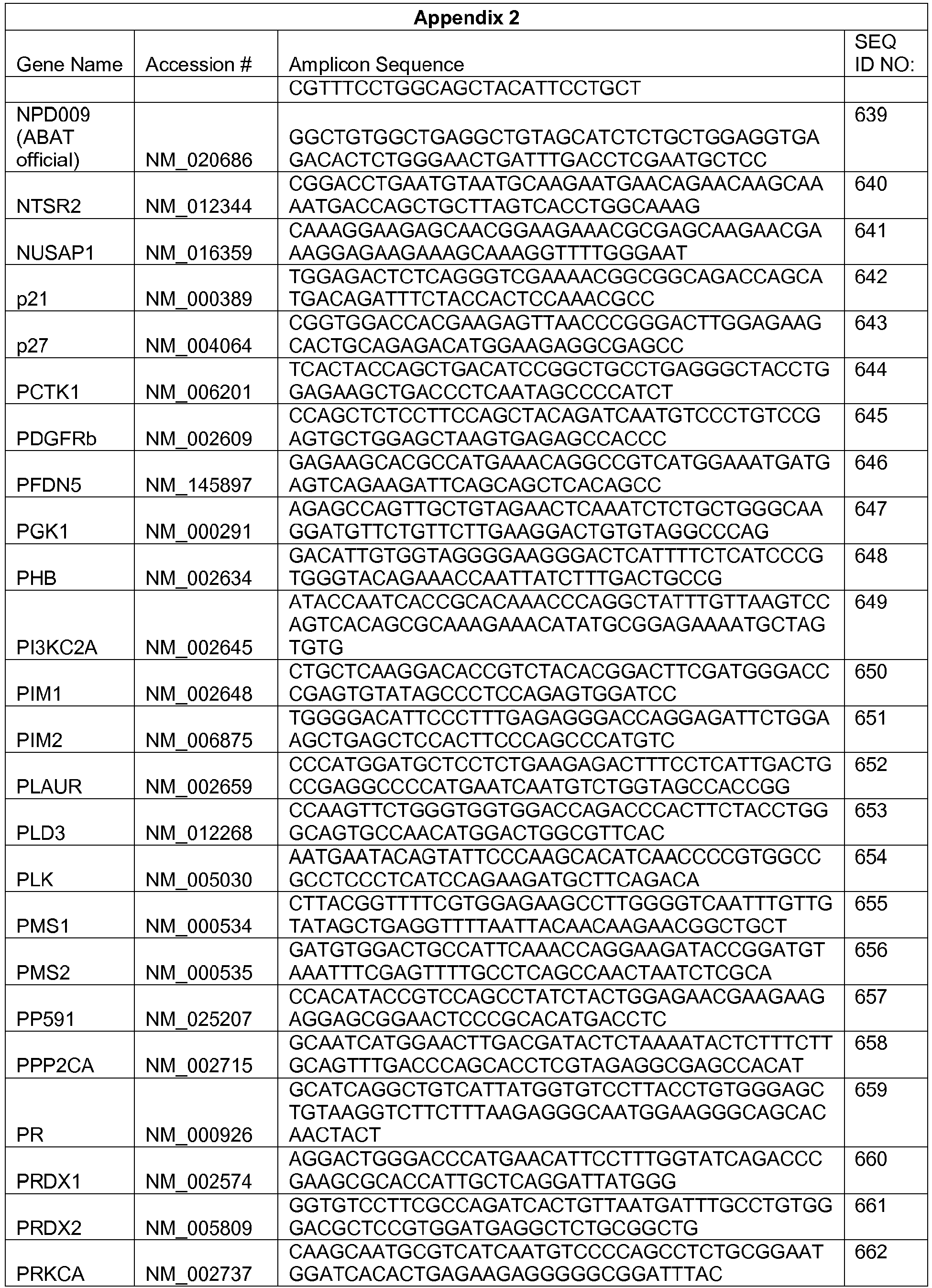

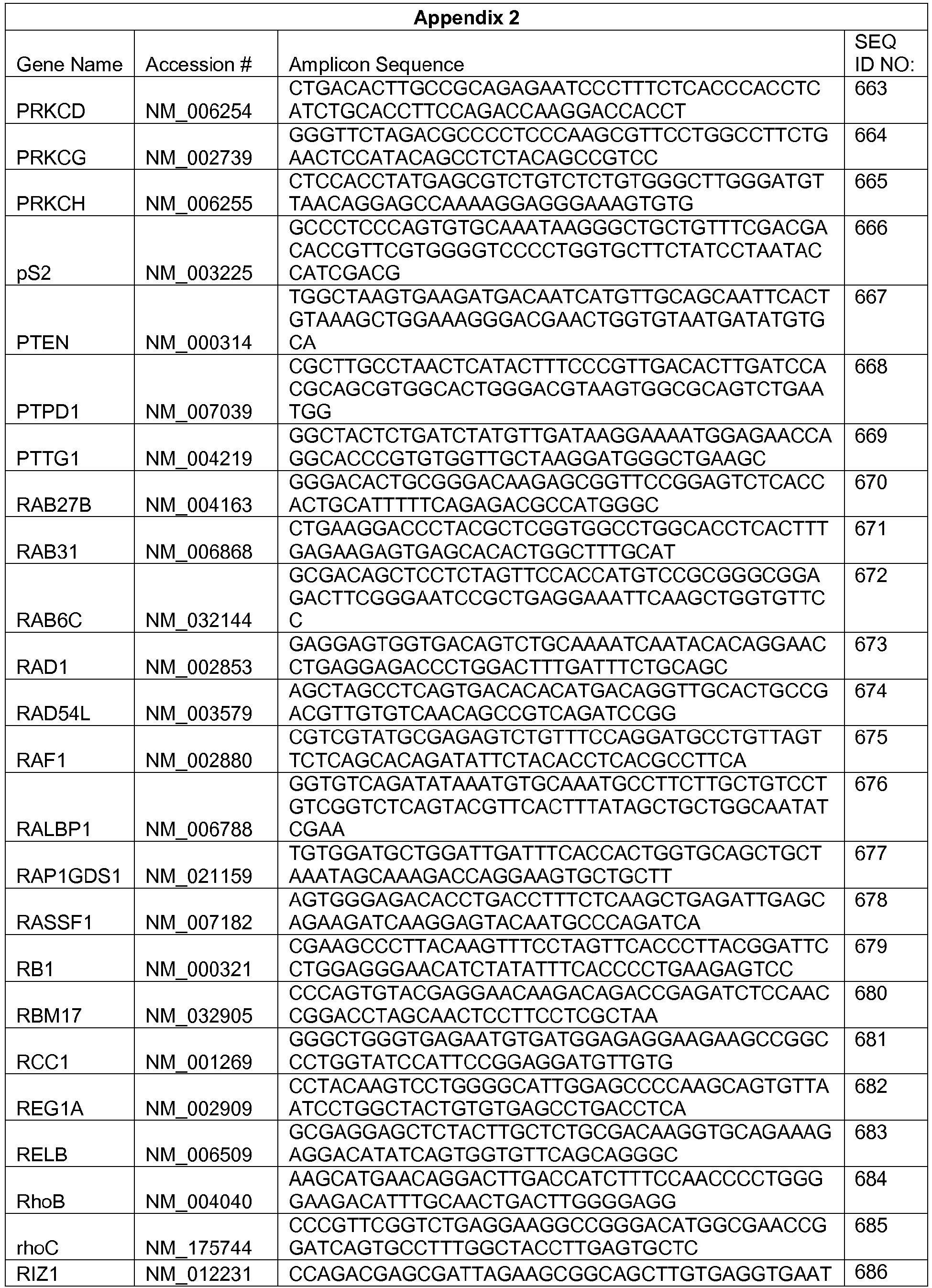

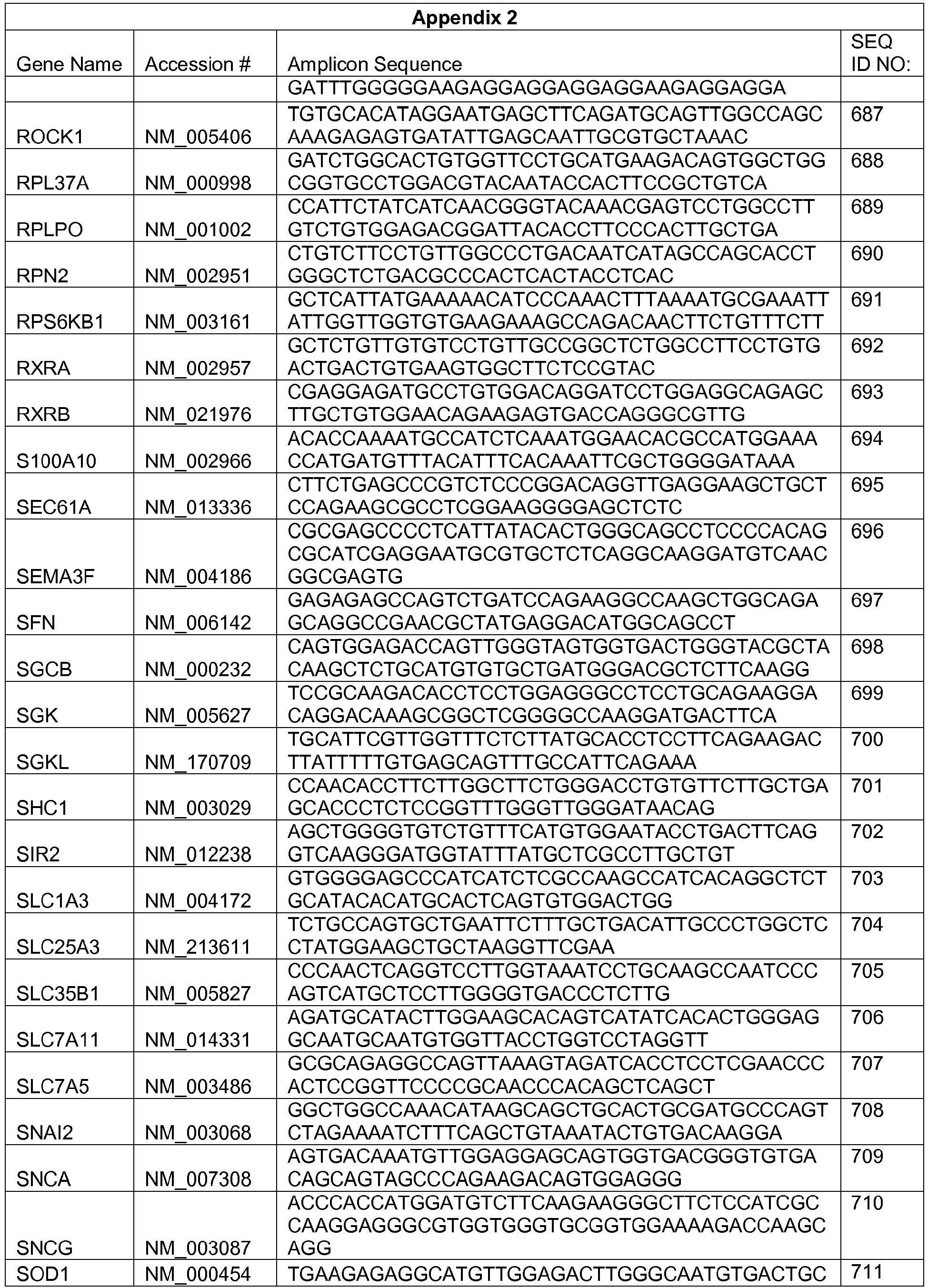

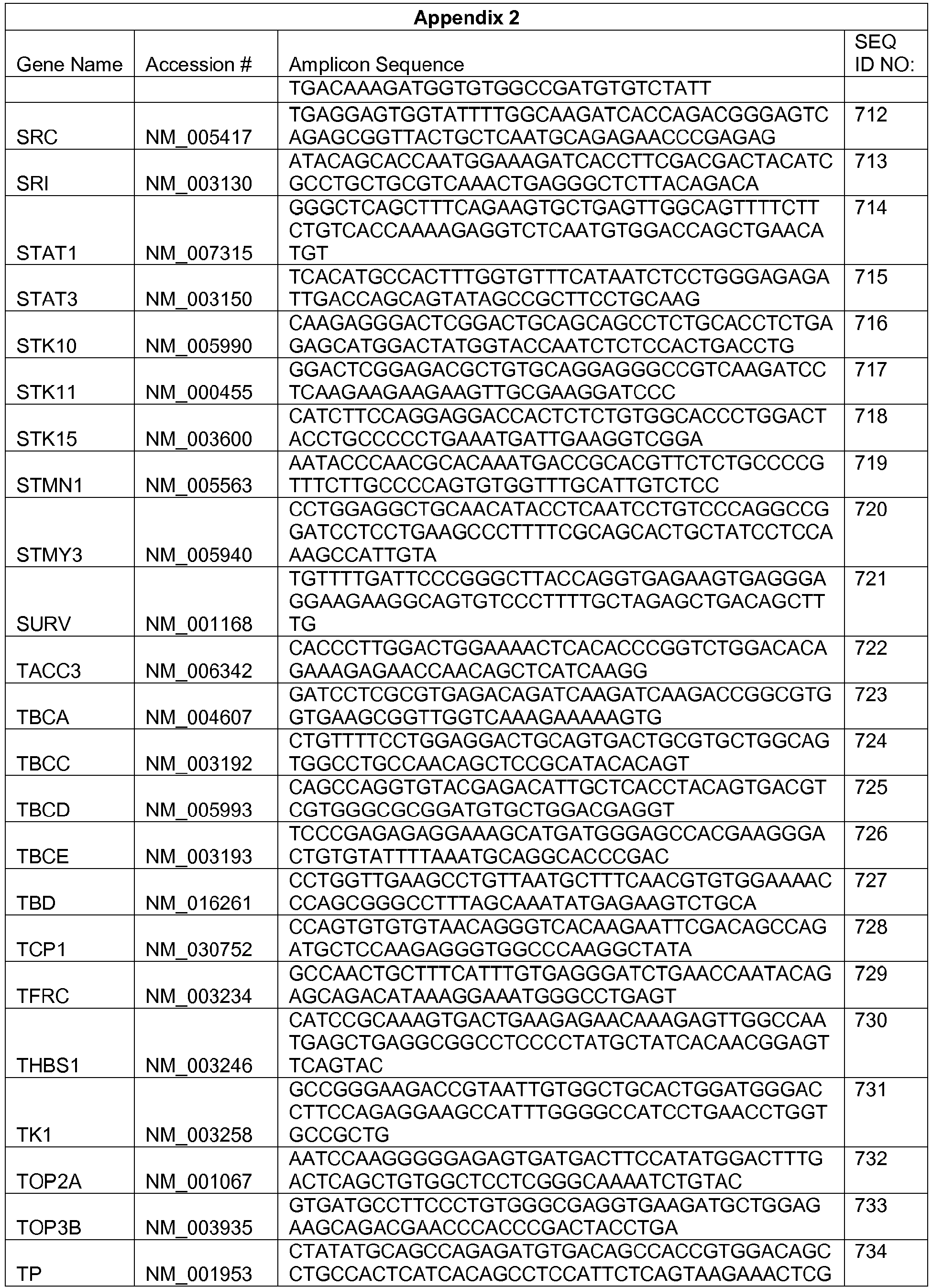

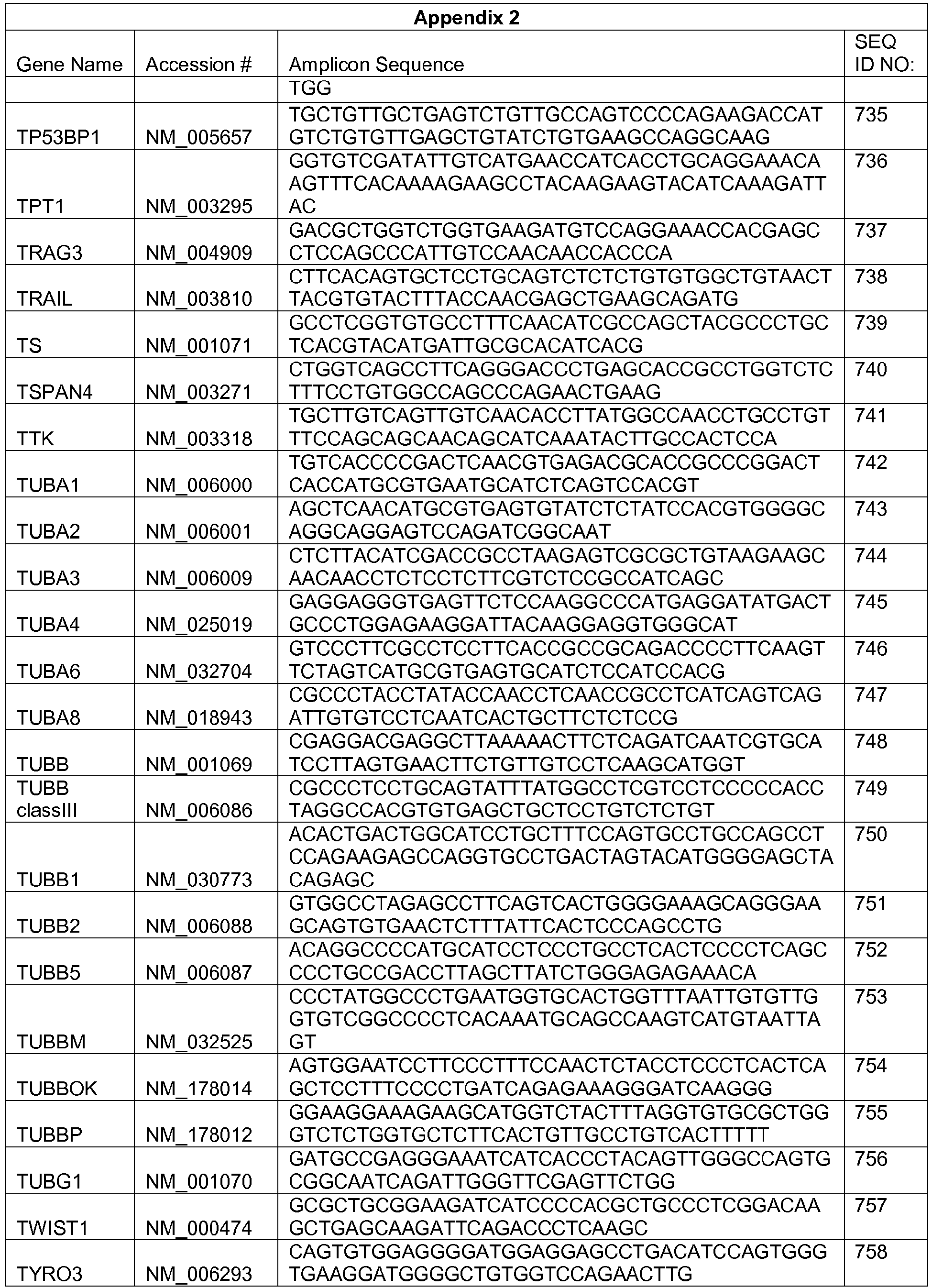

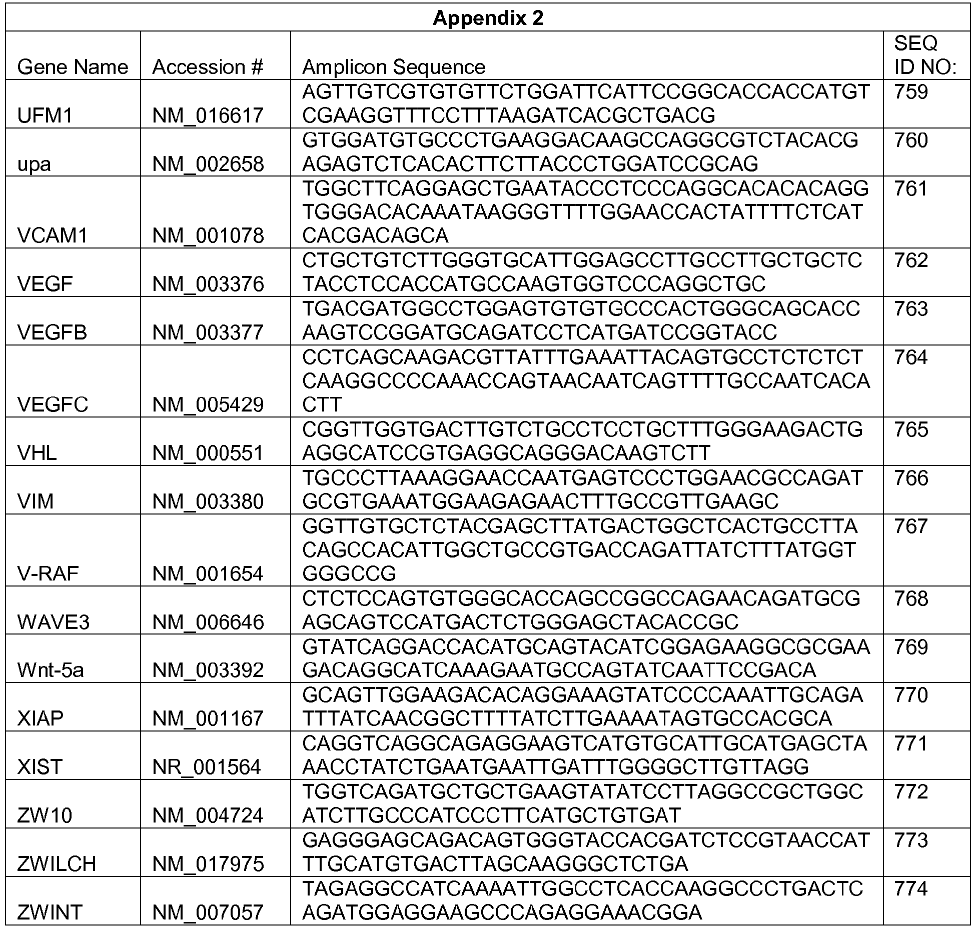

| **Table 1** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | SLC1A3 | 0.0002 | -0.7577 | 27 | RAD1 | 0.0115 | -0.6673 | 52 | ACTR2 | 0.0297 | -0.8754 |
| 2 | TBCC | 0.0006 | -1.0289 | 28 | MRE11A | 0.0120 | -0.6253 | 53 | WNT5A | 0.0321 | 0.5036 |
| 3 | EIF4E2 | 0.0009 | -1.2038 | 29 | DDR1 | 0.0122 | -0.5660 | 54 | HSPA1L | 0.0321 | -1.8702 |
| 4 | TUBB | 0.0017 | -0.7332 | 30 | STK10 | 0.0123 | -0.6002 | 55 | APOC1 | 0.0324 | -0.3434 |
| 5 | TSPAN4 | 0.0027 | -0.7211 | 31 | LILRB1 | 0.0125 | -0.4674 | 56 | ZWINT | 0.0326 | -0.3966 |
| 6 | VHL | 0.0034 | -0.7450 | 32 | BBC3 | 0.0128 | -0.4481 | 57 | APEX1 | 0.0330 | -0.7200 |
| 7 | BAX | 0.0039 | -1.0224 | 33 | BUB3 | 0.0144 | -0.5476 | 58 | KALPHA1 | 0.0351 | -0.7627 |
| 8 | CD247 | 0.0044 | -0.4656 | 34 | CDCA8 | 0.0145 | -0.3759 | 59 | ABCC10 | 0.0354 | -0.5667 |
| 9 | CAPZA1 | 0.0044 | -1.1182 | 35 | TOP3B | 0.0164 | -0.7292 | 60 | PHB | 0.0380 | -0.5832 |
| 10 | STMN1 | 0.0052 | -0.4350 | 36 | RPN2 | 0.0166 | -0.8121 | 61 | TUBB2C | 0.0380 | -0.6664 |
| 11 | ABCC1 | 0.0054 | -0.7653 | 37 | ILK | 0.0169 | -0.6920 | 62 | RALBP1 | 0.0382 | -0.5989 |
| 12 | ZW10 | 0.0055 | -0.8228 | 38 | GBP1 | 0.0170 | -0.3496 | 63 | VEGF | 0.0397 | -0.3673 |
| 13 | HSPA1B | 0.0058 | -0.4740 | 39 | TUBB3 | 0.0173 | -0.3037 | 64 | MCL1 | 0.0398 | -0.6137 |
| 14 | MAPRE1 | 0.0060 | -0.7833 | 40 | NTSR2 | 0.0175 | -2.4355 | 65 | HSPA1A | 0.0402 | -0.3451 |
| 15 | PLD3 | 0.0061 | -0.8595 | 41 | BID | 0.0175 | -0.6228 | 66 | BUB1 | 0.0404 | -0.2911 |
| 16 | APRT | 0.0062 | -0.7714 | 42 | BCL2L13 | 0.0189 | -0.7228 | 67 | MAD2L1 | 0.0412 | -0.3336 |
| 17 | BAK1 | 0.0064 | -0.7515 | 43 | TPX2 | 0.0196 | -0.3148 | 68 | CENPF | 0.0418 | -0.2979 |
| 18 | TUBA6 | 0.0067 | -0.7006 | 44 | ABCC5 | 0.0203 | -0.3906 | 69 | IL2RA | 0.0427 | -0.5023 |
| 19 | CST7 | 0.0069 | -0.4243 | 45 | HDAC6 | 0.0226 | -0.7782 | 70 | TUBA3 | 0.0429 | -0.4528 |
| 20 | SHC1 | 0.0080 | -0.6632 | 46 | CD68 | 0.0226 | -0.6531 | 71 | ACTB | 0.0439 | -0.8259 |
| 21 | ZWILCH | 0.0088 | -0.6902 | 47 | NEK2 | 0.0232 | -0.3657 | 72 | KIF22 | 0.0447 | -0.5427 |
| 22 | SRC | 0.0089 | -0.7011 | 48 | DICER1 | 0.0233 | -0.5537 | 73 | CXCR4 | 0.0462 | -0.4239 |
| 23 | GADD45B | 0.0102 | -0.5253 | 49 | RHOA | 0.0268 | -0.7407 | 74 | STAT1 | 0.0472 | -0.3555 |
| 24 | LIMK2 | 0.0106 | -0.7784 | 50 | TYMS | 0.0291 | -0.3577 | 75 | IL7 | 0.0473 | -0.3973 |
| 25 | CENPA | 0.0106 | -0.3588 | 51 | CCT3 | 0.0292 | -0.5989 | 76 | CHFR | 0.0499 | -0.5387 |
| 26 | CHEK2 | 0.0109 | -0.6737 | | | | | | | | |

| **Table 2** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.2307 | 34 | ILK | 0.0084 | -1.1481 | 66 | RXRA | 0.0247 | -0.7973 |
| 2 | EIF4E2 | 0.0001 | -1.8076 | 35 | NTSR2 | 0.0090 | -4.0522 | 67 | ABCD1 | 0.0259 | -0.7533 |
| 3 | TBCC | 0.0001 | -1.5303 | 36 | TOP3B | 0.0091 | -1.0744 | 68 | MAPK3 | 0.0269 | -0.7322 |
| 4 | STK10 | 0.0005 | -1.2320 | 37 | PLD3 | 0.0095 | -1.1126 | 69 | CDCA8 | 0.0275 | -0.5210 |
| 5 | ZW10 | 0.0006 | -1.3917 | 38 | DICER1 | 0.0095 | -0.8849 | 70 | DUSP1 | 0.0284 | -0.3398 |
| 6 | BBC3 | 0.0010 | -0.9034 | 39 | VHL | 0.0104 | -0.9357 | 71 | ABCC1 | 0.0287 | -0.8003 |
| 7 | BAX | 0.0011 | -1.4992 | 40 | GCLC | 0.0108 | -0.7822 | 72 | PRKCH | 0.0291 | -0.6680 |
| 8 | BAK1 | 0.0011 | -1.3122 | 41 | RAD1 | 0.0108 | -1.0141 | 73 | PRDX1 | 0.0301 | -0.8823 |
| 9 | TSPAN4 | 0.0013 | -1.1930 | 42 | GATA3 | 0.0112 | -0.4400 | 74 | TUBA3 | 0.0306 | -0.7331 |
| 10 | SLC1A3 | 0.0014 | -0.9828 | 43 | CXCR4 | 0.0120 | -0.7032 | 75 | VEGFB | 0.0317 | -0.7487 |
| 11 | SHC1 | 0.0015 | -1.1395 | 44 | NME6 | 0.0121 | -0.9873 | 76 | LILRB1 | 0.0320 | -0.5617 |
| 12 | CHFR | 0.0016 | -1.3371 | 45 | UFM1 | 0.0125 | -0.9686 | 77 | LAPTM4 B | 0.0321 | -0.4994 |
| 13 | RHOB | 0.0018 | -0.7059 | 46 | BUB3 | 0.0126 | -0.9054 | 78 | HSPA9B | 0.0324 | -0.9660 |
| 14 | TUBA6 | 0.0019 | -1.1071 | 47 | CD14 | 0.0130 | -0.8152 | 79 | ECGF1 | 0.0329 | -0.5807 |
| 15 | BCL2L13 | 0.0023 | -1.3181 | 48 | MRE11A | 0.0130 | -0.8915 | 80 | GDF15 | 0.0332 | -0.3646 |
| 16 | MAPRE1 | 0.0029 | -1.2233 | 49 | CST7 | 0.0131 | -0.5204 | 81 | ACTR2 | 0.0347 | -1.1827 |
| 17 | GADD45B | 0.0034 | -0.9174 | 50 | APOC1 | 0.0134 | -0.5630 | 82 | IL7 | 0.0349 | -0.5623 |
| 18 | HSPA1B | 0.0036 | -0.6406 | 51 | GNS | 0.0136 | -1.0979 | 83 | HDAC6 | 0.0380 | -0.9486 |
| 19 | FAS | 0.0037 | -0.8571 | 52 | ABCC5 | 0.0146 | -0.5595 | 84 | ZWILCH | 0.0384 | -0.7296 |
| 20 | TUBB | 0.0040 | -1.0178 | 53 | AKT2 | 0.0150 | -1.0824 | 85 | CHEK2 | 0.0392 | -0.7502 |
| 21 | HSPA1A | 0.0041 | -0.6648 | 54 | APRT | 0.0150 | -0.9231 | 86 | REG1A | 0.0398 | -3.4734 |
| 22 | MCL1 | 0.0041 | -1.1459 | 55 | PLAU | 0.0157 | -0.6705 | 87 | APC | 0.0411 | -0.8324 |
| 23 | CCT3 | 0.0048 | -1.0709 | 56 | RCC1 | 0.0163 | -0.9073 | 88 | SLC35B1 | 0.0411 | -0.6801 |
| 24 | VEGF | 0.0049 | -0.8411 | 57 | CAPZA1 | 0.0165 | -1.3542 | 89 | NEK2 | 0.0415 | -0.4609 |
| 25 | TUBB2C | 0.0051 | -1.4181 | 58 | RELA | 0.0168 | -0.8534 | 90 | ACTB | 0.0418 | -1.1482 |
| 26 | AKT1 | 0.0053 | -1.1175 | 59 | NFKB1 | 0.0179 | -0.9847 | 91 | BUB1 | 0.0423 | -0.4612 |
| 27 | MAD2L1BP | 0.0055 | -1.0691 | 60 | RASSF1 | 0.0186 | -0.8078 | 92 | PPP2CA | 0.0423 | -0.9474 |
| 28 | RPN2 | 0.0056 | -1.2688 | 61 | BCL2L11 | 0.0209 | -0.9394 | 93 | TNFRSF 10A | 0.0448 | -0.6415 |
| 29 | RHOA | 0.0063 | -1.3773 | 62 | CSNK1D | 0.0211 | -1.2276 | 94 | TBCD | 0.0456 | -0.6196 |
| 30 | MAP2K3 | 0.0063 | -0.9616 | 63 | SRC | 0.0220 | -0.8341 | 95 | ERBB4 | 0.0460 | -0.2830 |
| 31 | BID | 0.0067 | -1.0502 | 64 | LIMK2 | 0.0221 | -1.0830 | 96 | CDC25B | 0.0467 | -0.5660 |
| 32 | APOE | 0.0074 | -0.8130 | 65 | SIRT1 | 0.0229 | -0.7236 | 97 | STMN1 | 0.0472 | -0.4684 |
| 33 | ESR1 | 0.0077 | -0.3456 | | | | | | | | |

| **Table 3** | | | | | | | | | | | |
|---|---|---|---|---|---|---|---|---|---|---|---|
| | **Gene** | **p-value** | **Estimated Coefficient** | | **Gene** | **p-value** | **Estimated Coefficient** | | **Gene** | **p-value** | **Estimated Coefficient** |
| 1 | DDR1 | <.0001 | -1.3498 | 56 | APRT | 0.0027 | -1.2629 | 111 | RBM17 | 0.0171 | -1.3981 |
| 2 | ZW10 | <.0001 | -2.1657 | 57 | MAP2K3 | 0.0031 | -1.1297 | 112 | TP53BP1 | 0.0184 | -0.9442 |
| 3 | RELA | <.0001 | -1.5759 | 58 | MGC52057 | 0.0033 | -1.0906 | 113 | CD247 | 0.0188 | -0.5768 |
| 4 | BAX | <.0001 | -1.8857 | 59 | MAPK3 | 0.0033 | -1.0390 | 114 | ABCA9 | 0.0190 | -0.5489 |
| 5 | RHOB | <.0001 | -1.1694 | 60 | APC | 0.0034 | -1.2719 | 115 | NTSR2 | 0.0192 | -3.9043 |
| 6 | TSPAN4 | <.0001 | -1.7067 | 61 | RAD1 | 0.0036 | -1.2744 | 116 | FOS | 0.0195 | -0.4437 |
| 7 | BBC3 | <.0001 | -1.2017 | 62 | COL6A3 | 0.0039 | -0.8240 | 117 | TNFRSF10A | 0.0196 | -0.7666 |
| 8 | SHC1 | <.0001 | -1.4625 | 63 | RXRB | 0.0039 | -1.2638 | 118 | MSH3 | 0.0200 | -0.9585 |
| 9 | CAPZA1 | <.0001 | -2.4068 | 64 | CCT3 | 0.0040 | -1.3329 | 119 | PTEN | 0.0202 | -1.0307 |
| 10 | STK10 | 0.0001 | -1.4013 | 65 | ABCC3 | 0.0040 | -0.8170 | 120 | GBP2 | 0.0204 | -0.6414 |
| 11 | TBCC | 0.0001 | -1.6385 | 66 | GPX1 | 0.0042 | -1.5547 | 121 | STK11 | 0.0206 | -0.9807 |
| 12 | EIF4E2 | 0.0002 | -1.9122 | 67 | TUBB2C | 0.0042 | -1.6184 | 122 | ERBB4 | 0.0213 | -0.3933 |
| 13 | MCL1 | 0.0003 | -1.6617 | 68 | HSPA1A | 0.0043 | -0.7875 | 123 | TFF1 | 0.0220 | -0.2020 |
| 14 | RASSF1 | 0.0003 | -1.3201 | 69 | AKT1 | 0.0045 | -1.1777 | 124 | ABCC1 | 0.0222 | -0.9438 |
| 15 | VEGF | 0.0003 | -1.0800 | 70 | TUBA6 | 0.0046 | -1.2048 | 125 | IL7 | 0.0223 | -0.6920 |
| 16 | SLC1A3 | 0.0004 | -1.0855 | 71 | TOP3B | 0.0048 | -1.1950 | 126 | CDC25B | 0.0228 | -0.7338 |
| 17 | DICER1 | 0.0004 | -1.4236 | 72 | CSNK1D | 0.0049 | -1.6201 | 127 | TUBD1 | 0.0234 | -0.6092 |
| 18 | ILK | 0.0004 | -1.7221 | 73 | SOD1 | 0.0049 | -1.2383 | 128 | BIRC4 | 0.0236 | -0.9072 |
| 19 | FAS | 0.0005 | -1.1671 | 74 | BUB3 | 0.0050 | -1.0111 | 129 | ACTR3 | 0.0246 | -1.3384 |
| 20 | RAB6C | 0.0005 | -1.6154 | 75 | MAP4 | 0.0052 | -1.5220 | 130 | SLC35B1 | 0.0253 | -0.7793 |
| 21 | ESR1 | 0.0006 | -0.4845 | 76 | NFKB1 | 0.0060 | -1.2355 | 131 | COL1A1 | 0.0256 | -0.4945 |
| 22 | MRE11A | 0.0006 | -1.2537 | 77 | SEC61A1 | 0.0060 | -1.4777 | 132 | FOXA1 | 0.0262 | -0.4554 |
| 23 | APOE | 0.0006 | -1.0602 | 78 | MAD1L1 | 0.0060 | -1.1168 | 133 | DUSP1 | 0.0264 | -0.4205 |
| 24 | BAK1 | 0.0006 | -1.4288 | 79 | PRKCH | 0.0073 | -0.8259 | 134 | CXCR4 | 0.0265 | -0.6550 |
| 25 | UFM1 | 0.0006 | -1.4110 | 80 | RXRA | 0.0074 | -0.9693 | 135 | IL2RA | 0.0268 | -0.9731 |
| 26 | AKT2 | 0.0007 | -1.6213 | 81 | PLAU | 0.0074 | -0.7987 | 136 | GGPS1 | 0.0268 | -0.7915 |
| 27 | SIRT1 | 0.0007 | -1.1651 | 82 | CD63 | 0.0074 | -1.3830 | 137 | KNS2 | 0.0281 | -0.8758 |
| 28 | BCL2L13 | 0.0008 | -1.5059 | 83 | CD14 | 0.0075 | -0.9409 | 138 | RB1 | 0.0289 | -0.9291 |
| 29 | ACTR2 | 0.0008 | -1.9690 | 84 | RHOC | 0.0077 | -1.0341 | 139 | BCL2L1 | 0.0289 | -0.9123 |
| 30 | LIMK2 | 0.0009 | -1.6937 | 85 | STAT1 | 0.0093 | -0.7663 | 140 | XIST | 0.0294 | -0.6529 |
| 31 | HDAC6 | 0.0010 | -1.5715 | 86 | NPC2 | 0.0094 | -1.2302 | 141 | BIRC3 | 0.0294 | -0.4739 |
| 32 | RPN2 | 0.0010 | -1.5839 | 87 | NME6 | 0.0095 | -1.2091 | 142 | BID | 0.0303 | -0.8691 |
| 33 | PLD3 | 0.0010 | -1.5460 | 88 | PDGFRB | 0.0096 | -0.7932 | 143 | BCL2 | 0.0303 | -0.5525 |
| 34 | CHGA | 0.0011 | -0.8275 | 89 | MGMT | 0.0098 | -1.0325 | 144 | STAT3 | 0.0311 | -0.9289 |
| 35 | RHOA | 0.0011 | -1.6934 | 90 | GBP1 | 0.0098 | -0.5896 | 145 | PECAM1 | 0.0319 | -0.6803 |
| 36 | MAPK14 | 0.0014 | -1.6611 | 91 | ERCC1 | 0.0105 | -1.2240 | 146 | DIABLO | 0.0328 | -0.9572 |
| 37 | ECGF1 | 0.0014 | -0.8835 | 92 | RCC1 | 0.0107 | -1.0453 | 147 | CYBA | 0.0333 | -0.6642 |
| 38 | MAPRE1 | 0.0016 | -1.3329 | 93 | FUS | 0.0117 | -1.2869 | 148 | TBCE | 0.0336 | -0.7411 |
| 39 | HSPA1B | 0.0017 | -0.8048 | 94 | TUBA3 | 0.0117 | -0.8905 | 149 | CYP1B1 | 0.0337 | -0.6013 |
| 40 | GATA3 | 0.0017 | -0.6153 | 95 | CHEK2 | 0.0120 | -1.0057 | 150 | APEX1 | 0.0357 | -1.0916 |
| 41 | PPP2CA | 0.0017 | -1.6176 | 96 | APOC1 | 0.0123 | -0.6422 | 151 | TBCD | 0.0383 | -0.5893 |
| 42 | ABCD1 | 0.0018 | -1.1669 | 97 | ABCC10 | 0.0124 | -0.9400 | 152 | HRAS | 0.0390 | -0.8411 |
| 43 | MAD2L1BP | 0.0018 | -1.1725 | 98 | SRC | 0.0128 | -1.1170 | 153 | TNFRSF10B | 0.0394 | -0.7293 |
| 44 | VHL | 0.0022 | -1.1855 | 99 | TUBB | 0.0136 | -0.9398 | 154 | ELP3 | 0.0398 | -0.9560 |
| 45 | GCLC | 0.0023 | -1.1240 | 100 | FLAD1 | 0.0139 | -1.0396 | 155 | PIK3C2A | 0.0408 | -0.9158 |
| 46 | ACTB | 0.0023 | -1.8754 | 101 | MAD2L2 | 0.0141 | -1.0834 | 156 | HSPA5 | 0.0417 | -1.5232 |
| 47 | BCL2L11 | 0.0024 | -1.5415 | 102 | LAPTM4B | 0.0149 | -0.5932 | 157 | VEGFC | 0.0427 | -0.7309 |
| 48 | PRDX1 | 0.0025 | -1.3943 | 103 | REG1A | 0.0150 | -5.1214 | 158 | CRABP1 | 0.0440 | 0.2492 |
| 49 | LILRB1 | 0.0025 | -0.8462 | 104 | PRKCD | 0.0152 | -1.0120 | 159 | MMP11 | 0.0456 | -0.3894 |
| 50 | GNS | 0.0025 | -1.3307 | 105 | CST7 | 0.0157 | -0.5499 | 160 | SGK | 0.0456 | -0.6740 |
| 51 | CHFR | 0.0026 | -1.3292 | 106 | IGFBP2 | 0.0161 | -0.5019 | 161 | CTSD | 0.0463 | -0.7166 |
| 52 | CD68 | 0.0026 | -1.1941 | 107 | FYN | 0.0162 | -0.7670 | 162 | BAD | 0.0479 | -0.6436 |
| 53 | LIMK1 | 0.0026 | -1.5655 | 108 | KDR | 0.0168 | -0.8204 | 163 | PTPN21 | 0.0484 | -0.5636 |
| 54 | GADD45B | 0.0027 | -1.0162 | 109 | STMN1 | 0.0169 | -0.6791 | 164 | HSPA9B | 0.0487 | -0.9657 |
| 55 | VEGFB | 0.0027 | -1.1252 | 110 | ZWILCH | 0.0170 | -0.8897 | 165 | PMS1 | 0.0498 | -0.9283 |

| **Table 4** | | | | | | | | | | | |
|---|---|---|---|---|---|---|---|---|---|---|---|
| | Gene | p-value | Estimated Coefficient | | Gene | p-value | Estimated Coefficient | | Gene | p-value | Estimated Coefficient |
| 1 | CD247 | 0.0101 | -0.6642 | 4 | ACTG2 | 0.0280 | -0.2775 | 7 | CHEK2 | 0.0438 | -0.9595 |
| 2 | TYMS | 0.0225 | -0.5949 | 5 | CCND1 | 0.0355 | 0.4802 | 8 | STMN1 | 0.0441 | -0.5369 |
| 3 | IGF1R | 0.0270 | -0.5243 | 6 | CAPZA1 | 0.0401 | -1.1408 | 9 | ZWILCH | 0.0476 | -0.8264 |

| **Table 5** | | | |
|---|---|---|---|
| **Official Symbol** | **Name** | **Entrez** | **Role** |
| CHUK | Conserved helix-loop-helix ubiquitous kinase | 1147 | Activates |
| BCL3 | B-cell CLL/lymphoma 3 | 602 | Transcriptional co-activator |
| FADD | Fas (TNFRSF6)-associated via death domain | 8772 | Stimulates pathway |
| IKBKB | Inhibitor of kappa light polypeptide gene enhancer in B-cells, kinase beta | 3551 | Activates; triggers degradation of NFKBIA, NFKBIB |
| IKBKG | Inhibitor of kappa light polypeptide gene enhancer in B-cells, kinase gamma | 8517 | Activates; triggers degradation of NFKBIA, NFKBIB |
| IL1A | Interleukin 1, alpha | 3552 | Stimulates pathway |
| IL1R1 | Interleukin 1 receptor, type I | 3554 | Stimulates pathway |
| IRAK1 | Interleukin-1 receptor-associated kinase 1 | 3654 | Stimulates pathway |
| NFKB1 | Nuclear factor of kappa light polypeptide gene enhancer in B-cells 1 (p105) | 4790 | Core subunit |
| NFKB2 | Nuclear factor of kappa light polypeptide gene enhancer in B-cells 2 (p49/p100) | 4791 | Core subunit |
| NFKBIA | Nuclear factor of kappa light polypeptide gene enhancer in B-cells inhibitor, alpha | 4792 | Inhibits |
| NFKBIB | Nuclear factor of kappa light polypeptide gene enhancer in B-cells inhibitor, beta | 4793 | Inhibits |
| NFKBIE | nuclear factor of kappa light polypeptide gene enhancer in B-cells inhibitor, epsilon | 4794 | Inhibits |
| REL | v-rel reticuloendotheliosis viral oncogene homolog (avian) | 5966 | Transcriptional co-activator |
| RELA | V-rel reticuloendotheliosis viral oncogene homolog A, nuclear factor of kappa light polypeptide gene enhancer in B-cells 3, p65 (avian) | 5970 | Transcriptional co-activator |
| RELB | v-rel reticuloendotheliosis viral oncogene homolog B, nuclear factor of kappa light polypeptide gene enhancer in B-cells 3 (avian) | 5971 | Transcriptional co-activator |
| RHOC | ras homolog gene family, member C | 389 | Induce activation of pathway |
| TNFAIP3 | Tumor necrosis factor, alpha-induced protein 3 | 7128 | Activates |
| TNFRSF1A | Tumor necrosis factor receptor superfamily, member 1A | 7132 | Activates |
| TNFRSF1B | TNFRSF1A-associated via death domain | 7133 | Activates |
| TRAF6 | TNF receptor-associated factor 6 | 7189 | Activates CHUK |

According to a further aspect of the present invention, there is provided a method of predicting whether a hormone receptor (HR) positive cancer patient will exhibit a beneficial response to chemotherapy, comprising measuring an expression level of a gene, or its expression product, in a tumour sample obtained from the patient, wherein the gene is selected from the group consisting of ABCC1, ABCC5, ABCD1, ACTB, ACTR2, AKT1, AKT2, APC, APOC1, APOE, APRT, BAK1, BAX, BBC3, BCL2L11, BCL2L13, BID, BUB1, BUB3, CAPZA1, CCT3, CD14, CDC25B, CDCA8, CHEK2, CHFR, CSNK1D, CST7, CXCR4, DDR1, DICER1, DUSP1, ECGF1, EIF4E2, ERBB4, ESR1, FAS, GADD45B, GATA3, GCLC, GDF15, GNS, HDAC6, HSPA1A, HSPA1B, HSPA9B, IL7, ILK, LAPTM4B, LILRB1, LIMK2, MAD2L1BP, MAP2K3, MAPK3, MAPRE1, MCL1, MRE11A, NEK2, NFKB1, NME6, NTSR2, PLAU, PLD3, PPP2CA, PRDX1, PRKCH, RAD1, RASSF1, RCC1, REG1A, RELA, RHOA, RHOB, RPN2, RXRA, SHC1, SIRT1, SLC1A3, SLC35B1, SRC, STK10, STMN1, TBCC, TBCD, TNFRSF10A, TOP3B, TSPAN4, TUBA3, TUBA6, TUBB, TUBB2C, UFM1, VEGF, VEGFB, VHL, ZW10, and ZWILCH; using the expression level to determine a likelihood of a beneficial response to a treatment including a taxane, wherein expression of DDR1, EIF4E2, TBCC, STK10, ZW10, BBC3, BAX, BAK1, TSPAN4, SLC1A3, SHC1, CHFR, RHOB, TUBA6, BCL2L13, MAPRE1, GADD45B, HSPA1B, FAS, TUBB, HSPA1A, MCL1, CCT3, VEGF, TUBB2C, AKT1, MAD2L1BP, RPN2, RHOA, MAP2K3, BID, APOE ,ESR1, ILK, NTSR2, TOP3B, PLD3, DICER1, VHL, GCLC, RAD1, GATA3, CXCR4, NME6, UFM1, BUB3, CD14, MRE11A, CST7, APOC1, GNS, ABCC5, AKT2, APRT, PLAU, RCC1, CAPZA1, RELA, NFKB1, RASSF1, BCL2L11, CSNK1D, SRC, LIMK2, SIRT1, RXRA, ABCD1, MAPK3, DUSP1, ABCC1, PRKCH, PRDX1, TUBA3, VEGFB, LILRB1, LAPTM4B, HSPA9B, ECGF1, GDF15, ACTR2, IL7, HDAC6, CHEK2, REG1A, APC, SLC35B1, ACTB, PPP2CA, TNFRSF10A, TBCD, ERBB4, CDC25B, or STMN1 is positively correlated with increased likelihood of a beneficial response to a treatment including a taxane, and wherein expression of CDCA8, ZWILCH, NEK2, or BUB1 is negatively correlated with an increased likelihood of a beneficial response to a treatment including a taxane; and generating a report including information based on the likelihood of a beneficial response to chemotherapy including a taxane. The method may comprise using the expression level to determine a likelihood of a beneficial response to a treatment including a cyclophosphamide, wherein expression of ZW10, BAX, GADD45B, FAS, ESR1, NME6, MRE11A, AKT2, RELA, RASSF1, PRKCH, VEGFB, LILRB1, ACTR2, REG1A, or PPP2CA is positively correlated with increased likelihood of a beneficial response to a treatment including a cyclophosphamide, and wherein expression of DDR1, EIF4E2, TBCC, STK10, BBC3, BAK1, TSPAN4, SHC1, CHFR, RHOB, TUBA6, BCL2L13, MAPRE1, HSPA1, TUBB, HSPA1A, MCL1, CCT3, VEGF, TUBB2C, AKT1, MAD2L1BP, RPN2, RHOA, MAP2K3, BID, APOE, ILK, NTSR2, TOP3B, PLD3, DICER1, VHL, GCLC, RAD1, GATA3, CXCR4, UFM1, BUB3, CD14, CST7, APOC1, GNS, ABCC5, APRT, PLAU, RCC1, CAPZA1, NFKB1, BCL2L11, CSNK1D, SRC, LIMK2, SIRT1, RXRA, ABCD1, MAPK3, CDCA8, DUSP1, ABCC1, PRDX1, TUBA3, LAPTM4B, HSPA9B, ECGF1, GDF15, IL7, HDAC6, ZWILCH, CHEK2, APC, SLC35B1, NEK2, ACTB, BUB1, TNFRSF10A, TBCD, ERBB4, CDC25B, or STMN1 is negatively correlated with an increased likelihood of a beneficial response to a treatment including a cyclophosphamide, and wherein the report includes information based on the likelihood of a beneficial response to chemotherapy including a cyclophosphamide.

The chemotherapy may include an anthracycline, such as doxorubicin.

The taxane may be docetaxel.

The measuring may be by quantitative PCR.

The measuring may be by detection of an intron-based sequence of an RNA transcript of the gene, wherein the expression of which correlates with the expression of a corresponding exon sequence.

The tumour sample may be a formalin-fixed and paraffin-embedded (FPE) or a frozen tumour section.

According to another aspect, there is provided a method of predicting whether a hormone receptor (HR) positive cancer patient will exhibit a beneficial response to chemotherapy, comprising measuring an expression level of a gene, or its expression product, in a tumour sample obtained from the patient, wherein the gene is selected from the group consisting of ABCA9, ABCC1, ABCC10, ABCC3, ABCD1, ACTB, ACTR2, ACTR3, AKT1, AKT2, APC, APEX1, APOC1, APOE, APRT, BAD, BAK1, BAX, BBC3, BCL2, BCL2L1, BCL2L11, BCL2L13, BID, BIRC3, BIRC4, BUB3, CAPZA1, CCT3, CD14, CD247, CD63, CD68, CDC25B, CHEK2, CHFR, CHGA, COL1A1, COL6A3, CRABP1, CSNK1D, CST7, CTSD, CXCR4, CYBA, CYP1B1, DDR1, DIABLO, DICER1, DUSP1, ECGF1, EIF4E2, ELP3, ERBB4, ERCC1, ESR1, FAS, FLAD1, FOS, FOXA1, FUS, FYN, GADD45B, GATA3, GBP1, GBP2, GCLC, GGPS1, GNS, GPX1, HDAC6, HRAS, HSPA1A, HSPA1B, HSPA5, HSPA9B, IGFBP2, IL2RA, IL7, ILK, KDR, KNS2, LAPTM4B, LILRB1, LIMK1, LIMK2, MAD1L1, MAD2L1BP, MAD2L2, MAP2K3, MAP4, MAPK14, MAPK3, MAPRE1, MCL1, MGC52057, MGMT, MMP11, MRE11A, MSH3, NFKB1, NME6, NPC2, NTSR2, PDGFRB, PECAM1, PIK3C2A, PLAU, PLD3, PMS1, PPP2CA, PRDX1, PRKCD, PRKCH, PTEN, PTPN21, RAB6C, RAD1, RASSF1, RB1, RBM17, RCC1, REG1A, RELA, RHOA, RHOB, RHOC, RPN2, RXRA, RXRB, SEC61A1, SGK, SHC1, SIRT1, SLC1A3, SLC35B1, SOD1, SRC, STAT1, STAT3, STK10, STK11, STMN1, TBCC, TBCD, TBCE, TFF1, TNFRSF10A, TNFRSF10B, TOP3B, TP53BP1, TSPAN4, TUBA3, TUBA6, TUBB, TUBB2C, TUBD1, UFM1, VEGF, VEGFB, VEGFC, VHL, XIST, ZW10, and ZWILCH; using the expression level to determine a likelihood of a beneficial response to a treatment including a taxane, wherein expression of DDR1, ZW10, RELA, BAX, RHOB, TSPAN4, BBC3, SHC1, CAPZA1, STK10, TBCC, EIF4E2, MCL1, RASSF1, VEGF, SLC1A3, DICER1, ILK, FAS, RAB6C, ESR1, MRE11A, APOE, BAK1, UFM1, AKT2, SIRT1, BCL2L13, ACTR2, LIMK2, HDAC6, RPN2, PLD3, RHOA, MAPK14, ECGF1, MAPRE1, HSPA1B, GATA3, PPP2CA, ABCD1, MAD2L1BP, VHL, GCLC, ACTB, BCL2L11, PRDX1, LILRB1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, PRKCH, RXRA, PLAU, CD63, CD14, RHOC, STAT1, NPC2, NME6, PDGFRB, MGMTI, GBP1, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, RBM17, TP53BP1, CD247, ABCA9, NTSR2, FOS, TNFRSF10A, MSH3, PTEN, GBP2, STK11, ERBB4, TFF1, ABCC1, IL7, CDC25B, TUBD1, BIRC4, ACTR3, SLC35B1, COL1A1, FOXA1, DUSP1, CXCR4, IL2RA, GGPS1, KNS2, RB1, BCL2L1, XIST, BIRC3, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS1 is positively correlated with increased likelihood of a beneficial response to a treatment including a taxane, and wherein expression of CHGA, ZWILCH, or CRABP1 is negatively correlated with an increased likelihood of a beneficial response to a treatment including a taxane; and generating a report including information based on the likelihood of a beneficial response to chemotherapy including a taxane.

The method may comprise using the expression level to determine a likelihood of a beneficial response to a treatment including a cyclophosphamide, wherein expression of LILRB1, PRKCH, STAT1, GBP1, CD247, IL7, IL2RA, BIRC3, or CRABP1 is positively correlated with increased likelihood of a beneficial response to a treatment including a cyclophosphamide, and wherein expression of DDR1, ZW10, RELA, BAX, RHOB, TSPAN4, BBC3, SHC1, CAPZA1, STK10, TBCC, EIF4E2, MCL1, RASSF1, VEGF, DICER1, ILK, FAS, RAB6C, ESR1, MRE11A, APOE, BAK1, UFM1, AKT2, SIRT1, BCL2L13, ACTR2, LIMK2, HDAC6, RPN2, PLD3, CHGA, RHOA, MAPK14, ECGF1, MAPRE1, HSPA1B, GATA3, PPP2CA, ABCD1, MAD2L1BP, VHL, GCLC, ACTB, BCL2L11, PRDX1, GNS, CHFR, CD68, LIMK1, GADD45B, VEGFB, APRT, MAP2K3, MGC52057, MAPK3, APC, RAD1, COL6A3, RXRB, CCT3, ABCC3, GPX1, TUBB2C, HSPA1A, AKT1, TUBA6, TOP3B, CSNK1D, SOD1, BUB3, MAP4, NFKB1, SEC61A1, MAD1L1, RXRA, PLAU, CD63, CD14, RHOC, NPC2, NME6, PDGFRB, MGMTI, ERCC1, RCC1, FUS, TUBA3, CHEK2, APOC1, ABCC10, SRC, TUBB, FLAD1, MAD2L2, LAPTM4B, REG1A, PRKCD, CST7, IGFBP2, FYN, KDR, STMN1, ZWILCH, RBM17, TP53BP1, ABCA9, NTSR2, FOS, TNFRSF10A, MSH3, PTEN, GBP2, STK11, ERBB4, TFF1, ABCC1, CDC25B, TUBD1, BIRC4, ACTR3, SLC35B1, COL1A1, FOXA1, DUSP1, CXCR4, GGPS1, KNS2, RB1, BCL2L1, XIST, BID, BCL2, STAT3, PECAM1, DIABLO, CYBA, TBCE, CYP1B1, APEX1, TBCD, HRAS, TNFRSF10B, ELP3, PIK3C2A, HSPA5, VEGFC, MMP11, SGK, CTSD, BAD, PTPN21, HSPA9B, or PMS 1 is negatively correlated with an increased likelihood of a beneficial response to a treatment including a cyclophosphamide, and wherein the report includes information based on the likelihood of a beneficial response to chemotherapy including a cyclophosphamide.

The chemotherapy may include an anthracycline, such as doxorubicin.

The taxane may be docetaxel.

According to a further aspect of the present invention, there is provided a method of predicting whether a hormone receptor (HR) negative cancer patient will exhibit a beneficial response to chemotherapy, comprising measuring an expression level of a gene, or its expression product, in a tumour sample obtained from the patient, wherein the gene is selected from the group consisting of CD247, TYMS, IGF1R, ACTG2, CCND1, CAPZA1, CHEK2, STMN1, and ZWILCH using the expression level to determine a likelihood of a beneficial response to a treatment including a taxane, wherein expression of CD247, TYMS, IGF1R, ACTG2, CAPZA1, CHEK2, STMN1, or ZWILCH is positively correlated with increased likelihood of a beneficial response to a treatment including a taxane, and wherein expression of CCND1 is negatively correlated with an increased likelihood of a beneficial response to a treatment including a taxane; and generating a report including information based on the likelihood of a beneficial response to chemotherapy including a taxane.

The method may comprise using the expression level to determine a likelihood of a beneficial response to a treatment including a cyclophosphamide, wherein expression of CD247, CCND1, or CAPZA1 is positively correlated with increased likelihood of a beneficial response to a treatment including a cyclophosphamide, and wherein expression of TYMS, IGF1R, ACTG2, CHEK2, STMN1, or ZWILCH is negatively correlated with an increased likelihood of a beneficial response to a treatment including a cyclophosphamide, and wherein the report includes information based on the likelihood of a beneficial response to chemotherapy including a cyclophosphamide.

The chemotherapy may include an anthracycline, such as doxorubicin.

The taxane may be docetaxel.

According to a further aspect of the present invention, there is provided a method of predicting whether a cancer patient will exhibit a beneficial response to chemotherapy, comprising measuring an expression level of a gene, or its expression product, in a tumour sample obtained from the patient, wherein the gene is selected from the group consisting of ABCC1, ABCC10, ABCC5, ACTB, ACTR2, APEX1, APOC1, APRT, BAK1, BAX, BBC3, BCL2L13, BID, BUB1, BUB3, CAPZA1, CCT3, CD247, CD68, CDCA8, CENPA, CENPF, CHEK2, CHFR, CST7, CXCR4, DDR1, DICER1, EIF4E2, GADD45B, GBP1, HDAC6, HSPA1A, HSPA1B, HSPA1L, IL2RA, IL7, ILK, KALPHA1, KIF22, LILRB1, LIMK2, MAD2L1, MAPRE1, MCL1, MRE11A, NEK2, NTSR2, PHB, PLD3, RAD1, RALBP1, RHOA, RPN2, SHC1, SLC1A3, SRC, STAT1, STK10, STMN1, TBCC, TOP3B, TPX2, TSPAN4, TUBA3, TUBA6, TUBB, TUBB2C, TUBB3, TYMS, VEGF, VHL, WNT5A, ZW10, ZWILCH, and ZWINT; using the expression level to determine a likelihood of a beneficial response to a treatment including a taxane, wherein expression of SLC1A3, TBCC, EIF4E2, TUBB, TSPAN4, VHL, BAX, CD247, CAPZA1, STMN1, ABCC1, ZW10, HSPA1B, MAPRE1, PLD3, APRT, BAK1, CST7, SHC1, ZWILCH, SRC, GADD45B, LIMK2, CHEK2, RAD1, MRE11A, DDR1, STK10, LILRB1, BBC3, BUB3, TOP3B, RPN2, ILK, GBP1, TUBB3, NTSR2, BID, BCL2L13, ABCC5, HDAC6, CD68, DICER1, RHOA, CCT3, ACTR2, WNT5A, HSPA1L, APOC1, APEX1, KALPHA1, ABCC10, PHB, TUBB2C, RALBP1, MCL1, HSPA1A, IL2RA, TUBA3, ACTB, KIF22, CXCR4, STAT1, IL7, or CHFR is positively correlated with increased likelihood of a beneficial response to a treatment including a taxane, and wherein expression of CENPA, CDCA8, TPX2, NEK2, TYMS, ZWINT, VEGF, BUB1, MAD2L1, or CENPF is negatively correlated with an increased likelihood of a beneficial response to a treatment including a taxane; and generating a report including information based on the likelihood of a beneficial response to chemotherapy including a taxane.

The method may comprise using the expression level to determine a likelihood of a beneficial response to a treatment including a cyclophosphamide, wherein expression of SLC1A3, TSPAN4, BAX, CD247, CAPZA1, ZW10, CST7, SHC1, GADD45B, MRE11A, STK10, LILRB1, BBC3, BUB3, ILK, GBP1, BCL2L13, CD68, DICER1, RHOA, ACTR2, WNT5A, HSPA1L, APEX1, MCL1, IL2RA, ACTB, STAT1, IL7, or CHFR is positively correlated with increased likelihood of a beneficial response to a treatment including a cyclophosphamide, and wherein expression of TBCC, EIF4E2, TUBB, VHL, STMN1, ABCC1, HSPA1B, MAPRE1, APRT, BAK1, TUBA6, ZWILCH, SRC, LIMK2, CENPA, CHEK2, RAD1, DDR1, CDCA8, TOP3B, RPN2, TUBB3, NTSR2, BID, TPX2, ABCC5, HDAC6, NEK2, TYMS, CCT3, ZWINT, KALPHA1, ABCC10, PHB, TUBB2C, RALBP1, VEGF, HSPA1A, BUB1, MAD2L1, CENPF, TUBA3, KIF22, or CXCR4 is negatively correlated with an increased likelihood of a beneficial response to a treatment including a cyclophosphamide, and wherein the report includes information based on the likelihood of a beneficial response to chemotherapy including a cyclophosphamide.

According to another aspect of the present invention, there is provided a kit comprising one or more (1) extraction buffer/reagents and protocol; (2) reverse transcription buffer/reagents and protocol; and (3) qPCR buffer/reagents and protocol, suitable for performing the methods specified above.

## Claims

1. A method of predicting whether a breast cancer patient will exhibit a beneficial response to chemotherapy, comprising
measuring an expression level of EIF4E2 or its expression product, in a tumour sample obtained from the patient,
using the expression level to determine a likelihood of a beneficial response to a treatment including a taxane, wherein expression of EIF4E2 is positively correlated with increased likelihood of a beneficial response to a treatment including a taxane, and
generating a report including information based on the likelihood of a beneficial response to chemotherapy including a taxane.

2. A method as claimed in claim 1, wherein the method comprises using the expression level to determine a likelihood of a beneficial response to a treatment including a cyclophosphamide,
wherein expression of EIF4E2 is negatively correlated with an increased likelihood of a beneficial response to a treatment including a cyclophosphamide, and
wherein the report includes information based on the likelihood of a beneficial response to chemotherapy including a cyclophosphamide.

3. A method as claimed in claim 1 or 2, wherein the breast cancer is hormone-receptor positive.

4. A method as claimed in claim 1, wherein the chemotherapy includes an anthracycline.

5. A method as claimed in claim 4, wherein the anthracycline is doxorubicin.

6. A method as claimed in claim 1, wherein the taxane is docetaxel.

7. A method as claimed in any preceding claim, wherein said measuring is by quantitative polymerase chain reaction (qPCR).

8. A method as claimed in any preceding claim, wherein said measuring is by detection of an intron-based sequence of an RNA transcript of the gene, wherein the expression of which correlates with the expression of a corresponding exon sequence.

9. A method as claimed in any preceding claim, wherein the tumour sample is a formalin-fixed and paraffin-embedded (FPE) tumour sample.

10. A method as claimed in any of claims 1 to 8, wherein the tumour sample is a frozen tumour sample.

11. A method as claimed in any of claims 1 to 6, wherein said measuring is by microarray.

12. A method as claimed in any of claims 1 to 6, wherein said measuring is by nucleic acid sequencing.
